(19) Europäisches Patentamt
European Patent Office
Office européen des brevets

(11) **EP 4 508 047 B1**

(12) **EUROPEAN PATENT SPECIFICATION**

(45) Date of publication and mention of the grant of the patent:
**05.08.2026 Bulletin 2026/32**

(21) Application number: **23723109.7**

(22) Date of filing: **13.04.2023**

(51) International Patent Classification (IPC):
***C07D 405/14*** *(2006.01)* ***C07D 417/14*** *(2006.01)*
***A61K 31/437*** *(2006.01)* ***A61K 31/454*** *(2006.01)*
***A61K 31/497*** *(2006.01)* ***A61P 3/10*** *(2006.01)*
***A61P 9/12*** *(2006.01)* ***A61P 25/16*** *(2006.01)*

(52) Cooperative Patent Classification (CPC):
**C07D 405/14; C07D 417/14**

(86) International application number:
**PCT/CN2023/087997**

(87) International publication number:
**WO 2023/198140 (19.10.2023 Gazette 2023/42)**

(54) **HETEROCYCLIC GLP-1 AGONISTS**

HETEROCYCLISCHE GLP-1-AGONISTEN

AGONISTES HÉTÉROCYCLIQUES DE GLP-1

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **14.04.2022 PCT/CN2022/086863**

(43) Date of publication of application:
**19.02.2025 Bulletin 2025/08**

(73) Proprietor: **Gasherbrum Bio, Inc.**
**South San Francisco, CA 94080 (US)**

(72) Inventors:
- **LIN, Xichen**
  **Shanghai 200003 (CN)**
- **MENG, Qinghua**
  **Shanghai 200003 (CN)**
- **XING, Weiqiang**
  **Shanghai 200003 (CN)**
- **JENNINGS, Andrew**
  **San Francisco California 94080 (US)**

(74) Representative: **Carpmaels & Ransford LLP**
**One Southampton Row**
**London WC1B 5HA (GB)**

(56) References cited:
**WO-A1-2020/103815** **WO-A1-2022/078407**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### CROSS-REFERENCE TO RELATED APPLICATIONS

This application claims the benefit of International Patent Application No. PCT/CN2022/086863, filed on April 14, 2022.

### FIELD

This disclosure relates to GLP-1 agonists, pharmaceutical compositions, and methods of use thereof.

### BACKGROUND

Incretin metabolic hormones, including glucagon-like peptide-1 (GLP-1) and glucose-dependent insulinotropic polypeptide (GIP), are important in the regulation of glucose homeostasis. Medicaments targeting this family of intestinal peptides, such as GLP-1 agonists, have been shown to suppress glucagon production, decrease gastric motility, and increase satiety.

Diabetes mellitus refers to a group of metabolic disorders characterized by persistent hyperglycemia. The most common form, type 2 diabetes mellitus (T2DM) is an acquired condition that accounts for more than 90% of diabetes cases. Typical onset occurs in obese or otherwise sedentary adults and begins with insulin resistance. Though lifestyle changes can be useful in management of this disorder, patients with T2DM may be required to take antidiabetic medications, including dipeptidyl peptidase-4 inhibitors, SGLT2 inhibitors, and sulfonylureas, among others.

In healthy individuals, the incretin hormones glucose-dependent insulinotropic polypeptide (GIP) and glucagon-like peptide 1 (GLP-1) provide tandem modulation of insulin secretory response to glucose ingestion. While this incretin effect is significantly diminished (if at all present) in cases of T2DM, GLP-1 retains insulinotropic properties, even as endocrine pancreatic response to GIP is effectively halted. As such, incretin mimetics and other GLP-1-based therapies can help stimulate insulin production in T2DM patients.

### SUMMARY

The present application describes heterocyclic GLP-1 agonists, as well as pharmaceutical compositions comprising the compounds, or pharmaceutically acceptable salts or solvates thereof, disclosed herein. Also provided are compounds, or pharmaceutically acceptable salts or solvates thereof, or pharmaceutical compositions, for use in methods for treating GLP-1 associated diseases, disorders, and conditions. In the present description all references to methods of treatment are to be interpreted as being directed to compounds, or pharmaceutically acceptable salts or solvates thereof, or pharmaceutical compositions for use in such methods.

In one aspect, provided is a compound of Table 1, or a pharmaceutically acceptable salt or solvate thereof.

Also provided herein are pharmaceutical compositions comprising a compound of Table 1, or a pharmaceutically acceptable salt or solvate thereof, and a pharmaceutically acceptable excipient.

Also provided herein are methods for treating type 2 diabetes mellitus in a patient in need thereof, the methods comprising administering to the patient a therapeutically effective amount of a compound of Table 1, or a pharmaceutically acceptable salt or solvate thereof, or a pharmaceutical composition thereof.

Also provided herein are methods for treating type 2 diabetes mellitus in a patient, the methods comprising administering to a patient identified or diagnosed as having type 2 diabetes mellitus a therapeutically effective amount of a compound of Table 1, or a pharmaceutically acceptable salt or solvate thereof, or a pharmaceutical composition thereof.

Also provided herein are methods for treating diabetes mellitus in a patient, the methods comprising determining that the patient has type 2 diabetes mellitus; and administering to the patient a therapeutically effective amount of a compound of Table 1, or a pharmaceutically acceptable salt or solvate thereof, or a pharmaceutical composition thereof. In some embodiments, the step of determining that the patient has type 2 diabetes mellitus includes performing an assay to determine the level of an analyte in a sample from the patient, wherein the analyte is selected from the group consisting of hemoglobin A1c (HbA1c), fasting plasma glucose, non-fasting plasma glucose, or any combination thereof. In some embodiments, the level of HbA1c is greater than or about 6.5%. In some embodiments, the level of fasting plasma glucose is greater than or about 126 mg/dL. In some embodiments, the level of non-fasting plasma glucose is greater than or about 200 mg/dL.

In some embodiments, the methods further comprise obtaining a sample from the patient. In some embodiments, the sample is a body fluid sample. In some embodiments, the patient is about 40 to about 70 years old and is overweight or obese. In some embodiments, the patient has a body mass index (BMI) greater than or about 22 $kg/m^2$. In some embodiments, the patient has a BMI greater than or about 30 $kg/m^2$.

In some embodiments, the methods for the treatment of type 2 diabetes mellitus comprise a reduction in fasting plasma glucose levels. In some embodiments, the fasting plasma glucose levels are reduced to about or below 100 mg/dL.

In some embodiments, the methods for the treatment of type 2 diabetes mellitus comprise a reduction in HbA1c levels. In some embodiments, the HbA1c levels are reduced to about or below 5.7 %.

In some embodiments, the methods for the treatment of type 2 diabetes mellitus comprise a reduction in glucagon levels.

In some embodiments, the methods for the treatment of type 2 diabetes mellitus comprise an increase in insulin levels.

In some embodiments, the methods for the treatment of type 2 diabetes mellitus comprise a decrease in BMI. In some embodiments, the BMI is decreased to about or below 25 kg/$m^2$.

In some embodiments, the compound of Table 1, or a pharmaceutically acceptable salt or solvate thereof, or a pharmaceutical composition thereof, is administered orally.

In some embodiments, the methods of treatment for type 2 diabetes mellitus further comprise administering an additional therapy or therapeutic agent to the patient. In some embodiments, the additional therapy or therapeutic agent is selected from the group consisting of an antidiabetic agent, an anti-obesity agent, a GLP-1 receptor agonist, an agent to treat non-alcoholic steatohepatitis (NASH), an anti-emetic agent, gastric electrical stimulation, dietary monitoring, physical activity, or any combinations thereof. In some embodiments, the antidiabetic agent is selected from the group consisting of a biguanide, a sulfonylurea, a glitazar, a thiazolidinedione, a dipeptidyl peptidase 4 (DPP-4) inhibitor, a meglitinide, a sodium-glucose linked transporter 2 (SGLT2) inhibitor, a glitazone, a GRP40 agonist, a glucose-dependent insulinotropic peptide (GIP), an insulin or insulin analogue, an alpha glucosidase inhibitor, a sodium-glucose linked transporter 1 (SGLT1) inhibitor, or any combinations thereof. In some embodiments, the biguanide is metformin. In some embodiments, the anti-obesity agent is selected from the group consisting of neuropeptide Y receptor type 2 (NPYR2) agonist, a NPYR1 or NPYR5 antagonist, a human proislet peptide (HIP), a cannabinoid receptor type 1 (CB1R) antagonist, a lipase inhibitor, a melanocortin receptor 4 agonist, a farnesoid X receptor (FXR) agonist, phentermine, zonisamide, a norepinephrine/dopamine reuptake inhibitor, a GDF-15 analog, an opioid receptor antagonist, a cholecystokinin agonist, a serotonergic agent, a methionine aminopeptidase 2 (MetAP2) inhibitor, diethylpropion, phendimetrazine, benzphetamine, a fibroblast growth factor receptor (FGFR) modulator, an AMP-activated protein kinase (AMPK) activator, or any combinations thereof. In some embodiments, the GLP-1 receptor agonist is selected from the group consisting of liraglutide, exenatide, dulaglutide, albiglutide, taspoglutide, lixisenatide, semaglutide, or any combinations thereof. In some embodiments, the agent to treat NASH is selected from the group consisting of an FXR agonist, PF-05221304, a synthetic fatty acid-bile conjugate, an anti-lysyl oxidase homologue 2 (LOXL2) monoclonal antibody, a caspase inhibitor, a MAPK5 inhibitor, a galectin 3 inhibitor, a fibroblast growth factor 21 (FGF21) agonist, a niacin analogue, a leukotriene D4 (LTD4) receptor antagonist, an acetyl-CoA carboxylase (ACC) inhibitor, a ketohexokinase (KHK) inhibitor, an ileal bile acid transporter (IBAT) inhibitor, an apoptosis signal-regulating kinase 1 (ASK1) inhibitor, or any combinations thereof. In some embodiments, the compound of Table 1, or a pharmaceutically acceptable salt or solvate thereof, or a pharmaceutical composition thereof, and the additional therapeutic agent are administered as separate dosages sequentially in any order.

Also provided herein are methods for modulating insulin levels in a patient in need of such modulating, the method comprising administering to the patient an effective amount of a compound of Table 1, or a pharmaceutically acceptable salt or solvate thereof, or a pharmaceutical composition thereof. In some embodiments, the modulation results in an increase of insulin levels.

Also provided herein are methods for modulating glucose levels in a patient in need of such modulating, the method comprising administering to the patient an effective amount of a compound of Table 1, or a pharmaceutically acceptable salt or solvate thereof, or a pharmaceutical composition thereof. In some embodiments, the modulation results in a decrease of glucose levels.

Also provided herein are methods for treating a GLP-1 associated disease, disorder, or condition, the method comprising administering to a patient in need thereof an effective amount of a compound of Table 1, or a pharmaceutically acceptable salt or solvate thereof, or a pharmaceutical composition thereof. In some embodiments, the disease, disorder, or condition is selected from the group consisting of type 1 diabetes mellitus, type 2 diabetes mellitus, early onset type 2 diabetes mellitus, idiopathic type 1 diabetes mellitus (Type 1b), youth-onset atypical diabetes (YOAD), maturity onset diabetes of the young (MODY), latent autoimmune diabetes in adults (LADA), obesity, weight gain from use of other agents, gout, excessive sugar craving, hypertriglyceridemia, dyslipidemia, malnutrition-related diabetes, gestational diabetes, kidney disease, adipocyte dysfunction, sleep apnea, visceral adipose deposition, eating disorders, cardiovascular disease, congestive heart failure, myocardial infarction, left ventricular hypertrophy, peripheral arterial disease, stroke, hemorrhagic stroke, ischemic stroke, transient ischemic attacks, atherosclerotic cardiovascular disease, traumatic brain injury, peripheral vascular disease, endothelial dysfunction, impaired vascular compliance, vascular restenosis, thrombosis, hypertension, pulmonary hypertension. restenosis after angioplasty, intermittent claudication, hyperglycemia, post-prandial lipemia, metabolic acidosis, ketosis, hyperinsulinemia, impaired glucose metabolism, insulin resistance, hepatic insulin resistance, alcohol use disorder, chronic renal failure, metabolic syndrome, syndrome X, smoking

cessation, premenstrual syndrome, angina pectoris, diabetic nephropathy, impaired glucose tolerance, diabetic neuropathy, diabetic retinopathy, macular degeneration, cataract, glomerulosclerosis, arthritis, osteoporosis, treatment of addiction, cocaine dependence, bipolar disorder/major depressive disorder, skin and connective tissue disorders, foot ulcerations, psoriasis, primary polydipsia, non-alcoholic steatohepatitis (NASH), non-alcoholic fatty liver disease (NAFLD), ulcerative colitis, inflammatory bowel disease, colitis, irritable bowel syndrome, Crohn's disease, short bowel syndrome, Parkinson's, Alzheimer's disease, impaired cognition, schizophrenia, Polycystic Ovary Syndrome (PCOS), or any combination thereof. In some embodiments, the disease, disorder, or condition is selected from the group consisting of type 2 diabetes mellitus, early onset type 2 diabetes mellitus, obesity, weight gain from use of other agents, gout, excessive sugar craving, hypertriglyceridemia, dyslipidemia, gestational diabetes, kidney disease, adipocyte dysfunction, sleep apnea, visceral adipose deposition, eating disorders, cardiovascular disease, congestive heart failure, myocardial infarction, left ventricular hypertrophy, peripheral arterial disease, stroke, hemorrhagic stroke, ischemic stroke, transient ischemic attacks, atherosclerotic cardiovascular disease, hyperglycemia, post-prandial lipemia, metabolic acidosis, ketosis, hyperinsulinemia, impaired glucose metabolism, insulin resistance, hepatic insulin resistance, alcohol use disorder, chronic renal failure, metabolic syndrome, syndrome X, smoking cessation, premenstrual syndrome, angina pectoris, diabetic nephropathy, impaired glucose tolerance, diabetic neuropathy, diabetic retinopathy, bipolar disorder/-major depressive disorder, skin and connective tissue disorders, foot ulcerations, psoriasis, primary polydipsia, non-alcoholic steatohepatitis (NASH), non-alcoholic fatty liver disease (NAFLD), short bowel syndrome, Parkinson's disease, Polycystic Ovary Syndrome (PCOS), or any combination thereof. In some embodiments, the disease, disorder, or condition includes, but is not limited to type 2 diabetes mellitus, early onset type 2 diabetes mellitus, obesity, weight gain from use of other agents, gout, excessive sugar craving, hypertriglyceridemia, dyslipidemia, gestational diabetes, adipocyte dysfunction, visceral adipose deposition, myocardial infarction, peripheral arterial disease, stroke, transient ischemic attacks, hyperglycemia, post-prandial lipemia, metabolic acidosis, ketosis, hyperinsulinemia, impaired glucose metabolism, insulin resistance, hepatic insulin resistance, chronic renal failure, syndrome X, angina pectoris, diabetic nephropathy, impaired glucose tolerance, diabetic neuropathy, diabetic retinopathy, skin and connective tissue disorders, foot ulcerations, or any combination thereof.

## DETAILED DESCRIPTION

Before the present compounds and methods are described, it is to be understood that the disclosure is not limited to the methodologies, protocols, cell lines, assays, and reagents described, as these may vary. It is also to be understood that the terminology used herein is intended to describe embodiments of the present disclosure, and is in no way intended to limit the scope of the present disclosure as set forth in the appended claims.

### Definitions

It must be noted that as used herein, and in the appended claims, the singular forms "a," "an," and "the" include plural references unless the context clearly dictates otherwise.

Unless defined otherwise, all technical, and scientific terms used herein have the same meanings as commonly understood by one of ordinary skill in the art to which this disclosure pertains. Although any methods and materials similar or equivalent to those described herein can be used in the practice or testing of the present disclosure, exemplary methods, devices, and materials are now described. All publications cited herein are incorporated herein by reference in their entirety for the purpose of describing and disclosing the methodologies, reagents, and tools reported in the publications that might be used in connection with the disclosure.

Where values are described as ranges, it will be understood that such disclosure includes the disclosure of all possible sub-ranges within such ranges, as well as specific numerical values that fall within such ranges irrespective of whether a specific numerical value or specific sub-range is expressly stated.

Provided herein are heterocyclic GLP-1 agonists for use in the management of T2DM and other conditions where activation of GLP-1 activity is useful.

As used herein, the term "compound," is meant to include all stereoisomers, geometric isomers, tautomers, and isotopes of the structures depicted. In some embodiments, stereochemistry of a given compound is as depicted by the chemical structure shown in Table 1. Compounds herein identified by name or structure as one particular tautomeric form are intended to include other tautomeric forms unless otherwise specified.

The term "tautomer" as used herein refers to compounds whose structures differ markedly in arrangement of atoms, but which exist in easy and rapid equilibrium, and it is to be understood that compounds provided herein may be depicted as different tautomers, and when compounds have tautomeric forms, all tautomeric forms are intended to be within the scope of the disclosure, and the naming of the compounds does not exclude any tautomer.

A "solvate" refers to an association or complex of one or more solvent molecules and a compound of the disclosure. Examples of solvents that form solvates include, but are not limited to, water, isopropanol, ethanol, methanol,

dimethylsulfoxide, ethylacetate, acetic acid and ethanolamine. For example, compounds of Table 1 and salts thereof can exist in unsolvated as well as solvated forms with pharmaceutically acceptable solvents such as water, ethanol, and the like.

The term "pharmaceutically acceptable salt" of a given compound refers to salts that retain the biological effectiveness and properties of the given compound and which are not biologically or otherwise undesirable. "Pharmaceutically acceptable salts" or "physiologically acceptable salts" include, for example, salts with inorganic acids and salts with an organic acid. In addition, if the compounds described herein are obtained as an acid addition salt, the free base can be obtained by basifying a solution of the acid salt. Conversely, if the product is a free base, an addition salt, particularly a pharmaceutically acceptable addition salt, may be produced by dissolving the free base in a suitable organic solvent and treating the solution with an acid, in accordance with conventional procedures for preparing acid addition salts from base compounds. Those skilled in the art will recognize various synthetic methodologies that may be used to prepare nontoxic pharmaceutically acceptable addition salts. Pharmaceutically acceptable acid addition salts may be prepared from inorganic and organic acids. Salts derived from inorganic acids include, e.g., hydrochloric acid, hydrobromic acid, sulfuric acid, nitric acid, phosphoric acid and the like. Salts derived from organic acids include, e.g., acetic acid, propionic acid, gluconic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. Likewise, pharmaceutically acceptable base addition salts can be prepared from inorganic and organic bases. Salts derived from inorganic bases include, by way of example only, sodium, potassium, lithium, aluminum, ammonium, calcium, and magnesium salts. Salts derived from organic bases include, but are not limited to, salts of $NH_3$, or primary, secondary, tertiary amines, such as salts derived from a N-containing heterocycle, a N-containing heteroaryl, or derived from an amine of formula $N(R^N)_3$ (e.g., $HN^+(R^N)_3$ or (alkyl)$N^+(R^N)_3$) where each $R^N$ is independently hydrogen, alkyl, alkenyl, alkynyl, haloalkyl, cycloalkyl, heterocyclyl, aryl, or heteroaryl, wherein each is optionally substituted, such as by one or more (e.g., 1-5 or 1-3) substituents (e.g., halo, cyano, hydroxy, amino, alkyl, alkenyl, alkynyl, haloalkyl, alkoxy, or haloalkoxy). Specific examples of suitable amines include, by way of example only, isopropylamine, trimethyl amine, diethyl amine, tri(iso-propyl) amine, tri(n-propyl) amine, ethanolamine, 2-dimethylaminoethanol, piperazine, piperidine, morpholine, N-ethylpiperidine, and the like.

As used herein, the term "alkyl" refers to saturated linear or branched-chain monovalent hydrocarbon radicals, containing the indicated number of carbon atoms. For example, "($C_1$-$C_6$)alkyl" refers to saturated linear or branched-chain monovalent hydrocarbon radicals of one to six carbon atoms. Non-limiting examples of alkyl include methyl, ethyl, 1-propyl, isopropyl, 1-butyl, isobutyl, sec-butyl, tert-butyl, 2-methyl-2-propyl, pentyl, neopentyl, and hexyl.

As used herein, the term "alkylene" refers to a divalent alkyl containing the indicated number of carbon atoms. For example, "($C_1$-$C_3$)alkylene" refers to a divalent alkyl having one to three carbon atoms (e.g., $-CH_2-$, $-CH(CH_3)-$, $-CH_2CH_2-$, or $-CH_2CH_2CH_2-$). Similarly, the terms "cycloalkylene," "heterocycloalkylene," "arylene," and "heteroarylene" mean divalent cycloalkyl, heterocycloalkyl, aryl, and heteroaryl, respectively.

As used herein, the term "alkenyl" refers to a linear or branched mono-unsaturated hydrocarbon chain, containing the indicated number of carbon atoms. For example, "($C_2$-$C_6$)alkenyl" refers a linear or branched mono unsaturated hydrocarbon chain of two to six carbon atoms. Non-limiting examples of alkenyl include ethenyl, propenyl, butenyl, or pentenyl.

As used herein, the term "alkynyl" refers to a linear or branched di-unsaturated hydrocarbon chain, containing the indicated number of carbon atoms. For example, "($C_2$-$C_6$)alkynyl" refers to a linear or branched di-unsaturated hydrocarbon chain having two to six carbon atoms. Non-limiting examples of alkynyl include ethynyl, propynyl, butynyl, or pentynyl.

As used herein, the term "alkoxy" refers to an -O-alkyl radical, wherein the radical is on the oxygen atom. For example, "$C_{1-6}$ alkoxy" refers to an -O-($C_{1-6}$ alkyl) radical, wherein the radical is on the oxygen atom. Examples of alkoxy include methoxy, ethoxy, propoxy, isopropoxy, butoxy and tert-butoxy. Accordingly, as used herein, the term "haloalkoxy" refers to an -O-haloalkyl radical, wherein the radical is on the oxygen atom.

As used herein, the term "cycloalkyl" refers to a saturated or partially unsaturated cyclic hydrocarbon, containing the indicated number of carbon atoms. For example, "($C_3$-$C_6$)cycloalkyl" refers to a saturated or partially unsaturated cyclic hydrocarbon having three to six ring carbon atoms. Non-limiting examples of cycloalkyl include cyclopropyl, cyclobutyl, cyclopentyl, and cyclohexyl. Cycloalkyl may be partially unsaturated. Non-limiting examples of partially unsaturated cycloalkyl include cyclohexenyl, cyclopentenyl, cycloheptenyl, cyclooctenyl, and the like. Cycloalkyl may include multiple fused and/or bridged rings. Non-limiting examples of fused/bridged cycloalkyl includes: bicyclo[1.1.0]butane, bicyclo [2.1.0]pentane, bicyclo[1.1.1]pentane, bicyclo[3.1.0]hexane, bicyclo[2.1.1]hexane, bicyclo[3.2.0]heptane, bicyclo[4.1.0] heptane, bicyclo[2.2.1]heptane, bicyclo[3.1.1]heptane, bicyclo[4.2.0]octane, bicyclo[3.2.1]octane, bicyclo[2.2.2]octane, and the like. Cycloalkyl also includes spirocyclic rings (e.g., spirocyclic bicycle wherein two rings are connected through just one atom). Non-limiting examples of spirocyclic cycloalkyls include spiro[2.2]pentane, spiro[2.5]octane, spiro[3.5] nonane, spiro[3.5]nonane, spiro[3.5]nonane, spiro[4.4]nonane, spiro[2.6]nonane, spiro[4.5]decane, spiro[3.6]decane, spiro[5.5]undecane, and the like.

As used herein, the term "heterocycloalkyl" refers to a mono-, bi-, tri-, or polycyclic nonaromatic ring system containing indicated number of ring atoms (e.g., 3-8 membered monocyclic, 8-12 membered bicyclic, or 11-14 membered tricyclic ring system) having 1-3 heteroatoms if monocyclic, 1-6 heteroatoms if bicyclic, or 1-9 heteroatoms if tricyclic or polycyclic, the heteroatoms selected from O, N, S, or $S(O)_{1-2}$ (e.g., carbon atoms and 1-3, 1-6. or 1-9 heteroatoms of N, O, S, or $S(O)_{1-2}$ if monocyclic, bicyclic, or tricyclic, respectively), wherein 0, 1, 2 or 3 atoms of each ring may be substituted by a substituent. Examples of heterocycloalkyl groups include piperazinyl, pyrrolidinyl, dioxanyl, morpholinyl, tetrahydrofuranyl, and the like. Heterocycloalkyl groups may be partially unsaturated. Non-limiting examples of partially unsaturated heterocycloalkyl include dihydropyrrolyl, dihydropyridinyl, tetrahydropyridinyl, dihydrofuranyl, dihydropyranyl, and the like. Heterocycloalkyl may include multiple fused and bridged rings. Non-limiting examples of fused/bridged heterocyclyl includes: 2-azabicyclo[1.1.0]butane, 2-azabicyclo[2.1.0]pentane, 2-azabicyclo[1.1.1]pentane, 3-azabicyclo[3.1.0]hexane, 5-azabicyclo[2.1.1]hexane, 3-azabicyclo[3.2.0]heptane, octahydrocyclopenta[c]pyrrole, 3-azabicyclo[4.1.0]heptane, 7-azabicyclo[2.2.1]heptane, 6-azabicyclo[3.1.1]heptane, 7-azabicyclo[4.2.0]octane, 2-azabicyclo[2.2.2]octane, 3-azabicyclo[3.2.1]octane, 2-oxabicyclo[1.1.0]butane, 2-oxabicyclo[2.1.0]pentane, 2-oxabicyclo[1.1.1]pentane, 3-oxabicyclo[3.1.0]hexane, 5-oxabicyclo[2.1.1]hexane, 3-oxabicyclo[3.2.0]heptane, 3-oxabicyclo[4.1.0]heptane, 7-oxabicyclo [2.2.1]heptane, 6-oxabicyclo[3.1.1]heptane, 7-oxabicyclo[4.2.0]octane, 2-oxabicyclo[2.2.2]octane, 3-oxabicyclo[3.2.1] octane, and the like. Heterocycloalkyl also includes spirocyclic rings (e.g., spirocyclic bicycle wherein two rings are connected through just one atom). Non-limiting examples of spirocyclic heterocycloalkyl include 2-azaspiro[2.2]pentane, 4-azaspiro[2.5]octane, 1-azaspiro[3.5]nonane, 2-azaspiro[3.5]nonane, 7-azaspiro[3.5]nonane, 2-azaspiro[4.4]nonane, 6-azaspiro[2.6]nonane, 1,7-diazaspiro[4.5]decane, 7-azaspiro[4.5]decane 2,5-diazaspiro[3.6]decane, 3-azaspiro[5.5] undecane, 2-oxaspiro[2.2]pentane, 4-oxaspiro[2.5]octane, 1-oxaspiro[3.5]nonane, 2-oxaspiro[3.5]nonane, 7-oxaspiro [3.5]nonane, 2-oxaspiro[4.4]nonane, 6-oxaspiro[2.6]nonane, 1,7-dioxaspiro[4.5]decane, 2,5-dioxaspiro[3.6]decane, 1-oxaspiro[5.5]undecane, 3-oxaspiro[5.5]undecane, 3-oxa-9-azaspiro[5.5]undecane and the like.

As used herein, the term "aryl" refers to a mono-, bi-, tri- or polycyclic hydrocarbon group containing the indicated numbers of carbon atoms, wherein at least one ring in the system is aromatic (e.g., $C_6$ monocyclic, $C_{10}$ bicyclic, or $C_{14}$ tricyclic aromatic ring system). Examples of aryl groups include phenyl, naphthyl, tetrahydronaphthyl, and the like.

As used herein, the term "heteroaryl" refers to a mono-, bi-, tri- or polycyclic group having indicated numbers of ring atoms (e.g., 5-6 ring atoms; e.g., 5, 6, 9, 10, or 14 ring atoms); wherein at least one ring in the system is aromatic (but does not have to be a ring which contains a heteroatom, e.g. tetrahydroisoquinolinyl, e.g., tetrahydroquinolinyl), and at least one ring in the system contains one or more heteroatoms independently selected from the group consisting of N, O, and S. Heteroaryl groups can either be unsubstituted or substituted with one or more substituents. Examples of heteroaryl include thienyl, pyridinyl, furyl, oxazolyl, oxadiazolyl, pyrrolyl, imidazolyl, triazolyl, thiodiazolyl, pyrazolyl, isoxazolyl, thiadiazolyl, pyranyl, pyrazinyl, pyrimidinyl, pyridazinyl, triazinyl, thiazolyl benzothienyl, benzoxadiazolyl, benzofuranyl, benzimidazolyl, benzotriazolyl, cinnolinyl, indazolyl, indolyl, isoquinolinyl, isothiazolyl, naphthyridinyl, purinyl, thienopyridinyl, pyrido[2,3-*d*]pyrimidinyl, pyrrolo[2,3-*b*]pyridinyl, quinazolinyl, quinolinyl, thieno[2,3-*c*]pyridinyl, pyrazolo[3,4-*b*]pyridinyl, pyrazolo[3,4-*c*]pyridinyl, pyrazolo[4,3-*c*]pyridine, pyrazolo[4,3-*b*]pyridinyl, tetrazolyl, chromane, 2,3-dihydrobenzo[*b*][1,4]dioxine, benzo[*d*][1,3]dioxole, 2,3-dihydrobenzofuran, tetrahydroquinoline, 2,3-dihydrobenzo[*b*][1,4]oxathiine, isoindoline, and others.

As used herein, the term "halo" or "halogen" means -F (sometimes referred to herein as "fluoro" or "fluoros"), -Cl (sometimes referred to herein as "chloro" or "chloros"), -Br (sometimes referred to herein as "bromo" or "bromos"), and -I (sometimes referred to herein as "iodo" or "iodos").

As used herein, the term "haloalkyl" refers to an alkyl radical as defined herein, wherein one or more hydrogen atoms is replaced with one or more halogen atoms. Non-limiting examples include fluoromethyl, difluoromethyl, trifluoromethyl, 2-fluoroethyl, 2,2-difluoroethyl, 2,2,2-trifluoroethyl, chloromethyl, dichloromethyl, chloroethyl, trichloroethyl, bromomethyl, and iodomethyl.

As used herein, "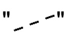" indicates an optional single or double bond, as allowed by valence. As used herein, "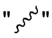" indicates the point of attachment to the parent molecule.

As used herein, when a ring is described as being "aromatic," it means the ring has a continuous, delocalized $\pi$-electron system. Typically, the number of out of plane $\pi$-electrons corresponds to the Hückel rule (4n+2). Examples of such rings include: benzene, pyridine, pyrimidine, pyrazine, pyridazine, pyridone, pyrrole, pyrazole, oxazole, thiazole, isoxazole, isothiazole, and the like. When a ring system comprising at least two rings is described as "aromatic," it means the ring system comprises one or more aromatic ring(s). Accordingly, when a ring system comprising at least two rings is described as "non-aromatic," none of the constituent rings of the ring system is aromatic.

As used herein, when a ring is described as being "partially unsaturated," it means the ring has one or more additional degrees of unsaturation (in addition to the degree of unsaturation attributed to the ring itself; e.g., one or more double bonds between constituent ring atoms), provided that the ring is not aromatic. Examples of such rings include: cyclopentene, cyclohexene, cycloheptene, dihydropyridine, tetrahydropyridine, dihydropyrrole, dihydrofuran, dihydrothiophene, and the like. When a ring system comprising at least two rings is described as "partially unsaturated," it means the ring system comprises one or more partially unsaturated ring(s), provided that none of the constituent rings of

the ring system is aromatic.

The term "GLP-1R" or "GLP-1 receptor" as used herein is meant to include, without limitation, nucleic acids, polynucleotides, oligonucleotides, sense and antisense polynucleotide strands, complementary sequences, peptides, polypeptides, proteins, homologous, and/or orthologous GLP-1R molecules, isoforms, precursors, mutants, variants, derivatives, splice variants, alleles, different species, and active fragments thereof.

The term "GLP-1 associated disease" as used herein is meant to include, without limitation, all those diseases, disorders, or conditions in which modulating glucagon-like peptide-1 (GLP-1) receptor signaling can alter the pathology and/or symptoms and/or progression of the disease, disorder, or condition.

The term "GLP-1 agonist" or "GLP-1 RA" as used herein refers to an agonist of the glucagon-like peptide-1 (GLP-1) receptor. GLP-1 RAs enhance glucose-dependent insulin secretion; suppress inappropriately elevated glucagon levels, both in fasting and postprandial states; and slow gastric emptying. Karla et al., Glucagon-like peptide-1 receptor agonists in the treatment of type 2 diabetes: Past, present, and future, Indian J Endocrinol Metab. 2016 Mar-Apr; 20(2): 254-267. GLP-1 RAs have been shown to treat type 2 diabetes. Examples of GLP-1 RAs include, but are not limited to, albiglutide (TANZEUM®), dulaglutide (LY2189265, TRULICITY®), efpeglenatide, exenatide (BYETTA®, BYDUREON®, Exendin-4), liraglutide (VICTOZA®, NN2211), lixisenatide (LYXUMIA®), semaglutide (OZEMPIC®), tirzepatide, ZP2929, NNC0113-0987, BPI-3016, and TT401. See, also, for example, additional GLP-1 receptor agonists described in U.S. Patent Nos. 10,370,426; 10,308,700; 10, 259,823; 10,208,019; 9,920,106; 9,839,664; 8,129,343; 8,536,122; 7,919,598; 6,414,126; 6,628,343; and RE45313.

The term "pharmaceutically acceptable" as used herein indicates that the compound, or salt or composition thereof is compatible chemically and/or toxicologically with the other ingredients comprising a formulation and/or the patient being treated therewith.

The term "therapeutic compound" as used herein is meant to include, without limitation, a compound disclosed herein (e.g., a compound of Table 1, or a pharmaceutically acceptable salt or solvate thereof), and all compositions (e.g., pharmaceutical compositions) wherein a compound of Table 1, or a pharmaceutically acceptable salt or solvate thereof) is a component of the composition.

The term "administration" or "administering" refers to a method of giving a dosage of a compound or pharmaceutical composition to a vertebrate or invertebrate, including a mammal, a bird, a fish, or an amphibian. The method of administration can vary depending on various factors, e.g., the components of the pharmaceutical composition, the site of the disease, and the severity of the disease.

The terms "effective amount" or "effective dosage" or "pharmaceutically effective amount" or "therapeutically effective amount," as used herein, refer to a sufficient amount of a chemical entity (e.g., a compound disclosed herein (e.g., a compound of Table 1, or a pharmaceutically acceptable salt or solvate thereof)) being administered which will relieve to some extent one or more of the symptoms of the disease or condition being treated, and can include curing the disease. "Curing" means that the symptoms of active disease are eliminated. The result includes reduction and/or alleviation of the signs, symptoms, or causes of a disease, or any other desired alteration of a biological system. For example, an "effective amount" for therapeutic uses is the amount of the composition comprising a compound as disclosed herein required to provide a clinically significant decrease in disease symptoms. An appropriate "effective" amount in any individual case is determined using any suitable technique, such as a dose escalation study. In some embodiments, a "therapeutically effective amount" of a compound as provided herein refers to an amount of the compound that is effective as a monotherapy or combination therapy.

The term "excipient" or "pharmaceutically acceptable excipient" means a pharmaceutically-acceptable material, composition, or vehicle, such as a liquid or solid filler, diluent, carrier, solvent, or encapsulating material. In some embodiments, each component is "pharmaceutically acceptable" in the sense of being compatible with the other ingredients of a pharmaceutical formulation, and suitable for use in contact with the tissue or organ of humans and animals without excessive toxicity, irritation, allergic response, immunogenicity, or other problems or complications, commensurate with a reasonable benefit/risk ratio. See, e.g., Remington: The Science and Practice of Pharmacy, 21st ed.; Lippincott Williams & Wilkins: Philadelphia, PA, 2005; Handbook of Pharmaceutical Excipients, 6th ed.; Rowe et al., Eds.; The Pharmaceutical Press and the American Pharmaceutical Association: 2009; Handbook of Pharmaceutical Additives, 3rd ed.; Ash and Ash Eds.; Gower Publishing Company: 2007; Pharmaceutical Preformulation and Formulation, 2nd ed.; Gibson Ed.; CRC Press LLC: Boca Raton, FL, 2009.

The term "pharmaceutical composition" refers to a mixture of a compound a compound disclosed herein, or a pharmaceutically acceptable salt or solvate thereof (e.g., a compound of a Table 1, or a pharmaceutically acceptable salt or solvate thereof) as described herein with other chemical components (referred to collectively herein as "excipients"), such as carriers, stabilizers, diluents, dispersing agents, suspending agents, and/or thickening agents. The pharmaceutical composition facilitates administration of the compound to an organism. Multiple techniques of administering a compound exist in the art including, but not limited to, rectal, oral, intravenous, aerosol, parenteral, ophthalmic, pulmonary, and topical administration.

The terms "treat," "treating," and "treatment," in the context of treating a disease, disorder, or condition, are meant

to include alleviating or abrogating a disorder, disease, or condition, or one or more of the symptoms associated with the disorder, disease, or condition; or to slowing the progression, spread or worsening of a disease, disorder or condition or of one or more symptoms thereof.

The term "preventing," as used herein, is the prevention of the onset, recurrence or spread, in whole or in part, of the disease or condition as described herein, or a symptom thereof.

The terms "subject," "patient" or "individual," as used herein, are used interchangeably and refers to any animal, including mammals such as mice, rats, other rodents, rabbits, dogs, cats, swine, cattle, sheep, horses, primates, and humans. In some embodiments, the term refers to a subject, particularly a mammalian subject, for whom diagnosis, prognosis, or therapy is desired or needed. In some embodiments, the patient is a human. In some embodiments, the subject has experienced and/or exhibited at least one symptom of the disease, disorder, or condition to be treated and/or prevented.

The terms "treatment regimen" and "dosing regimen" are used interchangeably to refer to the dose and timing of administration of each therapeutic agent in a combination described herein.

The term "pharmaceutical combination," as used herein, refers to a pharmaceutical treatment resulting from the mixing or combining of more than one active ingredient and includes both fixed and non-fixed combinations of the active ingredients.

The term "combination therapy" as used herein refers to a dosing regimen of two different therapeutically active agents (i.e., the components or combination partners of the combination), wherein the therapeutically active agents are administered together or separately in a manner prescribed by a medical care taker or according to a regulatory agency as defined herein.

The term "modulation," as used herein, refers to a regulation or an adjustment (e.g., increase or decrease) and can include, for example agonism, partial agonism or antagonism.

It is understood that the substituents as defined herein are not intended to include impermissible substitution patterns (*e.g.*, methyl substituted with 5 fluoro groups or a hydroxy group attached to an ethenylic or acetylenic carbon atom). Such impermissible substitution patterns are well known to the skilled artisan.

**Compounds**

Provided herein is a compound selected from Table 1, or a pharmaceutically acceptable salt or solvate thereof:

**Table 1**

| No. | Structure |
|---|---|
| 1 | 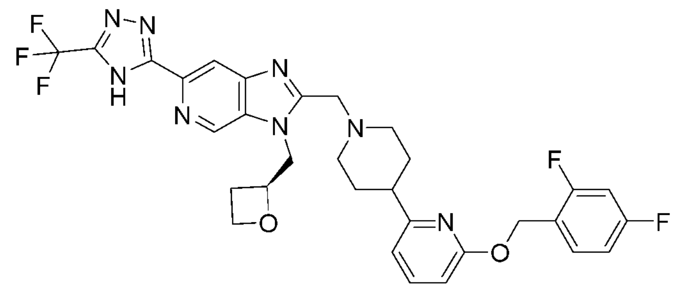 |
| 2 | 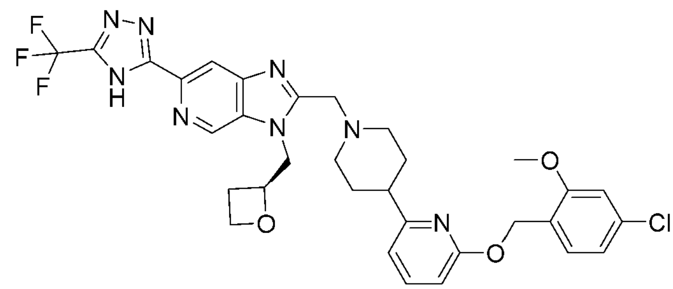 |

(continued)

| No. | Structure |
|---|---|
| 3 | 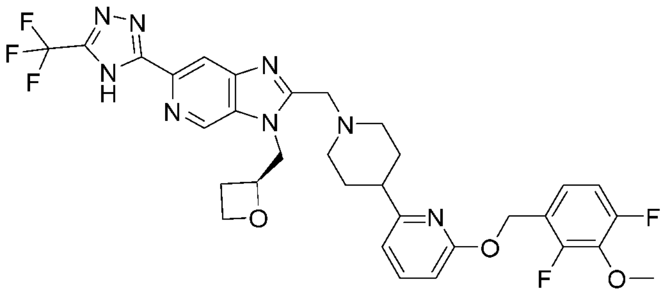 |
| 4 | 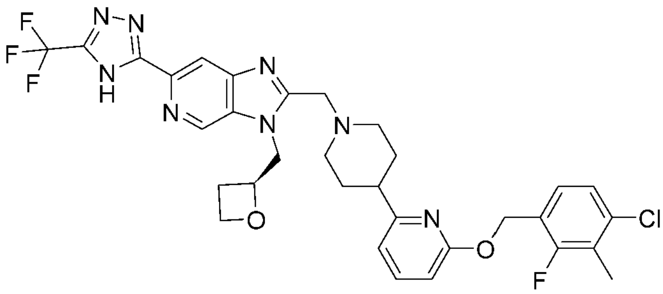 |
| 5 | 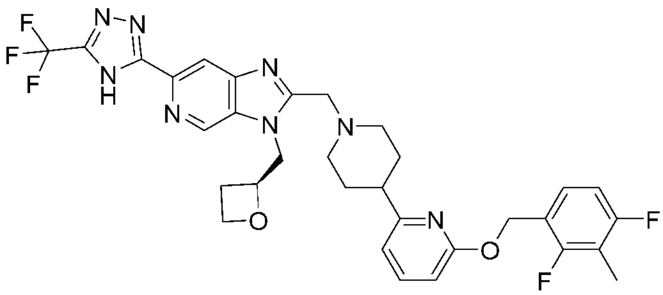 |
| 6 | 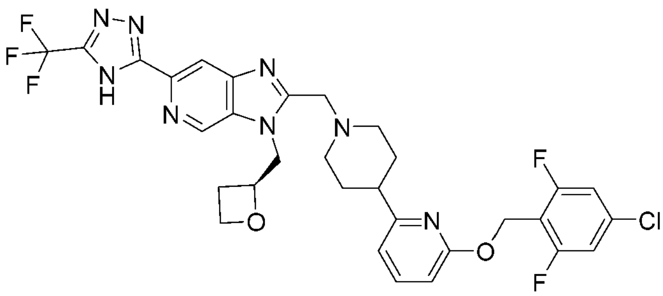 |
| 7 | 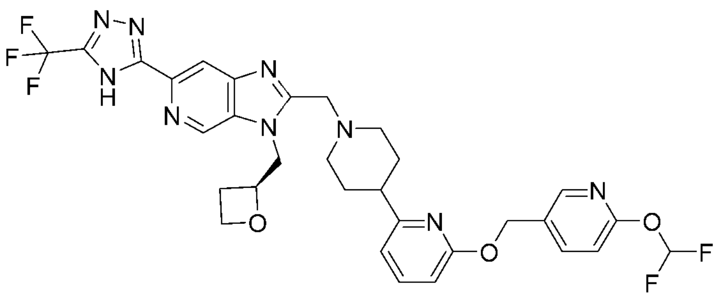 |

(continued)

| No. | Structure |
|---|---|
| 8 | 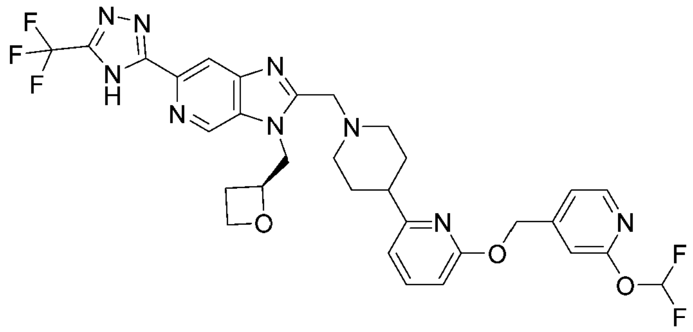 |
| 9 | 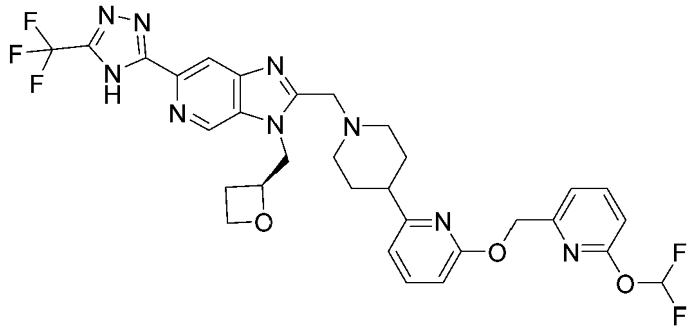 |
| 10 | 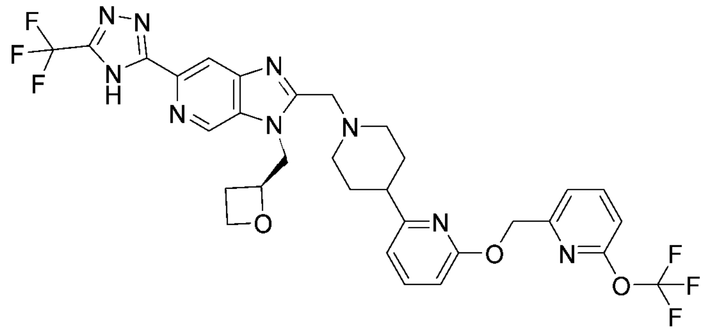 |
| 11 | 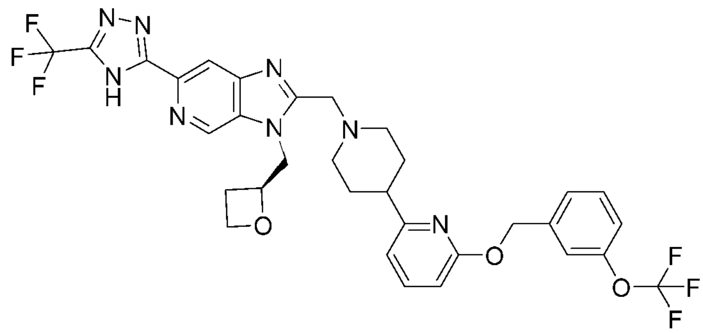 |
| 12 | 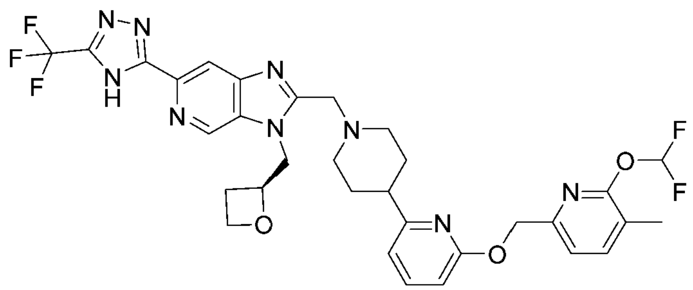 |

(continued)

| No. | Structure |
|---|---|
| 13 | 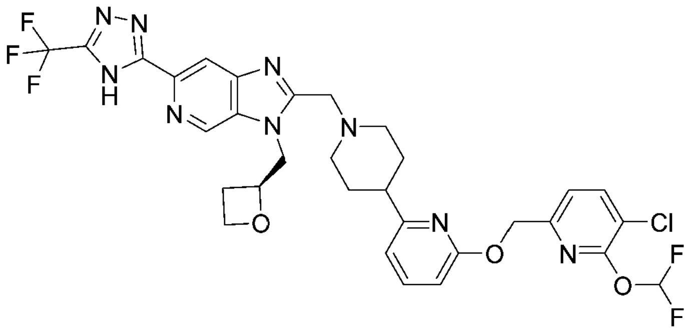 |
| 14 | 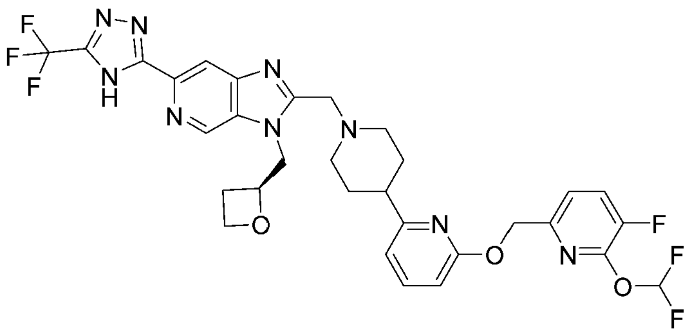 |
| 15 | 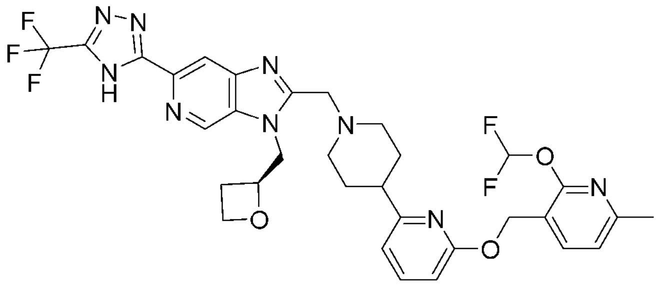 |
| 16 | 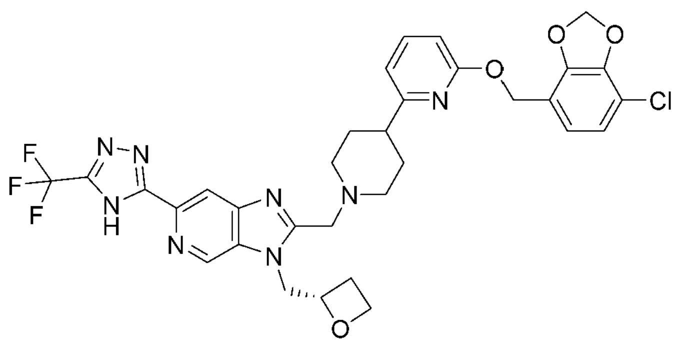 |
| 17 | 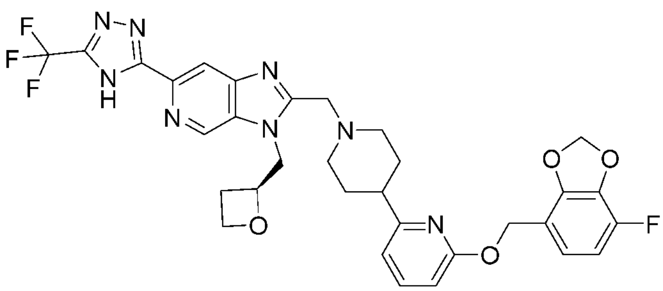 |

(continued)

| No. | Structure |
| --- | --- |
| 18 | 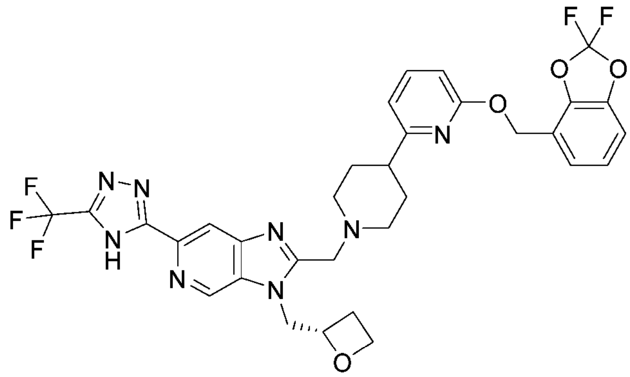 |
| 19 | 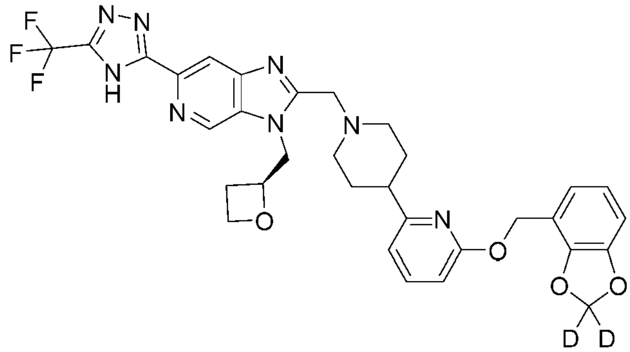 |
| 20 | 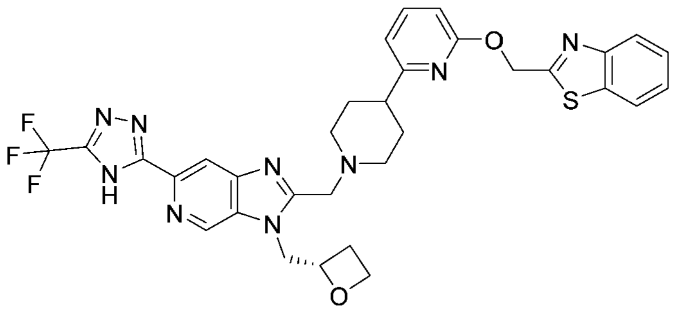 |
| 21 | 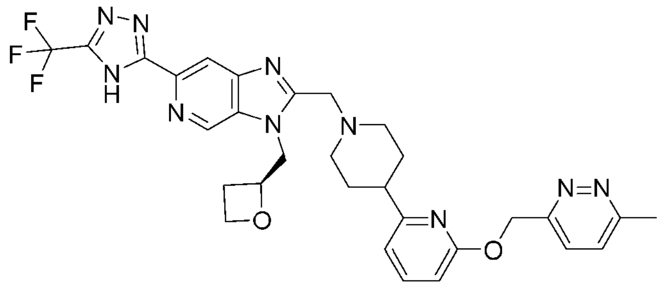 |
| 22 | 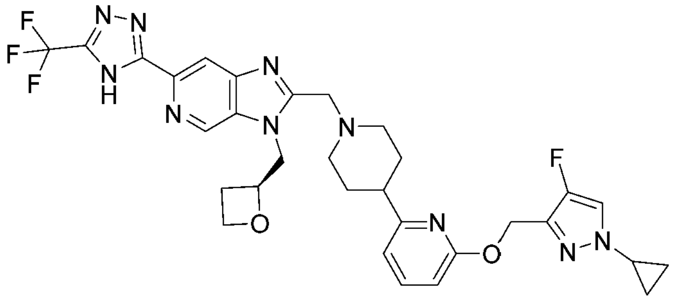 |

(continued)

| No. | Structure |
|---|---|
| 23 | 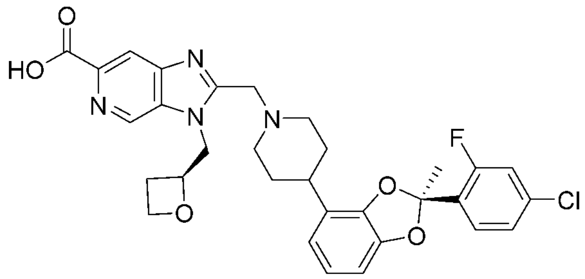 |
| 24 | 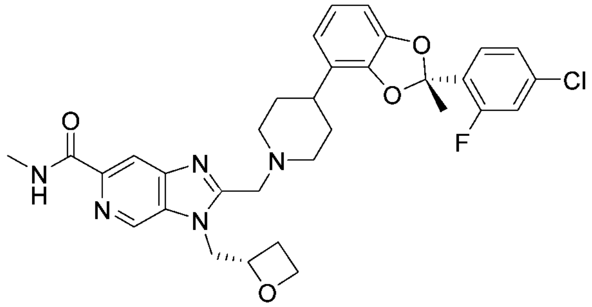 |
| 25 | 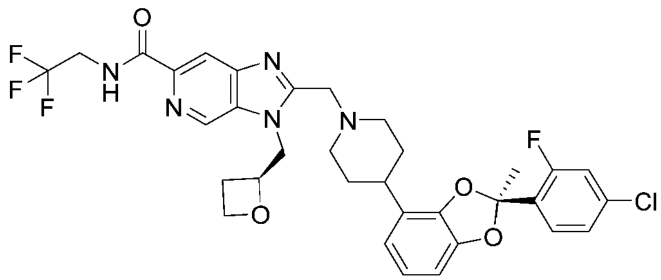 |
| 26 | 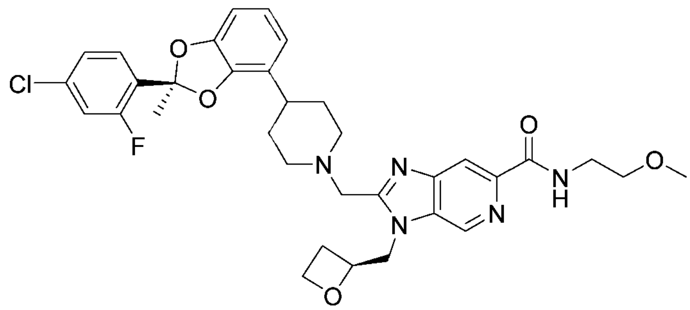 |
| 27 | 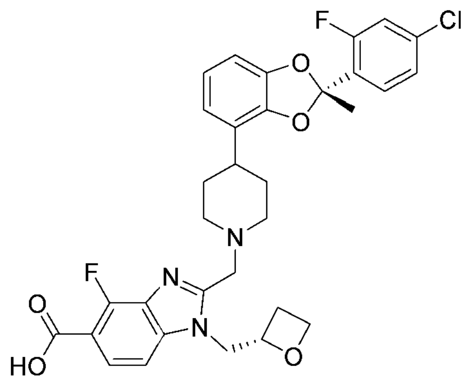 |

(continued)

| No. | Structure |
| --- | --- |
| 28 | 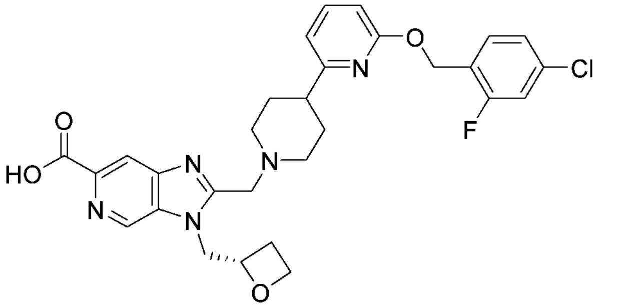 |
| 29 | 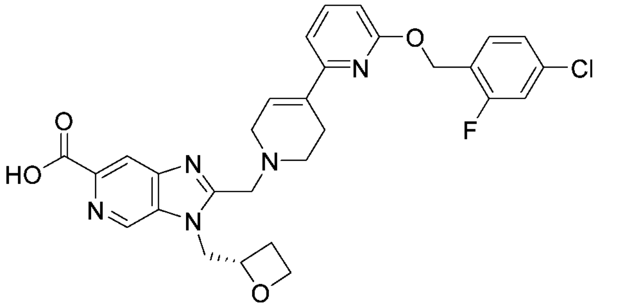 |
| 30 | 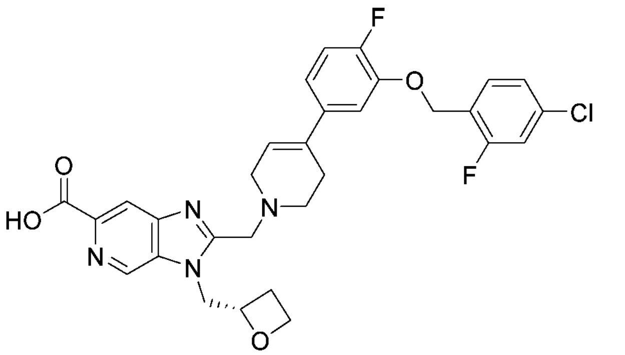 |
| 31 | 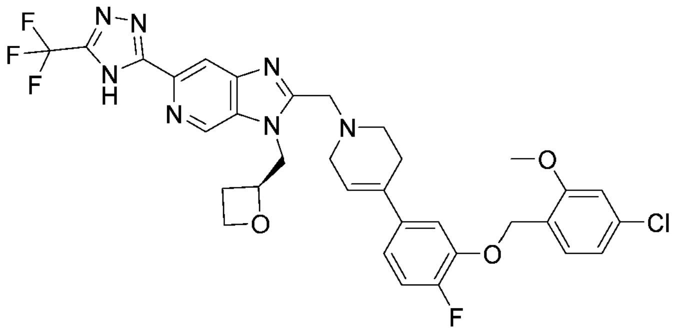 |

(continued)

| No. | Structure |
|---|---|
| 32 | 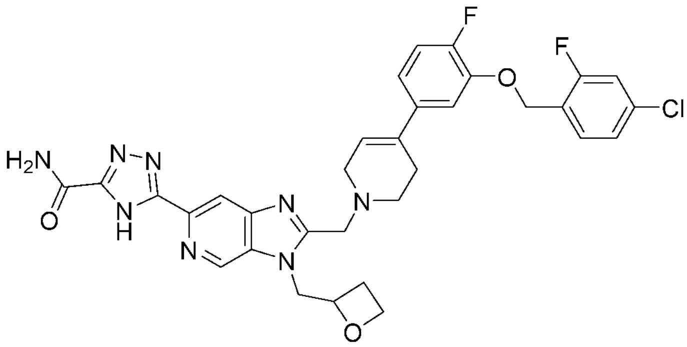 |
| 33 | 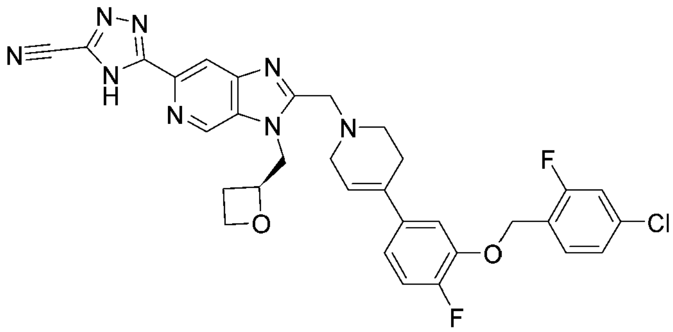 |
| 34 | 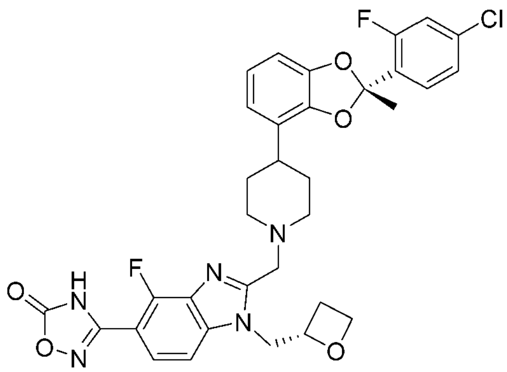 |
| 35 | 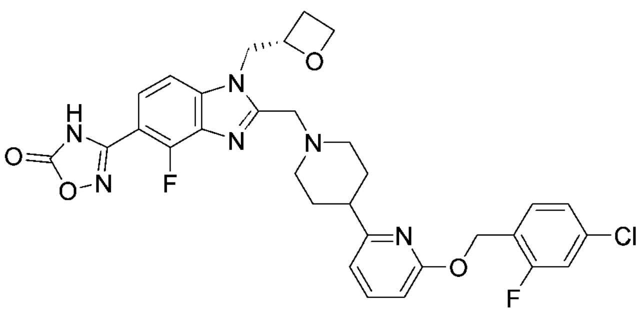 |

(continued)

| No. | Structure |
|---|---|
| 36 | 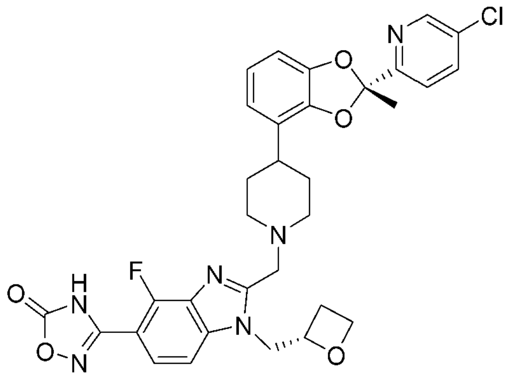 |
| 37 | 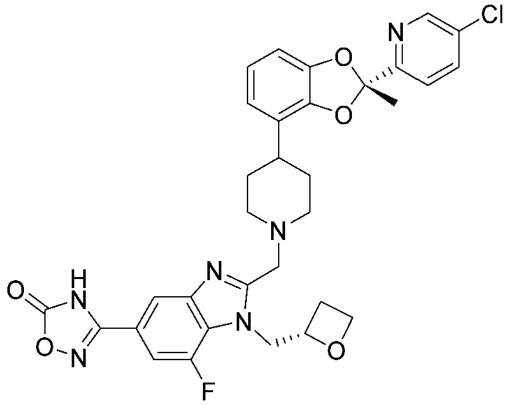 |
| 38 | 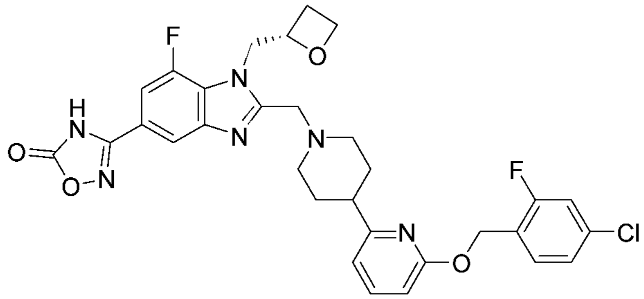 |
| 39 | 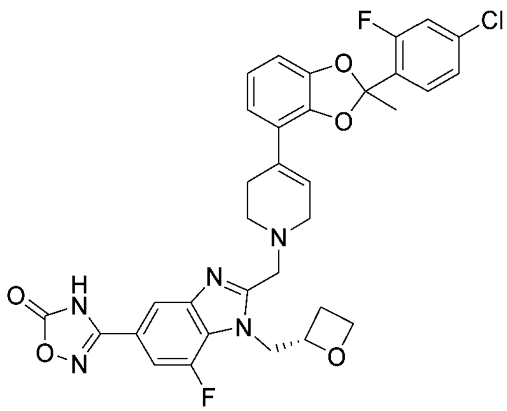 |

(continued)

| No. | Structure |
| --- | --- |
| 40 | 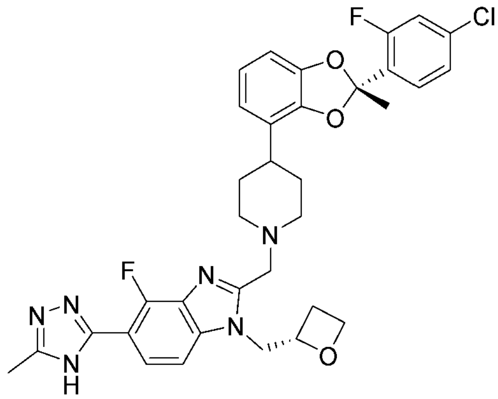 |
| 41 | 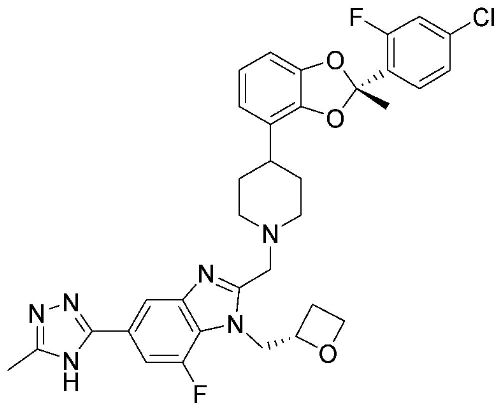 |
| 42 | 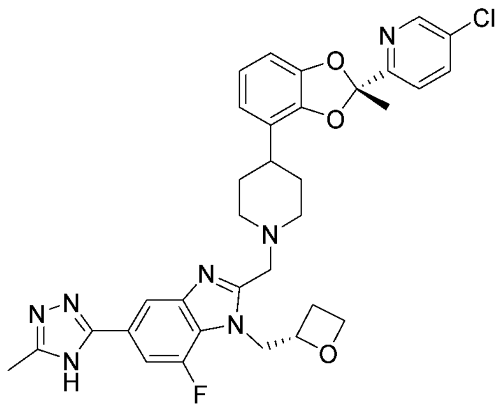 |
| 43 | 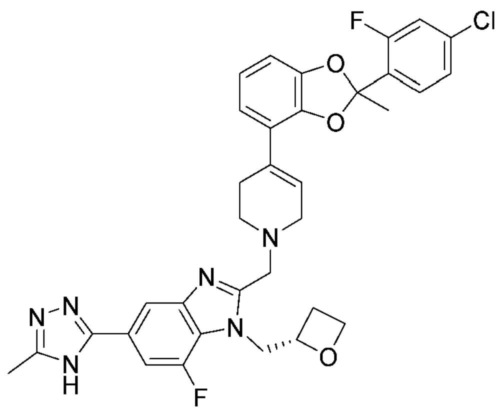 |

(continued)

| No. | Structure |
| --- | --- |
| 44 | 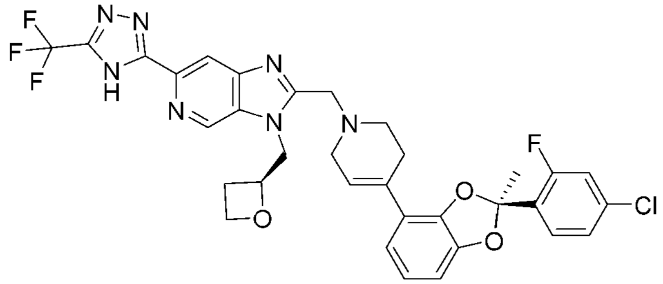 |
| 45 | 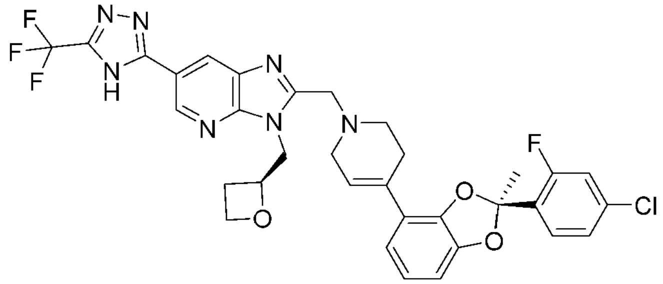 |
| 46 | 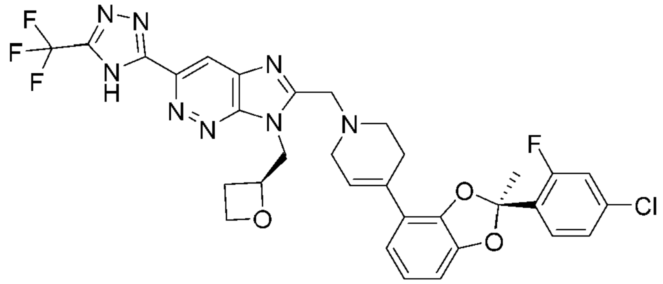 |
| 47 | 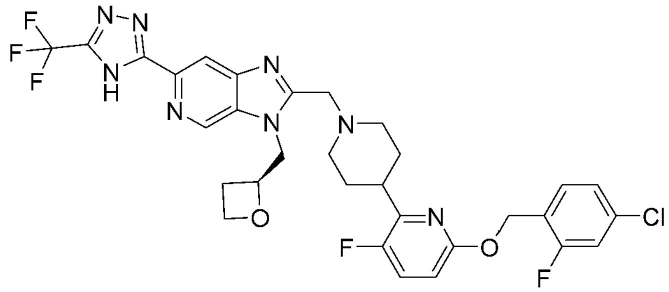 |
| 48 | 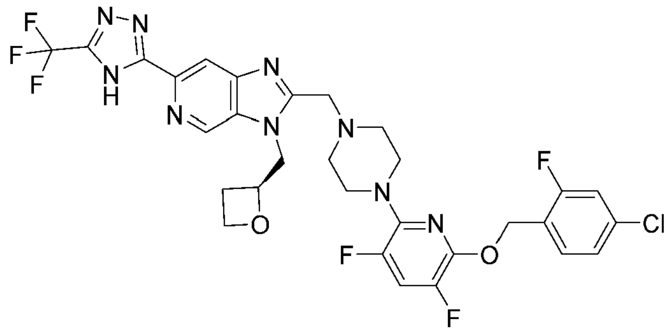 |

(continued)

| No. | Structure |
|---|---|
| 49 | 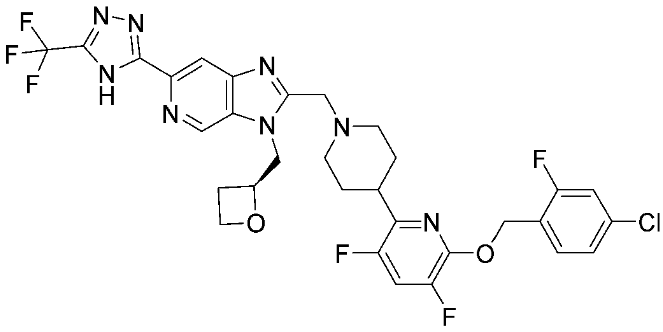 |
| 50 | 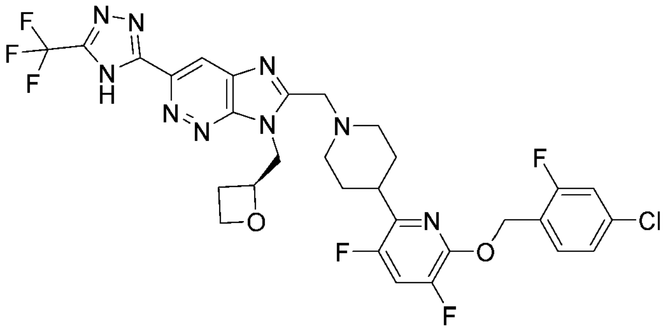 |
| 51 | 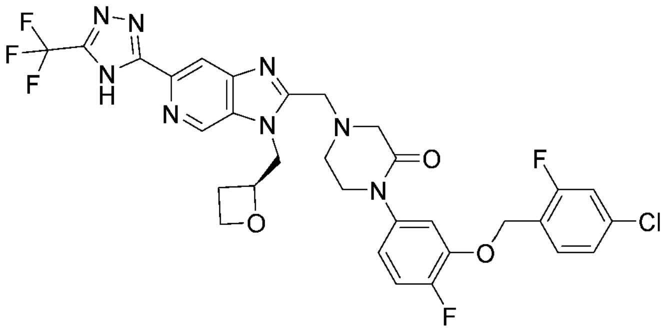 |
| 52 | 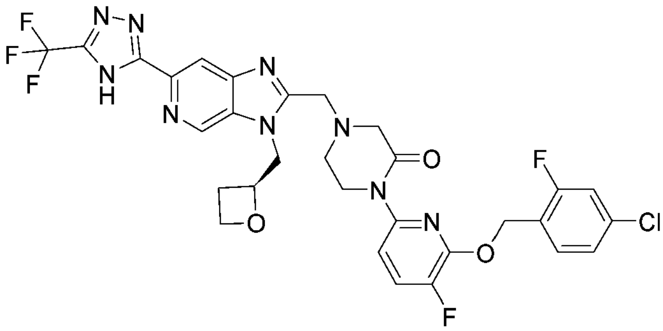 |
| 53 | 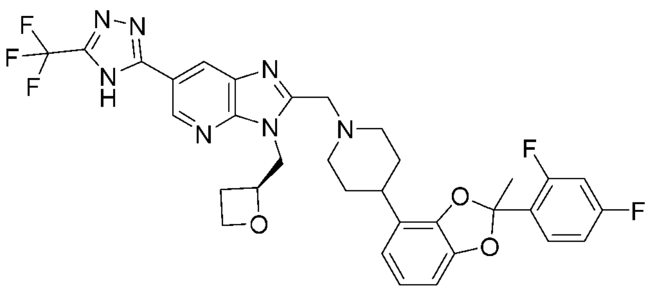 |

(continued)

| No. | Structure |
|---|---|
| 54 | 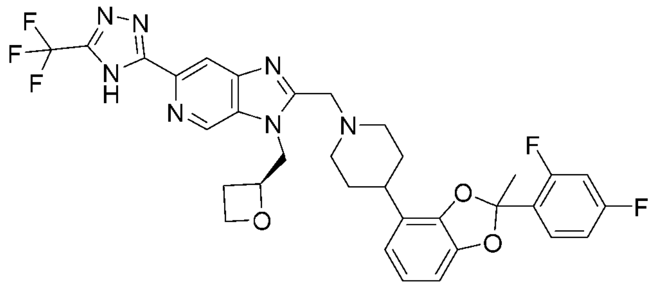 |
| 55 | 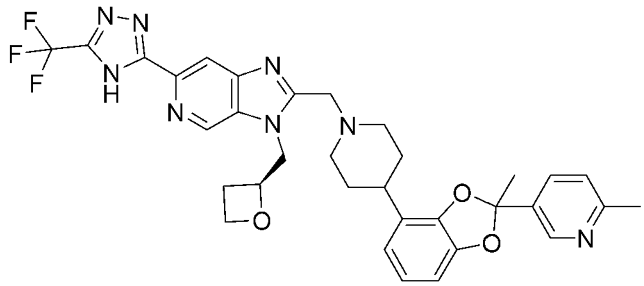 |
| 56 | 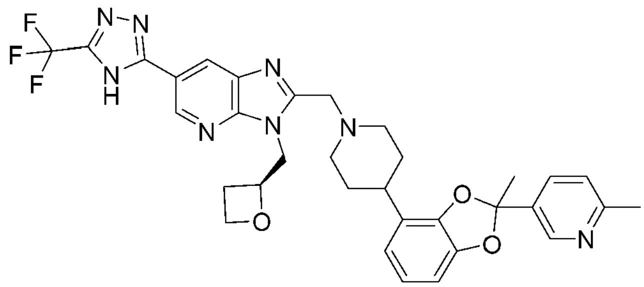 |
| 57 | 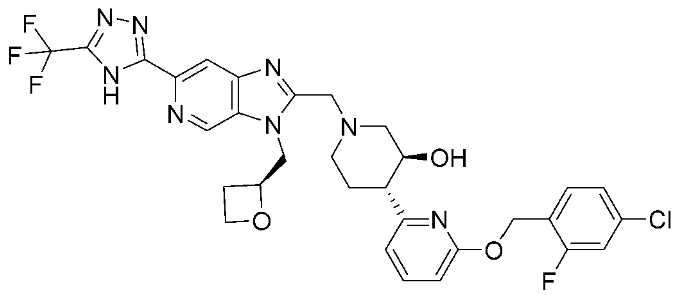 |
| 58 | 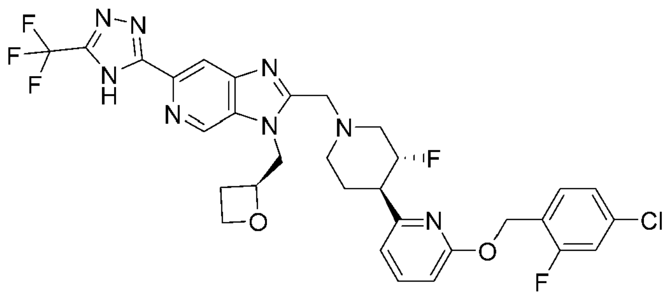 |

(continued)

| No. | Structure |
|---|---|
| 59 | 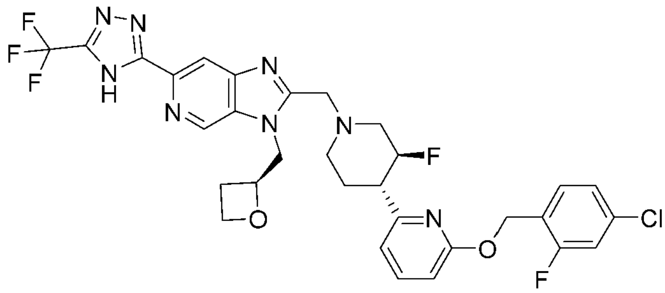 |
| 60 | 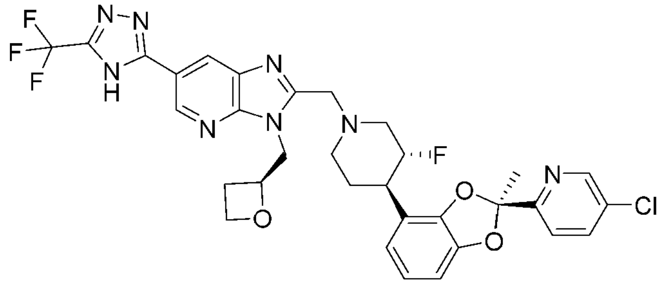 |
| 61 | 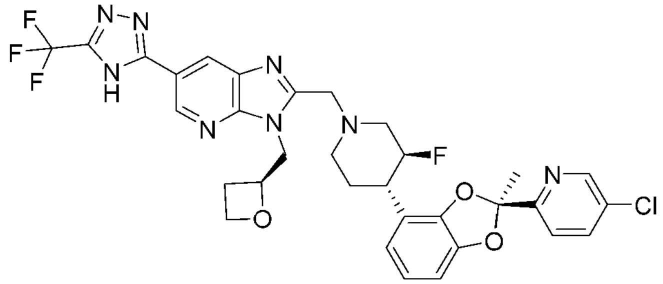 |
| 62 | 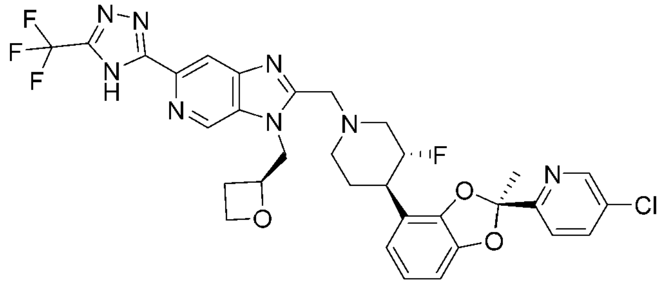 |
| 63 | 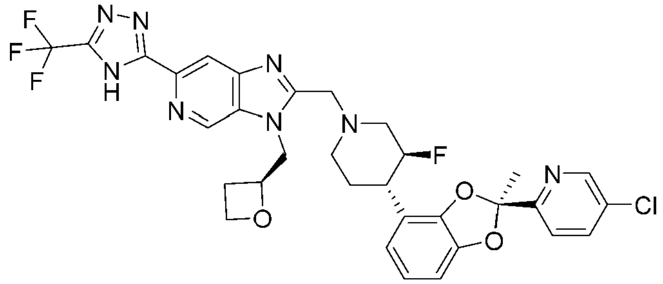 |

The compounds of Table 1 include pharmaceutically acceptable salts thereof. In addition, the compounds of Table 1 also include other salts of such compounds which are not necessarily pharmaceutically acceptable salts, and which may be useful as intermediates for preparing and/or purifying compounds of Table 1 and/or for separating enantiomers of compounds of Table 1. Non-limiting examples of pharmaceutically acceptable salts of compounds of Table 1 include trifluoroacetic acid salts.

It will further be appreciated that the compounds of Table 1 or their salts may be isolated in the form of solvates, and accordingly that any such solvate is included within the scope of the present disclosure.

In certain embodiments, the compound of Table 1 is the stereochemical form depicted, or a pharmaceutically acceptable salt thereof.

## Pharmaceutical Compositions and Administration

When employed as pharmaceuticals, compounds as described herein (e.g., a compound of Table 1, or a pharmaceutically acceptable salt or solvate thereof) can be administered in the form of a pharmaceutical compositions. These compositions can be prepared in a manner well known in the pharmaceutical art, and can be administered by a variety of routes, depending upon whether local or systemic treatment is desired and upon the area to be treated. Administration can be topical (including transdermal, epidermal, ophthalmic and to mucous membranes including intranasal, vaginal and rectal delivery), pulmonary (e.g., by inhalation or insufflation of powders or aerosols, including by nebulizer; intratracheal or intranasal), oral or parenteral. Oral administration can include a dosage form formulated for once-daily or twice-daily (BID) administration. Parenteral administration includes intravenous, intraarterial, subcutaneous, intraperitoneal intramuscular or injection or infusion; or intracranial, e.g., intrathecal or intraventricular, administration. Parenteral administration can be in the form of a single bolus dose, or can be, for example, by a continuous perfusion pump. Pharmaceutical compositions and formulations for topical administration can include transdermal patches, ointments, lotions, creams, gels, drops, suppositories, sprays, liquids and powders. Conventional pharmaceutical carriers, aqueous, powder or oily bases, thickeners and the like may be necessary or desirable.

Also provided herein are pharmaceutical compositions which contain, as the active ingredient, a compound of Table 1, or a pharmaceutically acceptable salt or solvate thereof, in combination with one or more pharmaceutically acceptable excipients (carriers). For example, a pharmaceutical composition prepared using a compound of Table 1, or a pharmaceutically acceptable salt or solvate thereof. In some embodiments, the composition is suitable for topical administration. In making the compositions provided herein, the active ingredient is typically mixed with an excipient, diluted by an excipient or enclosed within such a carrier in the form of, for example, a capsule, sachet, paper, or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, suppositories, sterile injectable solutions, and sterile packaged powders. In some embodiments, the composition is formulated for oral administration. In some embodiments, the composition is a solid oral formulation. In some embodiments, the composition is formulated as a tablet or capsule.

Further provided herein are pharmaceutical compositions containing a compound of Table 1, or a pharmaceutically acceptable salt or solvate thereof with a pharmaceutically acceptable excipient. Pharmaceutical compositions containing a compound of Table 1, or a pharmaceutically acceptable salt or solvate thereof as the active ingredient can be prepared by intimately mixing the compound of Table 1, or a pharmaceutically acceptable salt or solvate thereof with a pharmaceutical carrier according to conventional pharmaceutical compounding techniques. The carrier can take a wide variety of forms depending upon the desired route of administration (e.g., oral, parenteral, etc.). In some embodiments, the composition is a solid oral composition.

Suitable pharmaceutically acceptable carriers are well known in the art. Descriptions of some of these pharmaceutically acceptable carriers can be found in The Handbook of Pharmaceutical Excipients, published by the American Pharmaceutical Association and the Pharmaceutical Society of Great Britain.

Methods of formulating pharmaceutical compositions have been described in numerous publications such as Pharmaceutical Dosage Forms: Tablets, Second Edition, Revised and Expanded, Volumes 1-3, edited by Lieberman et al; Pharmaceutical Dosage Forms: Parenteral Medications, Volumes 1-2, edited by Avis et al; and Pharmaceutical Dosage Forms: Disperse Systems, Volumes 1-2, edited by Lieberman et al; published by Marcel Dekker, Inc.

In some embodiments, the compound or pharmaceutical composition can be administered in combination with one or more conventional pharmaceutical excipients. Pharmaceutically acceptable excipients include, but are not limited to, ion exchangers, alumina, aluminum stearate, lecithin, self-emulsifying drug delivery systems (SEDDS) such as d-α-tocopherol polyethylene glycol 1000 succinate, surfactants used in pharmaceutical dosage forms such as Tweens, poloxamers or other similar polymeric delivery matrices, serum proteins, such as human serum albumin, buffer substances such as phosphates, tris, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium-chloride, zinc salts, colloidal silica, magnesium trisilicate, polyvinyl pyrrolidone, cellulose-based substances, polyethylene glycol, sodium carboxymethyl cellulose, polyacrylates, waxes, polyethylene-polyoxypropylene-block polymers, and wool fat. Cyclodextrins such as α-, β, and γ-cyclodextrin, or chemically modified derivatives such as hydroxyalkylcyclodextrins, including 2- and 3-hydroxypropyl-β-cyclodextrins, or other solubilized

derivatives can also be used to enhance delivery of compounds described herein. Dosage forms or compositions containing a chemical entity as described herein in the range of 0.005% to 100% with the balance made up from non-toxic excipient may be prepared. The contemplated compositions may contain 0.001%-100% of a chemical entity provided herein, in one embodiment 0.1-95%, in another embodiment 75-85%, in a further embodiment 20-80%. Actual methods of preparing such dosage forms are known, or will be apparent, to those skilled in this art; for example, see Remington: The Science and Practice of Pharmacy, 22nd Edition (Pharmaceutical Press, London, UK. 2012).

In some embodiments, the compounds and pharmaceutical compositions described herein or a pharmaceutical composition thereof can be administered to patient in need thereof by any accepted route of administration. Acceptable routes of administration include, but are not limited to, buccal, cutaneous, endocervical, endosinusial, endotracheal, enteral, epidural, interstitial, intra-abdominal, intra-arterial, intrabronchial, intrabursal, intracerebral, intracisternal, intracoronary, intradermal, intraductal, intraduodenal, intradural, intraepidermal, intraesophageal, intragastric, intragingival, intraileal, intralymphatic, intramedullary, intrameningeal, intramuscular, intraovarian, intraperitoneal, intraprostatic, intrapulmonary, intrasinal, intraspinal, intrasynovial, intratesticular, intrathecal, intratubular, intratumoral, intrauterine, intravascular, intravenous, nasal (e.g., intranasal), nasogastric, oral, parenteral, percutaneous, peridural, rectal, respiratory (inhalation), subcutaneous, sublingual, submucosal, topical, transdermal, transmucosal, transtracheal, ureteral, urethral and vaginal. In some embodiments, a route of administration is parenteral (e.g., intratumoral).

In some embodiments, a compound of Table 1, or a pharmaceutically acceptable salt or solvate thereof as described herein, or pharmaceutical compositions thereof, can be formulated for parenteral administration, e.g., formulated for injection via the intraarterial, intrasternal, intracranial, intravenous, intramuscular, sub-cutaneous, or intraperitoneal routes. For example, such compositions can be prepared as injectables, either as liquid solutions or suspensions; solid forms suitable for use to prepare solutions or suspensions upon the addition of a liquid prior to injection can also be prepared; and the preparations can also be emulsified. The preparation of such formulations will be known to those of skill in the art in light of the present disclosure. In some embodiments, devices are used for parenteral administration. For example, such devices may include needle injectors, microneedle injectors, needle-free injectors, and infusion techniques.

In some embodiments, the pharmaceutical forms suitable for injectable use include sterile aqueous solutions or dispersions; formulations including sesame oil, peanut oil, or aqueous propylene glycol; and sterile powders for the extemporaneous preparation of sterile injectable solutions or dispersions. In some embodiments, the form must be sterile and must be fluid to the extent that it may be easily injected. In some embodiments, the form should be stable under the conditions of manufacture and storage and must be preserved against the contaminating action of microorganisms, such as bacteria and fungi.

In some embodiments, the carrier also can be a solvent or dispersion medium containing, for example, water, ethanol, polyol (for example, glycerol, propylene glycol, and liquid polyethylene glycol, and the like), suitable mixtures thereof, and vegetable oils. In some embodiments, the proper fluidity can be maintained, for example, by the use of a coating, such as lecithin, by the maintenance of the required particle size in the case of dispersion, and by the use of surfactants. In some embodiments, the prevention of the action of microorganisms can be brought about by various antibacterial and antifungal agents, for example, parabens, chlorobutanol, phenol, sorbic acid, thimerosal, and the like. In some embodiments, isotonic agents, for example, sugars or sodium chloride are included. In some embodiments, prolonged absorption of the injectable compositions can be brought about by the use in the compositions of agents delaying absorption, for example, aluminum monostearate and gelatin.

In some embodiments, sterile injectable solutions are prepared by incorporating a compound of Table 1, or a pharmaceutically acceptable salt or solvate thereof in the required amount in the appropriate solvent with various of the other ingredients enumerated above, as required, followed by filtered sterilization. In some embodiments, dispersions are prepared by incorporating the various sterilized active ingredients into a sterile vehicle which contains the basic dispersion medium and the required other ingredients from those enumerated above. In some embodiments, sterile powders are used for the preparation of sterile injectable solutions. In some embodiments, the methods of preparation are vacuum-drying and freeze-drying techniques, which yield a powder of the active ingredient, plus any additional desired ingredient from a previously sterile-filtered solution thereof.

In some embodiments, pharmacologically acceptable excipients usable in a rectal composition as a gel, cream, enema, or rectal suppository, include, without limitation, any one or more of cocoa butter glycerides, synthetic polymers such as polyvinylpyrrolidone, PEG (like PEG ointments), glycerine, glycerinated gelatin, hydrogenated vegetable oils, poloxamers, mixtures of polyethylene glycols of various molecular weights and fatty acid esters of polyethylene glycol, Vaseline, anhydrous lanolin, shark liver oil, sodium saccharinate, menthol, sweet almond oil, sorbitol, sodium benzoate, anoxid SBN, vanilla essential oil, aerosol, parabens in phenoxyethanol, sodium methyl p-oxybenzoate, sodium propyl p-oxybenzoate, diethylamine, carbomers, carbopol. methyloxybenzoate, macrogol cetostearyl ether, cocoyl caprylocaprate, isopropyl alcohol, propylene glycol, liquid paraffin, xanthan gum, carboxy-metabisulfite, sodium edetate, sodium benzoate, potassium metabisulfite, grapefruit seed extract, methyl sulfonyl methane (MSM) , lactic acid, glycine, vitamins, such as vitamin A and E and potassium acetate.

In some embodiments, suppositories can be prepared by mixing a compound of Table 1, or a pharmaceutically acceptable salt or solvate thereof, or pharmaceutical compositions as described herein with suitable non-irritating excipients or carriers such as cocoa butter, polyethylene glycol or a suppository wax which are solid at ambient temperature but liquid at body temperature and therefore melt in the rectum and release the active compound. In some embodiments, compositions for rectal administration are in the form of an enema.

In some embodiments, a compound of Table 1, or a pharmaceutically acceptable salt or solvate thereof, as described herein or a pharmaceutical composition thereof is formulated for local delivery to the digestive or GI tract by way of oral administration (e.g., solid or liquid dosage forms.).

In some embodiments, solid dosage forms for oral administration include capsules, tablets, pills, powders, and granules. In some embodiments, a compound of Table 1, or a pharmaceutically acceptable salt or solvate thereof, is mixed with one or more pharmaceutically acceptable excipients, such as sodium citrate or dicalcium phosphate and/or: a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. For example, in the case of capsules, tablets and pills, the dosage form may also comprise buffering agents. In some embodiments, solid compositions of a similar type may also be employed as fillers in soft and hard-filled gelatin capsules using such excipients as lactose or milk sugar as well as high molecular weight polyethylene glycols and the like.

In some embodiments, the pharmaceutical compositions will take the form of a unit dosage form such as a pill or tablet and thus the composition may contain, along with a compound of Table 1, or a pharmaceutically acceptable salt or solvate thereof as provided herein, a diluent such as lactose, sucrose, dicalcium phosphate, or the like; a lubricant such as magnesium stearate or the like; and a binder such as starch, gum acacia, polyvinylpyrrolidine, gelatin, cellulose, cellulose derivatives or the like. In some embodiments, another solid dosage form, a powder, marume, solution or suspension (e.g., in propylene carbonate, vegetable oils, PEG's, poloxamer 124 or triglycerides) is encapsulated in a capsule (gelatin or cellulose base capsule). In some embodiments, unit dosage forms in which one or more compounds and pharmaceutical compositions as provided herein or additional active agents are physically separated are also contemplated; e.g., capsules with granules (or tablets in a capsule) of each drug; two-layer tablets; two-compartment gel caps, etc. In some embodiments, enteric coated or delayed release oral dosage forms are also contemplated.

In some embodiments, other physiologically acceptable compounds may include wetting agents, emulsifying agents, dispersing agents or preservatives that are particularly useful for preventing the growth or action of microorganisms. For example, various preservatives are well known and include, for example, phenol and ascorbic acid.

In some embodiments, the excipients are sterile and generally free of undesirable matter. For example, these compositions can be sterilized by conventional, well-known sterilization techniques. In some embodiments, for various oral dosage form excipients such as tablets and capsules, sterility is not required. For example, the United States Pharmacopeia/National Formulary (USP/NF) standard can be sufficient.

In some embodiments, a compound of Table 1, or a pharmaceutically acceptable salt or solvate thereof as described herein or a pharmaceutical composition thereof is formulated for ocular administration. In some embodiments, ocular compositions can include, without limitation, one or more of any of the following: viscogens (e.g., carboxymethylcellulose, glycerin, polyvinylpyrrolidone, polyethylene glycol); stabilizers (e.g., pluronic (triblock copolymers), cyclodextrins); preservatives (e.g., benzalkonium chloride, EDTA, SofZia (boric acid, propylene glycol, sorbitol, and zinc chloride; Alcon Laboratories, Inc.), Purite (stabilized oxychloro complex; Allergan, Inc.)).

In some embodiments, a compound of Table 1, or a pharmaceutically acceptable salt or solvate thereof as described herein or a pharmaceutical composition thereof is formulated for topical administration to the skin or mucosa (e.g., dermally or transdermally). In some embodiments, topical compositions can include ointments and creams. In some embodiments, ointments are semisolid preparations that are typically based on petrolatum or other petroleum derivatives. In some embodiments, creams containing the selected active agent are typically viscous liquid or semisolid emulsions, often either oil-in-water or water-in-oil. For example, cream bases are typically water-washable, and contain an oil phase, an emulsifier and an aqueous phase. For example, the oil phase, also sometimes called the "internal" phase, is generally comprised of petrolatum and a fatty alcohol such as cetyl or stearyl alcohol; the aqueous phase usually, although not necessarily, exceeds the oil phase in volume, and generally contains a humectant. In some embodiments, the emulsifier in a cream formulation is generally a nonionic, anionic, cationic or amphoteric surfactant. In some embodiments, as with other carriers or vehicles, an ointment base should be inert, stable, nonirritating and non-sensitizing.

In any of the foregoing embodiments, pharmaceutical compositions as described herein can include one or more one or more of the following: lipids, interbilayer crosslinked multilamellar vesicles, biodegradable poly(D,L-lactic-co-glycolic acid) [PLGA]-based or poly anhydride-based nanoparticles or microparticles, and nanoporous particle-supported

lipid bilayers.

The amount of the compound in a pharmaceutical composition or formulation can vary within the full range employed by those skilled in the art. Typically, the formulation will contain, on a weight percent (wt %) basis, from about 0.01-99.99 wt % of a compound of this disclosure based on the total formulation, with the balance being one or more suitable pharmaceutical excipients. In one embodiment, the compound is present at a level of about 1-80 wt %. Representative pharmaceutical formulations are described below.

*Formulation Example 1 - Tablet formulation*

The following ingredients are mixed intimately and pressed into single scored tablets.

| Ingredient | Quantity per tablet, mg |
|---|---|
| compound of this disclosure | 400 |
| cornstarch | 50 |
| croscarmellose sodium | 25 |
| lactose | 120 |
| magnesium stearate | 5 |

*Formulation Example 2 - Capsule formulation*

The following ingredients are mixed intimately and loaded into a hard-shell gelatin capsule

| Ingredient | Quantity per capsule, mg |
|---|---|
| compound of this disclosure | 200 |
| lactose, spray-dried | 148 |
| magnesium stearate | 2 |

*Formulation Example 3 - Suspension formulation*

The following ingredients are mixed to form a suspension for oral administration.

| Ingredient | Amount |
|---|---|
| compound of this disclosure | 1.0 g |
| fumaric acid | 0.5 g |
| sodium chloride | 2.0 g |
| methyl paraben | 0.15 g |
| propyl paraben | 0.05 g |
| granulated sugar | 25.0 g |
| sorbitol (70% solution) | 13.00 g |
| Veegum K (Vanderbilt Co.) | 1.0 g |
| flavoring | 0.035 mL |
| coloring | 0.5 mg |
| distilled water | q.s. to 100 mL |

*Formulation Example 4 - Injectable formulation*

The following ingredients are mixed to form an injectable formulation.

| Ingredient | Amount |
|---|---|
| compound of this disclosure | 0.2 mg-20 mg |
| sodium acetate buffer solution, 0.4 M | 2.0 mL |
| HCl (1N) or NaOH (1N) | q.s. to suitable pH |
| water (distilled, sterile) | q.s. to 20 mL |

*Formulation Example 5 - Suppository Formulation*

A suppository of total weight 2.5 g is prepared by mixing the compound of this disclosure with Witepsol® H-15 (triglycerides of saturated vegetable fatty acid; Riches-Nelson, Inc., New York), and has the following composition:

| Ingredient | Amount |
|---|---|
| compound of this disclosure | 500 mg |
| Witepsol® H-15 | balance |

In some embodiments, the dosage for a compound of Table 1, or a pharmaceutically acceptable salt or solvate thereof, is determined based on a multiple factors including, but not limited to, type, age, weight, sex, medical condition of the patient, severity of the medical condition of the patient, route of administration, and activity of the compound or pharmaceutically acceptable salt or solvate thereof. In some embodiments, proper dosage for a particular situation can be determined by one skilled in the medical arts. In some embodiments, the total daily dosage may be divided and administered in portions throughout the day or by means providing continuous delivery.

In some embodiments, a compound of Table 1, or a pharmaceutically acceptable salt or solvate thereof, is administered at a dose from about 0.01 to about 1000 mg. For example, from about 0.1 to about 30 mg, about 10 to about 80 mg, about 0.5 to about 15 mg, about 50 mg to about 200 mg, about 100 mg to about 300 mg, about 200 to about 400 mg, about 300 mg to about 500 mg, about 400 mg to about 600 mg, about 500 mg to about 800 mg, about 600 mg to about 900 mg, or about 700 mg to about 1000 mg. In some embodiments, the dose is a therapeutically effective amount.

In some embodiments, a compound of Table 1, or a pharmaceutically acceptable salt or solvate thereof as described herein is administered at a dosage of from about 0.0002 mg/Kg to about 100 mg/Kg (e.g., from about 0.0002 mg/Kg to about 50 mg/Kg; from about 0.0002 mg/Kg to about 25 mg/Kg; from about 0.0002 mg/Kg to about 10 mg/Kg; from about 0.0002 mg/Kg to about 5 mg/Kg; from about 0.0002 mg/Kg to about 1 mg/Kg; from about 0.0002 mg/Kg to about 0.5 mg/Kg; from about 0.0002 mg/Kg to about 0.1 mg/Kg; from about 0.001 mg/Kg to about 50 mg/Kg; from about 0.001 mg/Kg to about 25 mg/Kg; from about 0.001 mg/Kg to about 10 mg/Kg; from about 0.001 mg/Kg to about 5 mg/Kg; from about 0.001 mg/Kg to about 1 mg/Kg; from about 0.001 mg/Kg to about 0.5 mg/Kg; from about 0.001 mg/Kg to about 0.1 mg/Kg; from about 0.01 mg/Kg to about 50 mg/Kg; from about 0.01 mg/Kg to about 25 mg/Kg; from about 0.01 mg/Kg to about 10 mg/Kg; from about 0.01 mg/Kg to about 5 mg/Kg; from about 0.01 mg/Kg to about 1 mg/Kg; from about 0.01 mg/Kg to about 0.5 mg/Kg; from about 0.01 mg/Kg to about 0.1 mg/Kg; from about 0.1 mg/Kg to about 50 mg/Kg; from about 0.1 mg/Kg to about 25 mg/Kg; from about 0.1 mg/Kg to about 10 mg/Kg; from about 0.1 mg/Kg to about 5 mg/Kg; from about 0.1 mg/Kg to about 1 mg/Kg; from about 0.1 mg/Kg to about 0.5 mg/Kg). In some embodiments, a compound of Table 1, or a pharmaceutically acceptable salt or solvate thereof as described herein is administered as a dosage of about 100 mg/Kg.

In some embodiments, the foregoing dosages of a compound of Table 1, or a pharmaceutically acceptable salt or solvate thereof, can be administered on a daily basis (e.g., as a single dose or as two or more divided doses) or non-daily basis (e.g., every other day, every two days, every three days, once weekly, twice weeks, once every two weeks, once a month).

In some embodiments, the period of administration of a compound of Table 1, or a pharmaceutically acceptable salt or solvate thereof as described herein is for 1 day, 2 days, 3 days, 4 days, 5 days, 6 days, 7 days, 8 days, 9 days, 10 days, 1 1 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 1 1 months, 12 months, or more. In some embodiments, a period of during which administration is stopped is for 1 day, 2 days, 3 days, 4 days, 5 days, 6 days, 7 days, 8 days, 9 days, 10 days, 1 1 days, 12 days, 13 days, 14 days, 3 weeks, 4 weeks, 5 weeks, 6 weeks, 7 weeks, 8 weeks, 9 weeks, 10 weeks, 1 1 weeks, 12 weeks, 4 months, 5 months, 6 months, 7 months, 8 months, 9 months, 10 months, 1 1 months, 12 months, or more. In some embodiments, a compound of Table 1, or a pharmaceutically acceptable salt or solvate thereof is administered to a patient for a period of time followed by a separate period of time where administration of the compound of Table 1, or a pharmaceutically acceptable salt or solvate thereof is stopped. In some

embodiments, a compound of Table 1, or a pharmaceutically acceptable salt or solvate thereof is administered for a first period and a second period following the first period, with administration stopped during the second period, followed by a third period where administration of the compound of Table 1, or a pharmaceutically acceptable salt or solvate thereof is started and then a fourth period following the third period where administration is stopped. For example, the period of administration of a compound of Table 1, or a pharmaceutically acceptable salt or solvate thereof followed by a period where administration is stopped is repeated for a determined or undetermined period of time. In some embodiments, a period of administration is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more. In some embodiments, a period of during which administration is stopped is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more.

In some embodiments, a compound of Table 1, or a pharmaceutically acceptable salt or solvate thereof, is orally administered to the patient one or more times per day (e.g., one time per day, two times per day, three times per day, four times per day per day or a single daily dose).

In some embodiments, a compound of Table 1, or a pharmaceutically acceptable salt or solvate thereof, is administered by parenteral administration to the patient one or more times per day (e.g., 1 to 4 times, one time per day, two times per day, three times per day, four times per day or a single daily dose).

In some embodiments, a compound of Table 1, or a pharmaceutically acceptable salt or solvate thereof, is administered by parenteral administration to the patient weekly.

**Methods of Treatment**

In some embodiments, this disclosure features methods for treating a patient (e.g., a human) having a disease, disorder, or condition in which modulation of GLP-1R (e.g., repressed or impaired and/or elevated or unwanted GLP-1R) is beneficial for the treatment of the underlying pathology and/or symptoms and/or progression of the disease, disorder, or condition. In some embodiments, the methods described herein can include or further include treating one or more conditions associated, co-morbid or sequela with any one or more of the conditions described herein.

Provided herein is a method for treating a GLP-1 associated disease, disorder, or condition, the method comprising administering to a patient in need thereof an effective amount of a compound of Table 1, or a pharmaceutically acceptable salt or solvate thereof, or a pharmaceutical composition as disclosed herein.

In some embodiments, the disease, disorder, or condition includes, but is not limited to type 1 diabetes mellitus, type 2 diabetes mellitus, early onset type 2 diabetes mellitus, idiopathic type 1 diabetes mellitus (Type 1b), youth-onset atypical diabetes (YOAD), maturity onset diabetes of the young (MODY), latent autoimmune diabetes in adults (LADA), obesity (including hypothalamic obesity and monogenic obesity), weight gain from use of other agents, idiopathic intracranial hypertension, Wolfram syndrome, gout, excessive sugar craving, hypertriglyceridemia, dyslipidemia, malnutrition-related diabetes, gestational diabetes, kidney disease, adipocyte dysfunction, sleep apnea, visceral adipose deposition, eating disorders, cardiovascular disease, congestive heart failure, myocardial infarction, left ventricular hypertrophy, peripheral arterial disease, stroke, hemorrhagic stroke, ischemic stroke, transient ischemic attacks, atherosclerotic cardiovascular disease, traumatic brain injury, peripheral vascular disease, endothelial dysfunction, impaired vascular compliance, vascular restenosis, thrombosis, hypertension, pulmonary hypertension, restenosis after angioplasty, intermittent claudication, hyperglycemia, post-prandial lipemia, metabolic acidosis, ketosis, hyperinsulinemia, impaired glucose metabolism, insulin resistance, hepatic insulin resistance, alcohol use disorder, chronic renal failure, metabolic syndrome, syndrome X, smoking cessation, premenstrual syndrome, angina pectoris, diabetic nephropathy, impaired glucose tolerance, diabetic neuropathy, diabetic retinopathy, macular degeneration, cataract, glomerulosclerosis, arthritis, osteoporosis, treatment of addiction, cocaine dependence, bipolar disorder/major depressive disorder, skin and connective tissue disorders, foot ulcerations, psoriasis, primary polydipsia, non-alcoholic steatohepatitis (NASH), non-alcoholic fatty liver disease (NAFLD), ulcerative colitis, inflammatory bowel disease, colitis, irritable bowel syndrome, Crohn's disease, short bowel syndrome, Parkinson's, Alzheimer's disease, impaired cognition, schizophrenia, and Polycystic Ovary Syndrome (PCOS).

In some embodiments, the disease, disorder, or condition includes, but is not limited to type 2 diabetes mellitus, early onset type 2 diabetes mellitus, obesity, idiopathic intracranial hypertension, Wolfram syndrome, weight gain from use of other agents, gout, excessive sugar craving, hypertriglyceridemia, dyslipidemia, gestational diabetes, kidney disease (e.g., acute kidney disorder, tubular dysfunction, proinflammatory changes to the proximal tubules), adipocyte dysfunction, sleep apnea, visceral adipose deposition, eating disorders, cardiovascular disease, congestive heart failure, myocardial infarction, left ventricular hypertrophy, peripheral arterial disease, stroke, hemorrhagic stroke, ischemic stroke, transient ischemic attacks, atherosclerotic cardiovascular disease, hyperglycemia, post-prandial lipemia, metabolic acidosis, ketosis, hyperinsulinemia, impaired glucose metabolism, insulin resistance, hepatic insulin resistance, alcohol use disorder, chronic renal failure, metabolic syndrome, syndrome X, smoking cessation, premenstrual syndrome, angina pectoris, diabetic nephropathy, impaired glucose tolerance, diabetic neuropathy, diabetic retinopathy, bipolar disorder/major depressive disorder, skin and connective tissue disorders, foot ulcerations, psoriasis, primary polydipsia, non-alcoholic steatohepatitis (NASH), non-alcoholic fatty liver disease (NAFLD), short bowel syndrome, Parkinson's disease, Polycystic Ovary Syndrome (PCOS), or any combination thereof.

In some embodiments, the disease, disorder, or condition includes, but is not limited to type 2 diabetes mellitus, early onset type 2 diabetes mellitus, obesity, idiopathic intracranial hypertension, Wolfram syndrome, weight gain from use of other agents, gout, excessive sugar craving, hypertriglyceridemia, dyslipidemia, gestational diabetes, adipocyte dysfunction, visceral adipose deposition, myocardial infarction, peripheral arterial disease, stroke, transient ischemic attacks, hyperglycemia, post-prandial lipemia, metabolic acidosis, ketosis, hyperinsulinemia, impaired glucose metabolism, insulin resistance, hepatic insulin resistance, chronic renal failure, syndrome X, angina pectoris, diabetic nephropathy, impaired glucose tolerance, diabetic neuropathy, diabetic retinopathy, skin and connective tissue disorders, foot ulcerations, or any combination thereof.

In some embodiments, the compounds and pharmaceutical compositions and methods for treating a patient described herein induce one or more of a reduction of blood glucose levels (e.g., reduce blood glucose levels), a reduction of blood hemoglobin A1c (HbA1c) levels, a promotion of insulin synthesis, a stimulation of insulin secretion, an increase in the mass of β-cells, a modulation of gastric acid secretion, a modulation of gastric emptying, a decrease in the body mass index (BMI), and/or a decrease in glucagon production (e.g., level). In some embodiments, the compounds and pharmaceutical compositions and methods for treating a patient described herein can reduce blood glucose levels, reduce blood hemoglobin A1c (HbAlc) levels, promote insulin synthesis, stimulate insulin secretion, increase the mass of β-cells, modulate gastric acid secretion. modulate gastric emptying, decrease the body mass index (BMI), decrease glucagon production (e.g., level), or any combination thereof. In certain embodiments, the compounds and pharmaceutical compositions and methods for treating a patient described herein stabilize serum glucose and serum insulin levels (e.g., serum glucose and serum insulin concentrations). Also provided herein are methods for modulating glucose or insulin levels in a patient in need of such modulating, the method comprising administering to the patient an effective amount of a compound of Table 1, or a pharmaceutically acceptable salt or solvate thereof, or a pharmaceutical composition as disclosed herein.

In some embodiments, provided herein is a method for reducing the risk (e.g., by about at least 20%, at least 30%, at least 40%, at least 50%, at least 60%, at least 70%, or at least 80%) of major adverse cardiovascular events (MACE) in a patient in need thereof, the method comprising administering to the patient an effective amount of a compound of Table 1, or a pharmaceutically acceptable salt or solvate thereof, or a pharmaceutical composition as disclosed herein. In certain of these embodiments, the patient is an adult that has been diagnosed with type 2 diabetes (T2D). In certain embodiments, the patient is an adult that has been diagnosed with a heart disease. In certain embodiments, the patient is an adult that has been diagnosed with type 2 diabetes (T2D) and a heart disease. In certain embodiments, the patient is an adult that has type 2 diabetes (T2D). In certain embodiments, the patient is an adult that has a heart disease. In certain embodiments, the patient has type 2 diabetes (T2D) and a heart disease.

### *Indications*

*Obesity*

In some embodiments, the condition, disease or disorder is obesity and conditions, diseases or disorders that are associated with or related to obesity. Non-limiting examples of obesity and obesity related conditions include symptomatic obesity, simple obesity, childhood obesity, morbid obesity, and abdominal obesity (central obesity characterized by abdominal adiposity). Non-limiting examples of symptomatic obesity include endocrine obesity (e.g., Cushing syndrome, hypothyroidism, insulinoma, obese type II diabetes, pseudohypoparathyroidism, hypogonadism), hypothalamic obesity, hereditary obesity (e.g., Prader-Willi syndrome, Laurence-Moon-Biedl syndrome), and drug-induced obesity (e.g., steroid, phenothiazine, insulin, sulfonylurea agent, or β-blocker-induced obesity).

In some embodiments, the condition, disease or disorder is associated with obesity. Examples of such conditions, diseases or disorders include, without limitation, glucose tolerance disorders, diabetes (e.g., type 2 diabetes, obese diabetes), lipid metabolism abnormality, hyperlipidemia, hypertension, cardiac failure, hyperuricemia, gout, fatty liver (including non-alcoholic steatohepatitis (NASH)), coronary heart disease (e.g., myocardial infarction, angina pectoris), cerebral infarction (e.g., brain thrombosis, transient cerebral ischemic attack), bone or articular disease (e.g., knee osteoarthritis, hip osteoarthritis, spondylitis deformans, lumbago), sleep apnea syndrome, obesity hypoventilation syndrome (Pickwickian syndrome), menstrual disorder (e.g., abnormal menstrual cycle, abnormality of menstrual flow and cycle, amenorrhea, abnormal catamenial symptom), visceral obesity syndrome, urine incontinence, and metabolic syndrome. In some embodiments, the chemical compound and pharmaceutical compositions described herein

can be used to treat patients exhibiting symptoms of both obesity and insulin deficiency.

*Diabetes*

In some embodiments, the condition, disease or disorder is diabetes. Non-limiting examples of diabetes include type 1 diabetes mellitus, type 2 diabetes mellitus (e.g., diet-treated type 2-diabetes, sulfonylurea-treated type 2-diabetes, a far-advanced stage type 2-diabetes, long-term insulin-treated type 2-diabetes), diabetes mellitus (e.g., non-insulin-dependent diabetes mellitus, insulin-dependent diabetes mellitus), gestational diabetes, obese diabetes, autoimmune diabetes, and borderline type diabetes. In some embodiments, the condition, disease or disorder is type 2 diabetes mellitus (e.g., diet-treated type 2-diabetes, sulfonylurea-treated type 2-diabetes, a far-advanced stage type 2-diabetes, long-term insulin-treated type 2-diabetes).

Provided herein is a method of treating a diabetes mellitus in a patient, the method comprising (a) determining that the patient has type 2 diabetes mellitus, and (b) administering to the patient a therapeutically effective amount of a compound of Table 1, or a pharmaceutically acceptable salt or solvate thereof, or a pharmaceutical composition as disclosed herein.

Provided herein is a method for treating type 2 diabetes mellitus in a patient, the method comprising administering to a patient identified or diagnosed as having type 2 diabetes mellitus a therapeutically effective amount of a compound of Table 1, or a pharmaceutically acceptable salt or solvate thereof, or a pharmaceutical composition as disclosed herein.

Also provided herein is a method of treating type 2 diabetes mellitus in a patient in need thereof, the method comprising administering to the patient a therapeutically effective amount of a compound of Table 1, or a pharmaceutically acceptable salt or solvate thereof, or a pharmaceutical composition as disclosed herein.

In some embodiments, the compounds and pharmaceutical compositions and methods for treating a patient with a condition, disease, or disorder (e.g., type 2 diabetes mellitus) described herein reduce fasting plasma glucose levels. In some embodiments, the compounds and pharmaceutical compositions and methods for treating a patient with a condition, disease, or disorder (e.g., type 2 diabetes mellitus) described herein reduce non-fasting plasma glucose levels. In some embodiments, the compounds and pharmaceutical compositions and methods for treating a patient with a condition, disease, or disorder (e.g., type 2 diabetes mellitus) described herein reduce HbA1c levels. In some embodiments, the compounds and pharmaceutical compositions and methods for treating a patient with a condition, disease, or disorder (e.g., type 2 diabetes mellitus) described herein reduce glucagon levels. In some embodiments, the compounds and pharmaceutical compositions and methods for treating a patient with a condition, disease, or disorder (e.g., type 2 diabetes mellitus) described herein increase insulin levels. In some embodiments, the compounds and pharmaceutical compositions and methods for treating a patient with a condition, disease, or disorder (e.g., type 2 diabetes mellitus) described herein reduce BMI.

In some embodiments, a reduction in fasting plasma glucose levels of about 5% to about 95% indicates treatment of type 2 diabetes mellitus. In some embodiments, a reduction in fasting plasma glucose levels of about 15% to about 80% indicates treatment of type 2 diabetes mellitus. In some embodiments, a reduction in fasting plasma glucose levels of about 25% to about 60% indicates treatment of type 2 diabetes mellitus. In some embodiments, a reduction in fasting plasma glucose levels to about or below 126 mg/dL, about or below 110 mg/dL, or about or below 90 mg/dL indicates treatment of the type 2 diabetes mellitus.

In some embodiments, a reduction in non-fasting plasma glucose levels of about 5% to about 95% indicates treatment of type 2 diabetes mellitus. In some embodiments, a reduction in non-fasting plasma glucose levels of about 15% to about 80% indicates treatment of type 2 diabetes mellitus. In some embodiments, a reduction in non-fasting plasma glucose levels of about 25% to about 60% indicates treatment of type 2 diabetes mellitus. In some embodiments, a reduction in non-fasting plasma glucose levels to about or below 200 mg/dL, about or below 150 mg/dL, or about or below 130 mg/dL indicates treatment of type 2 diabetes mellitus.

In some embodiments, a reduction in HbA1c levels of about 5% to about 95% indicates treatment of type 2 diabetes mellitus. In some embodiments, a reduction in HbA1c levels of about 15% to about 80% indicates treatment of type 2 diabetes mellitus. In some embodiments, a reduction in HbA1c levels of about 25% to about 60% indicates treatment of type 2 diabetes mellitus. In some embodiments, reduction in HbA1c levels to about or below 6.5%, about or below 6.0%, or about or below 5.0% indicates treatment of type 2 diabetes mellitus.

In some embodiments, a reduction in glucagon levels of about 5% to about 95% indicates treatment of type 2 diabetes mellitus. In some embodiments, a reduction in glucagon levels of about 15% to about 80% indicates treatment of type 2 diabetes mellitus. In some embodiments, a reduction in glucagon levels of about 25% to about 60% indicates treatment of type 2 diabetes mellitus. In some embodiments, an increase in insulin levels of about 5% to about 95% indicates treatment of type 2 diabetes mellitus. In some embodiments, an increase in insulin levels of about 15% to about 80% indicates treatment of type 2 diabetes mellitus. In some embodiments, an increase in insulin levels of about 25% to about 60% indicates treatment of type 2 diabetes mellitus.

In some embodiments, a reduction in BMI of about 5% to about 95% indicates treatment of type 2 diabetes

mellitus. In some embodiments, a reduction in BMI of about 15% to about 80% indicates treatment of the type 2 diabetes mellitus. In some embodiments, a reduction in BMI of about 25% to about 60% indicates treatment of type 2 diabetes mellitus. In some embodiments, a reduction in BMI of about 10%, about 15%, about 20%, about 25%, about 30%, about 35%, about 40%, about 45%, about 50%, about 55%, about 60%, about 65%, about 70%, about 75%, about 80%, about 85%, about 90%, or about 95% indicates treatment of type 2 diabetes mellitus. In some embodiments, a reduction in BMI to about or below 40, about or below 30, or about or below 20 indicates treatment of type 2 diabetes mellitus.

In some embodiments, the condition, disease or disorder is associated with diabetes (e.g., a complication of diabetes). Non-limiting examples of disorders associated with diabetes include obesity, obesity-related disorders, metabolic syndrome, neuropathy, nephropathy (e.g., diabetic nephropathy), retinopathy, diabetic cardiomyopathy, cataract, macroangiopathy, osteopenia, hyperosmolar diabetic coma, infectious disease (e.g., respiratory infection. urinary tract infection, gastrointestinal infection, dermal soft tissue infections, inferior limb infection), diabetic gangrene, xerostomia, hypacusis, cerebrovascular disorder, diabetic cachexia, delayed wound healing, diabetic dyslipidemia peripheral blood circulation disorder, cardiovascular risk factors. (e.g., coronary artery disease, peripheral artery disease, cerebrovascular disease, hypertension, and risk factors related to unmanaged cholesterol and/or lipid levels, and/or inflammation), NASH, bone fracture, and cognitive dysfunction

Other non-limiting examples of disorders related to diabetes include pre-diabetes, hyperlipidemia (e.g., hypertriglyceridemia, hypercholesterolemia, high LDL-cholesterolemia, low HDL-cholesterolemia, postprandial hyperlipemia), metabolic syndrome (e.g., metabolic disorder where activation of GLP-1R is beneficial, metabolic syndrome X), hypertension, impaired glucose tolerance (IGT), insulin resistance, and sarcopenia.

In some embodiments, the condition, disease or disorder is diabetes and obesity (diabesity). In some embodiments, the compounds described herein are also useful in improving the therapeutic effectiveness of metformin.

*Disorders of Metabolically Important Tissues*

In some embodiments, the condition, disease or disorder is a disorder of a metabolically important tissue. Non-limiting examples of metabolically important tissues include liver, fat, pancreas, kidney, and gut.

In some embodiments, the condition, disease or disorder is a fatty liver disease. Fatty liver diseases include, but are not limited to, non-alcoholic fatty acid liver disease (NAFLD), steatohepatitis, non-alcoholic steatohepatitis (NASH), fatty liver disease resulting from hepatitis, fatty liver disease resulting from obesity, fatty liver disease resulting from diabetes, fatty liver disease resulting from insulin resistance, fatty liver disease resulting from hypertriglyceridemia, abetalipoproteinemia, hyperlipoproteinemia, glycogen storage diseases, Weber-Christian disease, Wolman's disease, acute fatty liver of pregnancy, and lipodystrophy.

Non-alcoholic fatty liver disease (NAFLD) represents a spectrum of disease occurring in the absence of alcohol abuse and is typically characterized by the presence of steatosis (fat in the liver). NAFLD is believed to be linked to a variety of conditions, e.g., metabolic syndrome (including obesity, diabetes and hypertriglyceridemia) and insulin resistance. It can cause liver disease in adults and children and may ultimately lead to cirrhosis (Skelly et al., J Hepatol 2001; 35: 195-9; Chitturi et al., Hepatology 2002; 35(2):373-9). The severity of NAFLD ranges from the relatively benign isolated predominantly macrovesicular steatosis (i.e., nonalcoholic fatty liver or NAFL) to non-alcoholic steatohepatitis (NASH) (Angulo et al., J Gastroenterol Hepatol 2002; 17 Suppl:S186-90).

Other non-limiting examples of disorders in metabolically important tissues include joint disorders (e.g., osteoarthritis, secondary osteoarthritis), steatosis (e.g., in the liver); fibrosis (e.g., in the liver); cirrhosis (e.g., in the liver); gall stones; gallbladder disorders; gastroesophageal reflux; sleep apnea; hepatitis; fatty liver; bone disorder characterized by altered bone metabolism, such as osteoporosis, including post-menopausal osteoporosis, poor bone strength, osteopenia, Paget's disease, osteolytic metastasis in cancer patients, osteodistrophy in liver disease and the altered bone metabolism caused by renal failure or hemodialysis, bone fracture, bone surgery, aging, pregnancy, protection against bone fractures, and malnutrition polycystic ovary syndrome; renal disease (e.g., chronic renal failure, glomerulonephritis, glomerulosclerosis, nephrotic syndrome, hypertensive nephrosclerosis, end-stage renal disease); muscular dystrophy, angina pectoris, acute or chronic diarrhea, testicular dysfunction, respiratory dysfunction, frailty, sexual dysfunction (e.g., erectile dysfunction), and geriatric syndrome. In some embodiments, the compounds and pharmaceutical compositions described herein can be used for treating surgical trauma by improving recovery after surgery and/or by preventing the catabolic reaction caused by surgical trauma.

*Cardiovascular and Vascular Diseases*

In some embodiments, the condition, disease or disorder is a cardiovascular disease. Non-limiting examples of cardiovascular disease include congestive heart failure, atherosclerosis, arteriosclerosis, coronary heart disease, coronary artery disease, congestive heart failure, coronary heart disease, hypertension, cardiac failure, cerebrovascular disorder (e.g., cerebral infarction), vascular dysfunction, myocardial infarction, elevated blood pressure (e.g., 130/85 mm

Hg or higher), and prothrombotic state (exemplified by high fibrinogen or plasminogen activator inhibitor in the blood).

In some embodiments, the condition, disease or disorder is related to a vascular disease. Non-limiting examples of vascular diseases include peripheral vascular disease, macrovascular complications (e.g., stroke), vascular dysfunction, peripheral artery disease, abdominal aortic aneurysm, carotid artery disease, cerebrovascular disorder (e.g., cerebral infarction), pulmonary embolism, chronic venous insufficiency, critical limb ischemia, retinopathy, nephropathy, and neuropathy.

*Neurological Diseases*

In some embodiments, the condition, disease or disorder is a neurological disorder (e.g., neurodegenerative disorder) or a psychiatric disorder. Non-limiting examples of neurological disorders include idiopathic intracranial hypertension (IIH), brain insulin resistance, mild cognitive impairment (MCI), Alzheimer's disease (AD), Parkinson's disease (PD), anxiety, dementia (e.g., senile dementia), traumatic brain injury, Huntington's chores, tardive dyskinesia, hyperkinesia, mania, Morbus Parkinson, steel-Richard syndrome, Down's syndrome, myasthenia gravis, nerve trauma, brain trauma, vascular amyloidosis, cerebral hemorrhage I with amyloidosis, brain inflammation, Friedrich's ataxia, acute confusion disorder, amyotrophic lateral sclerosis (ALS), glaucoma, and apoptosis-mediated degenerative diseases of the central nervous system (e.g., Creutzfeld-Jakob Disease, bovine spongiform encephalopathy (mad cow disease), and chronic wasting syndrome). See, e.g., U.S. Publication No. 20060275288A1.

In some embodiments, the condition, disease or disorder is idiopathic intracranial hypertension. Idiopathic intracranial hypertension is characterized by increased intracranial pressure and papilloedema. See, e.g., Virdee et al. Ophthalmol Ther. 2020; 9(4):767-781. In some embodiments, the compounds and pharmaceutical compositions and methods described herein reduce cerebrospinal fluid secretion in a patient with idiopathic intracranial hypertension. In some embodiments, the compounds and pharmaceutical compositions and methods described herein reduce intracranial pressure in a patient with idiopathic intracranial hypertension. In some embodiments, the compounds and pharmaceutical compositions and methods described herein reduce one or more symptoms in a patient with idiopathic intracranial hypertension. Symptoms of idiopathic intracranial hypertension can include severe headaches and visual impairment. In some embodiments, the patient with idiopathic intracranial hypertension is female. In some embodiments, the patient with idiopathic intracranial hypertension is about 20 to about 30 years old. In some embodiments, the patient with idiopathic intracranial hypertension is obese.

In some embodiments, the condition, disease or disorder is Wolfram syndrome. Wolfram syndrome is caused by biallelic mutations of the Wolframin ER transmembrane glycoprotein (Wfs1) gene. See, e.g., Seppa et al. Sci Rep 9, 15742 (2019). Wolfram syndrome can first appear as diabetes mellitus, followed by optic nerve atrophy, deafness, and symptoms of neurodegeneration. Patients with Wolfram syndrome can have symptoms of ataxia, sleep apnea, dysphagia, hearing loss, and loss of taste due to brainstem atrophy. In some embodiments, the compounds and pharmaceutical compositions and methods described herein reduce neuroinflammation in a patient with Wolfram syndrome. In some embodiments, the neuroinflammation is reduced in the inferior olive in the patient. In some embodiments, the compounds and pharmaceutical compositions and methods described herein reduce retinal ganglion cell death in a patient with Wolfram syndrome. In some embodiments, the compounds and pharmaceutical compositions and methods described herein reduce axonal degeneration in a patient with Wolfram syndrome. In some embodiments, the compounds and pharmaceutical compositions and methods described herein reduce one or more symptoms (e.g., any of the symptoms described herein) in a patient with Wolfram syndrome.

Non-limiting examples of psychiatric disorders include drug dependence/addiction (narcotics and amphetamines and attention deficit/hyperactivity disorder (ADHD). The compounds and pharmaceutical compositions described herein can be useful in improving behavioral response to addictive drugs, decreasing drug dependence, prevention drug abuse relapse, and relieving anxiety caused by the absence of a given addictive substance. See, e.g., U.S. Publication No. 20120021979A1.

In some embodiments, the compounds and pharmaceutical compositions described herein are useful in improving learning and memory by enhancing neuronal plasticity and facilitation of cellular differentiation, and also in preserving dopamine neurons and motor function in Morbus Parkinson.

*Insulin-Related Conditions and Disorders*

In some embodiments, the condition, disease or disorder is impaired fasting glucose (IFG), impaired fasting glycemia (IFG), hyperglycemia, insulin resistance (impaired glucose homeostasis), hyperinsulinemia, elevated blood levels of fatty acids or glycerol, a hypoglycemic condition, insulin resistant syndrome, paresthesia caused by hyperinsulinemia, hyperlipidemia, hypercholesteremia, impaired wound healing, leptin resistance, glucose intolerance, increased fasting glucose, dyslipidemia (e.g., hyperlipidemia, atherogenic dyslipidemia characterized by high triglycerides and low HDL cholesterol), glucagonoma, hyperprolactinemia, hypoglycemia (e.g., nighttime hypoglycemia), and concomitant comatose endpoint associated with insulin.

In some embodiments, the compounds and pharmaceutical compositions described herein can reduce or slow down the progression of borderline type, impaired fasting glucose or impaired fasting glycemia into diabetes.

*Autoimmune Disorders*

In some embodiments, the condition, disease or disorder is an autoimmune disorder. Non-limiting examples of autoimmune disorders include multiple sclerosis, experimental autoimmune encephalomyelitis, autoimmune disorder is associated with immune rejection, graft versus host disease, uveitis, optic neuropathies, optic neuritis, transverse myelitis, inflammatory bowel disease, rheumatoid arthritis, ankylosing spondylitis, systemic lupus erythematosus, myasthenia gravis, and Graves' disease. See, e.g., U.S. Publication No. 20120148586A1.

*Stomach and Intestine-Related Disorders*

In some embodiments, the condition, disease or disorder is a stomach or intestine related disorder. Non-limiting examples of these disorders include ulcers of any etiology (e.g. peptic ulcers, Zollinger-Ellison syndrome, drug-induced ulcers, ulcers related to infections or other pathogens), digestion disorders, malabsorption, short bowel syndrome, cul-de-sac syndrome, inflammatory bowel diseases (Crohn's disease and ulcerative colitis), celiac sprue, hypogammaglobulinemic sprue, chemotherapy and/or radiation therapy-induced mucositis and diarrhea, gastrointestinal inflammation, short bowel syndrome, colitis ulcerosa, gastric mucosal injury (e.g., gastric mucosal injury caused by aspirin), small intestinal mucosal injury, and cachexia (e.g., cancerous cachexia, tuberculous cachexia, cachexia associated with blood disease, cachexia associated with endocrine disease, cachexia associated with infectious disease, and cachexia caused by acquired immunodeficiency syndrome).

*Body Weight*

In some embodiments, the compounds and pharmaceutical compositions described herein can be used to reduce body weight (e.g., excess body weight), prevent body weight gain, induce weight loss, decrease body fat, or reduce food intake in a patient (e.g., a patient in need thereof). In some embodiments, the weight increase in a patient may be attributed to excessive ingestion of food or unbalanced diets, or may be weight increase derived from a concomitant drug (e.g., insulin sensitizers having a PPARγ agonist-like action, such as troglitazone, rosiglitazone, englitazone, ciglitazone, pioglitazone and the like). In some embodiments, the weight increase may be weight increase before reaching obesity, or may be weight increase in an obese patient. In some embodiments, the weight increase may also be medication-induced weight gain or weight gain subsequent to cessation of smoking. In some embodiments, the weight gain is induced by the use of steroids or antipsychotics.

In some embodiments, the condition, disease or disorder is an eating disorder, such as hyperphagia, binge eating, bulimia, compulsive eating, or syndromic obesity such as Prader-Willi and Bardet-Biedl syndromes.

*Inflammatory Diseases*

In some embodiments, the condition, disease or disorder is an inflammatory disorder. Non-limiting examples of inflammatory disorders include chronic rheumatoid arthritis, spondylitis deformans, arthritis deformans, lumbago, gout, post-operational or post-traumatic inflammation, bloating, neuralgia, laryngopharyngitis, cystitis, pneumonia, pancreatitis, enteritis, inflammatory bowel disease (including inflammatory large bowel disease), inflammation in metabolically important tissues including liver, fat, pancreas, kidney and gut, and a proinflammatory state (e.g., elevated levels of proinflammatory cytokines or markers of inflammation-like C-reactive protein in the blood).

*Cancer*

In some embodiments, the condition, disease or disorder is cancer. Suitable examples of cancer include breast cancer (e.g., invasive ductal breast cancer, noninvasive ductal breast cancer, inflammatory breast cancer), prostate cancer (e.g., hormone-dependent prostate cancer, hormone-independent prostate cancer), pancreatic cancer (e.g., ductal pancreatic cancer), gastric cancer (e.g., papillary adenocarcinoma, mucous adenocarcinoma, adenosquamous carcinoma), lung cancer (e.g., non-small cell lung cancer, small-cell lung cancer, malignant mesothelioma), colon cancer (e.g., gastrointestinal stromal tumor), rectal cancer (e.g., gastrointestinal stromal tumor), colorectal cancer (e.g., familial colorectal cancer, hereditary non-polyposis colorectal cancer, gastrointestinal stromal tumor), small intestinal cancer (e.g., non-Hodgkin's lymphoma, gastrointestinal stromal tumor), esophageal cancer, duodenal cancer, tongue cancer, pharyngeal cancer (e.g., nasopharyngeal cancer, oropharynx cancer, hypopharyngeal cancer), salivary gland cancer,

brain tumor (e.g., pineal astrocytoma, pilocytic astrocytoma, diffuse astrocytoma, anaplastic astrocytoma), neurilemmoma, liver cancer (e.g., primary liver cancer, extrahepatic bile duct cancer), renal cancer (e.g., renal cell cancer, transitional cell cancer of the renal pelvis and ureter), bile duct cancer, endometrial cancer, uterine cervical cancer, ovarian cancer (e.g., epithelial ovarian cancer, extragonadal germ cell tumor, ovarian germ cell tumor, ovarian tumor of low malignant potential), bladder cancer, urethral cancer, skin cancer (e.g., intraocular (ocular) melanoma, Merkel cell carcinoma), hemangioma, malignant lymphoma, malignant melanoma, thyroid cancer (e.g., medullary thyroid cancer), parathyroid cancer, nasal cavity cancer, sinus cancer, bone tumor (e.g., osteosarcoma, Ewing tumor, uterine sarcoma, soft tissue sarcoma), angiofibroma, sarcoma of the retina, penis cancer, testicular tumor, pediatric solid tumor (e.g., Wilms' tumor, childhood kidney tumor), Kaposi's sarcoma, Kaposi's sarcoma caused by AIDS, tumor of maxillary sinus, fibrous histiocytoma, leiomyosarcoma, rhabdomyosarcoma, and leukemia (e.g., acute myeloid leukemia, acute lymphoblastic leukemia).

*Hypothalamic-pituitary disorders*

In some embodiments, the condition, disease or disorder is related to the hypothalamic-pituitary-gonadal axis. For example, the condition. disease or disorder is related to the hypothalamus-pituitary-ovary axis. In another example, the condition, disease or disorder is related to the hypothalamus-pituitary-testis axis. Hypothalamic-pituitary-gonadal axis diseases include, but are not limited to, hypogonadism, polycystic ovary syndrome, hypothyroidism, hypopituitarism, sexual dysfunction, and Cushing's disease.
In some embodiments, the condition, disease or disorder associated with diabetes is related to the hypothalamic-pituitary-gonadal axis.

*Pulmonary disease*

In some embodiments, the condition, disease or disorder is related to a pulmonary disease. Pulmonary diseases include, but are not limited to, asthma, idiopathic pulmonary fibrosis, pulmonary hypertension, obstructive sleep apnoea-hypopnoea syndrome, and chronic obstructive pulmonary disease (COPD) (e.g., emphysema, chronic bronchitis, and refractory (non-reversible) asthma).
In some embodiments, the condition, disease or disorder associated with diabetes is a pulmonary disease.

## Combination Therapy

In some embodiments, this disclosure contemplates both monotherapy regimens as well as combination therapy regimens.
In some embodiments, the methods described herein can further include administering one or more additional therapies (e.g., one or more additional therapeutic agents and/or one or more therapeutic regimens) in combination with administration of the compounds described herein.
In some embodiments, the methods described herein include administering a compound described herein in combination with one or more of a diet therapy (e.g., dietary monitoring, diet therapy for diabetes), an exercise therapy (e.g., physical activity), blood sugar monitoring, gastric electrical stimulation (e.g., TANTALUS®), and diet modifications.
In some embodiments, the compounds of Table 1, or a pharmaceutically acceptable salt or solvate thereof as described herein can be administered in combination with one or more additional therapeutic agents.
Representative additional therapeutic agents include, but are not limited to, anti-obesity agents, therapeutic agents for diabetes, therapeutic agents for diabetic complications, therapeutic agents for hyperlipidemia, antihypertensive agents, diuretics, chemotherapeutics, immunotherapeutics, antiinflammatory drugs, antithrombotic agents, anti-oxidants, therapeutic agents for osteoporosis, vitamins, antidementia drugs, erectile dysfunction drugs, therapeutic drugs for urinary frequency or urinary incontinence, therapeutic agents for NAFLD, therapeutic agents for NASH, therapeutic agents for dysuria and anti-emetic agents.
In some embodiments, the one or more additional therapeutic agents include those useful, for example, as anti-obesity agents. Non-limiting examples include monoamine uptake inhibitors (e.g., tramadol, phentermine, sibutramine, mazindol, fluoxetine, tesofensine), serotonin 2C receptor agonists (e.g., lorcaserin), serotonin 6 receptor antagonists, histamine H3 receptor modulator, GABA modulator (e.g., topiramate), including GABA receptor agonists (e.g., gabapentin, pregabalin), neuropeptide Y antagonists (e.g., velneperit), peptide YY or an analogue thereof, cannabinoid receptor antagonists (e.g., rimonabant, taranabant), ghrelin antagonists, ghrelin receptor antagonists, ghrelin acylation enzyme inhibitors, opioid receptor antagonists (e.g., GSK-1521498, naltrexone), orexin receptor antagonists, melanocortin 4 receptor agonists, 11β-hydroxysteroid dehydrogenase inhibitors (e.g., AZD-4017, BVT-3498, INCB-13739), pancreatic lipase inhibitors (e.g., orlistat, cetilistat), β3 agonists (e.g., N-5984), diacylglycerol acyltransferase 1 (DGAT1) inhibitors, acetylCoA carboxylase (ACC) inhibitors (e.g., compounds described in International Publication Nos. WO

2020/234726, WO 2020/044266, and U.S. Patent No. 8,859,577), stearoyl-CoA desaturated enzyme inhibitors, microsomal triglyceride transfer protein inhibitors (e.g., R-256918), sodium-glucose cotransporter 2 (SGLT-2) inhibitors (e.g., JNJ-28431754, dapagliflozin, AVE2268, TS-033, YM543, TA-7284, ASP1941, remogliflozin, empagliflozin, canagliflozin, ipragliflozin, tofogliflozin, sergliflozin etabonate, remogliflozin etabonate, or ertugliflozin), SGLT-1 inhibitors, MCR-4 agonists, monoamine reuptake inhibitors, melanocytestimulating hormone analogs, 5HT2c agonists, galanin antagonists, anorectic agents (such as a bombesin agonist), thyromimetic agents, dehydroepiandrosterone or analogs thereof, human agouti-related protein (AGRP) inhibitors, neuromedin U agonists, NFK inhibitors (e.g., HE-3286), PPAR agonists (e.g., GFT-505, DRF-11605, gemfibrozil, fenofibrate, balaglitazone, ciglitazone, darglitazone, englitazone, isaglitazone, pioglitazone, rosiglitazone, CLX-0940, GW-1536, GW-1 929, GW-2433, KRP-297, L-796449, LR-90, MK-0767, and SB-21 9994), phosphotyrosine phosphatase inhibitors (e.g., sodium vanadate, trodusquemin), GPR119 agonists (e.g., PSN-821, MBX-2982, APD597, compounds described in International Publication Nos. WO 2010/140092, WO 2010/128425, WO 2010/128414, WO 2010/106457), glucokinase activators (e.g., piragliatin, AZD-1656, AZD6370, TTP-355, TTP-399, TTP547, ARRY403, MK-0599, TAK-329, AZD5658 or GKM-001 compounds described in International Publication Nos. WO 2010/103437, WO 2010/103438, WO 2010/013161, WO 2007/122482, WO 2006/112549, WO 2007/028135, WO 2008/047821, WO 2008/050821, WO 2008/136428 and WO 2008/156757), leptin, leptin derivatives (e.g., metreleptin), leptin resistance improving drugs, CNTF (ciliary neurotrophic factor), BDNF (brain-derived neurotrophic factor), cholecystokinin agonists, amylin preparations (e.g., pramlintide, AC-2307), neuropeptide Y agonists (e.g., PYY3-36, derivatives of PYY3-36, obineptide, TM-30339, TM-30335), oxyntomodulin (OXM) preparations, appetite suppressants (e.g. ephedrine), FGF21 preparations (e.g., animal FGF21 preparations extracted from the pancreas of bovine or swine; human FGF21 preparations genetically synthesized using Escherichia coli or yeast; fragments or derivatives of FGF21), anorexigenic agents (e.g., P-57), human proislet peptide (HIP), melanocortin receptor 4 agonist (e.g., setmelanotide), melanin concentrating hormone receptor 1 antagonist, serotonergic agents (e.g. sibutramine, lorcaserin), farnesoid X receptor (FXR) agonist (e.g., obeticholic acid, tropifexor, cilofexor, LY2562175, Met409, TERN-101, EDP305, compounds described in International Publication Nos. WO 2020/234726 and WO 2020/044266), phentermine, zonisamide, norepinephrine/dopamine reuptake inhibitor (e.g., bupropion), GDF-15 analog, methionine aminopeptidase 2 (MetAP2) inhibitor (e.g., beloranib or ZGN-1061), diethylpropion, phendimetrazine, benzphetamine, fibroblast growth factor receptor (FGFR) modulator, biotin, a MAS receptor modulator, glucagon receptor agonist, CCKa agonists (e.g., compounds described in International Publication No. WO 2005/116034 and U.S. Publication No. 2005/0287100), and AMP-activated protein kinase (AMPK) activator.

In some embodiments, the one or more additional therapeutic agents include those useful, for example, as antidiabetic agents. Non-limiting examples include insulin and insulin preparations (e.g., animal insulin preparations extracted from the pancreas of bovine or swine; human insulin preparations genetically synthesized using Escherichia coli or yeast; zinc insulin; protamine zinc insulin; fragment or derivative of insulin (e.g., INS- 1), oral insulin preparation, synthetic human insulin), insulin sensitizers (e.g., pioglitazone or a salt thereof), biguanides (e.g., metformin, buformin or a salt thereof (e.g., hydrochloride, fumarate, succinate)), glucagon analogs (e.g., any of glucagon analogs described, e.g., in WO 2010/011439), agents which antagonize the actions of or reduce secretion of glucagon, sulfonylurea agents (e.g., chlorpropamide, tolazamide, glimepiride, tolbutamide, glibenclamide, gliclazide, acetohexamide, glyclopyramide, glybuzole, glyburide, glipizide), thiazolidinedione agents (e.g. rosiglitazone, lobeglitazone, troglitazone, balaglitazone, rivoglitazone, lobeglitazone or pioglitazone), glitazars (e.g., aleglitazar, chiglitazar, saroglitazar, muraglitazar, tesaglitazar), SGLT2 inhibitors (e.g., JNJ-28431754, dapagliflozin, AVE2268, TS-033, YM543, TA-7284, ASP1941, THR1474, TS-071, ISIS388626, LX4211, remogliflozin, empagliflozin, canagliflozin, ipragliflozin, tofogliflozin, sergliflozin etabonate, remogliflozin etabonate, ertugliflozin, compounds described in WO 2010/023594), GPR40 agonists (e.g., a FFAR1/FFA1 agonist, e.g. fasiglifam), α-glucosidase inhibitors (e.g., adiposin, camiglibose, pradimicin-Q, salbostatin, voglibose, acarbose, miglitol, emiglitate), insulin secretagogues, such as prandial glucose regulators (sometimes called "short-acting secretagogues"), e.g., meglitinides (e.g. repaglinide and nateglinide), cholinesterase inhibitors (e.g., donepezil, galantamine, rivastigmine, tacrine), NMDA receptor antagonists. dual GLP-1/GIP receptor agonists (e.g., LBT-2000, ZPD1-70), GLP-1R agonists (e.g., exenatide, liraglutide, albiglutide, dulaglutide, abiglutide, taspoglutide, lixisenatide, semaglutide, AVE-0010, S4P and Boc5), and dipeptidyl peptidase IV (DPP-4) inhibitors (e.g., vildagliptin, dutogliptin, gemigliptin, alogliptin, saxagliptin, sitagliptin, linagliptin, berberine, adogliptin, anagliptin (SK-0403), teneligliptin, omarigliptin, BI1356, GRC8200, MP-513, PF-00734200, PHX1149, ALS2-0426, TA-6666, TS-021, KRP-104, trelagliptin).

In some embodiments, the one or more additional therapeutic agents include those useful, for example, for treating NAFL and NASH. Non-limiting examples include FXR agonists (e.g., obeticholic acid), PF-05221304, PPAR α/δ agonists (e.g., elafibranor), a synthetic fatty acid-bile conjugate (e.g., aramchol), an anti-lysyl oxidase homologue 2 (LOXL2) monoclonal antibody (e.g., simtuzumab), a caspase inhibitor (e.g., emricasan), a MAPK5 inhibitor, a galectin 3 inhibitor (e.g., GR-MD-02), a fibroblast growth factor 21 (FGF21) (e.g., BMS-986036), a niacin analogue (e.g., ARJ 3037MO), a leukotriene D4 (LTD4) receptor antagonist (e.g., tipelukast), an acetyl-CoA carboxylase (ACC) inhibitor (e.g., NDI 010976 and compounds described in International Publication Nos. WO 2009/144554, WO 2003/072197, WO 2009/144555, and WO 2008/065508), a ketohexokinase (KHK) inhibitor, (e.g., compounds described in WO

2020/234726), an apoptosis signal-regulating kinase 1 (ASK1) inhibitor (selonsertib), an ileal bile acid transporter (IBAT) inhibitor, a dual antagonist of chemokine receptor 2 (CCR2) and CCR5 (e.g., cenicriviroc), diacylglyceryl acyltransferase 2 (DGAT2) inhibitor (e.g., compounds described in WO 2020/234726 and U.S. Publication No. 20180051012), a CB1 receptor antagonist, an anti-CB1R antibody, glycyrrhizin, schisandra extract, ascorbic acid, glutathione, silymarin, lipoic acid, and d-alpha-tocopherol, ascorbic acid, glutathione, vitamin B-complex, glitazones/thiazolidinediones (e.g., troglitazone, rosiglitazone, pioglitazone, balaglitazone, rivoglitazone, lobeglitazone), metformin, cysteamine, sulfonylureas, alpha-glucosidase inhibitors, meglitinides, vitamin E, tetrahydrolipstatin, milk thistle protein, anti-virals, and anti-oxidants.

In some embodiments, the one or more additional therapeutic agents include those useful, for example, for treating diabetic complications. Non-limiting examples include aldose reductase inhibitors (e.g., tolrestat, epalrestat, zopolrestat, fidarestat, CT-112, ranirestat, lidorestat), neurotrophic factor and increasing agents thereof (e.g., NGF, NT-3, BDNF, neurotrophic production/secretion promoting agents described in WO01/14372 (e.g., 4-(4-chlorophenyl)-2-(2-methyl-1-imidazolyl)-5-[3-(2-methylphenoxyl)propyl]oxazole), compounds described in WO2004/039365), PKC inhibitors (e.g., ruboxistaurin mesylate), AGE inhibitors (e.g., ALT946, N-phenacylthiazolium bromide (ALT766), EXO-226, pyridorin, pyridoxamine), serotonin and noradrenalin reuptake inhibitors (e.g., duloxetine), sodium channel inhibitors (e.g., lacosamide), active oxygen scavengers (e.g., thioctic acid), cerebral vasodilators (e.g., tiapuride, mexiletine), somatostatin receptor agonists (e.g., BIM23190), and apoptosis signal regulating kinase-1 (ASK-1) inhibitors.

In some embodiments, the one or more additional therapeutic agents include those useful, for example, for treating hyperlipidemia. Non-limiting examples include HMG-COA reductase inhibitors (e.g., pravastatin, simvastatin, lovastatin, atorvastatin, fluvastatin, rosuvastatin, pitavastatin or a salt thereof (e.g., sodium salt, calcium salt)), squalene synthase inhibitors (e.g., compounds described in WO97/10224, e.g., N-[[(3R,5S)-1-(3-acetoxy-2,2-dimethylpropyl)-7-chloro-5-(2,3-dimethoxyphenyl)-2-oxo- 1,2,3,5-tetrahydro-4,1-benzoxazepin-3-yl]acetyl]piperidin-4-acetic acid), fibrate compounds (e.g., bezafibrate, clofibrate, simfibrate, clinofibrate), anion exchange resin (e.g., colestyramine), nicotinic acid drugs (e.g., nicomol, niceritrol, niaspan), phytosterols (e.g., soysterol, gamma oryzanol ($\gamma$-oryzanol)), cholesterol absorption inhibitors (e.g., zechia), CETP inhibitors (e.g., dalcetrapib, anacetrapib) and $\omega$-3 fatty acid preparations (e.g., $\omega$-3-fatty acid ethyl esters 90).

In some embodiments, the one or more additional therapeutic agents include those useful, for example, as antihypertensive agents. Non-limiting examples include angiotensin converting enzyme inhibitors (e.g., captopril, zofenopril, fbsinopril, enalapril, ceranopril, cilazopril, delapril, pentopril, quinapril, ramipril, lisinopril), angiotensin II antagonists (e.g., candesartan cilexetil, candesartan, losartan, losartan potassium, eprosartan, valsartan, telmisartan, irbesartan, tasosartan, olmesartan, olmesartan medoxomil, azilsartan, azilsartan medoxomil), calcium antagonists (e.g., manidipine, nifedipine, amlodipine, efonidipine, nicardipine, cilnidipine) and $\beta$-blockers (e.g., metoprolol, atenolol, propranolol, carvedilol, pindolol). Further non-limiting examples of antihypertensive agents include: diuretics (e.g., chlorothiazide, hydrochlorothiazide. flumethiazide, hydroflumethiazide, bendroflumethiazide, methylchlorothiazide, trichloromethiazide, polythiazide, benzthiazide, ethacrynic acid tricrynafen, chlorthalidone, torsemide, furosemide, musolimine, bumetanide, triamtrenene, amiloride, spironolactone), alpha adrenergic blockers, beta adrenergic blockers, calcium channel blockers (e.g., diltiazem, verapamil, nifedipine and amlodipine), vasodilators (e.g., hydralazine), renin inhibitors, AT-1 receptor antagonists (e.g., losartan, irbesartan, valsartan), ET receptor antagonists (e.g., sitaxsentan, atrsentan, compounds disclosed in U.S. Patent Nos. 5,612,359 and 6,043,265), dual ET/AII antagonist (e.g., compounds disclosed in WO 2000/01389), neutral endopeptidase (NEP) inhibitors, If channel blocker ivabradinand, vasopepsidase inhibitors (dual NEP-ACE inhibitors) (e.g., gemopatrilat and nitrates).

In some embodiments, the one or more additional therapeutic agents include those useful, for example, as diuretics. Non-limiting examples include xanthine derivatives (e.g., theobromine sodium salicylate, theobromine calcium salicylate), thiazide preparations (e.g., ethiazide, cyclopenthiazide, trichloromethiazide, hydrochlorothiazide, hydroflumethiazide, benzylhydrochlorothiazide, penfluthiazide, polythiazide, methyclothiazide), antialdosterone preparations (e.g., spironolactone, triamterene), carbonic anhydrase inhibitors (e.g., acetazolamide) and chlorobenzenesulfonamide agents (e.g., chlortalidone, mefruside, indapamide).

In some embodiments, the one or more additional therapeutic agents include those useful, for example, as immunotherapeutic agents. Non-limiting examples include microbial or bacterial compounds (e.g., muramyl dipeptide derivative, picibanil), polysaccharides having immunoenhancing activity (e.g., lentinan, sizofiran, krestin), cytokines obtained by genetic engineering approaches (e.g., interferon, interleukin (IL) such as IL-1, IL-2, IL-12), and colony-stimulating factors (e.g., granulocyte colony-stimulating factor, erythropoietin).

In some embodiments, the one or more additional therapeutic agents include those useful, for example, as antithrombotic agents. Non-limiting examples include heparins (e.g., heparin sodium, heparin calcium, enoxaparin sodium, dalteparin sodium) warfarin (e.g., warfarin potassium); anti-thrombin drugs (e.g., aragatroban, dabigatran, boroarginine derivatives, boropeptides, heparins, hirudin, and melagatran), FXa inhibitors (e.g., rivaroxaban, apixaban, edoxaban, betrixaban, YM150, compounds described in WO02/06234, WO2004/048363, WO2005/030740, WO2005/058823, and WO2005/113504) thrombolytic agents (e.g., anistreplase, streptokinase, tenecteplase (TNK), lanoteplase (nPA), urokinase, tisokinase, alteplase, nateplase, monteplase, pamiteplase, factor VIIa inhibitors, PAI-1 inhibitors, alpha2-antiplasmin inhibitors, and anisoylated plasminogen streptokinase activator complex), and platelet aggregation inhibitors (e.g., ticlopidine hydrochloride, clopidogrel, prasugrel, E5555, SHC530348, cilostazol, ethyl icosapentate, beraprost sodium, and sarpogrelate hydrochloride).

In some embodiments, the one or more additional therapeutic agents include those useful, for example, for treating osteoporosis. Non-limiting examples include alfacalcidol, calcitriol, elcatonin, calcitonin salmon, estriol, ipriflavone, pamidronate disodium, alendronate sodium hydrate, incadronate disodium, and risedronate disodium. Suitable examples of vitamins include vitamin B1 and vitamin B12. Suitable examples of erectile dysfunction drugs include apomorphine and sildenafil citrate. Suitable examples of therapeutic agents for urinary frequency or urinary incontinence include flavorxate hydrochloride, oxybutynin hydrochloride and propiverine hydrochloride. Suitable examples of therapeutic agents for dysuria include acetylcholine esterase inhibitors (e.g., distigmine). Suitable examples of anti-inflammatory agents include nonsteroidal anti-inflammatory drugs such as aspirin, acetaminophen, indomethacin.

Other exemplary additional therapeutic agents include agents that modulate hepatic glucose balance (e.g., fructose 1,6-bisphosphatase inhibitors, glycogen phosphorylase inhibitors, glycogen synthase kinase inhibitors, glucokinase activators), agents designed to treat the complications of prolonged hyperglycemia, such as aldose reductase inhibitors (e.g. epalrestat and ranirestat), agents used to treat complications related to micro-angiopathies, anti-dyslipidemia agents, such as HMG-CoA reductase inhibitors (statins, e.g. rosuvastatin, pravastatin, pitavastatin, lovastatin, atorvastatin, simvastatin, fluvastatin, itavastatin, ZD-4522), HMG-CoA synthase inhibitors, cholesterol-lowering agents, bile acid sequestrants (e.g., cholestyramine, questran, colestipol, and colesevelam), cholesterol absorption inhibitors (e.g. plant sterols such as phytosterols), cholesteryl ester transfer protein (CETP) inhibitors, inhibitors of the ileal bile acid transport system (IBAT inhibitors), diacylglyceryl acyltransferase 1 (DGAT1) inhibitors (e.g., AZD7687, LCQ908, compounds described in WO 2009/016462, WO 2010/086820), monoacylglycerol O-acyltransferase inhibitors, $\alpha$-amylase inhibitors (e.g., tendamistat, trestatin, AL-3688), $\alpha$-glucoside hydrolase inhibitors, SIRT-1 activators, c-Jun N-terminal kinase (JNK) inhibitors, a VPAC2 receptor agonist, TGR5 receptor modulators (e.g., compounds described in ), GPBAR1 receptor modulators, GPR120 modulators, high affinity nicotinic acid receptor (HM74A) activators, carnitine palmitoyl transferase enzyme inhibitors, mineralocorticoid receptor inhibitors, inhibitors of TORC2, fatty acid synthetase inhibitors, serine palmitoyl transferase inhibitors, GPR81 modulators, GPR39 modulators, GPR43 modulators, GPR41 modulators, GPR105 modulators, Kv1.3 modulators, retinol binding protein 4 modulators, somatostain receptor modulators, PDHK2 modulators, PDHK4 modulators, MAP4K4 inhibitors, IL1 family modulators (e.g., ILI beta modulators), ACAT inhibitors, MTP inhibitors (e.g., diriotapide, mitratapide, and implitapide), lipooxygenase inhibitors, PCSK9 modulators (e.g., alirocumab and evolocumab), RXRalpha modulators, cysteamine, cystamine, an RNA antisense construct to inhibit protein tyrosine phosphatase PTPRU, vitamin B complex, pentraxin proteins, a protein tyrosine phosphatase-1 B (PTP-1 B) inhibitor (e.g., trodusquemine, hyrtiosal extract, and compounds described by Zhang et al. Drug Discovery Today. 2007, 12(9-10): 373-381), ezitimbe, betaine, pentoxifylline, alpha delta-9 desaturase, BCKDK inhibitors, branched-chain alpha keto acid dehydrogenase kinase (BCBK) inhibitors, PNPLA3 inhibitors, FGF1 9 analogs, SCD1 inhibitors, bile acid binding resins, nicotinic acid (niacin) and analogues thereof, antioxidants (e.g., probucol), omega-3 fatty acids, antihypertensive agents, including adrenergic receptor antagonists, such as beta blockers (e.g. atenolol), alpha blockers (e.g. doxazosin), and mixed alpha/beta blockers (e.g. labetalol), adrenergic receptor agonists, including alpha-2 agonists (e.g. clonidine), angiotensin converting enzyme (ACE) inhibitors (e.g. lisinopril), calcium channel blockers, such as dihydropyridines (e.g. nifedipine), phenylalkylamines (e.g. verapamil), and benzothiazepines (e.g. diltiazem), angiotensin II receptor antagonists (e.g. candesartan), aldosterone receptor antagonists (e.g. eplerenone, spironolactone), centrally acting adrenergic drugs, such as central alpha agonists (e.g. clonidine), diuretic agents (e.g. furosemide, torsemide, bemetanide, ethacrynic acid, thiazide-type diuretics (e.g., chlorothiazide, hydrochlorothiazide. benzthiazide, hydroflumethiazide, bendroflumethiazide, methychlorthiazide, polythiazide, trichlormethiazide, indapamide), phthalimidinetype diuretics (e.g., chlorthalidone, metolazone), quinazoline-type diuretics (e.g., quinethazone), potassium-sparing diuretics (e.g., triamterene and amiloride), thyroid receptor agonists (e.g., compounds described in WO 2020/117987), haemostasis modulators, including antithrombotics (e.g., activators of fibrinolysis), thrombin antagonists, factor VIIa inhibitors, anticoagulants (e.g., vitamin K antagonists such as warfarin), heparin and low molecular weight analogues thereof, factor Xa inhibitors, and direct thrombin inhibitors (e.g. argatroban), antiplatelet agents (e.g., cyclooxygenase inhibitors (e.g. aspirin), nonsteroidal anti-inflammatory drugs (NSAIDS), thromboxane-A2-receptor antagonists (e.g., ifetroban), thromboxane-A2-synthetase inhibitors, PDE inhibitors (e.g., Pletal, dipyridamole)), antagonists of purinergic receptors (e.g., P2Y1 and P2Y12), adenosine diphosphate (ADP) receptor inhibitors (e.g. clopidogrel), phosphodiesterase inhibitors (e.g. cilostazol), glycoprotein IIB/IIA inhibitors (e.g. tirofiban, eptifibatide, and abcixima), adenosine reuptake inhibitors (e.g. dipyridamole), noradrenergic agents (e.g. phentermine), serotonergic agents (e.g. sibutramine, lorcaserin), diacyl glycerolacyltransferase (DGAT) inhibitors, feeding behavior modifying agents, pyruvate dehydrogenase kinase (PDK) modulators, serotonin receptor modulators, monoamine transmission-modulating agents, such as selective serotonin reuptake inhibitors (SSRI) (e.g. fluoxetine), noradrenaline reuptake inhibitors (NARI), noradrenalineserotonin reuptake inhibitors (SNRI), and monoamine oxidase inhibitors (MAOI) (e.g. toloxatone and amiflamine), compounds described in WO 2007/013694, WO 2007/018314, WO 2008/093639 and WO 2008/099794, GPR40 agonists (e.g., fasiglifam or a hydrate

thereof, compounds described in WO 2004/041266, WO 2004/106276, WO 2005/063729, WO 2005/063725, WO 2005/087710, WO 2005/095338, WO 2007/013689 and WO 2008/001931), SGLT1 inhibitors, adiponectin or agonist thereof, IKK inhibitors (e.g., AS-2868), somatostatin receptor agonists, ACC2 inhibitors, cachexiaameliorating agents, such as a cyclooxygenase inhibitors (e.g., indomethacin), progesterone derivatives (e.g., megestrol acetate), glucocorticoids (e.g., dexamethasone), metoclopramide agents, tetrahydrocannabinol agents, agents for improving fat metabolism (e.g., eicosapentaenoic acid), growth hormones, IGF-1, antibodies against a cachexia-inducing factor TNF-α, LIF, IL-6, and oncostatin M, metabolism-modifying proteins or peptides such as glucokinase (GK), glucokinase regulatory protein (GKRP), uncoupling proteins 2 and 3 (UCP2 and UCP3), peroxisome proliferator-activated receptor α (PPARα), MC4r agonists, insulin receptor agonist, PDE 5 inhibitors, glycation inhibitors (e.g., ALT-711), nerve regeneration-promoting drugs (e.g., Y-128, VX853, prosaptide), antidepressants (e.g., desipramine, amitriptyline, imipramine), antiepileptic drugs (e.g., lamotrigine, trileptal, keppra, zonegran, pregabalin, harkoseride, carbamazepine), antiarrhythmic drugs (e.g., $K^+$ channel openers, mexiletine, propafenone, metoprolol, atenolol, carvadiol, propranolol, sotalol, dofetilide, amiodarone, azimilide, ibutilide, ditiazem, and verapamil), acetylcholine receptor ligands (e.g., ABT-594), endothelin receptor antagonists (e.g., ABT-627), narcotic analgesics (e.g., morphine), α2 receptor agonists (e.g., clonidine), local analgesics (e.g., capsaicin), antianxiety drugs (e.g., benzothiazepine), phosphodiesterase inhibitors (e.g., sildenafil), dopamine receptor agonists (e.g., apomorphine), cytotoxic antibodies (e.g., T-cell receptor and IL-2 receptor-specific antibodies), B cell depleting therapies (e.g., anti-CD20 antibody (e.g., rituxan), i-BLyS antibody), drugs affecting T cell migration (e.g., anti-integrin alpha 4/beta 1 antibody (e.g., tysabri), drugs that act on immunophilins (e.g., cyclosporine, tacrolimus, sirolimus, rapamicin), interferons (e.g., IFN-β), immunomodulators (e.g., glatiramer), TNF-binding proteins (e.g., circulating receptors), immunosupressants (e.g., mycophenolate), metaglidasen, AMG-131, balaglitazone, MBX-2044, rivoglitazone, aleglitazar, chiglitazar, saroglitazar, muraglitazar, tesaglitazar, lobeglitazone, PLX-204, PN-2034, GFT-505, THR-0921, exenatide, exendin-4, memantine, midazolam, ketoconazole, ethyl icosapentate, clonidine, azosemide, isosorbide, ethacrynic acid, piretanide, bumetanide, etoposide, piroxicam, NO donating agents (e.g., organonitrates), NO promoting agents (e.g., phosphodiesterase inhibitors).

In some embodiments, the one or more additional therapeutic agents include those useful, for example, as anti-emetic agents. As used herein, an "anti-emetic" agent refers to any agent that counteracts (e.g., reduces or removes) nausea or emesis (vomiting). While not wishing to be bound by theory, it is believed that administering one or more anti-emetic agents in combination with the Table 1 compounds described herein may allow higher dosages of the Table 1 compounds to be administered, e.g., because the patient may be able to have a normal food intake and thereby respond faster to the treatment.

Non-limiting examples of anti-emetic agents include 5HT3-receptor antagonists (serotonin receptor antagonists), neuroleptics/anti-psychotics, antihistamines, anticholinergic agents, steroids (e.g., corticosteroids), NK1 -receptor antagonists (e.g., Neurokinin 1 substance P receptor antagonists), antidopaminergic agents/dopamine receptor antagonists, benzodiazepines, and cannabinoids.

For example, the antiemetic agent can be selected from the group consisting of; neuroleptics, antihistamines, anti-cholinergic agents, steroids, 5HT-3-receptor antagonists, NK1 -receptor antagonists, anti- dopaminergic agents/-dopamine receptor antagonists, benzodiazepines and non- psychoactive cannabinoids.

In some embodiments, the anti-emetic agent is a 5HT3-receptor antagonist (serotonin receptor antagonist). Non-limiting examples of 5HT3-receptor antagonists (serotonin receptor antagonists) include: Granisetron (Kytril), Dolasetron, Ondansetron (Zofran), Tropisetron, Ramosetron, Palonosetron, Alosetron, azasetron, Bemesetron, Zatisetron, Batanopirde, MDL-73147EF; Metoclopramide, N-3389 (endo-3,9-dimethyl-3,9-diazabicyclo[3,3,1]non-7-yl-1 H-indazole-3-carboxamide dihydrochloride), Y-25130 hydrochloride, MDL 72222, Tropanyl-3,5-dimethylbenzoate, 3-(4-Allylpiperazin-1-yl)-2-quinoxalinecarbonitrile maleate, Zacopride hydrochloride, and Mirtazepine. Other non-limiting examples of 5HT3-receptor antagonists (serotonin receptor antagonists) include: cilansetron, clozapine, cyproheptadine, dazopride, hydroxyzine, lerisetron, metoclopramide, mianserin, olanzapine, palonosetron (+ netupitant), quetiapine, gamosetron, ramosteron, ricasetron, risperidone, ziprasidone, and zatosetron.

In certain embodiments, the 5HT-3-receptor antagonist is Granisetron, Dolasetron, Ondansetron hydrochloride, Tropisetron, Ramosetron, Palonosetron, Alosetron, Bemesetron, Zatisetron, Batanopirde, MDL-73147EF, Metoclopramide, N-3389, Y- 25130 hydrochloride, MDL 72222, Tropanyl-3,5-dimethylbenzoate 3-(4-AIIyl- piperazin-1-yl)-2-quinoxalinecarbonitrile maleate, Zacopride hydrochloride and Mirtazepine.

In certain embodiments, the 5HT-3-receptor antagonist is Granisetron, Dolasetron, Ondansetron hydrochloride, Tropisetron, Ramosetron, Palonosetron, Alosetron, Bemesetron, and Zatisetron.

In certain embodiments, the 5HT-3-receptor antagonist is Granisetron, Dolasetron and Ondansetron.

In certain embodiments, the 5HT-3-receptor antagonist is Granisetron.

In certain embodiments, the 5HT-3-receptor antagonist is Ondansetron.

In some embodiments, the anti-emetic agent is an antihistamine. Non-limiting examples of antihistamines include: piperazine derivatives (e.g.. cyclizine, meclizine, and cinnarizine); Promethazine; Dimenhydrinate (Dramamine, Gravol); Diphenhydramine; Hydroxyzine; Buclizine; and Meclizine hydrochloride (Bonine, Antivert), doxylamine, and

mirtazapine.

In some embodiments, the anti-emetic agent is an anticholinergic agent (Inhibitors of the acetylcholine receptors). Non-limiting examples of anticholinergic agents include: atropine, Scopolamine, Glycopyrron, Hyoscine, Artane (Trihexy-5 trihexyphenidyl hydrochloride), Cogentin (benztropine mesylate), Akineton (biperiden hydrochloride), Disipal (Norflex orphenadrine citrate), diphenhydramine, hydroxyzine, hyoscyamine, and Kemadrin (procyclidine hydrochloride).

In some embodiments, the anti-emetic agent is a steroid (e.g., a corticosteroid). Non-limiting examples of steroids include: betamethasone, Dexamethasone, Methylprednisolone, Prednisone®, and Trimethobenzamide (Tigan).

In some embodiments, the anti-emetic agent is an NK1 -receptor antagonists (e.g., Neurokinin 1 substance P receptor antagonists). Non-limiting examples of NK1 -receptor antagonists include: aprepitant, casopitant, ezlopitant, fosaprepitant, maropitant, netupitant, rolapitant, and vestipitant.

Other non-limiting examples of NK1-receptor antagonists include: MPC-4505, GW597599, MPC-4505, GR205171, L-759274, SR 140333, CP-96,345, BIIF 1149, NKP 608C, NKP 608A, CGP 60829, SR 140333 (Nolpitantium besilate/chloride), LY 303870 (Lanepitant), MDL-105172A, MDL-103896, MEN-11149, MEN-11467, DNK 333A, YM-49244, YM-44778, ZM-274773, MEN-10930, S-19752, Neuronorm, YM-35375, DA-5018, MK-869, L-754030, CJ-11974, L-758298, DNK-33A, 6b-1, CJ-11974 j. Benserazide and carbidopa k. TAK-637 [(aR,9R)-7-[3,5-bis(trifluoromethyl)benzyl]-8,9,10,11-tetrahydro-9-methyl-5-(4-methylphenyl)-7H-[1,4]diazocino[2,1-g] [1 ,7]naphthyridine-6,13-dione], PD 154075, ([(2-benzofuran)-$CH_2OCO$]-(R)-alpha-MeTrp-(S)-NHCH($CH_3$) Ph), FK888, and (D-Pro4, D-Trp7,9,10, Phe11)SP4-11.

In some embodiments, the anti-emetic agent is an anti- dopaminergic agents/dopamine receptor antagonist (e.g., dopamine receptor antagonist, e.g., D2 or D3 antagonists). Non-limiting examples include phenothiazines (e.g., promethazine, chlorpromazine, prochlorperazine, perphenazine, hydroxyzine, thiethylperazine, metopimazine,); benzamides (e.g., Metoclopramide, domperidone), butyrophenones (e.g., haloperidol, droperidol); alizapride, bromopride, clebopride, domperidone, itopride, metoclopramide, trimethobenzamide, and amisulpride.

In some embodiments, the anti-emetic agent is a non-psychoactive cannabinoids (e.g., Cannabidiol (CBD), Cannabidiol dimethylheptyl (CBD-DMH), Tetra-hydro-cannabinol (THC), Cannabinoid agonists such as WIN 55-212 (a CB1 and CB2 receptor agonist), Dronabinol (Marinol®), and Nabilone (Cesamet)).

Other exemplary anti-emetic agents include: c-9280 (Merck); benzodiazepines (diazepam, midazolam, lorazepam); neuroleptics/anti-psychotics (e.g., dixyrazine, haloperidol, and Prochlorperazine (Compazine®)); cerium oxalate; propofol; sodium citrate; dextrose; fructose(Nauzene); orthophosphoric acid; fructose; glucose (Emetrol); bismuth subsalicylate (Pepto Bismol); ephedrine; vitamin B6; peppermint, lavender, and lemon essential oils; and ginger.

Still other exemplary anti-emetic agents include those disclosed in US 20120101089A1; US 10,071,088 B2; US 6,673,792 B1; US 6,197,329 B1; US 10,828,297 B2; US 10,322,106 B2; US 10,525,033 B2; WO 2009080351 A1; WO 2019203753 A2; WO 2002020001 A2; US 8,119,697 B2; US 5,039,528; US20090305964A1; and WO 2006/111169.

In some embodiments, the additional therapeutic agent or regimen is administered to the patient prior to contacting with or administering the compounds and pharmaceutical compositions (e.g., about one hour prior, or about 6 hours prior, or about 12 hours prior, or about 24 hours prior, or about 48 hours prior, or about 1 week prior, or about 1 month prior).

In some embodiments, the additional therapeutic agent or regimen is administered to the patient at about the same time as contacting with or administering the compounds and pharmaceutical compositions. By way of example, the additional therapeutic agent or regimen and the compounds and pharmaceutical compositions are provided to the patient simultaneously in the same dosage form. As another example, the additional therapeutic agent or regimen and the compounds and pharmaceutical compositions are provided to the patient concurrently in separate dosage forms.

### *Patient Selection*

In some embodiments, the methods described herein further include the step of identifying a patient (e.g., a subject) in need of such treatment (e.g., by way of blood assay, body mass index, or other conventional method known in the art).

In some embodiments, the methods described herein further include the step of identifying a patient (e.g., patient) that has a disease, disorder, or condition as provided here (e.g., a GLP-1 associated disease, disorder, or condition).

In some embodiments, the methods described herein further include the step of identifying a patient (e.g., patient) that has type 2 diabetes mellitus. In some embodiments, determining if the patient has type 2 diabetes mellitus includes performing an assay to determine the level of hemoglobin A1c (HbA1c), fasting plasma glucose, non-fasting plasma glucose, or any combination thereof. In some embodiments, the level of HbA1c is about 6.5% to about 24.0%. In some embodiments, the level of HbA1c is greater than or about 6.5%. In some embodiments, the level of HbA1c is greater than or about 8.0%. In some embodiments, the level of HbA1c is greater than or about 10.0%. In some embodiments, the

level of HbA1c is greater than or about 12.0%. In some embodiments, the level of HbA1c is greater than or about 14.0%. In some embodiments, the level of HbA1c is greater than or about 16.0%. In some embodiments, the level of HbA1c is greater than or about 18.0%. In some embodiments, the level of HbA1c is greater than or about 20.0%. In some embodiments, the level of HbA1c is greater than or about 22.0%. In some embodiments, the level of HbA1c is greater than or about 24.0%.

In some embodiments, the level of fasting plasma glucose is greater than or about 120 mg/dL to greater than or about 750 mg/dL. In some embodiments, the level of fasting plasma glucose is greater than or about 200 mg/dL to greater than or about 500 mg/dL. In some embodiments, the level of fasting plasma glucose is greater than or about 300 mg/dL to greater than or about 700 mg/dL.

In some embodiments, the level of non-fasting plasma glucose is greater than or about 190 mg/dL to greater than or about 750 mg/dL. In some embodiments, the level of non-fasting plasma glucose is greater than or about 250 mg/dL to greater than or about 450 mg/dL. In some embodiments, the level of non-fasting plasma glucose is greater than or about 400 mg/dL to greater than or about 700 mg/dL.

In some embodiments, determining if the patient has type 2 diabetes mellitus further includes determining the patient's BMI. In some embodiments, the BMI of the patient is greater than or about 22 kg/m2 to greater than or about 100 kg/m2. In some embodiments, the BMI of the patient is greater than or about 30 kg/m2 to greater than or about 90 kg/m2. In some embodiments, the BMI of the patient is greater than or about 40 kg/m2 to greater than or about 80 kg/m2. In some embodiments, the BMI of the patient is greater than or about 50 kg/m2 to greater than or about 70 kg/m2.

In some embodiments, additional factors (e.g. risk factors) used for determining if the patient has type 2 diabetes mellitus further includes age and ethnicity of the patient. In some embodiments, the patient's age is greater than or about 10 years. In some embodiments, the patient's age is greater than or about 15 years. In some embodiments, the patient's age is greater than or about 20 years. In some embodiments, the patient's age is greater than or about 25 years. In some embodiments, the patient's age is greater than or about 30 years. In some embodiments, the patient's age is greater than or about 35 years. In some embodiments, the patient's age is greater than or about 40 years. In some embodiments, the patient's age is greater than or about 42 years. In some embodiments, the patient's age is greater than or about 44 years. In some embodiments, the patient's age is greater than or about 46 years. In some embodiments, the patient's age is greater than or about 48 years. In some embodiments, the patient's age is greater than or about 50 years. In some embodiments, the patient's age is greater than or about 52 years. In some embodiments, the patient's age is greater than or about 54 years. In some embodiments, the patient's age is greater than or about 56 years. In some embodiments, the patient's age is greater than or about 58 years. In some embodiments, the patient's age is greater than or about 60 years. In some embodiments, the patient's age is greater than or about 62 years. In some embodiments, the patient's age is greater than or about 64 years. In some embodiments, the patient's age is greater than or about 66 years. In some embodiments, the patient's age is greater than or about 68 years. In some embodiments, the patient's age is greater than or about 70 years. In some embodiments, the patient's age is greater than or about 72 years. In some embodiments, the patient's age is greater than or about 74 years. In some embodiments, the patient's age is greater than or about 76 years. In some embodiments, the patient's age is greater than or about 78 years. In some embodiments, the patient's age is greater than or about 80 years. In some embodiments, the patient's age is greater than or about 85 years. In some embodiments, the patient's age is greater than or about 90 years. In some embodiments, the patient's age is greater than or about 95 years. In some embodiments, the ethnicity of the patient may be African American, American Indian or Alaska Native, Asian American, Hispanics or Latinos, or Native Hawaiian or Pacific Islander.

In some embodiments, the patient is a pediatric patient. The term "pediatric patient" as used herein refers to a patient under the age of 21 years at the time of diagnosis or treatment. The term "pediatric" can be further be divided into various subpopulations including: neonates (from birth through the first month of life); infants (1 month up to two years of age); children (two years of age up to 12 years of age); and adolescents (12 years of age through 21 years of age (up to, but not including, the twenty-second birthday)). Berhman RE, Kliegman R, Arvin AM, Nelson WE. Nelson Textbook of Pediatrics, 15th Ed. Philadelphia: W.B. Saunders Company, 1996; Rudolph AM, et al. Rudolph's Pediatrics, 21st Ed. New York: McGraw-Hill, 2002; and Avery MD, First LR. Pediatric Medicine, 2nd Ed. Baltimore: Williams & Wilkins; 1994. In some embodiments, a pediatric patient is from birth through the first 28 days of life, from 29 days of age to less than two years of age, from two years of age to less than 12 years of age, or 12 years of age through 21 years of age (up to, but not including, the twenty-second birthday). In some embodiments, a pediatric patient is from birth through the first 28 days of life, from 29 days of age to less than 1 year of age, from one month of age to less than four months of age, from three months of age to less than seven months of age, from six months of age to less than 1 year of age, from 1 year of age to less than 2 years of age, from 2 years of age to less than 3 years of age, from 2 years of age to less than seven years of age, from 3 years of age to less than 5 years of age, from 5 years of age to less than 10 years of age, from 6 years of age to less than 13 years of age, from 10 years of age to less than 15 years of age, or from 15 years of age to less than 22 years of age. In some embodiments, the patient is an adult patient.

## Examples

General information: All evaporations were carried out in vacuo with a rotary evaporator. Analytical samples were dried in vacuo (1-5 mm Hg) at RT. Thin layer chromatography (TLC) was performed on silica gel plates, spots were visualized by UV light (214 and 254 nm). Purification by column and flash chromatography was carried out using silica gel (100-400 mesh). Solvent systems were reported as mixtures by volume. NMR spectra were recorded on a Bruker 400 or Varian (400 MHz) spectrometer. 1H chemical shifts are reported in $\delta$ values in ppm with the deuterated solvent as the internal standard. Data are reported as follows: chemical shift, multiplicity (s = singlet, d = doublet, t = triplet, q = quartet, br = broad, m = multiplet), coupling constant (Hz), integration. LCMS spectra were obtained on SHIMADZU LC20-MS2020 or Agilent 1260 series 6125B mass spectrometer or Agilent 1200 series, 6110 or 6120 mass spectrometer with electrospray ionization and excepted as otherwise indicated.

This disclosure is further understood by reference to the following examples, which are intended to be purely exemplary of the disclosure. The present disclosure is not limited in scope by the exemplified embodiments, which are intended as illustrations of single aspects of the disclosure only. Any methods that are functionally equivalent are within the scope of the disclosure. Various modifications of the disclosure in addition to those described herein will become apparent to those skilled in the art from the foregoing description and accompanying figures. Such modifications fall within the scope of the appended claims.

### Example 1: 2-[(2,4-difluorophenyl)methoxy]-6-{1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl}pyridine (Compound 1)

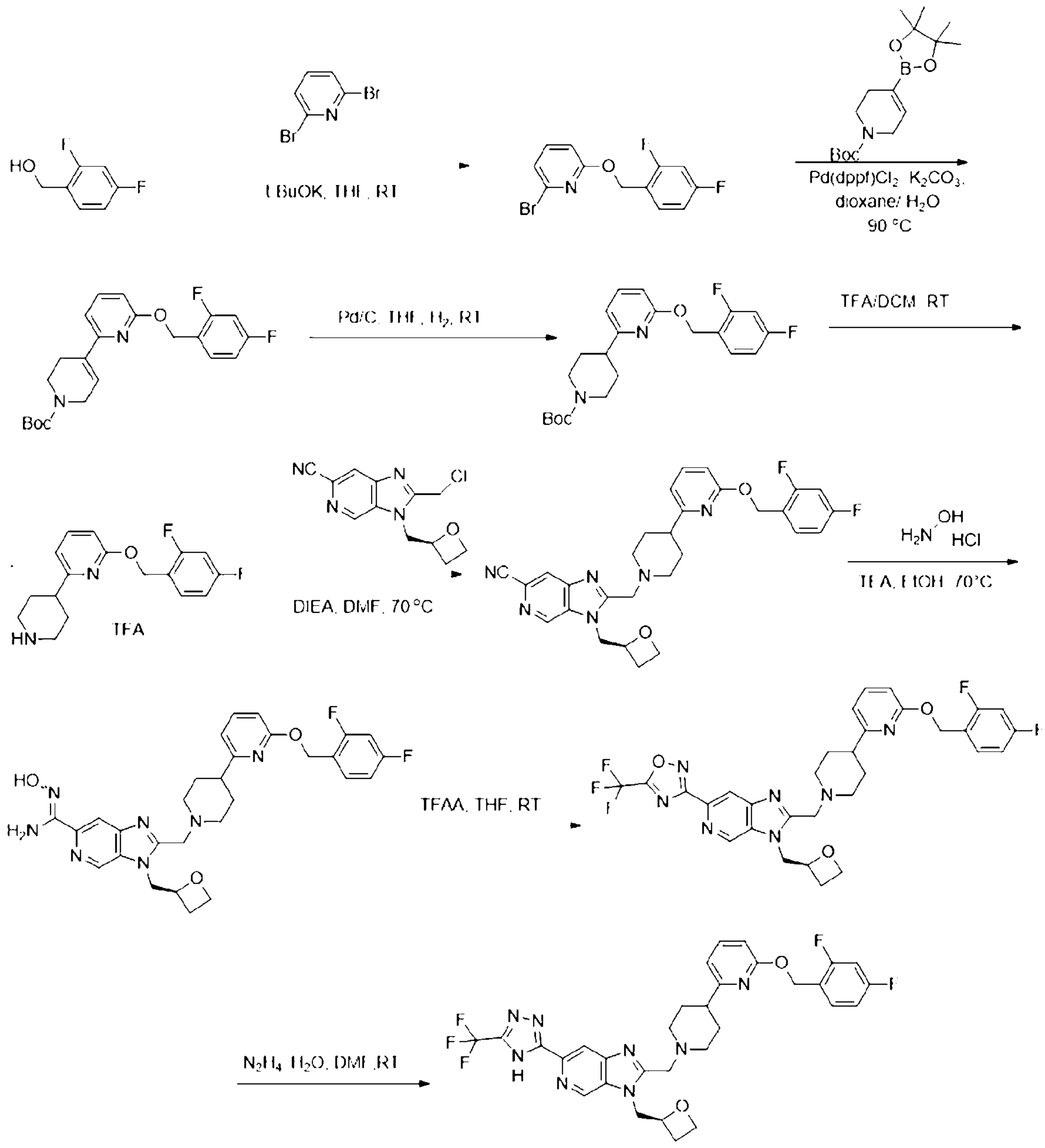


*Step A: 2-bromo-6-((2,4-difluorobenzyl)oxy)pyridine*

To a solution of (2,4-difluorophenyl)methanol (1.0 g, 6.97 mmol) in THF (15 mL) was added 2,6-dibromopyridine (1.64 g, 6.94 mmol) and t-BuOK (1 M in THF, 8.33 mL). The solution was stirred at RT for 2 hours. After the reaction was completed, the mixture was diluted with $H_2O$ (50 mL), extracted with EA (40 mL×2). The combined organic layer was dried over anhydrous sodium sulfate, filtered, and evaporated to dryness. The residue was purified by column chromatography on silica gel (PE/EA=20/1) to give 2-bromo-6-((2,4-difluorobenzyl)oxy)pyridine (2.3 g, yield: 92%). MS Calcd.: 299.0; MS Found: 299.8 $[M+H]^+$.

*Step B: tert-butyl 6-((2,4-difluorobenzyl)oxy)-3',6'-dihydro-[2,4'-bipyridine]-1'(2'H)-carboxylate*

A mixture of 2-bromo-6-((2,4-difluorobenzyl)oxy)pyridine (2.1 g, 7.02 mmol), tert-butyl 4-(4,4,5,5-tetramethyl-1,3,2-dioxaborolan-2-yl)-3,6-dihydropyridine-1(2H)-carboxylate (2.4 g, 7.73 mmol), $Pd(dppf)Cl_2$ (256 mg, 0.35 mmol), $K_2CO_3$ (2.9 g, 21.1 mmol) in dioxane/$H_2O$ (40.0 mL/4.0 mL) was stirred at 90 °C for 3 hours. After the reaction was completed, the mixture was diluted with $H_2O$ (100 mL), extracted with EA (50 mL×2). The combined organic layer was dried over anhydrous sodium sulfate, filtered and evaporated to dryness. The residue was purified by column chromatography on silica gel (PE/EA=20/1) to give tert-butyl 6-((2,4-difluorobenzyl)oxy)-3',6'-dihydro-[2,4'-bipyridine]-1'(2'H)-carboxylate (2.6 g, yield: 93%). MS Calcd.: 402.2; MS Found: 403.1 $[M+H]^+$.

*Step C: tert-butyl 4-(6-((2,4-difluorobenzyl)oxy)pyridin-2-yl)piperidine-1-carboxylate*

A solution of tert-butyl 6-((2,4-difluorobenzyl)oxy)-3',6'-dihydro-[2,4'-bipyridine]-1'(2'H)-carboxylate (200 mg, 0.50 mmol), Pd/C (20 mg, 10% on Carbon, wetted with ca. 55% water) in THF (4.0 mL) was stirred at RT for 1.5 h. After the reaction was completed, the mixture was filtered and the solid dried to give tert-butyl 4-(6-((2,4-difluorobenzyl)oxy) pyridin-2-yl)piperidine-1-carboxylate (210 mg, crude). MS Calcd.: 404.2; MS Found: 405.2 $[M+H]^+$.

*Step D: 2-((2,4-difluorobenzyl)oxy)-6-(piperidin-4-yl)pyridine TFA Salt*

To a solution of tert-butyl 4-(6-((2,4-difluorobenzyl)oxy)pyridin-2-yl)piperidine-1-carboxylate (210 mg, 0.52 mmol) in DCM (4 mL) was added TFA (0.8 mL). The solution was stirred at room temperature for 1 hour. The reaction was removed in vacuo to give 2-((2,4-difluorobenzyl)oxy)-6-(piperidin-4-yl)pyridine TFA Salt (250 mg, crude). MS Calcd.: 304.1; MS Found: 305.1 $[M+H]^+$.

*Step E: (S)-2-((4-(6-((2,4-difluorobenzyl)oxy)pyridin-2-yl)piperidin- 1-yl)methyl)-3-(oxetan-2-ylmethyl)-3H-imidazo[4,5-c]pyridine-6-carbonitrile*

A mixture of 2-((2,4-difluorobenzyl)oxy)-6-(piperidin-4-yl)pyridine TFA Salt (250 mg, crude), (S)-2-(chloromethyl)-3-(oxetan-2-ylmethyl)-3H-imidazo[4,5-c]pyridine-6-carbonitrile (100 mg, 0.38 mmol), DIEA (492 mg, 3.82 mmol) in DMF (4.0 mL) was stirred at 70°C for 1 hour. After the reaction was completed, the mixture was diluted with $H_2O$ (20 mL), extracted with EA (15 mL×2). The combined organic layer was dried over anhydrous sodium sulfate, filtered and evaporated to dryness. The residue was purified by column chromatography on silica gel (DCM/MeOH=100/1) to give (S)-2-((4-(6-((2,4-difluorobenzyl)oxy)pyridin-2-yl)piperidin-1-yl)methyl)-3-(oxetan-2-ylmethyl)-3H-imidazo[4,5-c]pyridine-6-carbonitrile (130 mg, yield: 64%). MS Calcd.: 530.2; MS Found: 531.0 $[M+H]^+$.

*Step F: (S)-2-((4-(6-((2,4-difluorobenzyl)oxy)pyridin-2-yl)piperidin-1-yl)methyl)-N'-hydroxy-3-(oxetan-2-ylmethyl)-3H-imidazo[4,5-c]pyridine-6-carboximidamide*

A solution of (S)-2-((4-(6-((2,4-difluorobenzyl)oxy)pyridin-2-yl)piperidin-1-yl)methyl)-3-(oxetan-2-ylmethyl)-3H-imidazo[4,5-c]pyridine-6-carbonitrile (130 mg, 0.25 mmol), $HONH_2 \cdot HCl$ (100 mg, 1.47 mmol), TEA (99 mg, 0.98 mmol) in EtOH (4.0 mL) was stirred at 70°C for 1 h. After the reaction was completed, the mixture was filtered and the solid was dried to give (S)-2-((4-(6-((2,4-difluorobenzyl)oxy)pyridin-2-yl)piperidin-1-yl)methyl)-N'-hydroxy-3-(oxetan-2-ylmethyl)-3H-imidazo[4,5-c]pyridine-6-carboximidamide (115 mg, yield: 83%). MS Calcd.: 563.2; MS Found: 564.2 $[M+H]^+$.

*Step G: (S)-2-((4-(6-((2,4-difluorobenzyl)oxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine*

To a solution of (S)-2-((4-(6-((2,4-difluorobenzyl)oxy)pyridin-2-yl)piperidin-1-yl)methyl)-N'-hydroxy-3-(oxetan-2-ylmethyl)-3H-imidazo[4,5-c]pyridine-6-carboximidamide (115 mg, 0.20 mmol) in THF (4.0 mL) was added TFAA (86 mg, 0.41 mmol) and the mixture stirred at RT for 1 hour. After the reaction was completed, the reaction was quenched with sat. sodium bicarbonate aqueous solution (5.0 mL), extracted with ethyl acetate (15 mL×3). The organic layers were combined, dried over $Na_2SO_4$, filtered and concentrated under vacuum to dryness to give (S)-2-((4-(6-((2,4-difluorobenzyl)oxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (140 mg, crude). MS Calcd.: 641.2; MS Found: 642.2 $[M+H]^+$.

*Step H: 2-[(2,4-difluorophenyl)methoxy]-6-{1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl}pyridine*

A mixture of (S)-2-((4-(6-((2,4-difluorobenzyl)oxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (140 mg, 0.22 mmol) and $N_2H_4.H_2O$ (22 mg, 0.44 mmol) in DMF (2.0 mL) was stirred at RT for 1 hour. After the reaction was completed, the mixture was purified directly by Prep-HPLC (0.1% FA/$H_2O$/$CH_3CN$) to give 2-[(2,4-difluorophenyl)methoxy]-6-{1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl}pyridine (54 mg, yield: 39%). MS Calcd.: 640.2; MS Found: 641.0 $[M+H]^+$.

$^1H$ NMR (400 MHz, DMSO-d6): δ 9.17 (s, 1 H), 8.29 (s, 1 H), 7.55-7.67 (m, 2 H), 7.22-7.33 (m, 1 H), 7.04-7.15 (m, 1 H), 6.88 (d, *J*=7.6 Hz, 1 H), 6.66 (d, *J*=8.4 Hz, 1 H), 5.37 (s, 2 H), 5.14-5.24 (m, 1 H), 4.88-4.99 (m, 1 H), 4.73-4.83 (m, 1 H),

4.39-4.55 (m, 2 H), 4.01 (d, *J*=14.0 Hz, 1 H), 3.86 (d, *J*=13.6 Hz, 1 H), 2.99-3.08 (m, 1 H), 2.83-2.92 (m, 1 H), 2.56-2.80 (m, 2 H), 2.40-2.53 (m, 1 H), 2.17-2.35 (m, 2 H), 1.64-1.88 (m, 4 H). $^{19}$F NMR(377 MHz, DMSO-d6): δ -63.68, -63.72, -110.21,-110.23, - 113.85, -113.88.

**Example 2: (S)-2-((4-(6-((4-chloro-2-methoxybenzyl)oxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (Compound 2)**

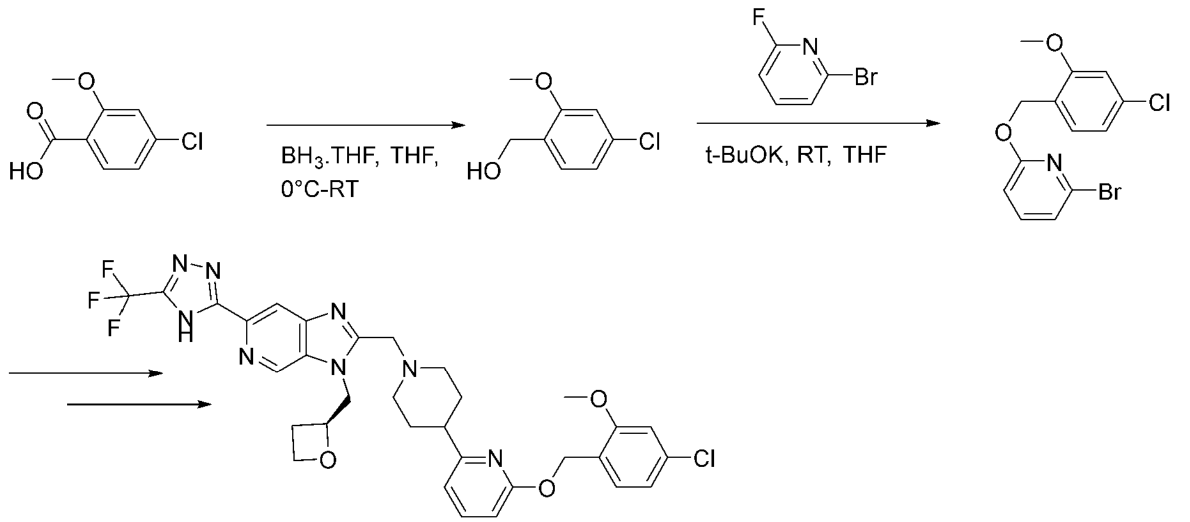


*Step A: (4-chloro-2-methoxyphenyl)methanol*

To a solution of 4-chloro-2-methoxybenzoic acid (2.0 g, 10.7 mmol) in THF (10.0 mL) was added dropwise BH$_3$.THF (22 mL, 22 mmol, 1 M in toluene) at 0°C. The solution was stirred at room temperature for 2 hours. After the reaction was completed, the reaction mixture was quenched by the MeOH (8 mL). The mixture was added with brine (30 ml x 2), extracted with ethyl acetate (30 ml x 3). The organic layers were dried over sodium sulfate, filtered and concentrated in vacuum. The residue was purified by column chromatography on silica gel (PE/EA=2/1) to give (4-chloro-2-methoxyphenyl)methanol (1.8 g, 97.3% yield).

*Step B: 2-bromo-6-((4-chloro-2-methoxybenzyl)oxy)pyridine*

To a solution of (4-chloro-2-methoxyphenyl)methanol (500 mg, 2.9 mmol) in THF (5.0 mL) was added 2-bromo-6-fluoropyridine (511 mg, 2.9 mmol) and t-BuOK(389 mg, 3.5 mmol) at 0°C. The resulting mixture was stirred at RT for 1h. After the reaction was completed, the reaction mixture was quenched by the addition of the saturated aqueous $NH_4Cl$ and extracted with ethyl acetate (30 ml x 3). The organic layers were combined and washed with brine (30 ml x 2), dried over sodium sulfate, filtered, and concentrated in vacuum to give 2-bromo-6-((4-chloro-2-methoxybenzyl)oxy)pyridine (1.0 g, crude). MS Calcd.: 327.0; MS Found: 328.0 [M+H]$^+$.

*Step C: (S)-2-((4-(6-((4-chloro-2-methoxybenzyl)oxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine*

(S)-2-((4-(6-((4-chloro-2-methoxybenzyl)oxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (12.9 mg) was obtained with the similar method of Example 1. MS Calcd.: 668.2; MS Found: 669.0 [M+H]$^+$.

$^1$H NMR (400 MHz, DMSO-d6): δ 9.15 (s, 1 H), 8.28 (s, 1 H), 7.62 (t, *J*=8.0 Hz, 1 H), 7.40 (d, *J*=8.0 Hz, 1 H), 7.12 (m, 1 H), 7.00 (dd, *J*=8.4 Hz, 2.0 Hz, 1 H), 6.86 (d, *J*=7.2 Hz, 1 H), 6.66 (d, *J*=8.0 Hz, 1 H), 5.29 (s, 2 H), 5.18 (m, 1 H), 4.87-4.96 (m, 1 H), 4.71-4.80 (m, 1 H), 4.38-4.53 (m, 2 H), 4.01 (d, *J*=13.6 Hz, 1 H), 3.81-3.89 (m, 4 H), 2.99-3.07 (m, 1 H), 2.84-2.91 (m, 1 H), 2.73-2.77 (m, 1 H), 2.56-2.68 (m, 1 H), 2.40-2.48 (m, 1 H), 2.16-2.40 (m, 2 H), 1.63-1.87 (m, 4 H). $^{19}$F NMR (377 MHz, DMSO-d6): δ -63.54.

**Example 3: (S)-2-((4-(6-((2,4-difluoro-3-methoxybenzyl)oxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (Compound 3)**

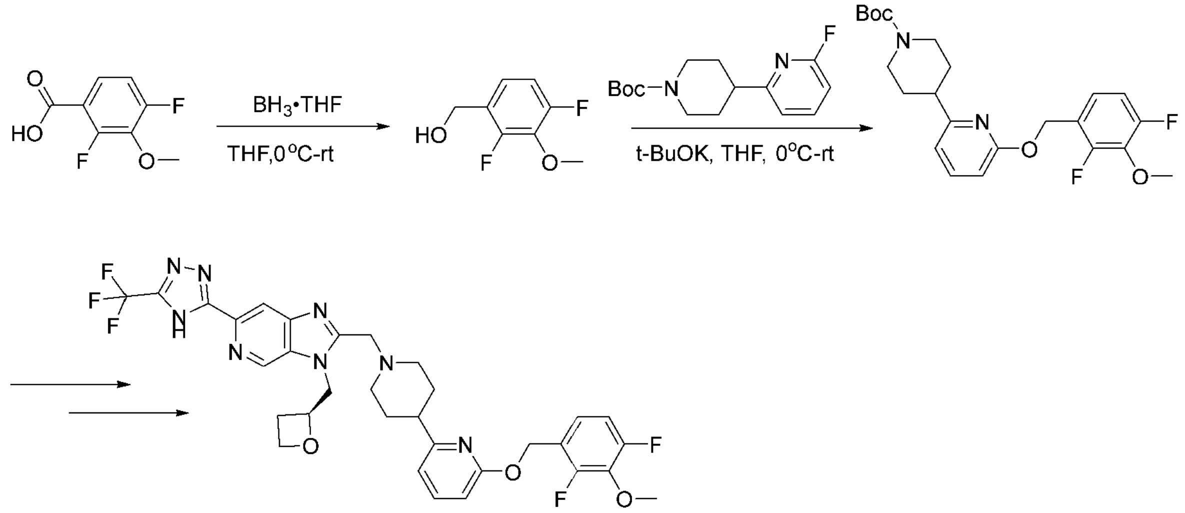


*Step A: (2,4-difluoro-3-methoxyphenyl)methanol*

To a solution of 2,4-difluoro-3-methoxybenzoic acid (1.2 g, 6.4 mmol) in THF (13 ml) was added dropwise $BH_3$.THF (19.1 ml, 19.1 mmol) at 0 °C. The mixture was stirred at room temperature for 2 hours. The mixture was quenched with $H_2O$ (50 mL), extracted with EA (3 x 20 mL). The combined organic layers were washed with brine (30 ml), dried over $Na_2SO_4$, filtered and concentrated under reduced pressure. The residue was purified by silica gel column chromatography, eluted with PE/EA (3/1) to give (2,4-difluoro-3-methoxyphenyl)methanol (920 mg, yield: 83%).

*Step B: tert-butyl 4-(6-((2,4-difluoro-3-methoxybenzyl)oxy)pyridin-2-yl)piperidine-1-carboxylate*

To a solution of (2,4-difluoro-3-methoxyphenyl)methanol (920 mg, 5.3 mmol) and t-BuOK (5.3 ml, 5.3 mmol) in THF (16 ml) was added tert-butyl 4-(6-fluoropyridin-2-yl)piperidine-1-carboxylate (888 mg, 3.2 mmol) at 0°C. The solution was stirred at room temperature for an hour. The mixture was quenched with $H_2O$ (50 mL), extracted with EA (3 x 20 mL). The combined organic layers were washed with brine (30 ml). The organic layer was dried over $Na_2SO_4$, filtered and concentrated under reduced pressure. The residue was purified by silica gel column chromatography, eluted with PE/EA (3/1) to give tert-butyl 4-(6-((2,4-difluoro-3-methoxybenzyl)oxy)pyridin-2-yl)piperidine-1-carboxylate (1.01 g, yield: 44%).

*Step C: (S)-2-((4-(6-((2,4-difluoro-3-methoxybenzyl)oxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine*

(S)-2-((4-(6-((2,4-difluoro-3-methoxybenzyl)oxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (66.6 mg) was obtained with the similar method of Example 1. MS Calcd.: 670.0; MS Found: 671.0 [M+H]+.

1 H NMR: δ 9.16 (s, 1 H), 8.29 (s, 1 H), 7.63 (t, *J*=8.0 Hz, 1 H), 7.22-7.30 (m, 1 H), 7.09-7.18 (m, 1 H), 6.88 (d, *J*=7.2 Hz, 1 H), 6.67 (d, *J*=8.0 Hz, 1 H), 5.36 (s, 2 H), 5.14-5.24 (m, 1 H), 4.89-4.99 (m, 1 H), 4.72-4.81 (m, 1 H), 4.38-4.55 (m, 2 H), 4.02 (d, *J*=14.0 Hz, 1 H), 3.90 (s, 3 H), 3.87 (d, *J*=13.6 Hz, 1 H), 2.98-3.07 (m, 1 H), 2.84-2.93 (m, 1 H), 2.69-2.80 (m, 1 H), 2.57-2.67 (m, 1 H), 2.39-2.50 (m, 1 H), 2.17-2.34 (m, 2 H), 1.64-1.87 (m, 4 H). $^{19}$F NMR(377 MHz, DMSO-d6): δ -63.68, -128.73, -132.96.

**Example 4: 2-[(4-chloro-2-fluoro-3-methylphenyl)methoxy]-6-{1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl}pyridine (Compound 4)**

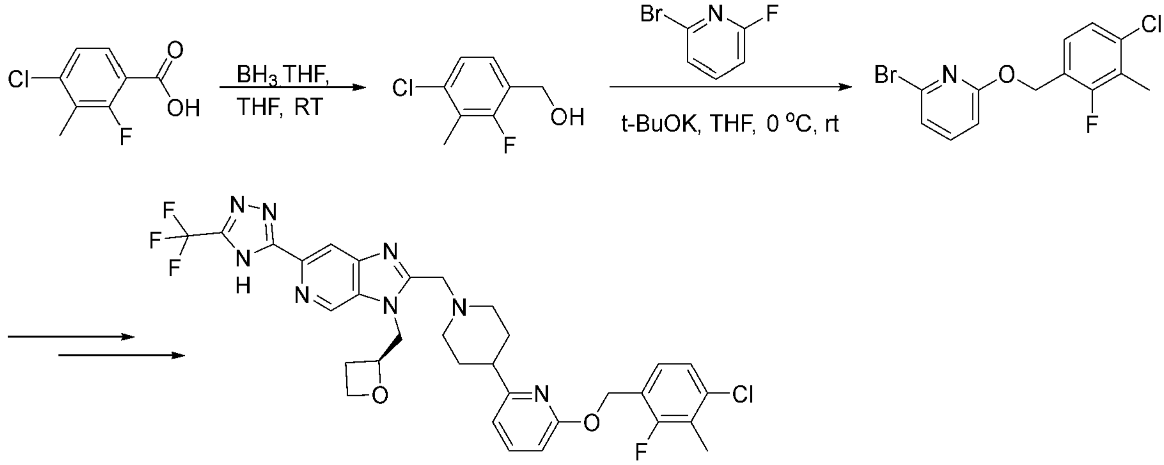


*Step A: (4-chloro-2-fluoro-3-methylphenyl)methanol*

To a solution of 4-chloro-2-fluoro-3-methylbenzoic acid (400 mg, 2.1 mmol) in THF (4 mL) was added Borane-tetrahydrofuran complex (1 M in THF, 6.5 mL, 6.5 mmol) at 0°C. The mixture was stirred at RT. for 2 hours, quenched with $NH_4Cl$ aq. (30 mL), extracted with ethyl acetate (40 mL*2). The organic layer was dried over anhydrous sodium sulfate, filtered and evaporated to dryness to give (4-chloro-2-fluoro-3-methylphenyl)methanol (330 mg, crude).

*Step B: 2-bromo-6-((4-chloro-2-fluoro-3-methylbenzyl)oxy)pyridine*

To a solution of (4-chloro-2-fluoro-3-methylphenyl)methanol (280 mg) in THF (2.0 mL) was added 2-bromo-6-fluoropyridine (312 mg, 1.77 mmol) and t-BuOK (217 mg, 1.93 mmol) at 0°C. The mixture was stirred at RT. for 2 h, quenched with $NH_4Cl$ aq. (30 mL), extracted with ethyl acetate (40 mL*2). The organic layer was dried over anhydrous sodium sulfate, filtered and evaporated to dryness. The residue was purified by column chromatography on silica gel (PE/EA=1/0) to give 2-bromo-6-((4-chloro-2-fluoro-3-methylbenzyl)oxy)pyridine (330 mg, yield: 62.5%). MS Calcd.: 329.0; MS Found: 330.0 [M+H]+.

*Step C: 2-[(4-chloro-2-fluoro-3-methylphenyl)methoxy]-6-{1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl}pyridine*

2-[(4-chloro-2-fluoro-3-methylphenyl)methoxy]-6-{1-[(3-{[(2S)-oxetan-2-yl]methyl}-6-[5-(trifiluoromethyl)-4H-1,2,4-triazol-3-yl]-3H-imidazo[4,5-c]pyridin-2-yl)methyl]piperidin-4-yl}pyridine (26.2 mg) was obtained with the similar method of Example 1. MS Calcd.: 670.2; MS Found: 671.0 [M+H]+.

$^1$H NMR (400 MHz, DMSO-d6): δ 9.14 (s, 1 H), 8.27 (s, 1 H), 7.63 (t, *J*=8.0 Hz, 1 H), 7.39 (t, *J*=8.4 Hz, 1 H), 7.29 (d, *J*=8.4 Hz, 1 H), 6.87 (d, *J*=7.2 Hz, 1 H), 6.67 (d, *J*=8.0 Hz, 1 H), 5.38 (s. 2 H), 5.12-5.24 (m. 1 H), 4.87-4.97 (m. 1 H), 4.71-4.82 (m, 1 H), 4.47-4.55 (m, 1 H), 4.38-4.46 (m, 1 H), 4.00 (d, *J*=13.6 Hz, 1 H), 3.86 (d, *J*=13.6 Hz, 1 H), 2.98-3.08 (m, 1 H), 2.84-2.93 (m, 1 H), 2.68-2.78 (m, 1 H), 2.55-2.68 (m, 1 H), 2.39-2.51 (m, 1 H), 2.15-2.33 (m, 5 H), 1.62-1.87 (m, 4 H). $^{19}$F NMR (377 MHz, DMSO-d6): δ -63.26, -116.98.

**Example 5: 2-[(2,4-difluoro-3-methylphenyl)methoxy]-6-{1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl}pyridine (Compound 5)**

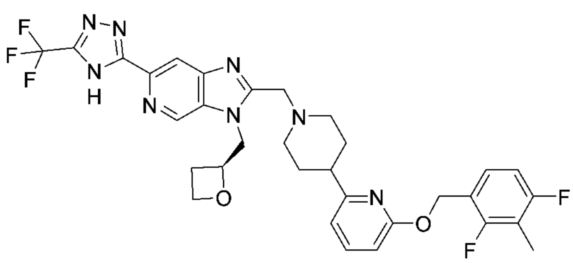

Starting from 2,4-difluoro-3-methylbenzoic acid, 2-[(2,4-difluoro-3-methylphenyl)methoxy]-6-{1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl}pyridine was obtained with the similar method as described herein.

MS Calcd.: 654.2; MS Found: 655.2 [M+H]+.

$^1$H NMR (400 MHz, DMSO-d6): δ 9.15 (s, 1 H), 8.28 (s, 1 H), 7.62 (t, *J*=7.6 Hz 1 H), 7.38-7.46 (m, 1 H), 7.05 (t, *J*=8.4 Hz, 1 H), 6.87 (d, *J*=7.2 Hz, 1 H), 6.65 (d, *J* = 8.0 Hz, 1 H), 5.35 (s, 2 H), 5.14-5.23 (m, 1 H), 4.88-4.97 (m, 1 H), 4.73-4.81 (m, 1 H), 4.39-4.53 (m, 2 H), 4.01 (d, *J*=13.6 Hz, 1 H), 3.86 (d, *J*=13.2 Hz, 1 H), 2.99-3.08 (m, 1 H), 2.85-2.92 (m, 1H), 2.70-2.80 (m, 1 H), 2.58-2.70 (m, 1 H), 2.18-2.35 (m, 3 H), 2.15 (s, 3 H), 1.69-1.88 (m, 4 H). $^{19}$F NMR (377 MHz, DMSO-d6): δ -63.49, - 114.79,-114.81, -118.52,-118.54.

**Example 6: (S)-2-((4-(6-((4-chloro-2,6-difluorobenzyl)oxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (Compound 6)**

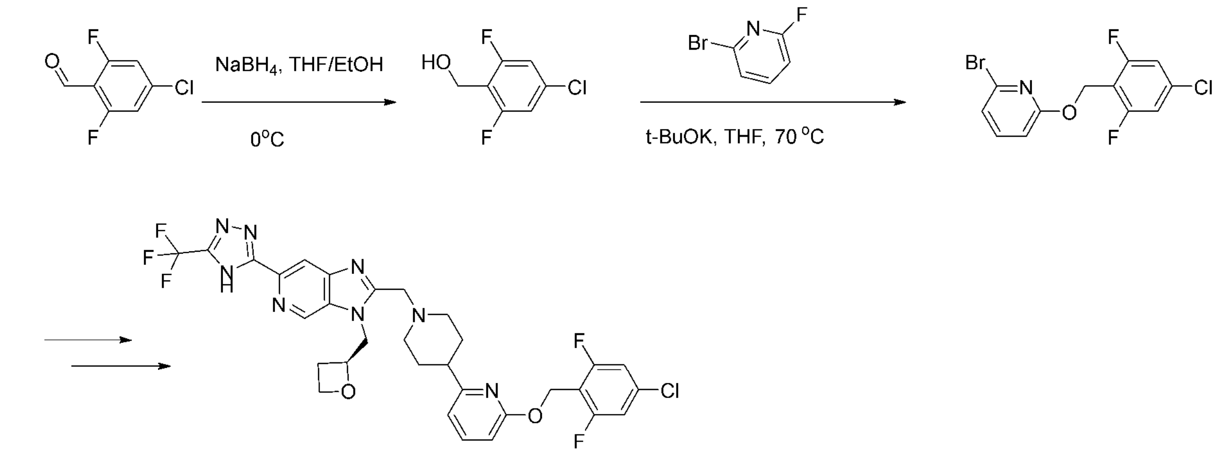


*Step A: (4-chloro-2,6-difluorophenyl)methanol*

A mixture of 4-chloro-2,6-difluorobenzaldehyde (1.0 g, 5.66 mmol) in THF/EtOH (15 mL/2 mL) was added $NaBH_4$ (215 mg, 5.68 mmol) at 0°C. The reaction was stirred at 0°C for 20 min. After the reaction was completed, the mixture was quenched with water (20 mL), extracted with ethyl acetate (20 mL×3). The organic layers were combined, dried over $Na_2SO_4$, filtered, and concentrated under vacuum to dryness. The residue was purified by silica gel (PE/EA=10/1) to give (4-chloro-2,6-difluorophenyl)methanol (849 mg, yield: 84.2 %).

*Step B: 2-bromo-6-((4-chloro-2,6-dijluorobenzyl)oxy)pyridine*

A mixture of (4-chloro-2,6-difluorophenyl)methanol (400 mg, 2.2 mmol), 2-bromo-6-fluoropyridine (435 mg, 2.5 mmol), t-BuOK (504 mg, 4.5 mmol) in THF (8 mL) was stirred at 70°C for 1day. After the reaction was completed, the mixture was quenched with water (10 mL), extracted with ethyl acetate (10 mL×3). The organic layers were combined, dried over $Na_2SO_4$, filtered, and concentrated under vacuum to dryness. The residue was purified by column chromatography on silica gel (PE =100%) to give 2-bromo-6-((4-chloro-2,6-difluorobenzyl)oxy)pyridine (272 mg, yield: 36.4 %). MS Calcd.: 332.9; MS Found: 333.9 $[M+1]^+$.

*Step C: (S)-2-((4-(6-((4-chloro-2,6-difluorobenzyl)oxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine*

(S)-2-((4-(6-((4-chloro-2,6-difluorobenzyl)oxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (64.6 mg) was obtained with the similar method of Example 1. MS Calcd.: 674.2; MS Found: 675.2 [M+H]+.

$^1$H NMR (400 MHz, DMSO-d6): δ 9.16 (s, 1 H), 8.28 (d, *J*=0.8 Hz, 1 H), 7.63 (t, *J*=8.0 Hz, 1 H), 7.37-7.45 (m, 2 H), 6.89 (d, *J*=7.2 Hz, 1 H), 6.64 (d, *J*=8.0 Hz, 1 H), 5.37 (s, 2 H), 5.14-5.25 (m, 1 H), 4.89-4.99 (m, 1 H), 4.73-4.84 (m, 1 H), 4.39-4.55 (m, 2 H), 4.02 (d, *J*=14.0 Hz, 1 H), 3.87 (d, *J*=13.6 Hz, 1 H), 2.99-3.08 (m , 1 H), 2.84-2.93 (m, 1 H), 2.69-2.80 (m, 1 H), 2.57-2.68 (m, 1 H), 2.43-2.48 (m, 1 H), 2.17-2.35 (m, 2 H); 1.67-1.88 (m, 4 H). $^{19}$F NMR (377 MHz, DMSO-d6): δ -63.54,

-112.14.

**Example 7: 2-{[6-(difluoromethoxy)pyridin-3-yl]methoxy}-6-{1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl}pyridine (Compound 7)**

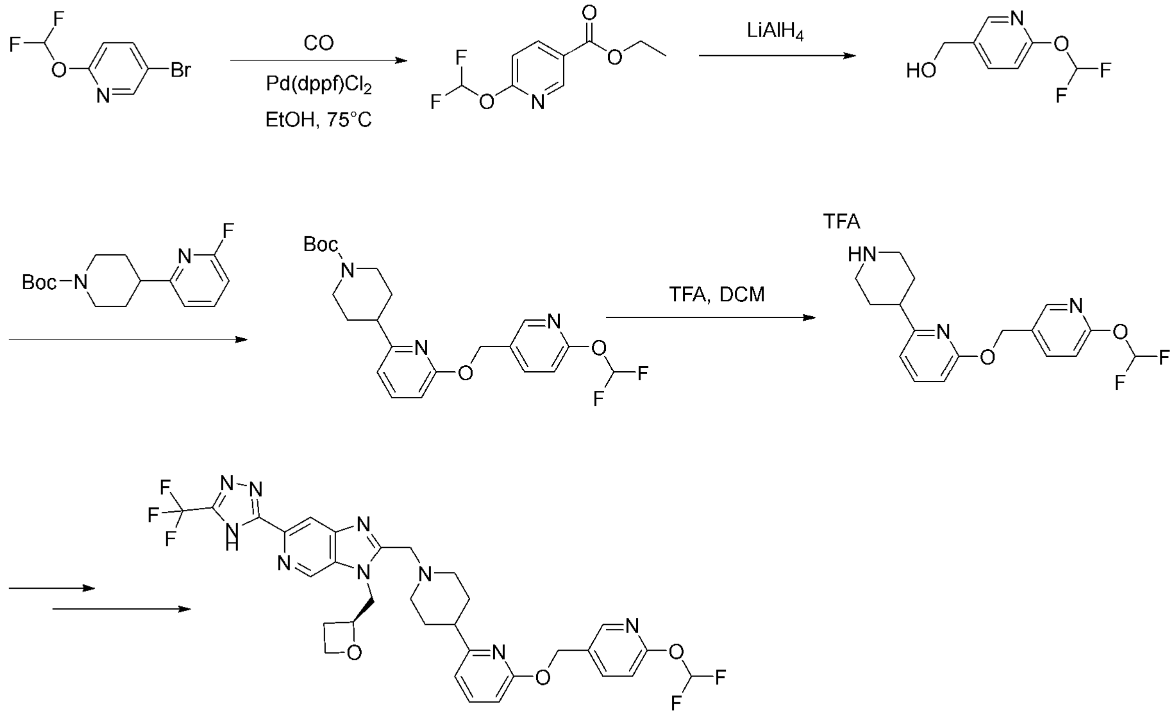


*Step A: ethyl 6-(difluoromethoxy)nicotinate*

A mixture of 5-bromo-2-(difluoromethoxy)pyridine (900 mg, 4.0 mmol) and Pd(dppf)$Cl_2$ (294 mg, 0.4 mmol) and KOAc (788 mg, 8.0 mmol) in EtOH (10 mL) was stirred at 75°C for 2 hours under CO (1 atm). After the reaction was completed, the reaction was filtered and concentrated in vacuum. The residue was purified by column chromatography (PE:EA=4:1) to give ethyl 6-(difluoromethoxy)nicotinate (800 mg, 91% yield). MS Calcd.: 217.1; MS Found: 218.1 [M+H]+.

*Step B: (6-(difluoromethoxy)pyridin-3-yl)methanol*

To a solution of ethyl 6-(difluoromethoxy)nicotinate (650 mg, 3.0 mmol) in THF (10 mL) was added $LiAlH_4$ (285 mg, 7.5 mmol). The reaction was stirred at room temperature for 1 hour. After the reaction was completed, the reaction was quenched with EA (10 mL) and NaOH solution (2M, 5 mL). The mixture was dried and filtered. The reside was concentrated in vacuum and purified by column chromatography (PE:EA=1:1) to give (6-(difluoromethoxy)pyridin-3-yl)methanol (400 mg, 76% yield). MS Calcd.: 175.0; MS Found: 176.1 [M+H]+.

*Step C: tert-butyl 4-(6-((6-(difluoromethoxy)pyridin-3-yl)methoxy)pyridin-2-yl)piperidine-1-carboxylate*

A mixture of (6-(difluoromethoxy)pyridin-3-yl)methanol (400 mg, 2.3 mmol) and tert-butyl 4-(6-fluoropyridin-2-yl) piperidine-1-carboxylate (424 mg, 1.5 mmol) and NaOH (121 mg, 3.0 mmol) in DMF (5 mL) was stirred at 70°C for 16 hours. After the reaction was completed, the reaction was filtered and concentrated in vacuum. The residue was purified by column chromatography (PE:EA=3:1) to give tert-butyl 4-(6-((6-(difluoromethoxy)pyridin-3-yl)methoxy)pyridin-2-yl)piperidine-1-carboxylate (400 mg, 61% yield). MS Calcd.: 435.2; MS Found: 458.2 $[M+Na]^+$.

*Step D: 2-(difluoromethoxy)-5-(((6-(piperidin-4-yl)pyridin-2-yl)oxy)methyl)pyridine TFA salt*

To a solution of tert-butyl 4-(6-((6-(difluoromethoxy)pyridin-3-yl)methoxy)pyridin-2-yl)piperidine-1-carboxylate (200 mg, 0.46 mmol) in DCM (3 mL) was added TFA (1 mL) at room temperature. The reaction was stirred at room

temperature for 1 hour. After the reaction was completed, the reaction was concentrated in vacuum to give 2-(difluoromethoxy)-5-(((6-(piperidin-4-yl)pyridin-2-yl)oxy)methyl)pyridine TFA salt (210 mg, crude). MS Calcd.: 335.1; MS Found:336.1 [M+H]+.

*Step E: 2-{[6-(difluoromethoxy)pyridin-3-yl]methoxy}-6-(1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl}pyridine (Compound 7)*

2-{[6-(difluoromethoxy)pyridin-3-yl]methoxy}-6-{1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl}pyridine (29.9 mg) was obtained with the similar method of Example 1.

MS Calcd.: 671.2; MS Found: 672.0 [M+H]+.

$^1$H NMR (400 MHz, DMSO-$d_6$): 1H NMR: δ 9.16 (d, *J*=0.8 Hz, 1 H), 8.38 (d, *J*=2.0 Hz, 1 H), 8.29 (d, *J*=0.8 Hz, 1 H), 8.02 (dd, *J*=8.4 Hz, *J*=2.4 Hz, 1 H), 7.51-7.87 (m, 2 H), 7.10 (d, *J*=8.4 Hz, 1 H), 6.88 (d, *J*=7.2 Hz, 1 H), 6.67 (d, *J*=8.0 Hz, 1 H), 5.37 (s, 2 H), 5.14-5.23 (m, 1 H), 4.89-4.98 (m, 1 H), 4.73-4.82 (m, 1 H), 4.38-4.54 (m, 2 H), 4.02 (d, *J*=14.0 Hz, 1 H), 3.87 (d, *J*=13.6 Hz, 1 H), 3.00-3.07 (m, 1 H), 2.85-2.93 (m, 1 H), 2.68-2.80 (m, 1 H), 2.58-2.64 (m, 1 H), 2.43-2.53 (m, 1 H), 2.17-2.34 (m, 2 H), 1.67-1.88 (m, 4 H). $^{19}$F NMR (377 MHz, DMSO-$d_6$): δ -63.66, -87.30.

**Example 8: (S)-2-((4-(6-((2-(difluoromethoxy)pyridin-4-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (Compound 8)**

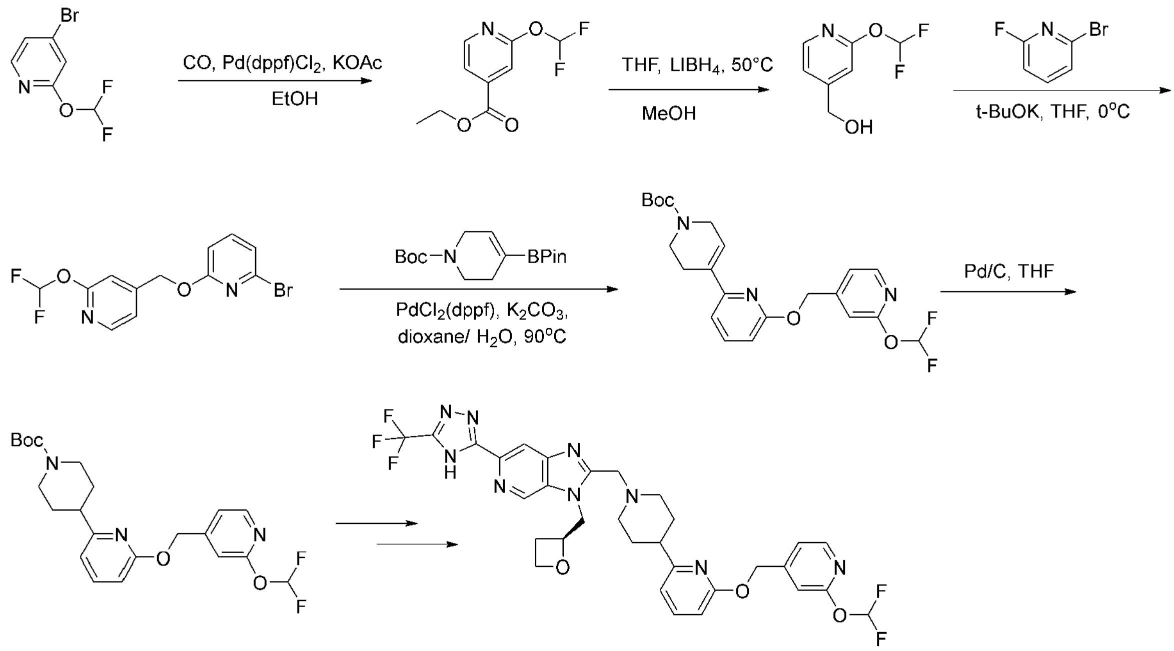


*Step A: Ethyl 2-(difluoromethoxy)isonicotinate*

To a mixture of 4-bromo-2-(difluoromethoxy)pyridine (1.0 g, 4.46 mmol) in EtOH (16.0 mL) was added $PdCl_2$ (dppf) (326 mg, 0.44 mmol) and KOAc (875 mg, 8.93 mmol). The mixture was stirred at 75°C for 2 hours under CO. After the reaction was completed, the reaction was quenched with $H_2O$ (50 mL) and extracted with ethyl acetate (60 mL x 3). The organic layer was combined and washed with brine (40 mL x 2), dried over sodium sulfate, filtered and concentrated in vacuum. The reaction was purified by column chromatography (PE/EA=10/1) to give ethyl 2-(difluoromethoxy)isonicotinate (740 mg, yield:76%). MS Calcd.: 217.1; MS Found: 218.1 [M+H]+.

*Step B: (2-(Difluoromethoxy)pyridin-4-yl)methanol*

To a solution of ethyl 2-(difluoromethoxy)isonicotinate (740 mg, 3.41 mmol) in THF (8 mL) was added $LiBH_4$ (225 mg, 10.23 mmol). The solution was stirred at 40°C overnight. The mixture was quenched with MeOH (8 mL) and $H_2O$ (30ml), extracted with EA (50 mL×2). The organic layers were combined, dried over $Na_2SO_4$, filtered and concentrated

under vacuum. The reaction was purified by column chromatography (DCM/MeOH=97/3) to give to (2-(difluoromethoxy) pyridin-4-yl)methanol (370 mg, yield: 66%). MS Calcd.: 175.0; MS Found: 176.1 $[M+H]^+$.

*Step C: 4-(((6-Bromopyridin-2-yl)oxy)methyl)-2-(difluoromethoxy)pyridine*

To a solution of 2-bromo-6-fluoropyridine (143 mg, 0.82 mmol) in THF (4 mL) was added (2-(difluoromethoxy) pyridin-4-yl)methanol (130 mg, 0.74 mmol) and t-BuOK (1 M in THF, 0.66 mL). The solution was stirred at 0°C for 2 hours. After the reaction was completed, the reaction was quenched with $H_2O$ (20 mL) and extracted with ethyl acetate (30 mL x 3). The organic layer was combined and washed with brine (25 mL x 2), dried over sodium sulfate, filtered and concentrated in vacuum. The reaction was purified by column chromatography (PE/EA=10/1) to give 4-(((6-bromopyridin-2-yl)oxy) methyl)-2-(difluoromethoxy)pyridine (180 mg, yield: 73%). MS Calcd.: 330.0; MS Found: 331.0 $[M+H]^+$.

*Step D: tert-Butyl 6-((2-(difluoromethoxy)pyridin-4-yl)methoxy)-3',6'-dihydro-[2,4'-bipyridine]-1'(2'H)-carboxylate*

A mixture of 4-(((6-bromopyridin-2-yl)oxy)methyl)-2-(difluoromethoxy)pyridine (180 mg, 0.54 mmol), tert-butyl 4-(4,4,5,5-tetramethyl-1,3,2-dioxaborolan-2-yl)-3,6-dihydropyridine-1(2H)-carboxylate (360 mg, 1.16 mmol), Pd(dppf) $Cl_2$ (20 mg, 0.027 mmol), $K_2CO_3$ (226 mg, 1.64 mmol) in dioxane/$H_2O$ (10.0 mL/1.0 mL) was stirred at 90 °C for 3 hours. After the reaction was completed, the reaction was quenched with $H_2O$ (20 mL) and extracted with ethyl acetate (30 mL x 3). The organic layer was combined and washed with brine (25 mL x 2), dried over sodium sulfate, filtered and concentrated in vacuum. The reaction was purified by column chromatography (PE/EA=15/1) to give tert-butyl 6-((2-(difluoromethoxy) pyridin-4-yl)methoxy)-3',6'-dihydro-[2,4'-bipyridine]-1'(2'H)-carboxylate (190 mg, yield:72%). MS Calcd.: 433.2; MS Found: 378.3 $[M-56+H]^+$.

*Step E: tert-Butyl 4-(6-((2-(difluoromethoxy)pyridin-4-yl)methoxy)pyridin-2-yl)piperidine-1-carboxylate*

A solution of tert-butyl 6-((2-(difluoromethoxy)pyridin-4-yl)methoxy)-3',6'-dihydro-[2,4'-bipyridine]-1'(2'H)-carboxylate (190 mg, 0.44 mmol), Pd/C (95 mg, 10% on Carbon, wetted with ca. 55% water) in THF (5.0 mL) was stirred at RT for 30 min. After the reaction was completed, the mixture was filtered and the solid dried to give tert-butyl 4-(6-((2-(difluoromethoxy)pyridin-4-yl)methoxy)pyridin-2-yl)piperidine-1-carboxylate (80 mg, yield: 42%). MS Calcd.: 435.2; MS Found: 458.2 $[M+Na]^+$.

*Step F: (S)-2-((4-(6-((2-(difluoromethoxy)pyridin-4-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine*

(S)-2-((4-(6-((2-(difluoromethoxy)pyridin-4-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (14.8 mg) was then obtained with the similar method of Example 1. MS Calcd.: 671.2; MS Found: 672.4 [M+H]+.

$^1H$ NMR: δ 9.15 (s, 1 H), 8.28 (s, 1 H), 8.23 (d, *J*=5.2 Hz, 1 H), 7.49-7.90 (m, 2 H), 7.29 (d, *J*=5.2 Hz, 1 H), 7.08 (s, 1 H), 6.90 (d, *J*=7.2 Hz, 1 H), 6.77 (d, *J*=8.0 Hz, 1 H), 5.43 (s, 2 H), 5.12-5.20 (m, 1 H), 4.87-4.96 (m, 1 H), 4.73-4.77 (m, 1 H), 4.48-4.54 (m, 1 H), 4.38-4.45 (m, 1 H), 3.98 (d, *J*=14.0 Hz, 1 H), 3.85 (d, *J*=14.0 Hz, 1 H), 2.95-3.02 (m, 1 H), 2.81-2.88 (m, 1 H), 2.66-2.78 (m, 1 H), 2.54-2.63 (m, 1 H), 2.38-2.45 (m, 1 H), 2.14-2.31 (m, 2 H), 1.57-1.83 (m, 4 H). $^{19}F$ NMR(377 MHz, DMSO-d6): δ -63.58, -87.32.

**Example 9: (S)-2-((4-(6-((6-(difluoromethoxy)pyridin-2-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (Compound 9)**

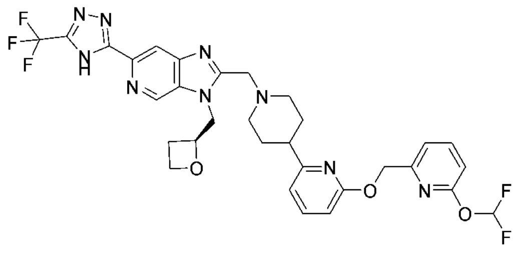

Starting from 2-bromo-6-(difluoromethoxy)pyridine, (S)-2-((4-(6-((6-(difluoromethoxy)pyridin-2-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine was obtained with the similar method of Example 8. MS Calcd.: 671.2; MS Found: 672.0 [M+H]+.

$^1$H NMR (400 MHz, DMSO-d6): δ 9.15 (s, 1 H), 8.28 (m, 1 H), 8.46-8.21 (m, 3 H), 7.30 (d, *J*=7.6 Hz, 1 H), 6.99 (d, *J*=8.4 Hz, 1 H), 6.89 (d, *J*=7.2 Hz, 1 H), 6.76 (d, *J*=8.4 Hz, 1 H), 5.38 (s, 2 H), 5.12-5.23 (m, 1 H), 4.85-4.96 (m, 1 H), 4.69-4.79 (m, 1 H), 4.38-4.55 (m, 2 H), 3.99 (d, *J*=13.6 Hz, 1 H), 3.83 (d, *J*=13.6 Hz, 1 H), 2.94-3.03 (m, 1 H), 2.80-2.88 (m, 1 H), 2.67-2.79 (m, 1 H), 2.55-2.64 (m, 1 H), 2.39-2.45 (m, 1 H), 2.13-2.30 (m, 2 H), 1.59-1.82 (m, 4 H). $^{19}$F NMR(377 MHz, DMSO-d6): δ -63.58, - 87.21.

**Example 10: (S)-3-(oxetan-2-ylmethyl)-2-((4-(6-((6-(trifluoromethoxy)pyridin-2-yl)methoxy)pyridin-2-yl)piperidin-1-yl)methyl)-6-(5-(trifluoromethyl)-4H-1,2,4-triazol-3-yl)-3H-imidazo[4,5-c]pyridine (Compound 10)**

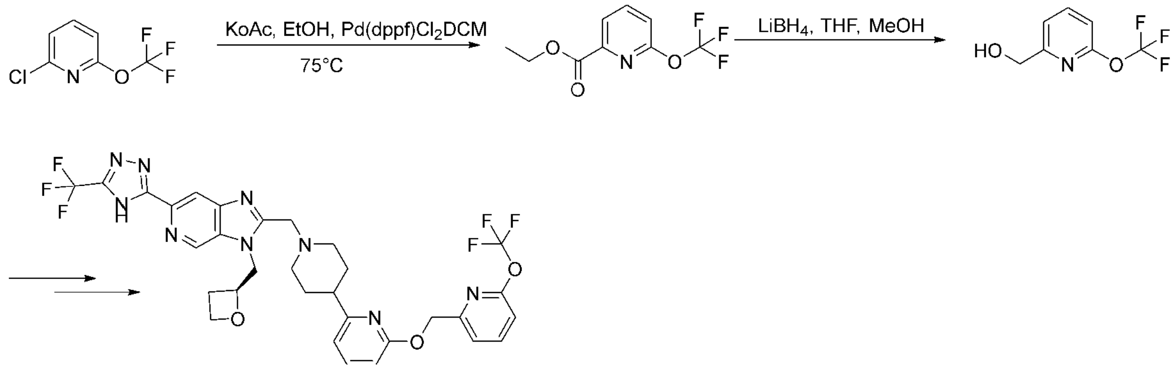


*Step A: Ethyl 6-(trifluoromethoxy)picolinate*

To a mixture of 2-chloro-6-(trifluoromethoxy)pyridine (1.26 g, 6.4 mmol) in EtOH (20 mL) was added $PdCl_2$(dppf) (467 mg, 0.64 mmol) and KOAc (1.25 g, 12.8 mmol). The mixture was stirred at 75°C for 2 hours under CO. After the reaction was completed, the reaction mixture was filtered. The filtrate was diluted with $H_2O$ (80 mL) and extracted with ethyl acetate (70 mL x 3). The organic layer was combined and washed with brine (50 mL x 2), dried over sodium sulfate, filtered and concentrated in vacuum. The reaction was purified by column chromatography (PE/EA=1/10) to give ethyl 6-(trifluoromethoxy)picolinate (1.13 g, yield: 75%). MS Calcd.: 235.0; MS Found: 236.3 [M+H]+.

*Step B: (6-(Trifluoromethoxy)pyridin-2-yl)methanol*

To a solution of ethyl 6-(trifluoromethoxy)picolinate (1.13 g, 4.81mmol ) in THF (10 mL) and MeOH (10 mL) was added $LiBH_4$ (317 mg, 14.42 mmol). The solution was stirred at room temperature for 2 hours. After the reaction was completed, the reaction mixture was quenched with $H_2O$ (40 mL) and extracted with ethyl acetate (40 mL x 3). The organic layers were combined and washed with brine (30 mL x 2), dried over sodium sulfate, filtered and concentrated in vacuum. The residue was purified by column chromatography (PE/EA=1/4) to give (6-(trifluoromethoxy)pyridin-2-yl)methanol (740 mg, yield: 80%). MS Calcd.: 193.0; MS Found: 194.1 $[M+H]^+$.

*Step C: (S)-3-(oxetan-2-ylmethyl)-2-((4-(6-((6-(trifluoromethoxy)pyridin-2-yl)methoxy)pyridin-2-yl)piperidin-1-yl)methyl)-6-(5-(trifluoromethyl)-4H-1,2,4-triazol-3-yl)-3H-imidazo[4,5-c]pyridine*

(S)-3-(oxetan-2-ylmethyl)-2-((4-(6-((6-(trifluoromethoxy)pyridin-2-yl)methoxy)pyridin-2-yl)piperidin-1-yl)methyl)-6-(5-(trifluoromethyl)-4H-1,2,4-triazol-3-yl)-3H-imidazo[4,5-c]pyridine (63.7 mg) was then obtained with the similar method of Example 3. MS Calcd.: 689.2; MS Found: 690.4 [M+H]+.

$^1$H NMR (400 MHz, DMSO-d6): δ 9.15 (s, 1 H), 8.28 (s, 1 H), 8.00 (t, *J*=8.0 Hz, 1 H), 7.66 (t, *J*=8.0 Hz, 1 H), 7.44 (d, *J*=7.2 Hz, 1 H), 7.19 (d, *J*=8.4 Hz, 1 H), 6.88 (d, *J*=7.2 Hz, 1 H), 6.77 (d, *J*=8.4 Hz, 1 H), 5.41 (s, 2 H), 5.12-5.22 (m, 1 H), 4.85-4.97 (m, 1 H), 4.71-4.76 (m, 1 H), 4.36-4.56 (m, 2 H), 3.98 (d, *J*=13.6 Hz, 1 H), 3.83 (d, *J*=13.6 Hz, 1 H), 2.92-3.02 (m, 1 H), 2.78-2.85 (m, 1 H), 2.67-2.77 (m, 1 H), 2.55-2.62 (m, 1 H), 2.39-2.46 (m, 1 H), 2.12-2.31 (m, 2 H), 1.53-1.78 (m, 4 H). $^{19}$F NMR (377 MHz, DMSO-d6): δ -55.12, -63.62.

**Example 11: (S)-3-(oxetan-2-ylmethyl)-2-((4-(6-((3-(trifluoromethoxy)benzyl)oxy)pyridin-2-yl)piperidin-1-yl)methyl)-6-(5-(trifluoromethyl)-4H-1,2,4-triazol-3-yl)-3H-imidazo[4,5-c]pyridine (Compound 11)**

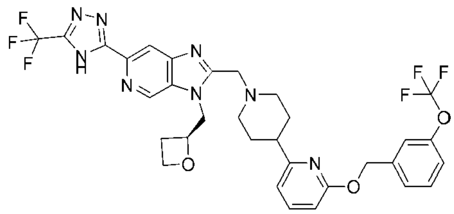

Starting from (3-(trifluoromethoxy)phenyl)methanol, (S)-3-(oxetan-2-ylmethyl)-2-((4-(6-((3-(trifluoromethoxy)benzyl)oxy)pyridin-2-yl)piperidin-1-yl)methyl)-6-(5-(trifluoromethyl)-4H-1 ,2,4-triazol-3-yl)-3H-imidazo[4,5-c]pyridine was obtained with the similar method of Example 3. MS Calcd.: 688.2; MS Found: 689.1 [M+H]+.

$^{1}$H NMR (400 MHz, DMSO-d6) δ 9.30 (s, 1 H), 8.41 (s, 1 H), 7.71 (t, *J*=8.0 Hz, 1 H), 7.49-7.55 (m, 2 H), 7.43 (s, 1 H), 7.31 (d, *J*=7.6 Hz, 1 H), 6.94 (d, *J*=7.2 Hz, 1 H), 6.78 (d, *J*=8.0 Hz, 1 H), 5.45 (s, 2 H), 5.08-5.14 (m, 1 H), 4.85-4.97 (m, 3 H), 4.73-4.77 (m, 1 H), 4.50-4.56 (m, 1 H), 4.37-4.43 (m, 1 H), 3.74-3.84 (m, 2 H), 3.26-3.43 (m, 2 H), 2.91-3.01 (m, 1 H), 2.70-2.81 (m, 1 H), 2.33-2.39 (m, 1 H), 2.01-2.17 (m, 4 H). $^{19}$F NMR (377 MHz, DMSO-d6): δ -56.70, -63.76.

**Example 12: (S)-2-((4-(6-((6-(difluoromethoxy)-5-methylpyridin-2-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (Compound 12)**

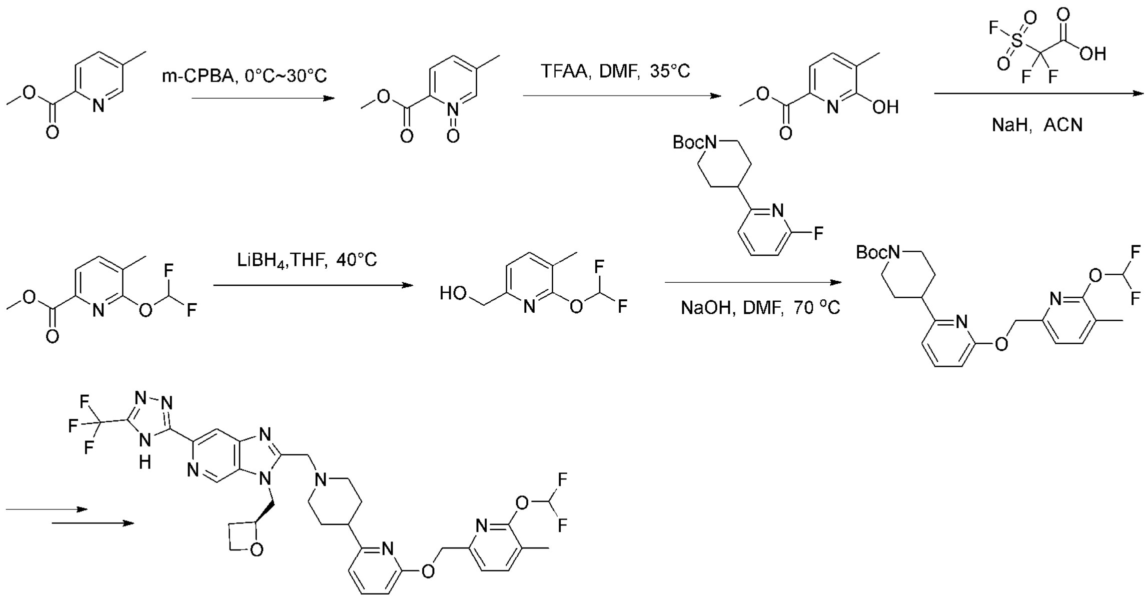


*Step A: 2-(Methoxycarbonyl)-5-methylpyridine 1-oxide*

To a mixture of methyl 5-methylpicolinate (2.0 g, 13.2 mmol) in DCM (50 mL) was added m-CPBA (4.6 g, 26.5 mmol) at 0 °C under $N_2$. The mixture was stirred at 30 °C for 2 hours. After the reaction was completed, the reaction was cooled to 0°C. The mixture was quenched with $NaHCO_3$ (eq, 50 mL), extracted with DCM and MeOH (DCM/MeOH=20/1, 3 x 80 mL). The combined organic layers were washed with brine (60 ml). The organic layer was dried over $Na_2SO_4$, filtered and concentrated under reduced pressure. The residue was purified by column chromatography on silica gel (EA/PE=1/1) to give 2-(methoxycarbonyl)-5-methylpyridine 1-oxide (2.1 g, yield: 93.1%). MS Calcd.: 167.1; MS Found: 168.1 [M+H]+.

*Step B: Methyl 6-hydroxy-5-methylpicolinate*

To a mixture of 2-(methoxycarbonyl)-5-methylpyridine 1-oxide (2.1 g, 12.3 mmol) in DMF (20 mL) was added

TFAA (15.5 g, 74.0 mmol) at RT. The mixture was stirred at 35°C for 2 h. After the reaction was completed, the reaction was quenched with sat. sodium bicarbonate aqueous solution (50 mL), extracted with ethyl acetate (50 mL×3). The organic layers were combined, dried over $Na_2SO_4$, filtered and concentrated under vacuum to dryness. The residue was purified by column chromatography on silica gel (EA/PE=1/1) to give methyl *6-hydroxy-5-methylpicolinate* (1.7 g, yield: 81.1%). MS Calcd.: 167.1; MS Found: 168.1 $[M+H]^+$.

*Step C: Methyl 6-(difluoromethoxy)-5-methylpicolinate*

To a mixture of methyl 6-hydroxy-5-methylpicolinate (600 mg, 3.6 mmol) in ACN (10 mL) was added NaH (259 mg, 10.79 mmol) at 0°C under $N_2$. The mixture was stirred at 0°C for 30 min, added with 2,2-difluoro-2-(fluorosulfonyl) acetic acid (959 mg, 5.4 mmol). The mixture was stirred at RT for 3 hours. After the reaction was completed, the reaction was quenched with saturated ammonium chloride solution (50 mL), extracted with ethyl acetate (50 mL×3). The organic layers were combined, dried over $Na_2SO_4$, filtered and concentrated under vacuum to dryness. The residue was purified by column chromatography on silica gel (EA/PE=1/3) to give methyl 6-(difluoromethoxy)-5-methylpicolinate (770 mg, yield: 98.8%). MS Calcd.: 217.1; MS Found: 218.1 [M+H]+.

*Step D: (6-(Difluoromethoxy)-5-methylpyridin-2-yl)methanol*

To a mixture of methyl 6-(difluoromethoxy)-5-methylpicolinate (740 mg, 3.4 mmol) in THF (8 mL) was added $LiBH_4$ (300 mg, 13.6 mmol) at RT under $N_2$. The mixture was stirred at 40°C for 30 min. After the reaction was completed, the reaction was quenched with Saturated ammonium chloride solution (50 mL), extracted with ethyl acetate (50 mL×3). The organic layers were combined, dried over $Na_2SO_4$, filtered and concentrated under vacuum to dryness. The residue was purified by column chromatography on silica gel (EA/PE=1/3) to give (6-(difluoromethoxy)-5-methylpyridin-2-yl) methanol (470 mg, 72.9%). MS Calcd.: 189.1; MS Found: 190.1 $[M+H]^+$.

*Step E: tert-Butyl 4-(6-((6-(difluoromethoxy)-5-methylpyridin-2-yl)methoxy)pyridin-2-yl)piperidine-1-carboxylate*

To a solution of (6-(difluoromethoxy)-5-methylpyridin-2-yl)methanol (470 mg, 2.5 mmol) in DMF (10 mL) was added tert-butyl 4-(6-fluoropyridin-2-yl)piperidine-1-carboxylate (836 mg, 3.0 mmol), NaOH (199 mg, 5.0 mmol) at RT. The resulting mixture was stirred at 70°C for 16 h. After the reaction was completed, the reaction was quenched with sat. sodium bicarbonate aqueous solution (50 mL), extracted with ethyl acetate (50 mL×3). The organic layers were combined, dried over $Na_2SO_4$, filtered and concentrated under vacuum to dryness. The residue was purified by column chromatography on silica gel (EA/PE=2/1) to give tert-butyl 4-(6-((6-(difluoromethoxy)-5-methylpyridin-2-yl)methoxy) pyridin-2-yl)piperidine-1-carboxylate (620 mg, 55.5% yield). MS Calcd.: 449.2; MS Found: 472.2 $[M+H+23]^+$.

*Step F: (S)-2-((4-(6-((6-(difluoromethoxy)-5-methylpyridin-2-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine*

(S)-2-((4-(6-((6-(difluoromethoxy)-5-methylpyridin-2-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (86.8 mg) was obtained with the similar method of Example 1. MS Calcd.: 685.2; MS Found: 686.1 [M+H]+.

$^1$H NMR (400 MHz, DMSO-d6): δ 9.15 (s, 1 H), 8.28 (s, 1 H), 7.45-7.88 (m, 3 H), 7.23 (d, *J*=7.6 Hz, 1 H), 6.89 (d, *J*=7.2 Hz, 1 H), 6.74 (d, *J*=8.0 Hz, 1 H), 5.33 (s, 2 H), 5.12-5.22 (m, 1 H), 4.85-4.96 (m, 1 H), 4.70-4.79 (m, 1 H), 4.51-4.55 (m, 1 H), 4.37-4.43 (m, 1 H), 3.99 (d, *J*=14.0 Hz, 1 H), 3.84 (d, *J*=13.6 Hz, 1 H), 2.95-3.04 (m, 1 H), 2.80-2.89 (m, 1 H), 2.68-2.78 (m, 1 H), 2.53-2.64 (m, 2 H), 2.21-2.34 (m, 2 H), 2.19 (s, 3 H), 1.60-1.84 (m, 4 H). $^{19}$F NMR(377 MHz, DMSO-d6): δ -63.58, -86.96.

**Example 13: (S)-2-((4-(6-((5-chloro-6-(difluoromethoxy)pyridin-2-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (Compound 13)**

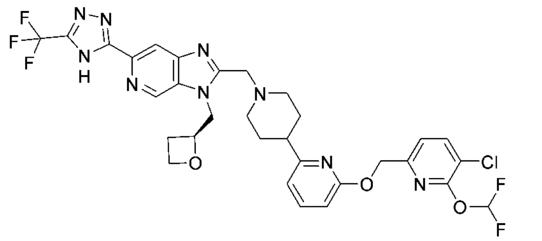

Starting from 5-chloropicolinic acid, (S)-2-((4-(6-((5-chloro-6-(difluoromethoxy)pyridin-2-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (31.6 mg) was obtained with the similar method as described herein. MS Calcd.: 705.20; MS Found: 705.9 [M+H]+.

$^1$H NMR (400 MHz, DMSO-d6): δ 9.15 (s, 1 H), 8.28 (s, 1 H), 8.10 (d, *J*=8.0 Hz, 1 H), 7.51-7.90 (m, 2 H), 7.33 (d, *J*=8.0 Hz, 1 H), 6.90 (d, *J*=7.2 Hz, 1 H), 6.77 (d, *J*=8.4 Hz, 1 H), 5.37 (s, 2 H), 5.13-5.22 (m, 1 H), 4.87-4.94 (m, 1 H), 4.71-4.77 (m, 1 H), 4.38-4.54 (m, 2 H), 3.49 (d, *J*=13.6 Hz, 1 H), 3.84 (d, *J*=13.6 Hz, 1 H), 2.94-3.03 (m, 1 H), 2.80-2.88 (m, 1 H), 2.65-2.77 (m, 1 H), 2.53-2.63 (m, 1 H), 2.41-2.47 (m, 1 H), 2.13-2.30 (m, 2 H), 1.55-1.83 (m, 4 H). $^{19}$F NMR(377 MHz, DMSO-d6): δ -63.59, - 87.67.

**Example 14: (S)-2-((4-(6-((6-(difluoromethoxy)-5-fluoropyridin-2-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (Compound 14)**

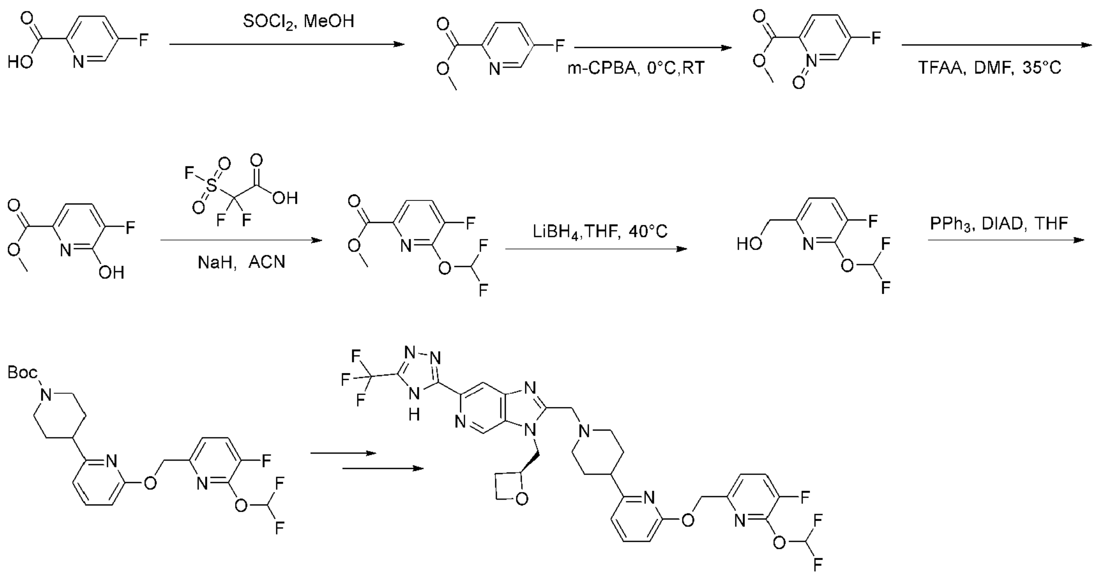


*Step A: Methyl 5-fluoropicolinate*

To a mixture of 5-fluoropicolinic acid (2.0 g, 14.2 mmol) in MeOH (20 mL) was added $SOCl_2$ (10.1 g, 85.1 mmol) at 0°C under $N_2$. The mixture was stirred at 0°C for 10 min, warmed up to rt for 10 min, and then heated to 70°C for 16h. After the reaction was completed, the reaction was concentrated under vacuum to dryness to give methyl 5-fluoropicolinate (2.0 g, yield: 93.3%). MS Calcd.: 155.0; MS Found: 156.1 [M+H]+.

*Step B: 5-Fluoro-2-(methoxycarbonyl)pyridine 1-oxide*

To a mixture of methyl 5-fluoropicolinate (2.0 g, 12.9 mmol) in DCM (20 mL) was added m-CPBA (6.7 g, 38.7 mmol) at 0 °C under $N_2$. The mixture was stirred at RT for 16 h. After the reaction was completed, the reaction was cooled to 0°C and filtered. The filtrate was concentrated under vacuum to dryness. The residue was purified by column chromatography on silica gel (EA/PE=1/1) to give 5-fluoro-2-(methoxycarbonyl)pyridine 1-oxide (1.2 g, yield: 54.5%). MS Calcd.: 171.0; MS Found: 172.1 $[M+H]^+$.

*Step C: Methyl 5-fluoro-6-hydroxypicolinate*

To a mixture of 5-fluoro-2-(methoxycarbonyl)pyridine 1-oxide (1.1 g, 6.4 mmol) in DMF (10 mL) was added TFAA (7.7 g, 38.6 mmol) at RT. The mixture was stirred at 35°C for 2 h. After the reaction was completed, the reaction was quenched with sat. sodium bicarbonate aqueous solution (50 mL), extracted with ethyl acetate (50 mL×3). The organic layers were combined, dried over $Na_2SO_4$, filtered and concentrated under vacuum to dryness. The residue was purified by column chromatography on silica gel (EA/PE=1/1) to give methyl 5-fluoro-6-hydroxypicolinate (950 mg, yield: 86.4%). MS Calcd.: 171.0; MS Found: 172.1 $[M+H]^+$.

*Step D: Methyl 6-(difluoromethoxy)-5-fluoropicolinate*

To a mixture of methyl 5-fluoro-6-hydroxypicolinate (920 mg, 5.4 mmol) in ACN (10 mL) was added NaH (387 mg, 16.1 mmol) at 0°C under $N_2$. The mixture was stirred at 0°C for 30 min, added with 2,2-difluoro-2-(fluorosulfonyl)acetic acid (1.43 g, 8.1 mmol). The mixture was stirred at RT for 3 h. After the reaction was completed, the reaction was quenched with Saturated ammonium chloride solution (50 mL), extracted with ethyl acetate (50 mL×3). The organic layers were combined, dried over $Na_2SO_4$, filtered and concentrated under vacuum to dryness. The residue was purified by column chromatography on silica gel (EA/PE=1/3) to give methyl 6-(difluoromethoxy)-5-fluoropicolinate (720 mg, yield: 60.6%). MS Calcd.: 221.0; MS Found: 222.1 $[M+H]^+$.

*Step E: (6-(difluoromethoxy)-5-fluoropyridin-2-yl)methanol*

To a mixture of methyl 6-(difluoromethoxy)-5-fluoropicolinate (720 mg, 3.3 mmol) in THF (5 mL) was added $LiBH_4$ (287 mg, 13.0 mmol) at RT under $N_2$. The mixture was stirred at 40°C for 30 min. After the reaction was completed, the reaction was quenched with Saturated ammonium chloride solution (50 mL), extracted with ethyl acetate (50 mL×3). The organic layers were combined, dried over $Na_2SO_4$, filtered and concentrated under vacuum to dryness. The residue was purified by column chromatography on silica gel (EA/PE=1/3) to give (6-(difluoromethoxy)-5-fluoropyridin-2-yl)methanol (660 mg, crude). MS Calcd.: 193.0; MS Found: 194.1 $[M+H]^+$.

*Step F: tert-Butyl 4-(6-((6-(difluoromethoxy)-5-fluoropyridin-2-yl)methoxy)pyridin-2-yl)piperidine-1-carboxylate*

To a solution of (6-(difluoromethoxy)-5-fluoropyridin-2-yl)methanol (200 mg, 1.0 mmol) in THF (10 mL) was added tert-butyl 4-(6-hydroxypyridin-2-yl)piperidine-1-carboxylate (518 mg, 1.9 mmol), $PPh_3$ (543 mg, 2.1 mmol) and DIAD (418 mg, 2.072 mmol) at 0°C. The resulting mixture was stirred at RT for 2h. After the reaction was completed, the reaction was quenched with sat. sodium bicarbonate aqueous solution (50 mL), extracted with ethyl acetate (50 mL×3). The organic layers were combined, dried over $Na_2SO_4$, filtered and concentrated under vacuum to dryness. The residue was purified by column chromatography on silica gel (EA/PE=2/1) to give tert-butyl 4-(6-((6-(difluoromethoxy)-5-fluoropyridin-2-yl)methoxy)pyridin-2-yl)piperidine-1-carboxylate (180 mg, 38.3% yield). MS Calcd.: 453.2; MS Found: 454.1 $[M+H]^+$.

*Step G: (S)-2-((4-(6-((6-(difluoromethoxy)-5-fluoropyridin-2-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine*

(S)-2-((4-(6-((6-(difluoromethoxy)-5-fluoropyridin-2-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (13.3 mg) was obtained with the similar method of Example 1. MS Calcd.: 689.2; MS Found: 690.0 $[M+H]^+$.

$^1$H NMR (400 MHz, DMSO-d6): δ 9.14 (s, 1 H), 8.27 (s, 1 H), 7.50-7.97 (m, 3 H), 7.37 (dd, *J*=8.4 Hz, *J*=3.2 Hz, 1 H), 6.90 (d, *J*=7.6 Hz, 1 H). 6.75 (d, *J*=8.0 Hz, 1 H), 5.34 (s, 2 H), 5.12-5.22 (m, 1 H), 4.85-4.96 (m, 1 H), 4.67-4.78 (m, 1 H), 4.47-4.55 (m, 1 H), 4.39-4.46 (m, 1 H), 3.99 (d, *J*=14.0 Hz, 1 H), 3.84 (d, *J*=13.6 Hz, 1 H), 2.95-3.03 (m, 1 H), 2.86-2.95 (m, 1 H), 2.69-2.81 (m, 1 H), 2.55-2.63 (m, 1 H), 2.42-2.47 (m, 1 H), 2.14-2.31 (m, 2 H), 1.59-1.84 (m, 4 H). $^{19}$F NMR(377 MHz, DMSO-d6): δ - 63.40, -86.99, -140.71.

**Example 15: (S)-2-((4-(6-((2-(difluoromethoxy)-6-methylpyridin-3-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (Compound 15)**

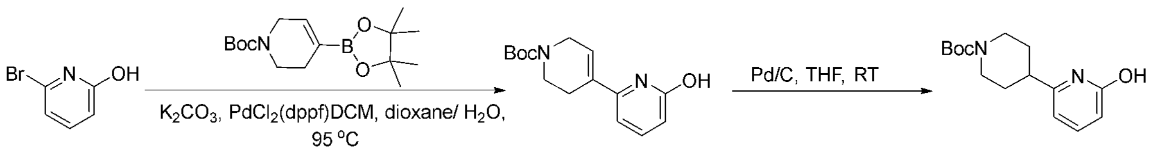


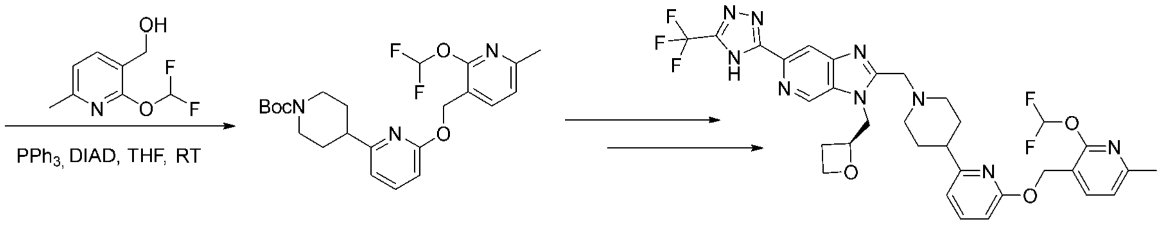


*Step A:tert-butyl 6-hydroxy-3',6'-dihydro-[2,4'-bipyridine]-1'(2'H)-carboxylate*

To a mixture of 6-bromopyridin-2-ol (2.0 g, 11.5 mmol), tert-butyl 4-(4,4,5,5-tetramethyl-1,3,2-dioxaborolan-2-yl)-3,6-dihydropyridine-1(2H)-carboxylate (5.33g,17.2mmol)and$K_2CO_3$ (3.2g, 23.0 mmol) in dioxane/$H_2O$ (10 mL/1 mL) was added Pd(dppf)$Cl_2$·DCM (938 mg, 1.15 mmol) at RT under $N_2$. The mixture was stirred at 95°C for 16 h. After the reaction was completed, the reaction was quenched with sat. sodium bicarbonate aqueous solution (50 mL), extracted with ethyl acetate (50 mL×3). The organic layers were combined, dried over $Na_2SO_4$, filtered and concentrated under vacuum to dryness. The residue was purified by column chromatography on silica gel (EA/PE=1/1) to give tert-butyl 6-hydroxy-3',6'-dihydro-[2,4'-bipyridine]-]'(2'H)-carboxylate (1.0 g, yield: 31.5%). MS Calcd.: 276.2; MS Found: 277.2 $[M+H]^+$.

*Step B: tert-butyl 4-(6-((4-chloro-2-methoxybenzyl)oxy)pyridin-2-yl)piperidine-1-carboxylate*

To a mixture of tert-butyl 6-hydroxy-3',6'-dihydro-[2,4'-bipyridine]-1'(2'H)-carboxylate (1.0 g, 3.62 mmol) in THF (10 mL) was added Pd/C (500 mg, 10% on Carbon, wetted with ca. 55% water) at RT. The mixture was stirred at RT for 1 h under $H_2$. After the reaction was completed, the mixture was filtered, and the filtrate evaporated to give tert-butyl 4-(6-hydroxypyridin-2-yl)piperidine-1-carboxylate (1.5 g, crude). MS Calcd.: 278.2; MS Found: 279.2 $[M+H]^+$.

*Step C: tert-butyl 4-(6-((2-(difluoromethoxy)-6-methylpyridin-3-yl)methoxy)pyridin-2-yl)piperidine-1-carboxylate*

To a solution of tert-butyl 4-(6-hydroxypyridin-2-yl)piperidine-1-carboxylate (264 mg, 0.95 mmol), (2-(difluoromethoxy)-6-methylpyridin-3-yl)methanol (150 mg, 0.79 mmol) and $PPh_3$ (415 mg, 1.6 mmol) in THF (5.0 mL) was added dropwise DIAD (319 mg, 1.6 mmol) successively at 0°C. The resulting mixture was stirred at RT for 2h. After the reaction was completed, the reaction was quenched with sat. sodium bicarbonate aqueous solution (50 mL), extracted with ethyl acetate (50 mL×3). The organic layers were combined, dried over $Na_2SO_4$, filtered and concentrated under vacuum to dryness. The residue was purified by column chromatography on silica gel (EA/PE=1/1) to give tert-butyl 4-(6-((2-(difluoromethoxy)-6-methylpyridin-3-yl)methoxy)pyridin-2-yl)piperidine-1-carboxylate (40 mg, 11.2% yield). MS Calcd.: 449.2; MS Found: 450.2 $[M+H]^+$.

*Step D: (S)-2-((4-(6-((2-(difluoromethoxy)-6-methylpyridin-3-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine*

(S)-2-((4-(6-((2-(difluoromethoxy)-6-methylpyridin-3-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (4.8 mg) was then obtained with the similar method of Example 1. MS Calcd.: 685.2; MS Found: 686.0 $[M+H]^+$.

$^1$H NMR (400 MHz, DMSO-d6): δ9.15 (s, 1 H), 8.28 (s, 1 H), 7.51-7.95 (m, 3 H), 7.12 (d, *J*=7.6 Hz, 1 H), 6.88 (d, *J*=7.2 Hz, 1 H), 6.68 (d, *J*=8.0 Hz, 1 H), 5.32 (s, 2 H), 5.13-5.23 (m, 1 H), 4.87-4.97 (m, 1 H), 4.71-4.80 (m, 1 H), 4.37-4.55 (m, 2 H), 4.00 (d, *J*=14.0 Hz, 1 H), 3.85 (d, *J*=13.6 Hz, 1 H), 2.98-3.06 (m, 1 H), 2.83-2.91 (m, 1 H), 2.68-2.79 (m, 1 H), 2.58-2.65 (m, 1 H), 2.37-2.47 (m, 4 H), 2.16-2.35 (m, 2 H), 1.59-1.87 (m, 4 H).

$^{19}$F NMR (377 MHz, DMSO-d6): δ -63.51, -86.91.

**Example 16: (S)-2-((4-(6-((7-chlorobenzo[d][1,3]dioxol-4-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (Compound 16)**

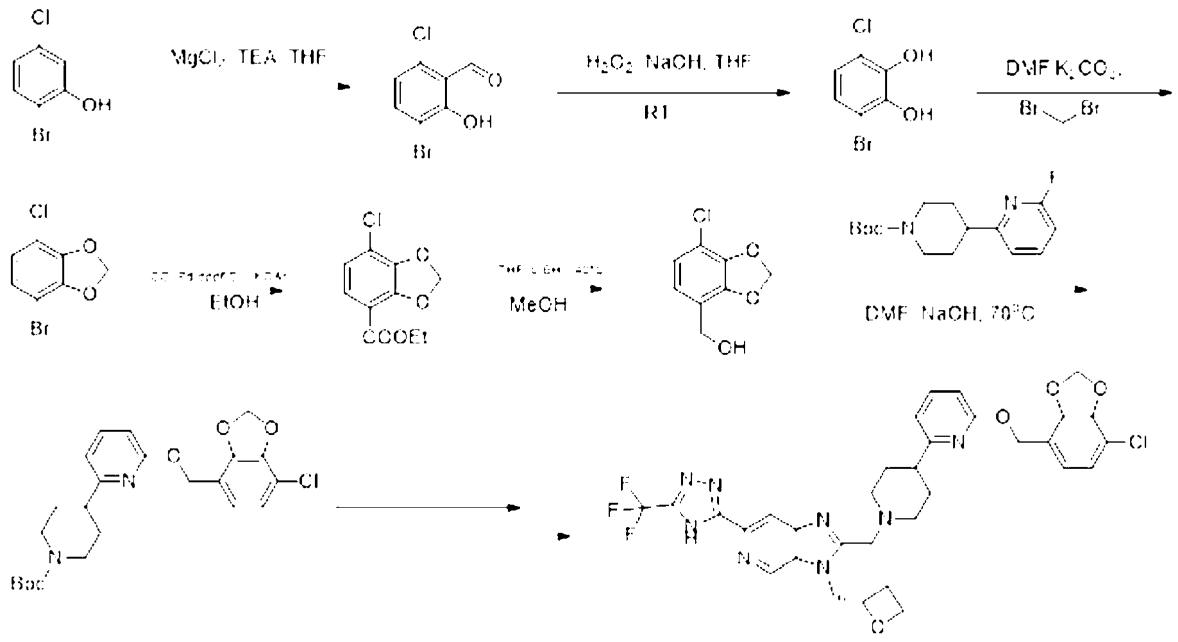


*Step A: 3-bromo-6-chloro-2-hydroxybenzaldehyde*

To a mixture of paraformaldehyde (11.2 g, 124.4 mmol), $MgCl_2$ (7.88 g, 82.9 mmol) in THF (150 mL) and TEA (11.5 ml) was stirred at 70°C for 15 min under Ar. Then, 2-bromo-5-chlorophenol (8.5 g, 41.5 mmol) was added, the reaction was stirred at 70°C for overnight under Ar. After the reaction was completed, the reaction mixture was filtered and quenched with $H_2O$ (150 mL) and extracted with ethyl acetate (150 mL x 3). The organic layer was combined and washed with brine (80 mL x 2), dried over sodium sulfate, filtered, and concentrated in vacuum. The reaction was purified by column chromatography (PE=100%) to give 3-bromo-6-chloro-2-hydroxybenzaldehyde (6.6 g, yield: 68%).

*Step B: 3-bromo-6-chlorobenzene-1,2-diol*

To a mixture of 3-bromo-6-chloro-2-hydroxybenzaldehyde (2.0 g, 8.55 mmol) in $H_2O_2$ (5 ml, 30% aqueous solution), THF (20 ml) was added NaOH (5 ml, 2 M in water) and stirred at room temperature for overnight. After the reaction was completed, the reaction mixture was quenched with $H_2O$ (40 mL) and extracted with ethyl acetate (40 mL x 3). The organic layer was combined and washed with brine (30 mL x 2), dried over sodium sulfate, filtered and concentrated in vacuum. The reaction was purified by column chromatography (PE/EA=1/3) to give 3-bromo-6-chlorobenzene-1,2-diol (1.33 g, yield: 70%).

*Step C: 4-bromo-7-fluorobenzo[d][1,3]dioxole*

To a solution of 3-bromo-6-fluorobenzene-1,2-diol (550 mg, 2.5 mmol) in DMF (16 mL) was added dibromomethane (4.3 g, 24.77 mmol) and $K_2CO_3$ (1.03 g, 7.46 mmol). The reaction was stirred at 110 °C for 2 hours. After the reaction was completed, the mixture was quenched with $H_2O$ (40 mL) and extracted with ethyl acetate (40 mL x 3). The organic layer was combined and washed with brine (30 mL x 2), dried over sodium sulfate, filtered and concentrated in vacuum. The reaction was purified by column chromatography (PE/EA=1/10) to give 4-bromo-7-fluorobenzo[d][1,3] dioxole (470 mg, yield: 89%).

*Step D: ethyl 7-chlorobenzo[d][1,3]dioxole-4-carboxylate*

To a mixture of 4-bromo-7-fluorobenzo[d][1,3]dioxole (470 mg, 2.00 mmol) in EtOH (8.0 mL) was added $PdCl_2$ (dppf) (147 mg, 0.20 mmol) and KOAc (394 mg, 4.02 mmol). The mixture was stirred at 75 °C for 2 hours under CO. After

the reaction was completed, the mixture was quenched with $H_2O$ (50 mL) and extracted with ethyl acetate (50 mL x 3). The organic layer was combined and washed with brine (30 mL x 2), dried over sodium sulfate, filtered and concentrated in vacuum. The reaction was purified by column chromatography (PE/EA=10/1) to give ethyl 7-chlorobenzo[d][1,3]dioxole-4-carboxylate (360 mg, yield: 79%).

*Step E: (7-chlorobenzo[d][1,3]dioxol-4-yl)methanol*

To a solution of ethyl 7-fluorobenzo[d][1,3]dioxole-4-carboxylate (360 mg, 1.58 mmol) in THF (3 mL) and MeOH (3 ml) was added $LiBH_4$ (144.7 mg, 6.58 mmol). The solution was stirred at room temperature for 1.5 hours. After the reaction was completed, the mixture was quenched with $H_2O$ (30 mL) and extracted with ethyl acetate (30 mL x 3). The organic layer was combined and washed with brine (20 mL x 2), dried over sodium sulfate, filtered and concentrated in vacuum. The reaction was purified by column chromatography (PE/EA=3/1) to give (7-chlorobenzo[d][1,3]dioxol-4-yl) methanol (190 mg, yield: 65%).

*Step F: tert-butyl 4-(6-((7-chlorobenzo[d][1,3]dioxol-4-yl)methoxy)pyridin-2-yl)piperidine-1-carboxylate*

A mixture of (7-chlorobenzo[d][1,3]dioxol-4-yl)methanol (190 mg, 1.02 mmol), tert-butyl 4-(6-fluoropyridin-2-yl) piperidine-1-carboxylate (343.2 mg, 1.22 mmol), NaOH (81.72 mg, 2.04 mmol) in DMF (4.5 mL) was stirred at 70°C for overnight. After the reaction was completed, the mixture was quenched with $H_2O$ (40 mL) and extracted with ethyl acetate (30 mL x 3). The organic layer was combined and washed with brine (20 mL x 2), dried over sodium sulfate, filtered and concentrated in vacuum. The reaction was purified by column chromatography (PE/EA=3/1) to give tert-butyl 4-(6-((7-chlorobenzo[d][1,3]dioxol-4-yl)methoxy)pyridin-2-yl)piperidine-1-carboxylate (270 mg, yield: 59%). MS Calcd.: 446.2; MS Found: 447.2 $[M+H]^+$.

*Step G: (S)-2-((4-(6-((7-chlorobenzo[d][1,3]dioxol-4-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine*

(S)-2-((4-(6-((7-chlorobenzo[d][1,3]dioxol-4-yl)methoxy)pyridin-2-yl)piperidin-1 -yl)methyl)-3-(oxetan-2-ylmethyl)-6-(5-(trifluoromethyl)-4H-1,2,4-triazol-3-yl)-3H-imidazo[4,5-c]pyridine (65.7 mg) was obtained with the similar procedure of Example 1. MS Calcd.: 682.2; MS Found: 683.3 $[M+H]^+$.

1H NMR: δ 9.15 (s, 1 H), 8.28 (s, 1 H), 7.62 (t, *J*=8.0 Hz, 1 H), 6.98 (d, *J*=8.4 Hz, 1 H), 6.92 (d, *J*=8.4 Hz, 1 H), 6.88 (d, *J*=7.2 Hz, 1 H), 6.67 (d, *J*=8.4 Hz, 1 H), 6.18 (s, 2 H), 5.28 (s, 2 H), 5.14-5.22 (m, 1 H), 4.87-4.96 (m, 1 H), 4.74-4.78 (m, 1 H), 4.39-4.53 (m, 2 H), 4.01 (d, *J*=13.6 Hz, 1 H), 3.86 (d, *J*=13.6 Hz, 1 H), 3.00-3.08 (m, 1 H), 2.84-2.92 (m, 1 H), 2.69-2.78 (m, 1 H), 2.58-2.65 (m, 1 H), 2.38-2.45 (m, 1 H), 2.17-2.31 (m , 2 H), 1.65-1.87 (m, 4 H).

$^{19}F$ NMR (377 MHz, DMSO-d6): δ -63.56.

**Example 17: (S)-2-((4-(6-((7-fluorobenzo[d][1,3]dioxol-4-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (Compound 17)**

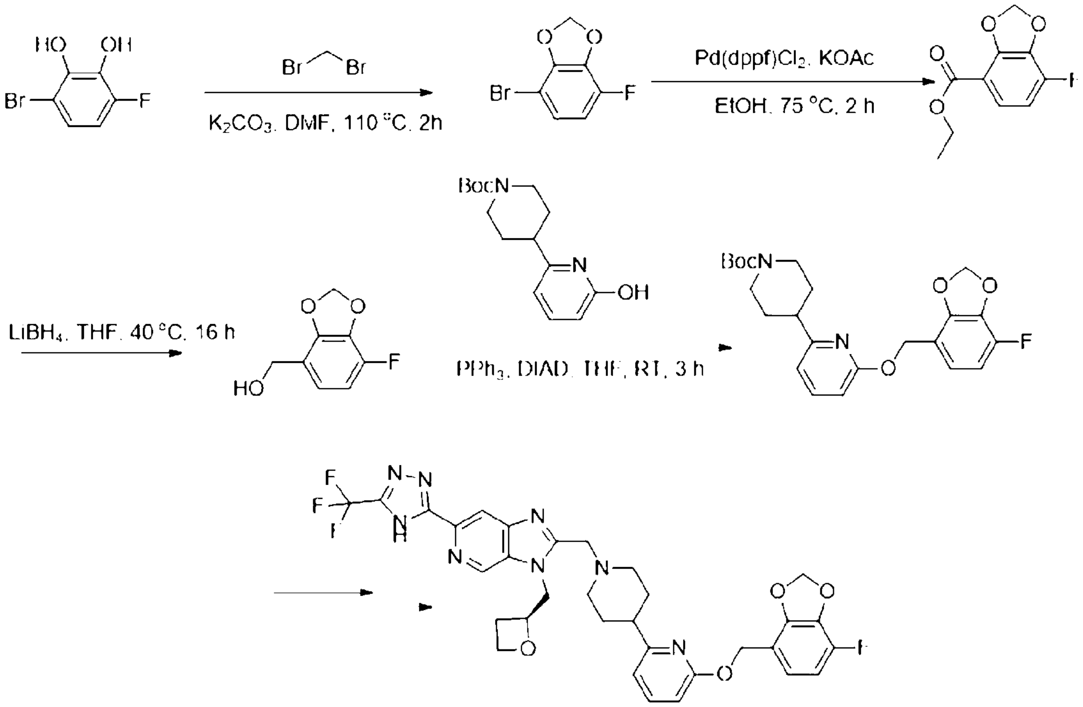


*Step A: 4-Bromo-7-fluorobenzo[d][1,3]dioxole*

To a solution of 3-bromo-6-fluorobenzene-1,2-diol (1.50 g, 7.28 mmol) in DMF (30 mL) was added dibromomethane (6.33 g, 36.41 mmol) and $K_2CO_3$ (3.01 g, 21.84 mmol). The solution was stirred at 110 °C for 2 hours. After the reaction was completed, the mixture was diluted with $H_2O$ (50 mL), extracted with EA (60 mL×2). The combined organic layer was dried over anhydrous sodium sulfate, filtered and evaporated to dryness to give 4-bromo-7-fluorobenzo[d][1,3]dioxole (1.47 g, yield: 93%).

*Step B: Ethyl 7-fluorobenzo[d][1,3]dioxole-4-carboxylate*

To a mixture of 4-bromo-7-fluorobenzo[d][1,3]dioxole (700 mg, 3.21 mmol) in EtOH (10.0 mL) was added $PdCl_2$(dppf) (469.89 mg, 0.64 mmol) and KOAc (943.74 g, 9.63 mmol). The mixture was stirred at 75°C for 2 hours under CO. After the reaction was completed, the reaction mixture was filtered, and the filtrate evaporated to dryness. The residue was purified by column chromatography on silica gel (PE/EA=8/1) to give ethyl 7-fluorobenzo[d][1,3]dioxole-4-carboxylate (530 mg, yield: 78%).

*Step C: (7-Fluorobenzo[d][1,3]dioxol-4-yl)methanol*

To a solution of ethyl 7-fluorobenzo[d][1,3]dioxole-4-carboxylate (500 mg, 2.36 mmol) in THF (4 mL) was added $LiBH_4$ (102.74 mg, 4.72 mmol). The solution was stirred at 40 °C for 16 hours. After the reaction was completed, the mixture was diluted with $NH_4Cl$ aq (30 mL), extracted with DCM (30 mL×2). The organic layers were combined, dried over $Na_2SO_4$, filtered and concentrated under vacuum to dryness to give (7-fluorobenzo[d][1,3]dioxol-4-yl)methanol (340 mg, yield: 85%).

*Step D: tert-Butyl 4-(6-((7-fluorobenzo[d][1,3]dioxol-4-yl)methoxy)pyridin-2-yl)piperidine-1-carboxylate*

A mixture of (7-fluorobenzo[d][1,3]dioxol-4-yl)methanol (290 mg, 1.7 mmol), tert-butyl 4-(6-hydroxypyridin-2-yl)piperidine-1-carboxylate (569 mg, 2.0 mmol), $PPh_3$ (896 mg, 3.4 mmol), DIAD (691 mg, 3.4 mmol) in THF (6 mL) was stirred at RT for 3 hours. After the reaction was completed, the mixture was diluted with $H_2O$ (20 mL), extracted with EA (30 mL×2). The combined organic layer was dried over anhydrous sodium sulfate, filtered and evaporated to dryness. The residue was purified by column chromatography on silica gel (PE/EA=8/1) to give tert-butyl 4-(6-((7-fluorobenzo[d][1,3]dioxol-4-yl)methoxy)pyridin-2-yl)piperidine-1-carboxylate (100 mg, yield: 14%). MS Calcd.: 430.2; MS Found: 431.0 $[M+H]^+$.

*Step E: (S)-2-((4-(6-((7-fluorobenzo[d][1,3]dioxol-4-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine*

(S)-2-((4-(6-((7-fluorobenzo[d][1,3]dioxol-4-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine was obtained with the similar method of Example 1 (6.8 mg). MS Calcd.: 666.2; MS Found: 667.1 $[M+H]^+$.

$^1$H NMR (400 MHz, DMSO-d6): δ 9.15 (s, 1 H), 8.28 (s, 1 H), 7.63 (t, *J*=8.0 Hz, 1 H), 6.96-7.02 (m, 1 H), 6.80-6.90 (m, 2 H), 6.65 (d, *J*=8.0 Hz, 1 H), 6.17 (s, 2 H), 5.27 (s, 2 H), 5.15-5.21 (m, 1 H), 4.88-4.96 (m, 1 H), 4.73-4.80 (m, 1 H), 4.39-4.53 (m, 2 H), 4.02 (d, *J*=13.2 Hz, 1 H), 3.86 (d, *J*=13.6 Hz, 1 H), 3.01-3.08 (m, 1 H), 2.86-2.93 (m, 1 H), 2.70-2.79 (m, 2 H), 2.43-2.48 (m, 1 H), 2.18-2.35 (m, 2 H), 1.71-1.87 (m, 4 H).$^{19}$F NMR(377 MHz, DMSO-d6): δ -63.37, -139.35.

**Example 18: (S)-2-((4-(6-((2,2-difluorobenzo[d][1,3]dioxol-4-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (Compound 18)**

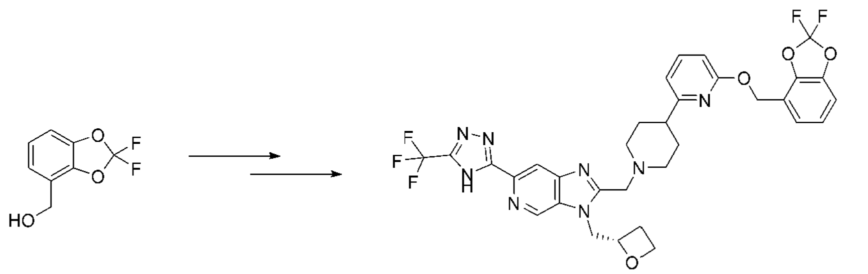

Starting from (2,2-difluorobenzo[d][1,3]dioxol-4-yl)methanol, (S)-2-((4-(6-((2,2-difluorobenzo[d][1,3]dioxol-4-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine was obtained with the similar method of Example 3. MS Calcd.: 684.2; MS Found: 685.4 [M+H]+.

1H NMR: δ 9.16 (s, 1 H), 8.28 (s, 1 H), 7.64 (t, *J*=8.0 Hz, 1 H), 7.35 (dd, *J*=1.2 Hz, J=8.0 Hz, 1 H), 7.29 (d, *J*=7.2 Hz, 1 H), 7.19 (t, *J*=8.0 Hz, 1 H), 6.88 (d, *J*=7.2 Hz, 1 H), 6.70 (d, *J*=8.0 Hz, 1 H), 5.44 (s, 2 H), 5.14-5.21 (m, 1 H), 4.88-4.96 (m, 1 H), 4.74-4.78 (m, 1 H), 4.47-4.54 (m, 1 H), 4.38-4.46 (m, 1 H), 4.00 (d, *J*=13.6 Hz, 1 H), 3.85 (d, *J*=13.6 Hz, 1 H), 2.96-3.04 (m, 1 H), 2.83-2.89 (m, 1 H), 2.70-2.78 (m, 1 H), 2.55-2.66 (m, 1 H), 2.38-2.44 (m, 1 H), 2.14-2.31 (m, 2 H), 1.60-1.84 (m, 4 H). $^{19}$F NMR:(377 MHz, DMSO-d6): δ -48.85, -63.63.

**Example 19: (*S*)-2-((4-(6-((benzo[d][1,3]dioxol-4-yl-2,2-d2)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (Compound 19)**

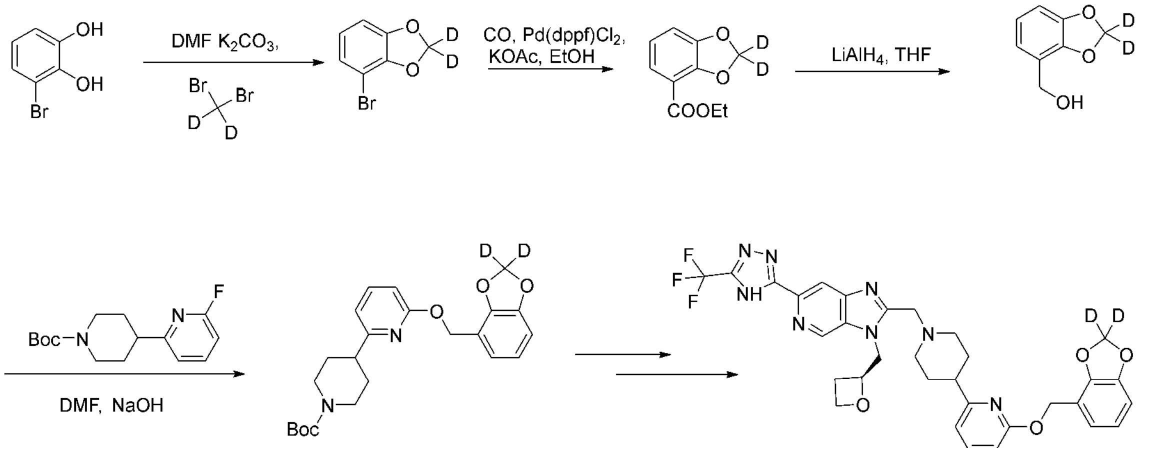


*Step A: 4-Bromobenzo[d][1,3]dioxole-2,2-d2*

To a solution of 3-bromobenzene-1,2-diol (1.0 g, 5.3 mmol) in DMF (15 mL) was added dibromomethane-d2 (1.8

g, 10.6 mmol) and $K_2CO_3$ (2.2 g, 15.9 mmol) at room temperature. The reaction was stirred at 110°C for 2 hours. After the reaction was completed, the mixture was quenched with $H_2O$ (100 mL) and extracted with ethyl acetate (60 mL x 3). The organic layer was combined and washed with brine (50 mL x 2), dried over sodium sulfate, filtered and concentrated in vacuum. The reaction was purified by column chromatography (PE/EA=1/100) to give 4-bromobenzo[d][1,3]dioxole-2,2-d2 (829 mg, yield: 77%).

*Step B: Ethyl benzo[d][1,3]dioxole-4-carboxylate-2,2-d2*

To a mixture of 4-bromobenzo[d][1 ,3]dioxole-2,2-d2 (829 mg, 4.1 mmol) in EtOH (12 mL) was added $PdCl_2$(dppf) (300 mg, 0.41 mmol) and KOAc (1.0 g, 10.2 mmol) at room temperature. The mixture was stirred at 75°C for 2 hours under CO. After the reaction was completed, the mixture was quenched with $H_2O$ (50 mL) and extracted with ethyl acetate (50 mL x 3). The organic layer was combined and washed with brine (30 mL x 2), dried over sodium sulfate, filtered and concentrated in vacuum. The reaction was purified by column chromatography (PE/EA=10/1) to give ethyl benzo[d][1,3] dioxole-4-carboxylate-2,2-d2 (660 mg, yield: 81.6%) as a white solid. MS Calcd.: 196.1; MS Found: 197.1 [M+H]+.

*Step C: (Benzo[d][1,3]dioxol-4-yl-2,2-d2)methanol*

To a solution of ethyl benzo[d][1,3]dioxole-4-carboxylate-2,2-d2 (560 mg, 2.86 mmol) in THF (10 mL) was added $LiAlH_4$ (130 mg, 3.42 mmol) at room temperature. The solution was stirred at room temperature for 1.5 hours. After the reaction was completed, the mixture was added EA (20 mL) and $H_2O$ (0.15 mL), stirred at room temperature for 10 min, then add with NaOH solution (2 M, 0.15 mL) and $H_2O$ (0.35 ml). The resulting mixture was stirred at room temperature for 15 min. Finally, the mixture was dried over sodium sulfate, stirred at room temperature for 15 min filtered and concentrated in vacuum. The reaction was purified by column chromatography (PE/EA=4/1) to give (benzo[d][1,3]dioxol-4-yl-2,2-d2) methanol (366 mg, yield: 83%).

*Step D: tert-Butyl 4-(6-((benzo[d][1,3]dioxol-4-yl-2,2-d2)methoxy)pyridin-2-yl)piperidine-1-carboxylate*

A mixture of (benzo[d][1,3]dioxol-4-yl-2,2-d2)methanol (366 mg, 2.38 mmol), tert-butyl 4-(6-fluoropyridin-2-yl) piperidine-1-carboxylate (560 mg, 2.00 mmol), NaOH (160 mg, 4.00 mmol) in DMF (10.0 mL) at room temperature and the mixture was stirred at 70°C for 4 hours. After the reaction was completed, the mixture was quenched with $H_2O$ (60 mL) and extracted with ethyl acetate (40 mL x 3). The organic layer was combined and washed with brine (30 mL x 2), dried over sodium sulfate, filtered and concentrated in vacuum. The reaction was purified by column chromatography (PE/EA=97/4) to give tert-butyl 4-(6-((benzo[d][1,3]dioxol-4-yl-2,2-d2)methoxy)pyridin-2-yl)piperidine-1-carboxylate (550 mg, yield: 56%). MS Calcd.: 414.2; MS Found: 415.3 [M+H]+.

*Step E: (S)-2-((4-(6-((benzo[d][1,3]dioxol-4-yl-2,2-d2)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine*

(S)-2-((4-(6-((benzo[d][1,3]dioxol-4-yl-2,2-d2)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (61.3 mg) was obtained with the similar method of Example 1. MS Calcd.: 650.3; MS Found: 651.4 [M+H]+.

1H NMR (400 MHz, DMSO-d6) δ 9.16 (s, 1 H), 8.29 (s, 1 H), 7.62 (t, *J*=5.6 Hz, 1 H), 6.92-6.98 (m, 1 H), 6.78-6.90 (m, 3H), 6.65 (d, *J*=8.0 Hz, 1 H), 5.30 (s, 2 H), 5.11-5.23 (m, 1 H), 4.86-4.97 (m, 1 H), 4.74-4.78 (m, 1 H), 4.36-4.53 (m, 2 H), 4.01 (d, *J*=13.2 Hz, 1 H), 3.86 (d, *J*=13.2 Hz, 1 H), 2.98-3.08 (m, 1 H), 2.82-2.93 (m, 1 H), 2.68-2.78 (m, 1 H), 2.57-2.67 (m, 1 H), 2.14-2.37 (m, 3 H), 1.63-1.91 (m, 4 H). $^{19}F$ NMR (377 MHz, DMSO-d6): δ -63.64.

**Example 20: (S)-2-(((6-(1-((3-(oxetan-2-ylmethyl)-6-(5-(trifluoromethyl)-4H-1,2,4-triazol-3-yl)-3H-imidazo[4,5-c] pyridin-2-yl)methyl)piperidin-4-yl)pyridin-2-yl)oxy)methyl)benzo[d]thiazole (Compound 20)**

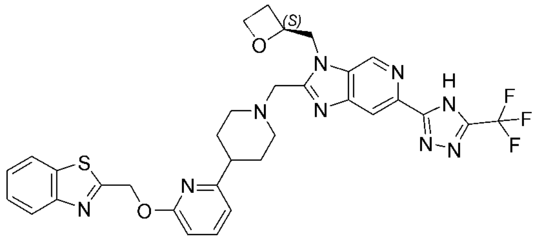

Starting from benzo[d]thiazol-2-ylmethanol, (S)-2-(((6-(1-((3-{oxetan-2-ylmethyl)-6-(5-(trifluoromethyl)-4H-1,2,4-triazol-3-yl)-3H-imidazo[4,5-c]pyridin-2-yl)methyl)piperidin-4-yl)pyridin-2-yl)oxy)methyl)benzo[d]thiazole was obtained with the similar method of Example 3. MS Calcd.: 661.22; MS Found: 662.2 [M+H]$^+$.

$^1$H NMR (400 MHz, DMSO-d6): δ 9.15 (s, 1 H), 8.27 (s, 1 H), 8.06 (d, *J*=7.6 Hz, 1 H), 7.98 (d, *J*=8.0 Hz, 1 H), 7.67-7.75 (m, 1 H), 7.45-7.51 (m, 1 H), 7.38-7.44 (m, 1 H), 6.94 (d, *J*=7.6 Hz, 1 H), 6.81 (d, *J*=8.0 Hz, 1 H), 5.81 (s, 2 H), 5.11-5.21 (m, 1 H), 4.84-4.96 (m, 1 H), 4.68-4.77 (m, 1 H), 4.36-4.50 (m, 2 H), 3.98 (d, J=13.6 Hz, 1 H), 3.82 (d, *J*=13.6 Hz, 1 H), 2.94-3.01 (m, 1 H), 2.82-2.85 (m, 1 H), 2.66-2.75 (m, 1 H), 2.56-2.63 (m, 1 H), 2.37-2.46 (m, 1 H), 2.12-2.29 (m, 2 H), 1.59-1.85 (m, 4 H). $^{19}$F NMR(377 MHz, DMSO-d6): δ -63.54.

**Example 21: 3-methyl-6-{[(6-{1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl}pyridin-2-yl)oxy]methyl}pyridazine (Compound 21)**

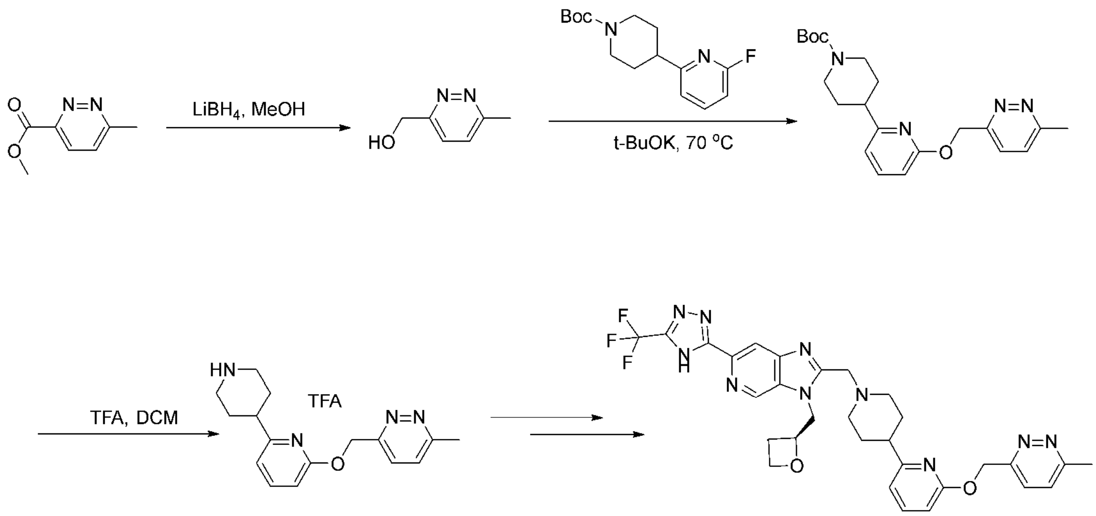


*Step A: (6-methylpyridazin-3-yl)methanol*

To a solution of methyl 6-methylpyridazine-3-carboxylate (500 mg, 3.29 mmol) in MeOH (10 mL) was added $LiBH_4$ (217 mg, 9.87 mmol) slowly. The mixture was stirred at room temperature for 2 hours. Upon completion, the reaction mixture was quenched with water (1 mL) and filtrated. The filtrate was concentrated under reduced pressure. The mixture was purified by column chromatography on silica gel (DCM/MeOH=12/1) to give (6-methylpyridazin-3-yl)methanol (350 mg, 86.0% yield). MS Calcd.: 124.1. MS Found: 125.2[M+H]$^+$.

*Step B: tert-butyl 4-(6-((6-methylpyridazin-3-yl)methoxy)pyridin-2-vl)piperidine-1-carboxylate*

A mixture of (6-methylpyridazin-3-yl)methanol (350 mg, 2.82mmol), tert-butyl 4-(6-fluoropyridin-2-yl)piperidine-1-carboxylate (541 mg, 2.82 mmol) and t-BuOK (1.9 g, 16.9 mmol) in THF (10 mL) was stirred at 70 °C for 5 hours. After the reaction was completed, the mixture was diluted with EA (20 mL), washed with $H_2O$ (10 mL) and extracted with EA (50 mL x 2). The combined organic layer was washed by brine (20 mL x 2) and dried over anhydrous sodium sulfate, filtered and evaporated to dryness. The residue was purified by column chromatography on silica gel (PE/EA=3/1) to give tert-butyl 4-(6-((6-methylpyridazin-3-yl)methoxy)pyridin-2-yl)piperidine-1-carboxylate (600 mg, 55.4% yield). MS Calcd.: 384.2; MS Found: 385.3[M+H]$^+$.

*Step C: 3-methyl-6-(((6-(piperidin-4-yl)pyridin-2-yl)oxy)methyl)pyridazine TFA salt*

A solution of tert-butyl 4-(6-((6-methylpyridazin-3-yl)methoxy)pyridin-2-yl)piperidine-1-carboxylate (222 mg, 0.577 mmol), TFA (0.1 mL) in DCM (2 mL) was stirred at room temperature for 2 hours. Upon completion, the reaction mixture was concentrated to give 3-methyl-6-(((6-(piperidin-4-yl)pyridin-2-yl)oxy)methyl)pyridazine TFA salt (250 mg, crude). MS Calcd.: 284.2. MS Found: 285.2[M+H]$^+$.

*Step D: 3-methyl-6-{[(6-{1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl}pyridin-2-yl)oxy]methyl)pyridazine*

3-methyl-6-{[(6-{1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl}pyridin-2-yl)oxy]methyl}pyridazine (Compound 21) (67.6 mg) was obtained with the similar method of Example 1. MS Calcd.: 620.3; MS Found: 621.4[M+H]$^+$.

1H NMR (400 MHz, DMSO-d6): δ 9.31 (s, 1 H), 8.40 (s, 1 H), 7.73 (t, *J*=8.0 Hz, 1 H), 7.66 (d, *J*=8.4 Hz, 1 H), 7.59 (d, *J*=8.8 Hz, 1 H), 6.92-7.00 (m, 1 H), 6.82 (d, *J*=8.0 Hz, 1 H), 5.63 (s, 2 H), 5.08-5.17 (m, 1 H), 4.84-4.99 (m. 3 H), 4.73-4.82 (m, 1 H), 4.48-4.56 (m, 1 H), 4.36-4.46 (m, 1 H), 3.65-3.86 (m, 2 H), 3.21-3.41 (m, 2 H), 2.82-3.05 (m, 1 H), 2.70-2.81 (m, 1 H), 2.62 (s, 3 H), 2.30-2.43 (m, 1 H), 2.00-2.15 (m, 4 H). $^{19}$F NMR(377 MHz, DMSO-d6): δ -63.71.

**Example 22: (S)-2-((4-(6-((1-cyclopropyl-4-fluoro-1H-pyrazol-3-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (Compound 22)**

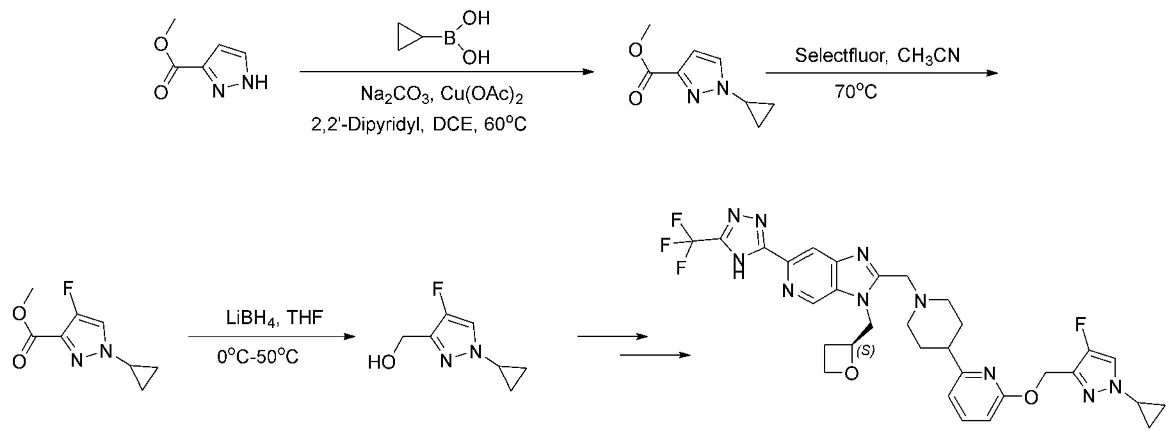


*Step A: Methyl 1-cyclopropyl-1H-pyrazole-3-carboxylate*

A mixture of methyl 1H-pyrazole-3-carboxylate (2.0 g, 15.9 mmol), cyclopropylboronic acid (2.7 g, 31.7 mmol), $Na_2CO_3$ (3.4 g, 31.8 mmol), $Cu(OAc)_2$ (2.9 g, 15.9 mmol) and 2,2'-Dipyridyl (2.5 g, 15.9 mmol) in DCE (30 mL) was stirred at 60°C for 16 hours. The reaction mixture was cooled to room temperature, quenched with $H_2O$ (60 mL), extracted with EA (3 x 40 mL). The combined organic layers were washed with brine (40 mL). The organic layer was dried over $Na_2SO_4$, filtered and concentrated under reduced pressure. The residue was purified by silica gel column chromatography, eluted with PE/EA (3/1) to give methyl 1-cyclopropyl-1H-pyrazole-3-carboxylate (1.7 g, yield: 64.9%). MS Calcd.: 166.1; MS Found: 167.1 [M+H]+.

*Step B: methyl 1-cyclopropyl-4-fluoro-1H-pyrazole-3-carboxylate*

A mixture of methyl 1-cyclopropyl-1H-pyrazole-3-carboxylate (1.5 g, 9.0 mmol) and (9.6 g, 27.1 mmol) in $CH_3CN$ (20 mL) was stirred at 70°C for 36 hours under $N_2$. The reaction mixture was cooled to room temperature. The mixture was quenched with $H_2O$ (50 mL), extracted with EA (3 x 30 mL). The combined organic layers were washed with brine (30 mL). The organic layer was dried over $Na_2SO_4$, filtered and concentrated under reduced pressure. The residue was purified by silica gel column chromatography, eluted with PE/EA (4/1) to give methyl 1-cyclopropyl-4-fluoro-1H-pyrazole-3-carboxylate (1.5 g, yield: 90.3%).

*Step C: (1-Cyclopropyl-4-fluoro-1H-pyrazol-3-yl)methanol*

To a solution of methyl 1-cyclopropyl-4-fluoro-1H-pyrazole-3-carboxylate (1.11 g, 6.03 mmol) in THF (15 mL) was added $LiBH_4$ (0.265 g, 12.06 mmol) at 0°C. The mixture was stirred at 50°C for 5 hours. The reaction mixture was cooled to room temperature, quenched with $H_2O$ (50 mL), extracted with EA (3 x 30 mL). The combined organic layers were washed with brine (30 mL). The organic layer was dried over $Na_2SO_4$, filtered and concentrated under reduced pressure. The residue was purified by silica gel column chromatography, eluted with PE/EA (4/1) to give (1-cyclopropyl-4-fluoro-1H-pyrazol-3-yl)methanol (510 mg, yield: 54.2%).

*Step D: (S)-2-((4-(6-((1-cyclopropyl-4-fluoro-1H-pyrazol-3-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine*

(S)-2-((4-(6-((1-cyclopropyl-4-fluoro-1H-pyrazol-3-yl)methoxy)pyridin-2-yl)piperidin-1-yl)methyl)-3-(oxetan-2-ylmethyl)-6-(5-(trifluoromethyl)-4H-1,2,4-triazol-3-yl)-3H-imidazo[4,5-c]pyridine (30.8 mg) was obtained with the similar method of Compound 3. MS Calcd.: 652.26; MS Found: 653.0 [M+H]+.

$^1H$ NMR (400 MHz, DMSO-d6) : δ 9.15 (s, 1 H), 8.28 (s, 1 H), 7.91 (d, *J*=4.4 Hz, 1 H), 7.61 (t, *J*=7.6 Hz, 1 H), 6.87 (d, *J*=7.6 Hz, 1 H), 6.62 (d, *J*=8.0 Hz, 1 H), 5.25 (s, 2 H), 5.15-5.22 (m, 1 H), 4.90-4.96 (m, 1 H), 4.73-4.79 (m, 1 H), 4.47-4.53 (m, 1 H), 4.40-4.45 (m, 1 H), 4.01 (d, *J*=13.6 Hz, 1 H), 3.86 (d, *J*=13.6 Hz, 1 H), 3.62-3.68 (m, 1 H), 2.99-3.09 (m, 1 H), 2.84-2.94 (m, 1 H), 2.71-2.79 (m, 1 H), 2.59-2.65 (m, 1 H), 2.44-2.48 (m, 1 H), 2.16-2.35 (m, 2 H), 1.71-1.90 (m, 4 H), 0.96-1.03 (m, 2 H), 0.86-0.94(m, 2 H). $^{19}F$ NMR(377 MHz, DMSO-d6) : δ -63.53, -176.75.

**Example 23: 2-((4-((S)-2-(4-chloro-2-fluorophenyl)-2-methylbenzo[d][1,3]dioxol-4-yl)piperidin-1-yl) methyl)-3-(((S)-oxetan-2-yl)methyl)-3H-imidazo[4,5-c]pyridine-6-carboxylic acid (Compound** 23)

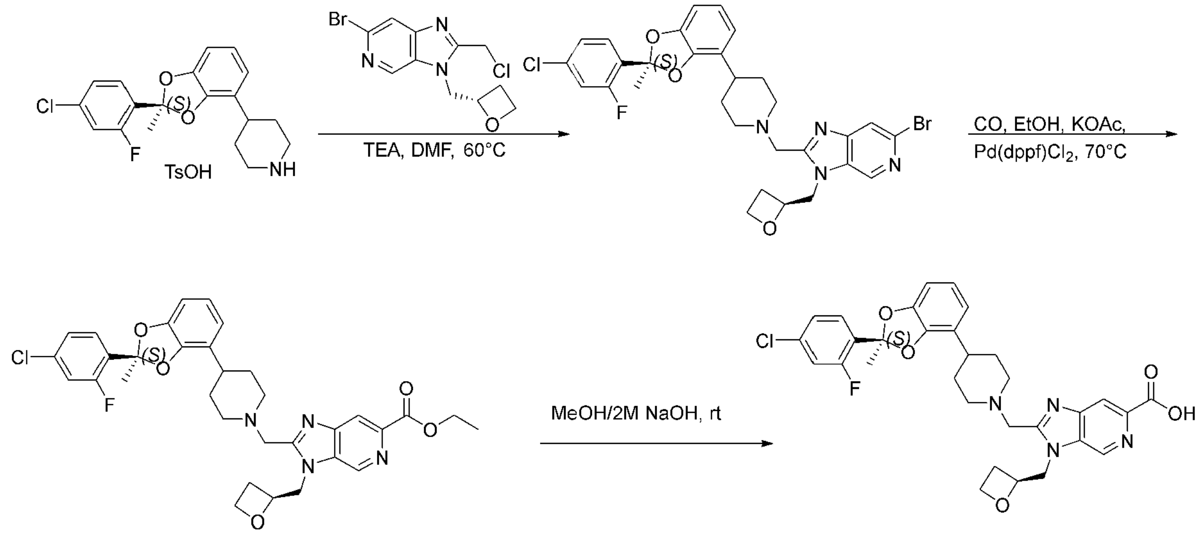


*Step A: 6-Bromo-2-((4-((S)-2-(4-chloro-2-fluorophenyl)-2-methylbenzo[d][1,3]dioxol-4-yl)piperidin-1-yl) methyl)-3-(((S)-oxetan-2-yl)methyl)-3H-imidazo[4,5-c]pyridine*

To a solution of (S)-4-(2-(4-chloro-2-fluorophenyl)-2-methylbenzo[d][1,3]dioxol-4-yl)piperidine TsOH salt (545 mg, 1.0 mmol) in DMF (10 mL) was added TEA (377 mg, 3.1 mmol). After stirring 2 minutes, (S)-6-bromo-2-(chloromethyl)-3-(oxetan-2-ylmethyl)-3H-imidazo[4,5-c]pyridine (332 mg, 1.0 mmol) was added. The resulting mixture was stirred at 60 °C for 2 hours. After the reaction was completed, the mixture was diluted with EA (100 mL), washed with $H_2O$ (2 x 50 mL). The organic layer was dried over anhydrous sodium sulfate, filtered and evaporated to dryness. The residue was purified by column chromatography on silica gel (DCM/MeOH=10/1) to give 6-bromo-2-((4-((S)-2-(4-chloro-2-fluorophenyl)-2-methylbenzo[d][1,3]dioxol-4-yl)piperidin-1-yl)methyl)-3-(((S)-oxetan-2-yl)methyl)-3H-imidazo[4,5-c]pyridine (535 mg, yield: 81.6%). MS Calcd.: 626.1; MS Found: 627.1 $[M+H]^+$.

*Step B: Ethyl 2-((4-((S)-2-(4-chloro-2-fluorophenyl)-2-methylbenzo[d][1,3]dioxol-4-yl)piperidin-1-yl)methyl)-3-(((S)-oxetan-2-yl)methyl)-3H-imidazo[4,5-c]pyridine-6-carboxylate*

To a mixture of 6-bromo-2-((4-((S)-2-(4-chloro-2-fluorophenyl)-2-methylbenzo[d][1,3]dioxol-4-yl)piperidin-1-yl)methyl)-3-(((S)-oxetan-2-yl)methyl)-3H-imidazo[4,5-c]pyridine (535 mg, 0.85 mmol) in EtOH (10 mL) was added Pd(dppf)$Cl_2$ (125 mg, 0.17 mmol) and KOAc (250 mg, 2.56 mmol). The mixture was stirred at 70°C for 16 hours under an atmosphere of CO (balloon). The solvent was removed in vacuo and the residue was purified by column chromatography on silica gel (DCM/MeOH=20/1) to give ethyl 2-((4-((S)-2-(4-chloro-2-fluorophenyl)-2-methylbenzo[d][1,3]dioxol-4-yl)piperidin-1-yl)methyl)-3-(((S)-oxetan-2-yl)methyl)-3H-imidazo[4,5-c]pyridine-6-carboxylate (400 mg, yield: 75.6%). MS Calcd.: 620.2; MS Found: 620.9 $[M+H]^+$.

*Step C: 2-((4-((S)-2-(4-chloro-2-fluorophenyl)-2-methylbenzo[d][1,3]dioxol-4-yl)piperidin-1-yl)methyl)-3-(((S)-oxetan-2-yl)methyl)-3H-imidazo[4,5-c]pyridine-6-carboxylic acid*

To a solution of ethyl 2-((4-((S)-2-(4-chloro-2-fluorophenyl)-2-methylbenzo[d][1,3]dioxol-4-yl)piperidin-1-yl)methyl)-3-(((S)-oxetan-2-yl)methyl)-3H-imidazo[4,5-c]pyridine-6-carboxylate (400 mg, 0.64 mmol) in MeOH (6 mL) was added NaOH (2 mL, 2 M solution in water). The mixture was stirred at room temperature for 2 hours. The reaction mixture was diluted with water (5 mL), then the mixture was adjusted to pH 5-6 with 1 N HCl (aq.). The solid was collected, dried to give 140 mg of crude product as a white solid. 40 mg of crude product was purified by Prep-HPLC to give 2-((4-((S)-2-(4-chloro-2-fluorophenyl)-2-methylbenzo[d][1,3]dioxol-4-yl)piperidin-1-yl)methyl)-3-(((S)-oxetan-2-yl)methyl)-3H-imidazo[4,5-c]pyridine-6-carboxylic acid (6.3 mg, yield: 16.5%). MS Calcd.: 592.2; MS Found: 593.0 [M+H]+.

$^1H$ NMR (400 MHz, DMSO-d6) δ 9.09 (s, 1 H), 8.27 (s, 1 H), 7.49-7.59 (m, 2 H), 7.29-7.34 (m, 1 H), 6.69-6.80 (m, 3 H), 5.13-5.18 (m, 1 H), 4.86-4.92 (m, 1 H), 4.72-4.75 (m, 1 H), 4.46-4.51 (m, 1 H), 4.38-4.44 (m, 1 H), 3.99-4.02 (m, 1 H), 3.83-3.86 (m, 1 H), 2.97-3.01 (m, 1 H), 2.85-2.88 (m, 1 H), 2.63-2.74 (m, 2 H), 2.21-2.27 (m, 2 H), 1.98-2.02 (m, 4 H), 1.65-1.77 (m, 4 H). $^{19}F$ NMR (377 MHz, DMSO-d6): δ -110.78.

**Example 24: 2-((4-((S)-2-(4-chloro-2-fluorophenyl)-2-methylbenzo[d][1,3]dioxol-4-yl)piperidin-1-yl)methyl)-N-methyl-3-(((S)-oxetan-2-yl)methyl)-3H-imidazo[4,5-c]pyridine-6-carboxamide (Compound 24)**

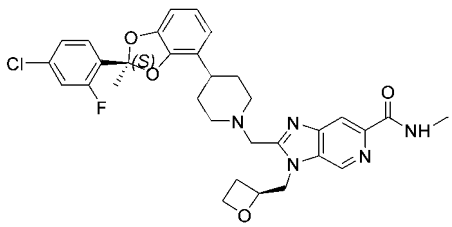

A mixture of (S)-2-((4-(2-(4-chloro-2-fluorophenyl)-2-methylbenzo[d][1,3]dioxol-4-yl)piperidin-1-yl)methyl)-3-(cyclobutylmethyl)-3H-imidazo[4,5-c]pyridine-6-carboxylic acid (50 mg, 0.08 mmol), methanamine (8 mg, 0.13 mmol), HATU (32 mg, 0.13 mmol), DIEA (48 mg, 0.38 mmol) in DMF (1.5 mL) was stirred at room temperature for an hour. After the reaction was completed, the mixture was purified directly by Prep-HPLC to give 2-((4-((S)-2-(4-chloro-2-fluorophenyl)-2-methylbenzo[d][1,3]dioxol-4-yl)piperidin-1-yl)methyl)-N-methyl-3-(((S)-oxetan-2-yl)methyl)-3H-imidazo[4,5-c]pyridine-6-carboxamide (15.93 mg, yield: 31.2%). MS Calcd.: 605.2; MS Found: 606.1 [M+H]+.

$^1H$ NMR (400 MHz, DMSO-d6) δ 9.02 (s, 1 H), 8.71 (q, *J*=4.8 Hz, 1 H), 8.21 (s, 1 H), 7.53-7.59 (m, 2 H), 7.33 (dd, *J*=8.4 Hz, *J*=1.6 Hz, 1 H), 6.73-6.80 (m, 3 H), 5.13-5.19 (m, 1 H), 4.88-4.92 (m, 1 H), 4.73 (dd, *J*=15.2 Hz, *J*=2.8 Hz, 1 H), 4.47-4.52 (m, 1 H), 4.38-4.43 (m, 1 H), 3.99 (d, *J*=13.6 Hz, 1 H), 3.83 (d, *J*=13.6 Hz, 1 H), 3.02-3.04 (m, 1 H), 2.84-2.86 (m, 4 H), 2.62-2.76 (m, 2 H), 2.41-2.48 (m, 1 H), 2.19-2.31 (m, 2 H), 2.03 (s, 3 H), 1.65-1.80 (m, 4 H). $^{19}F$ NMR (377 MHz, DMSO-d6): δ -110.79.

**Example 25: 2-((4-((S)-2-(4-chloro-2-fluorophenyl)-2-methylbenzo[d][1,3]dioxol-4-yl)piperidin-1-yl)methyl)-3-(((S)-oxetan-2-yl)methyl)-N-(2,2,2-trifluoroethyl)-3H-imidazo[4,5-c]pyridine-6-carboxamide (Compound 25)**

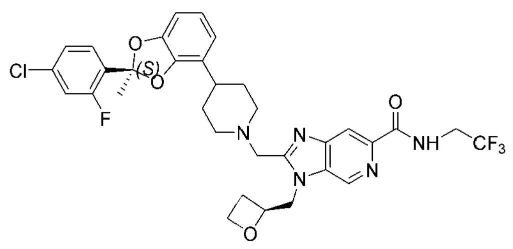

A mixture of 2-((4-((S)-2-(4-chloro-2-fluorophenyl)-2-methylbenzo[d][1,3]dioxol-4-yl)piperidin-1-yl) methyl)-3-(((S)-oxetan-2-yl)methyl)-3H-imidazo[4,5-c]pyridine-6-carboxylic acid (50 mg, 0.08 mmol), 2,2,2-trifluoroethan-1-amine (12 mg, 0.13 mmol), HATU (48 mg, 0.13 mmol), DIEA (16 mg, 0.13 mmol) in DMF (1.5 mL) was stirred at room temperature for an hour. After the reaction was completed, the mixture was purified directly by Prep-HPLC to give 2-((4-((S)-2-(4-chloro-2-fluorophenyl)-2-methylbenzo[d][1,3]dioxol-4-yl)piperidin-1-yl)methyl)-3-(((S)-oxetan-2-yl) methyl)-N-(2,2,2-trifluoroethyl)-3H-imidazo[4,5-c]pyridine-6-carboxamide (24.3 mg, yield: 42.8%). MS Calcd.: 673.21; MS Found: 674.1 $[M+H]^+$.

$^1$H NMR (400 MHz, DMSO-d6) δ 9.33 (t, *J*=6.4 Hz, 1 H), 9.22 (s, 1 H), 8.39 (s, 1 H), 7.56-7.64 (m, 2 H), 7.35 (dd, *J*=8.4 Hz, *J*=1.6 Hz, 1 H), 6.86 (d, *J*=4.0 Hz, 2 H), 6.73-6.81 (m, 1 H), 5.05-5.15 (m, 1 H), 4.84-4.96 (m, 3 H), 4.73-4.76 (m, 1 H), 4.47-4.55 (m, 1 H), 4.34-4.42 (m, 1 H), 4.08-4.19 (m, 2 H), 3.69-3.84 (m, 2 H), 3.29-3.41 (m, 2 H), 2.94-3.05 (m, 1 H), 2.71-2.80 (m, 1 H), 2.33-2.40 (m, 1 H), 1.98-2.20 (m, 7 H). $^{19}$F NMR (377 MHz, DMSO-d6): δ -70.35, -110.50.

**Example 26: 2-((4-((S)-2-(4-chloro-2-fluorophenyl)-2-methylbenzo[d][1,3]dioxol-4-yl)piperidin-1-yl) methyl)-N-(2-methoxyethyl)-3-(((S)-oxetan-2-yl)methyl)-3H-imidazo[4,5-c]pyridine-6-carboxamide (Compound 26)**

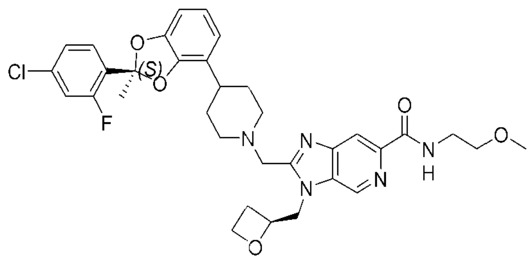

A mixture of 2-((4-((S)-2-(4-chloro-2-fluorophenyl)-2-methylbenzo[d][1,3]dioxol-4-yl)piperidin-1-yl) methyl)-3-(((S)-oxetan-2-yl)methyl)-3H-imidazo[4,5-c]pyridine-6-carboxylic acid (90 mg, 0.15 mmol), 2-methoxyethan-1-amine ( 23 mg. 0.30 mmol), $T_3P$ (95 mg, 0.30 mmol), DIEA (58 mg, 0.45 mmol) in DMF (1.5 mL) was stirred at room temperature for 2 hours. After the reaction was completed, the mixture was diluted with EA (30 mL), washed with $H_2O$ (2 x 20 mL). The combined organic layer was dried over anhydrous sodium sulfate, filtered and evaporated to dryness, the mixture was purified directly by Prep-HPLC to give 2-((4-((S)-2-(4-chloro-2-fluorophenyl)-2-methylbenzo[d][1,3] dioxol-4-yl)piperidin-1-yl)methyl)-N-(2-methoxyethyl)-3-(((S)-oxetan-2-yl)methyl)-3H-imidazo[4,5-c]pyridine-6-carboxamide (34.3 mg, yield: 62.5%). MS Calcd.: 649.25; MS Found: 650.0 $[M+H]^+$.

$^1$H NMR (400 MHz, DMSO-d6) δ 9.17 (d, *J*=0.8 Hz, 1 H), 8.69-8.75 (m, 1 H), 8.34 (d, *J*=0.8 Hz, 1 H), 7.56-7.64 (m, 2 H), 7.35 (dd, *J*=8.4 Hz, 2.0 Hz, 1 H), 6.86-6.87 (m, 2 H), 6.74-6.80 (m, 1 H), 5.05-5.14 (m, 1 H), 4.84-4.96 (m, 3 H), 4.73-4.77 (m, 1 H), 4.48-4.53 (m, 1 H), 4.35-4.40 (m, 1 H), 3.61-3.77 (m, 6 H), 3.49-3.52 (m, 3 H), 3.31-3.42 (m, 2 H), 2.95-3.06 (m, 1 H), 2.71-2.79 (m, 1 H), 2.31-2.39 (m, 1 H), 1.96-2.21 (m, 7 H). $^{19}$F NMR (377 MHz, DMSO-d6): δ -110.50.

**Example 27: 2-((4-((S)-2-(4-chloro-2-fluorophenyl)-2-methylbenzo[d][1,3]dioxol-4-yl)piperidin-1-yl)methyl)-4-fluoro-1-(((S)-oxetan-2-yl)methyl)-1H-benzo[d]imidazole-5-carboxylic acid (Compound 27)**

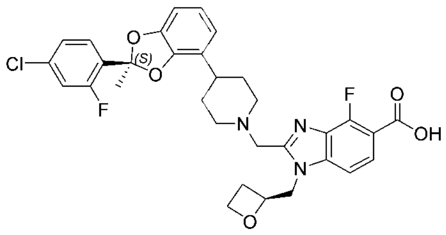

Starting from (S)-4-(2-(4-chloro-2-fluorophenyl)-2-methylbenzo[d][1,3]dioxol-4-yl)piperidine TsOH salt and (S)-5-bromo-2-(chloromethyl)-4-fluoro-1-(oxetan-2-ylmethyl)-1H-benzo[d]imidazole

2-((4-((S)-2-(4-chloro-2-fluorophenyl)-2-methylbenzo[d][1,3]dioxol-4-yl)piperidin-1-yl)methyl)-4-fluoro-1-(((S)-oxetan-2-yl)methyl)-1H-benzo[d]imidazole-5-carboxylic acid was obtained with the similar method of Example 23. MS Calcd.: 609.2; MS Found: 610.0 [M+H]+.

$^1$H NMR (400 MHz, DMSO-d6) δ: 7.69-7.76 (m, 1 H), 7.50-7.59 (m, 3 H), 7.33 (dd, *J*=8.4 Hz, 2.0 Hz, 1 H), 6.72-6.82 (m, 3 H), 5.08-5.16 (m, 1 H), 4.73-4.79 (m, 1 H), 4.59-4.66 (m, 1 H), 4.35-4.50 (m, 2 H), 3.95 (d, *J*=13.6 Hz, 1 H), 3.79 (d, *J*=13.2 Hz, 1 H), 2.97-3.05 (m, 1 H), 2.82-2.90 (m, 1 H), 2.61-2.74 (m, 2 H), 2.39-2.46 (m, 1 H), 2.14-2.30 (m, 2 H), 2.03 (s, 1 H), 1.65-1.83 (m, 4 H). $^{19}$F NMR (377 MHz, DMSO-d6): δ -110.79, -124.83.

**Example 28: (S)-2-((4-(6-((4-chloro-2-fluorobenzyl)oxy)pyridin-2-yl)piperidin-1-yl)methyl)-3-(oxetan-2-ylmethyl)-3H-imidazo[4,5-c]pyridine-6-carboxylic acid (Compound 28)**

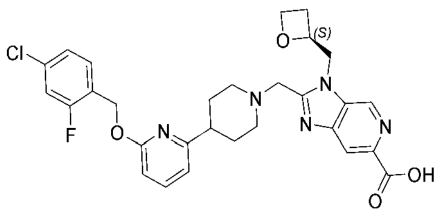

Starting from 2-((4-chloro-2-fluorobenzyl)oxy)-6-(piperidin-4-yl)pyridine TFA salt,

(S)-2-((4-(6-((4-chloro-2-fluorobenzyl)oxy)pyridin-2-yl)piperidin-1-yl)methyl)-3-(oxetan-2-ylmethyl)-3H-imidazo[4,5-c]pyridine-6-carboxylic acid was obtained with the similar method of Example 23. MS Calcd.: 565.2; MS Found: 566.2 [M+H]$^+$.

$^1$H NMR (400 MHz, DMSO-d6) δ 9.23 (s, 1 H), 8.39 (d, *J*=0.4 Hz, 1 H), 7.70 (t, *J*=7.6 Hz, 1 H), 7.60 (t, *J*=8.0 Hz, 1 H), 7.49 (dd, *J*=10.0 Hz, 2.0 Hz, 1 H), 7.33 (dd, *J*=8.4 Hz, 2.0 Hz, 1 H), 6.92-6.97 (m, 1 H), 6.75 (d, *J*=8.4 Hz, 1 H), 5.41 (s, 2 H), 5.06-5.12 (m, 1 H), 4.85-4.96 (m, 3 H), 4.71-4.79 (m, 1 H), 4.48-4.54 (m, 1 H), 4.35-4.41 (m, 1 H), 3.75-3.82 (m, 2 H), 3.22-3.36 (m, 2 H), 2.89-3.01 (m, 1 H), 2.69-2.79 (m, 1 H), 2.31-2.39 (m, 1 H), 2.02-2.19 (m, 4 H). $^{19}$F NMR (377 MHz, DMSO-d6): δ - 115.20.

**Example 29: (S)-2-((6-((4-chloro-2-fluorobenzyl)oxy)-3',6'-dihydro-[2,4'-bipyridin]-1'(2'H)-yl)methyl)-3-(oxetan-2-ylmethyl)-3H-imidazo[4,5-c]pyridine-6-carboxylic acid (Compound 29)**

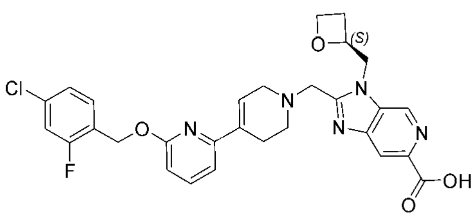

Starting from 6-((4-chloro-2-fluorobenzyl)oxy)-1',2',3',6'-tetrahydro-2,4'-bipyridine HCl salt, (S)-2-((6-((4-chloro-2-fluorobenzyl)oxy)-3',6'-dihydro-[2,4'-bipyridin]-1'(2'H)-yl)methyl)-3-(oxetan-2-ylmethyl)-3H-imidazo[4,5-c]pyridine-6-carboxylic acid was obtained with the similar method of Example 23. MS Calcd.: 563.2; MS Found: 564.0 [M+H]$^+$.

$^1$H NMR (400 MHz, DMSO-d6) δ 9.08 (s, 1 H), 8.28 (s, 1 H), 7.69 (t, *J*=7.6 Hz, 1 H), 7.55 (t, *J*=8.0 Hz, 1 H), 7.46 (dd,

J=10.0 Hz, 2.0 Hz, 1 H), 7.30 (dd, J=8.4 Hz, 1.6 Hz, 1 H), 7.09 (d, J=7.6 Hz, 1 H), 6.70-6.78 (m, 2 H), 5.40 (s, 2 H), 5.06-5.15 (m, 1 H), 4.84-4.94 (m, 1 H), 4.69-4.78 (m, 1 H), 4.45-4.51 (m, 1 H), 4.35-4.41 (m, 1 H), 4.11 (d, J=13.6 Hz, 1 H), 3.99 (d, J=13.6 Hz, 1 H), 3.18-3.26 (m, 4 H), 2.75-2.82 (m, 2 H), 2.64-2.70 (m, 1 H), 2.37-2.44 (m, 1 H). $^{19}F$ NMR (377 MHz, DMSO-d6): δ - 115.27.

**Example 30: (S)-2-((4-(3-((4-chloro-2-fluorobenzyl)oxy)-4-fluorophenyl)-3,6-dihydropyridin-1(2H)-yl)methyl)-3-(oxetan-2-ylmethyl)-3H-imidazo[4,5-c]pyridine-6-carboxylic acid (Compound 30)**

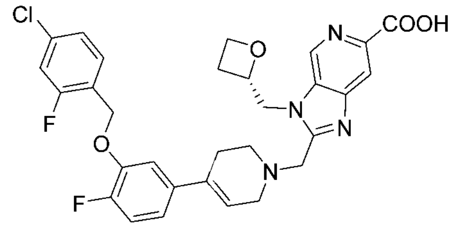

Starting from 4-(3-((4-chloro-2-fluorobenzyl)oxy)-4-fluorophenyl)-1,2,3,6-tetrahydropyridine TFA salt,

(S)-2-((4-(3-((4-chloro-2-fluorobenzyl)oxy)-4-fluorophenyl)-3,6-dihydropyridin-1(2H)-yl)methyl)-3-(oxetan-2-ylmethyl)-3H-imidazo[4,5-c]pyridine-6-carboxylic acid was obtained with the similar method of Example 23. MS Calcd: 580.2; MS Found: 581.1 $[M+H]^+$

$^1H$ NMR (400 MHz, $CD_3OD-d_4$): δ 9.10 (s, 1 H), 8.51 (s, 1 H), 7.54 (t, J = 7.4 Hz, 1 H), 7.29 - 7.17 (m, 3 H), 7.12 - 7.02 (m, 2 H), 6.09 (s, 1 H), 5.28 - 5.16 (m, 3 H), 4.69 - 4.59 (m, 2 H), 4.52 - 4.39 (m, 4 H), 3.55 - 3.48 (m, 2 H), 3.21 - 3.01 (m, 3 H), 2.84 - 2.77 (m, 1 H), 2.70 - 2.63 (m, 1 H), 2.56 - 2.48 (m, 1 H). $^{19}F$ NMR (377 MHz, $CD_3OD-d_4$): δ (-117.54).

**Example 31: (S)-2-((4-(3-((4-chloro-2-methoxybenzyl)oxy)-4-fluorophenyl)-3,6-dihydropyridin-1(2H)-yl)methyl)-3-(oxetan-2-ylmethyl)-6-(5-(trifluoromethyl)-4H-1,2,4-triazol-3-yl)-3H-imidazo[4,5-c]pyridine (Compound 31)**

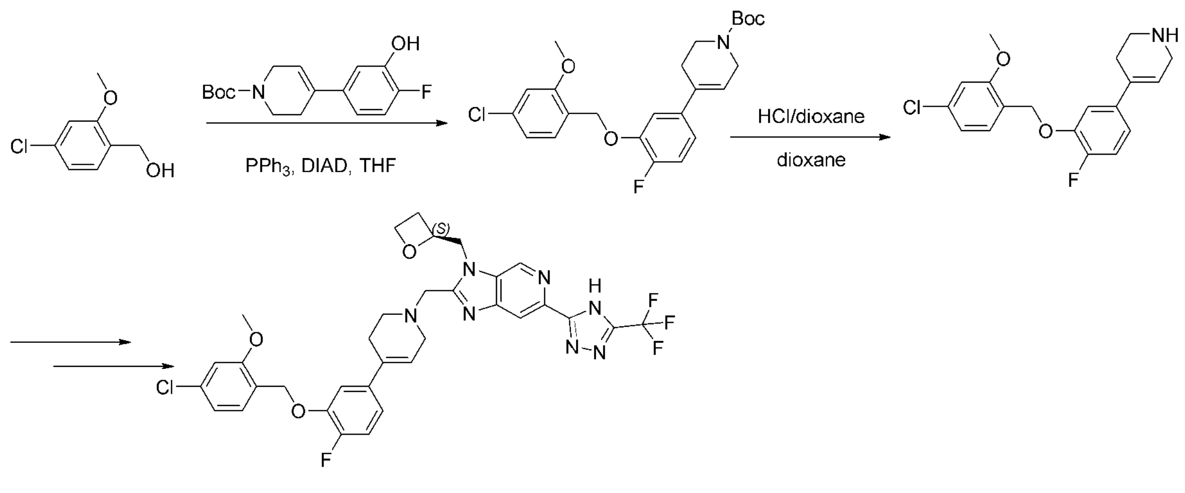


*Step A: tert-butyl 4-(3-((4-chloro-2-methoxybenzyl)oxy)-4-fluorophenyl)-3,6-dihydropyridine-1(2H)-carboxylate*

To a solution of (4-chloro-2-methoxyphenyl)methanol (100 mg, 0.58 mmol), tert-butyl 4-(4-fluoro-3-hydroxyphenyl)-3,6-dihydropyridine-1(2H)-carboxylate (170 mg, 0.58 mmol) in THF (5.0 ml) was added $PPh_3$ (183 mg, 0.70 mmol) and DIAD (178 mg, 0.88 mmol) at 0 °C, then the mixture was stirred at 0°C for 3 hours. After the reaction was completed, the mixture was quenched with $H_2O$ (40 mL) and extracted with ethyl acetate (30 mL x 3). The organic layer was combined and washed with brine (25 mL x 2), dried over sodium sulfate, filtered and concentrated in vacuum. The reaction was purified by column chromatography (PE/EA= 5/1) to give tert-butyl 4-(3-((4-chloro-2-methoxybenzyl)oxy)-4-fluorophenyl)-3,6-dihydropyridine-1(2H)-carboxylate (170 mg, yield: 65%). MS Calcd.:447.2 ; MS Found: 391.9$[M-56+H]^+$.

*Step B: 4-(3-((4-chloro-2-methoxybenzyl)oxy)-4-fluorophenyl)-1,2,3,6-tetrahydropyridine HCl salt*

To a solution of tert-butyl 4-(3-((4-chloro-2-fluoro-6-methoxybenzyl)oxy)-4-fluorophenyl)-3,6-dihydropyridine-1(2H)-carboxylate (150 mg, 0.34 mmol) in dioxane (2 mL) was added HCl /dioxane (4 M, 2 mL) at room temperature. The solution was stirred at room temperature for 5 hours. The mixture was concentrated under reduced pressure to give 4-(3-((4-chloro-2-methoxybenzyl)oxy)-4-fluorophenyl)-1,2,3,6-tetrahydropyridine HCl salt (168 mg, crude). MS Calcd.: 347.1; MS Found: 348.2[M+H]$^+$.

*Step C: (S)-2-((4-(3-((4-chloro-2-methoxybenzyl)oxy)-4-fluorophenyl)-3,6-dihydropyridin-1(2H)-yl)methyl)-3-(oxetan-2-ylmethyl)-6-(5-(trifluoromethyl)-4H-1,2,4-triazol-3-yl)-3H-imidazo[4,5-c]pyridine*

(S)-2-((4-(3-((4-chloro-2-methoxybenzyl)oxy)-4-fluorophenyl)-3,6-dihydropyridin-1(2H)-yl)methyl)-3-(oxetan-2-ylmethyl)-6-(5-(trifluoromethyl)-4H-1,2,4-triazol-3-yl)-3H-imidazo[4,5-c]pyridine (26.9 mg) was obtained with the similar method as described herein. MS Calcd.: 683.2; MS Found: 684.5 [M+H]+.

1H NMR (400 MHz, DMSO-d6) δ 9.16 (s, 1 H), 8.30 (s, 1 H), 7.42 (d, *J*=8.0 Hz, 1 H), 7.25 (dd, *J*=2.0 Hz, *J*=8.4 Hz, 1 H), 7.12-7.20 (m, 2 H), 7.03-7.09 (m, 1 H), 6.94-7.01 (m, 1 H), 6.12-6.16 (m, 1 H), 5.13 (s, 3 H), 4.88-4.97 (m, 1 H), 4.71-4.81 (m, 1 H), 4.46-4.53 (m, 1 H), 4.37-4.44 (m, 1 H), 4.11 (d, *J*=13.6 Hz, 1 H), 3.99 (d, *J*=13.6 Hz, 1 H), 3.84 (s, 3 H), 3.14-3.26 (m, 2 H), 2.63-2.83 (m, 3 H), 2.33-2.45 (m, 3 H). $^{19}$F NMR(377 MHz, DMSO-d6): δ -63.60, -136.52.

**Example 32: (S)-5-(2-((4-(3-((4-chloro-2-fluorobenzyl)oxy)-4-fluorophenyl)-3,6-dihydropyridin-1(2H)-yl)methyl)-3-(oxetan-2-ylmethyl)-3H-imidazo[4,5-c]pyridin-6-yl)-4H-1,2,4-triazole-3-carboxamide (Compound 32)**

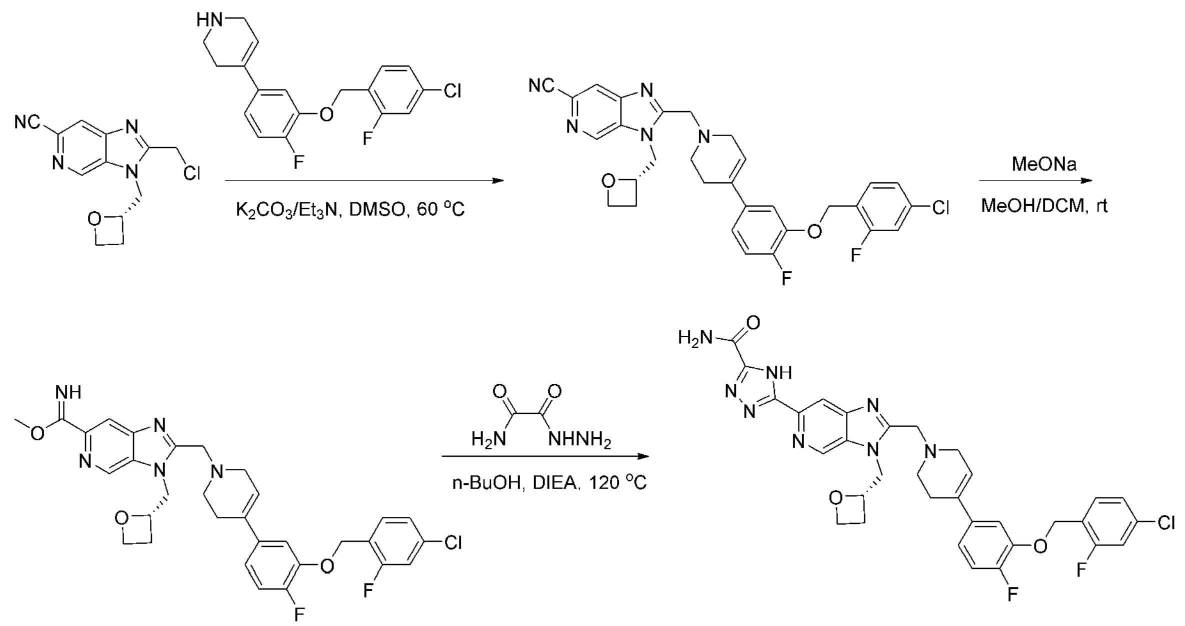


*Step A: (S)-2-((4-(3-((4-chloro-2-fluorobenzyl)oxy)-4-fluorophenyl)-3,6-dihydropyridin-1(2H)-yl)methyl)-3-(oxetan-2-ylmethyl)-3H-imidazo[4,5-c]pyridine-6-carbonitrile*

A mixture of (S)-2-(chloromethyl)-3-(oxetan-2-ylmethyl)-3H-imidazo[4,5-c]pyridine-6-carbonitrile (220 mg, 0.84 mmol), 4-(3-((4-chloro-2-fluorobenzyl)oxy)-4-fluorophenyl)-1,2,3,6-tetrahydropyridine 2,2,2-trifluoroacetic acid salt (378 mg, 0.84 mmol), TEA (0.35 mL, 2.52 mmol) and $K_2CO_3$ (348 mg, 2.52 mmol) in DMSO (3 mL) was stirred at 60 °C for 3 hours. The mixture was poured into water (50 mL) and extracted with DCM (2 x 50 mL), the combined organic layer was washed with brine, dried over sodium sulfate, filtered and the residue was concentrated to obtain (S)-2-((4-(3-((4-chloro-2-fluorobenzyl)oxy)-4-fluorophenyl)-3,6-dihydropyridin-1(2H)-yl)methyl)-3-(oxetan-2-ylmethyl)-3H-imidazo[4,5-c]pyridine-6-carbonitrile (480 mg). MS Calcd: 561.2; MS Found: 562.2 [M+H]$^+$

*Step B: methyl (S)-2-((4-(3-((4-chloro-2-fluorobenzyl)oxy)-4-fluorophenyl)-3,6-dihydropyridin-1(2H)-yl)methyl)-3-(oxetan-2-ylmethyl)-3H-imidazo[4,5-c]pyridine-6-carbimidate*

A mixture of (S)-2-((4-(3-((4-chloro-2-fluorobenzyl)oxy)-4-fluorophenyl)-3,6-dihydropyridin-1(2H)-yl)methyl)-3-(oxetan-2-ylmethyl)-3H-imidazo[4,5-c]pyridine-6-carbonitrile (240 mg, 0.428 mmol) and NaOMe (240 mg, 4.28 mmol) in DCM/MeOH (2 mL/2 mL) was stirred at room temperature for 16 h. The mixture was concentrated, and the residue was purified by column chromatography on silica gel to obtain methyl (S)-2-((4-(3-((4-chloro-2-fluorobenzyl)oxy)-4-fluorophenyl)-3,6-dihydropyridin-1(2H)-yl)methyl)-3-(oxetan-2-ylmethyl)-3H-imidazo[4,5-c]pyridine-6-carbimidate (105 mg, yield: 48.7%). MS Calcd: 593.2; MS Found: 594.2 $[M+H]^+$

*Step C: (S)-5-(2-((4-(3-((4-chloro-2-fluorobenzyl)oxy)-4-fluorophenyl)-3,6-dihydropyridin-1(2H)-yl)methyl)-3-(oxetan-2-ylmethyl)-3H-imidazo[4,5-c]pyridin-6-yl)-4H-1,2,4-triazole-3-carboxamide*

A mixture of methyl (S)-2-((4-(3-((4-chloro-2-fluorobenzyl)oxy)-4-fluorophenyl)-3,6-dihydropyridin-1(2H)-yl)methyl)-3-(oxetan-2-ylmethyl)-3H-imidazo[4,5-c]pyridine-6-carbimidate (105 mg, 0.18 mmol), 2-hydrazineyl-2-oxoacetamide (36 mg, 0.35 mmol) and DIEA (0.15 mL, 0.88 mmol) in n-BuOH (5 mL) was stirred at 120 °C for 16 h. The mixture was purified by prep-HPLC to obtain (S)-5-(2-((4-(3-((4-chloro-2-filuorobenzyl)oxy)-4-filuorophenyl)-3,6-dihydropyridin-1(2H)-yl)methyl)-3-(oxetan-2-ylmethyl)-3H-imidazo[4,5-c]pyridin-6-yl)-4H-1,2,4-triazole-3-carboxamide (47.5 mg, yield: 41.5%). MS Calcd: 646.2; MS Found: 647.2 $[M+H]^+$

$^1H$ NMR (400 MHz, $CD_3OD$-$d_4$): δ 9.12 (s, 1 H), 8.48 (s, 1 H), 7.54 (t, J = 8.0 Hz, 1 H), 7.27 - 7.24 (m, 2 H), 7.20 (d, J =9.2 Hz, 1 H), 7.09 - 7.02 (m, 2 H), 6.11 - 6.05 (m, 1 H), 5.31 - 5.25 (m, 1 H), 5.20 (s, 2 H), 4.99 - 4.93 (m, 1 H), 4.83 - 4.78 (m, 1 H), 4.69 - 4.63 (m, 1 H), 4.53 - 4.46 (m, 1 H), 4.18 (d, J = 14.0 Hz, 1 H), 4.05 (d, J = 14.0 Hz, 1 H), 3.29 - 3.19 (m, 2 H), 2.91 - 2.76 (m, 3 H), 2.59 - 2.53 (m, 3 H). $^{19}F$ NMR (377 MHz, $CD_3OD$-$d_4$): δ (-117.52), (-137.79).

**Example 33: (S)-5-(2-((4-(3-((4-chloro-2-fluorobenzyl)oxy)-4-fluorophenyl)-3,6-dihydropyridin-1(2H)-yl)methyl)-3-(oxetan-2-ylmethyl)-3H-imidazo[4,5-c]pyridin-6-yl)-4H-1,2,4-triazole-3-carbonitrile (Compound 33)**

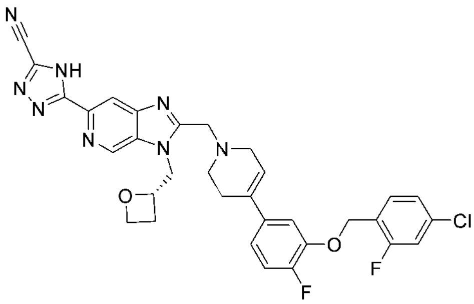

A mixture of (S)-5-(2-((4-(3-((4-chloro-2-fluorobenzyl)oxy)-4-fluorophenyl)-3,6-dihydropyridin-1(2H)-yl)methyl)-3-(oxetan-2-ylmethyl)-3H-imidazo[4,5-c]pyridin-6-yl)-4H-1 ,2,4-triazole-3-carboxamide (30 mg, 0.046 mmol) and burgess (300 mg) in DCM (3 mL) was stirred at room temperature for 2 h. The mixture was poured into water (30 mL) and extracted with DCM (2 x 30 mL), the combined organic layer was washed with brine. dried over sodium sulfate, filtered and the residue was concentrated. The residue was purified by prep-HPLC to obtain (S)-5-(2-((4-(3-((4-chloro-2-fluorobenzyl)oxy)-4-fluorophenyl)-3,6-dihydropyridin-1(2H)-yl)methyl)-3-(oxetan-2-ylmethyl)-3H-imidazo[4,5-c]pyridin-6-yl)-4H-1,2,4-triazole-3-carbonitrile (3.9 mg, yield: 13.5%). MS Calcd: 628.2; MS Found: 629.2 $[M+H]^+$

$^1H$ NMR (400 MHz, $CD_3OD$-$d_4$): δ 9.06 (s, 1 H), 8.35 (s, 1 H), 7.54 (t, J = 8.0 Hz, 1 H), 7.26 (d, J =7.6, 2 H), 7.20 (d, J = 8.0 Hz, 1 H), 7.09 - 7.05 (m, 2 H), 6.08 (s, 1 H), 5.31 - 5.24 (m, 1 H), 5.20 (s, 2 H), 4.98 - 4.92 (m, 1 H), 4.81 - 4.75 (m, 1 H), 4.68 - 4.62 (m, 1 H), 4.52 - 4.46 (m, 1 H), 4.17 (d, J = 13.6 Hz, 1 H), 4.05 (d, J = 13.6 Hz, 1 H), 3.28 - 3.22 (m, 2 H), 2.89 - 2.83 (m, 2 H), 2.82 - 2.76 (m, 1 H), 2.60 - 2.50 (m, 3 H). $^{19}F$ NMR (377 MHz, $CD_3OD$-$d_4$): δ (-117.52), (-137.88).

**Example 34: 3-(2-((4-((S)-2-(4-chloro-2-fluorophenyl)-2-methylbenzo[d][1,3]dioxol-4-yl)piperidin-1-yl)methyl)-4-fluoro-1-(((S)-oxetan-2-yl)methyl)-1H-benzo[d]imidazol-5-yl)-1,2,4-oxadiazol-5(4H)-one (Compound 34)**

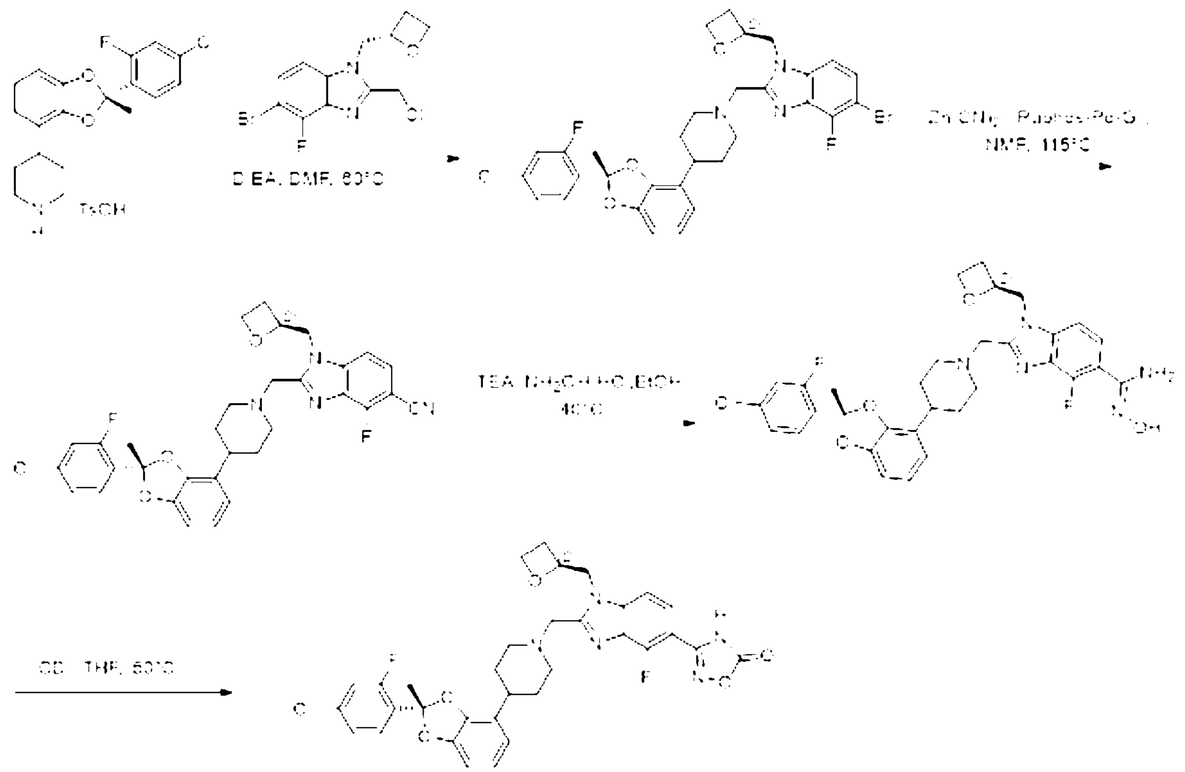


*Step A: 5-bromo-2-((4-((S)-2-(4-chloro-2-fluorophenyl)-2-methylbenzo[d][1,3]dioxol-4-yl)piperidin-1-yl)methyl)-4-fluoro-1-(((S)-oxetan-2-yl)methyl)-1H-benzo[d]imidazole*

To a mixture of (S)-4-(2-(4-chloro-2-fluorophenyl)-2-methylbenzo[d][1,3]dioxol-4-yl)piperidine TsOH salt (187.20 mg, 0.36 mmol) in DMF (4.0 mL) was added (S)-5-bromo-2-(chloromethyl)-4-fluoro-1-(oxetan-2-ylmethyl)-1H-benzo[d]imidazole (100 mg, 0.30 mmol) and DIEA (388.55 mg, 3.01 mmol). The mixture was stirred at 60 °C for 1 h. After the reaction was completed, the mixture was diluted with $H_2O$ (40 mL), extracted with EA (30 mL×2). The combined organic layer was dried over anhydrous sodium sulfate, filtered and evaporated to dryness. The residue was purified by silica gel column chromatography, eluted with (DCM/MeOH=100/1) to give 5-bromo-2-((4-((S)-2-(4-chloro-2-fluorophenyl)-2-methylbenzo[d][1,3]dioxol-4-yl)piperidin-1-yl)methyl)-4-fluoro-1-(((S)-oxetan-2-yl)methyl)-1H-benzo[d]imidazole (170 mg, yield: 87.8%). MS Calcd.: 643.10; MS Found: 644.3/646.3 $[M+H]^+$.

*Step B: 2-((4-((S)-2-(4-chloro-2-fluorophenyl)-2-methylbenzo[d][1,3]dioxol-4-yl)piperidin-1-yl)methyl)-4-fluoro-1-(((S)-oxetan-2-yl)methyl)-1H-benzo[d]imidazole-5-carbonitrile*

A solution of 5-bromo-2-((4-((S)-2-(4-chloro-2-fluorophenyl)-2-methylbenzo[d][1,3]dioxol-4-yl)piperidin-1-yl)methyl)-4-fluoro-1-(((S)-oxetan-2-yl)methyl)-1H-benzo[d]imidazole (130 mg, 0.20 mmol), $Zn(CN)_2$ (11.87 mg, 0.10 mmol), Ruphos-Pd-$G_3$ (33.44 mg, 0.04 mmol) in NMP (2.0 mL) was stirred at 115°C for 1.5 h. After the reaction was completed, the mixture was diluted with $H_2O$ (20 mL), extracted with EA (20 mL×2). The combined organic layer was dried over anhydrous sodium sulfate, filtered and evaporated to dryness. The residue was purified by silica gel column chromatography, eluted with (DCM/MeOH=80/1) to give 2-((4-((S)-2-(4-chloro-2-fluorophenyl)-2-methylbenzo[d][1,3]dioxol-4-yl)piperidin-1-yl)methyl)-4-fluoro-1-(((S)-oxetan-2-yl)methyl)-1H-benzo[d]imidazole-5-carbonitrile (120 mg impure, 59% pure, yield: 100%). MS Calcd.: 590.2; MS Found: 591.5 $[M+H]^+$.

*Step C: (Z)-2-((4-((S)-2-(4-chloro-2-fluorophenyl)-2-methylbenzo[d][1,3]dioxol-4-yl)piperidin-1-yl)methyl)-4-fluoro-N'-hydroxy-1-(((S)-oxetan-2-yl)methyl)-1H-benzo[d]imidazole-5-carboximidamide*

A solution of 2-((4-((S)-2-(4-chloro-2-fluorophenyl)-2-methylbenzo[d][1,3]dioxol-4-yl)piperidin-1-yl)methyl)-4-fluoro-1-(((S)-oxetan-2-yl)methyl)-1H-benzo[d]imidazole-5-carbonitrile (80 mg, 0.14 mmol), $HONH_2 \cdot HCl$ (59.64 mg, 0.81 mmol), TEA (56.67 mg, 0.56 mmol) in EtOH (4.0 mL) was stirred at 40 °C for 5 hours. After the reaction was completed, the mixture was diluted with $H_2O$ (20 mL) and extracted with EA (15 mL×2). The combined organic layer was dried over anhydrous sodium sulfate, filtered and evaporated to give (Z)-2-((4-((S)-2-(4-chloro-2-fluorophenyl)-2-methylbenzo[d][1,3]dioxol-4-yl)piperidin-1-yl)methyl)-4-fluoro-N'-hydroxy-1-(((S)-oxetan-2-yl)methyl)-1H-benzo[d]imidazole-5-carboximidamide (86 mg, crude). MS Calcd.: 623.21; MS Found: 624.2 $[M+H]^+$.

*Step D: 3-(2-((4-((S)-2-(4-chloro-2-fluorophenyl)-2-methylbenzo[d][1,3]dioxol-4-yl)piperidin-1-yl)methyl)-4-fluoro-1-(((S)-oxetan-2-yl)methyl)-1H-benzo[d]imidazol-5-yl)-1,2,4-oxadiazol-5(4H)-one*

A mixture of (Z)-2-((4-((S)-2-(4-chloro-2-fluorophenyl)-2-methylbenzo[d][1,3]dioxol-4-yl)piperidin-1-yl) methyl)-4-fluoro-N'-hydroxy-1-(((S)-oxetan-2-yl)methyl)-1H-benzo[d]imidazole-5-carboximidamide (86 mg, 0.14 mmol) and CDI (112 mg, 0.69 mmol) in THF (2.0 mL) was stirred at 50 °C for 3 hours. After the reaction was completed, the mixture was purified directly by Prep-HPLC (0.1% FA/$H_2O$/$CH_3CN$) to give 3-(2-((4-((S)-2-(4-chloro-2-fluorophenyl)-2-methylbenzo[d][1,3]dioxol-4-yl)piperidin-1-yl)methyl)-4-fluoro-1-(((S)-oxetan-2-yl)methyl)-1H-benzo[d]imidazol-5-yl)-1,2,4-oxadiazol-5(4H)-one (11.41mg, yield: 12.8%). MS Calcd.: 649.2; MS Found: 650.1 $[M+H]^+$.

$^1$H NMR (400 MHz, DMSO-d6): δ 6.69 (d, *J*=8.8 Hz, 1 H), 7.50-7.62 (m, 3 H), 7.34 (d, *J*=8.8 Hz, 1 H), 6.71-6.84 (m, 3 H), 5.09-5.17 (m, 1 H), 4.75-4.84 (m, 1 H), 4.61-4.71 (m, 1 H), 4.35-4.53 (m, 2 H), 3.99 (d, *J*=13.2 Hz, 1 H), 3.83 (d, *J*=13.6 Hz, 1 H), 2.98-3.09 (m, 1 H), 2.84-2.93 (m, 1 H), 2.61-2.76 (m, 2 H), 2.38-2.47 (m, 1 H), 2.16-2.35 (m, 2 H), 2.03 (s, 2 H), 1.66-1.84 (m, 4 H). $^{19}$F NMR (377 MHz, DMSO-d6): δ -110.78, -126.12.

**Example 35: (S)-3-(2-((4-(6-((4-chloro-2-fluorobenzyl)oxy)pyridin-2-yl)piperidin-1-yl)methyl)-4-fluoro-1-(oxetan-2-ylmethyl)-1H-benzo[d]imidazol-5-yl)-1,2,4-oxadiazol-5(4H)-one (Compound 35)**

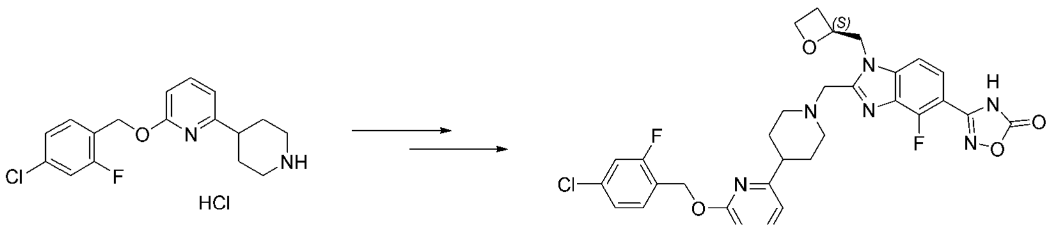

Starting from 2-((4-chloro-2-fluorobenzyl)oxy)-6-(piperidin-4-yl)pyridine HCl salt, (S)-3-(2-((4-(6-((4-chloro-2-fluorobenzyl)oxy)pyridin-2-yl)piperidin-1-yl)methyl)-4-fluoro-1-(oxetan-2-ylmethyl)-1H-benzo[d]imidazol-5-yl)-1,2,4-oxadiazol-5(4H)-one was obtained with the similar method of Example 34.

MS Calcd.: 622.19; MS Found: 623.2 [M+H]+.

$^1$H NMR (400 MHz, DMSO-d6): δ 12.74 (br s, 1 H), 7.69 (d, *J*=8.4 Hz, 1 H), 7.63 (t, *J*=7.6 Hz, 1 H), 7.53-7.60 (m, 2 H), 7.46 (dd, *J*=9.6 Hz, 2.0 Hz, 1 H), 7.30 (dd, *J*=8.4 Hz, 2.0 Hz, 1 H), 6.88 (d, *J*=7.6 Hz, 1 H), 6.68 (d, *J*=8.0 Hz, 1 H), 5.38 (s, 2 H), 5.09-5.17 (m, 1 H), 4.76-4.85 (m, 1 H), 4.64-4.71 (m, 1 H), 4.44-4.51 (m, 1 H), 4.36-4.44 (m, 1 H), 3.99 (d, *J*=14.0 Hz, 1 H), 3.85 (d, *J*=13.6 Hz, 1 H), 2.98-3.06 (m, 1 H), 2.85-2.92 (m, 1 H), 2.61-2.75 (m, 2 H), 2.39-2.46 (m, 1 H), 2.20-2.33 (m, 2 H), 1.66-1.84 (m, 4 H). $^{19}$F NMR(377 MHz, DMSO-d6): δ -63.63, -111.38.

**Example 36: 3-(2-((4-((S)-2-(5-chloropyridin-2-yl)-2-methylbenzo[d][1,3]dioxol-4-yl)piperidin-1-yl)methyl)-4-fluoro-1-(((S)-oxetan-2-yl)methyl)-1H-benzo[d]imidazol-5-yl)-1,2,4-oxadiazol-5(4H)-one (Compound 36)**

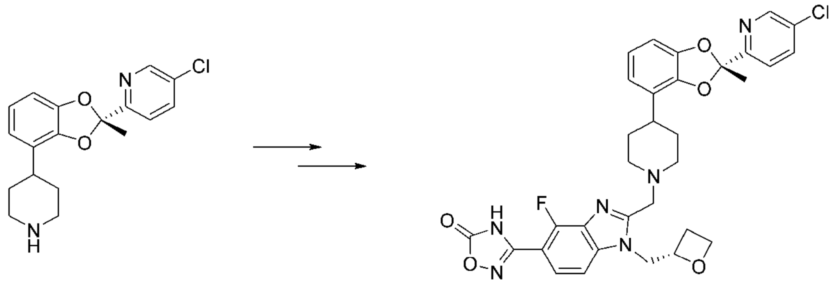

Starting from (S)-5-chloro-2-(2-methyl-4-(piperidin-4-yl)benzo[d][1,3]dioxol-2-yl)pyridine, 3-(2-((4-((S)-2-(5-chloropyridin-2-yl)-2-methylbenzo[d][1,3]dioxol-4-yl)piperidin-1-yl)methyl)-4-fluoro-1-(((S)-oxetan-2-yl)methyl)-1H-benzo[d]imidazol-5-yl)-1,2,4-oxadiazol-5(4H)-one was obtained with the similar method of Example 34.

MS Calcd.: 632.20; MS Found: 633.2 [M+H]+.

$^{1}$H NMR (400 MHz, DMSO-d6): δ 12.89 (br s, 1 H), 8.73 (d, *J*=2.4 Hz, 1 H), 8.02 (dd, *J*=8.4 Hz, 2.4 Hz, 1 H), 7.82 (d, *J*=8.4 Hz, 1 H), 7.61-7.72 (m, 2 H), 6.83-6.89 (m, 2 H), 6.72-6.80 (m, 1 H), 5.01-5.11 (m, 1 H), 4.76-4.87 (m, 2 H), 4.61-4.70 (m, 1 H), 4.45-4.53 (m, 1 H), 4.30-4.38 (m, 1 H), 3.65-3.81 (m, 2 H), 3.21-3.34 (m, 2 H), 2.89-3.04 (m, 1 H), 2.64-2.77 (m, 2 H), 2.25-2.38 (m, 1 H), 1.93-2.18 (m, 7 H). $^{19}$F NMR (377 MHz, DMSO-d6): δ -73.73, -124.85.

**Example 37: 3-(2-((4-((S)-2-(5-chloropyridin-2-yl)-2-methylbenzo[d][1,3]dioxol-4-yl)piperidin-1-yl)methyl)-7-fluoro-1-(((S)-oxetan-2-yl)methyl)-1H-benzo[d]imidazol-5-yl)-1,2,4-oxadiazol-5(4H)-**one (Compound 37)

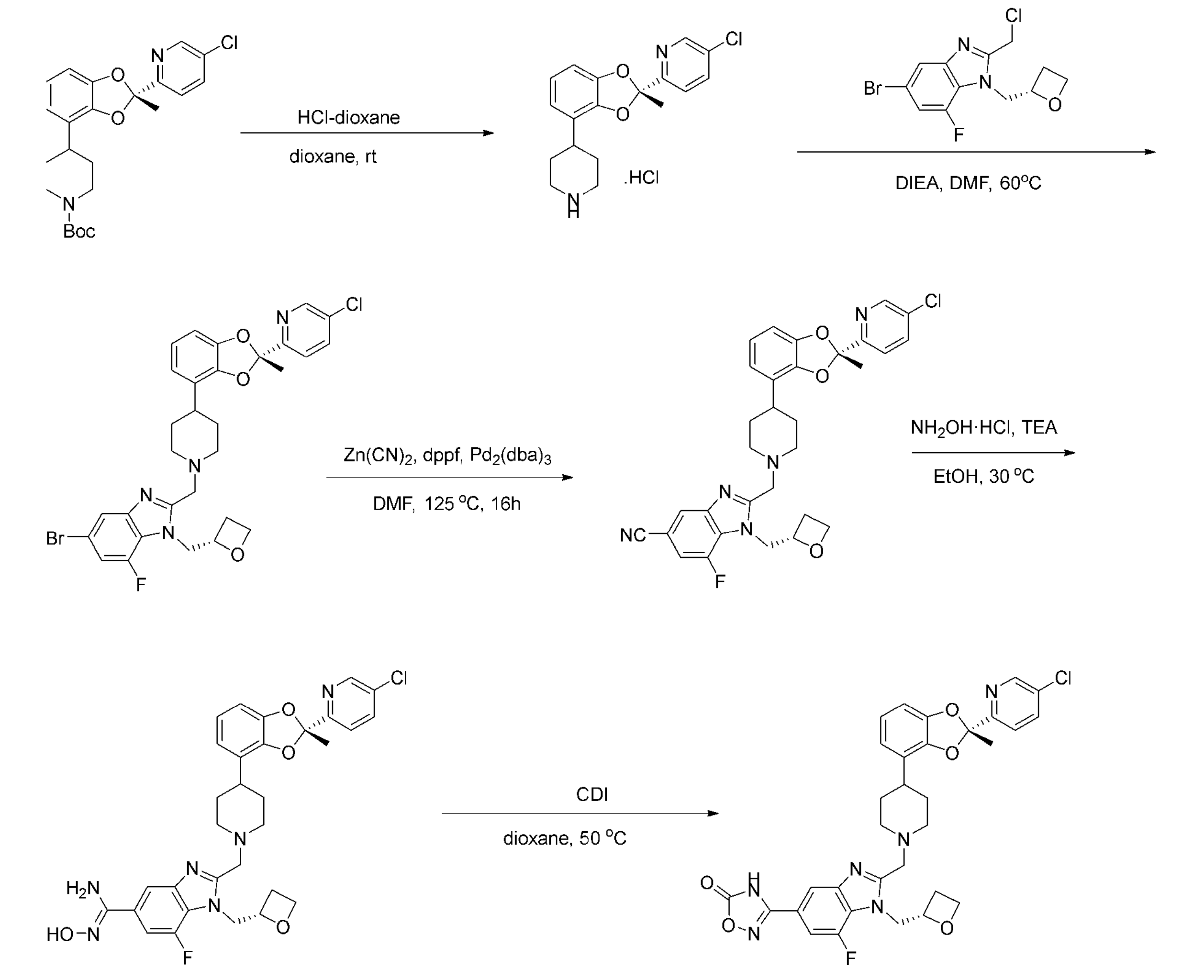


*Step A: (S)-5-chloro-2-(2-methyl-4-(piperidin-4-yl)benzo[d][1,3]dioxol-2-yl)pyridine HCl salt*

To a solution of tert-butyl (S)-4-(2-(5-chloropyridin-2-yl)-2-methylbenzo[d][1,3]dioxol-4-yl)piperidine-1-carboxylate (100 mg, 0.23 mmol) in dioxane (1 mL) was added HCl-dioxane (4M, 1 mL) at room temperature. The solution was stirred at room temperature for 1 hour. The reaction was concentrated in vacuum to give (S)-5-chloro-2-(2-methyl-4-(piperidin-4-yl)benzo[d][1,3]dioxol-2-yl)pyridine HCl salt (120 mg, crude). MS Calcd.: 330.11; MS Found:331.3 [M+H]$^{+}$.

*Step B: 5-bromo-2-((4-((S)-2-(5-chloropyridin-2-yl)-2-methylbenzo[d][1,3]dioxol-4-yl)piperidin-1-yl)methyl)-7-fluoro-1-(((S)-oxetan-2-yl)methyl)-1H-benzo[d]imidazole*

A mixture of (S)-5-chloro-2-(2-methyl-4-(piperidin-4-yl)benzo[d][1,3]dioxol-2-yl)pyridine HCl salt (120 mg, crude), (S)-5-bromo-2-(chloromethyl)-7-fluoro-1-(oxetan-2-ylmethyl)-1H-benzo[d]imidazole (77 mg, 0.23 mmol), DIEA (91 mg, 0.70 mmol) in DMF (3.0 mL) was stirred at 60°C for 2 hours. After the reaction was completed, the reaction was

concentrated in vacuum. The reaction was purified by column chromatography (DCM:MeOH=95:5) to give 5-bromo-2-((4-((S)-2-(5-chloropyridin-2-yl)-2-methylbenzo[d][1,3]dioxol-4-yl)piperidin-1-yl)methyl)-7-fluoro-1-(((S)-oxetan-2-yl)methyl)-1H-benzo[d]imidazole (120 mg, yield: 82.8%). MS Calcd.: 626.11; MS Found: 627.5[M+H]+.

*Step C: 2-((4-((S)-2-(5-chloropyridin-2-yl)-2-methylbenzo[d][1,3]dioxol-4-yl)piperidin-1-yl)methyl)-7-fluoro-1-(((S)-oxetan-2-yl)methyl)-1H-benzo[d]imidazole-5-carbonitrile*

A solution of 5-bromo-2-((4-((S)-2-(5-chloropyridin-2-yl)-2-methylbenzo[d][1,3]dioxol-4-yl)piperidin-1-yl) methyl)-7-fluoro-1-(((S)-oxetan-2-yl)methyl)-1H-benzo[d]imidazole (120 mg, 0.19 mmol), $Zn(CN)_2$ (66 mg, 0.38 mmol), dppf (8.0 mg, 0.015 mmol) and $Pd_2(dba)_3$ (11 mg, 0.0096 mmol) in DMF (2 mL) was stirred at 125°C for 16 hours. After the reaction was completed, the reaction mixture was washed with $H_2O$ (10 mL) and extracted with ethyl acetate (20 mL x 3). The organic layer was combined and washed with brine (10 mL x 2), dried over sodium sulfate, filtered and concentrated in vacuum. The reaction was purified by column chromatography (DCM:MeOH=95:5) to give 2-((4-((S)-2-(5-chloropyridin-2-yl)-2-methylbenzo[d] [1 ,3]dioxol-4-yl)piperidin-1-yl)methyl)-7-fluoro-1-(((S)-oxetan-2-yl)methyl)-1H-benzo[d]imidazole-5-carbonitrile (100 mg, 91.7% yield). MS Calcd.: 573.2; MS Found: 574.4[M+H]+.

*Step D: 2-((4-((S)-2-(5-chloropyridin-2-yl)-2-methylbenzo[d][1,3]dioxol-4-yl)piperidin-1-yl)methyl)-7-fluoro-N-hydroxy-1-(((S)-oxetan-2-yl)methyl)-1H-benzo[d]imidazole-5-carboximidamide*

A mixture of 2-((4-((S)-2-(5-chloropyridin-2-yl)-2-methylbenzo[d][1,3]dioxol-4-yl)piperidin-1-yl)methyl)-7-fluoro-1-(((S)-oxetan-2-yl)methyl)-1H-benzo[d]imidazole-5-carbonitrile (100 mg, 0.17 mmol), $NH_2OH \cdot HCl$(18 mg, 0.26 mmol) and TEA(53 mg, 0.52 mmol)in EtOH (3.0 mL) was stirred at 30°C for 16 hours. After the reaction was completed, the reaction was washed with water (10 mL) and extracted with ethyl acetate (20 mL x 3). The organic layer was combined and washed with brine (10 mL), dried over sodium sulfate, filtered and concentrated in vacuum. The reaction was purified by column chromatography (DCM:MeOH=95:5) to give 2-((4-((S)-2-(5-chloropyridin-2-yl)-2-methylbenzo[d][1,3]dioxol-4-yl) piperidin-1-yl)methyl)-7-fluoro-N-hydroxy-1-(((S)-oxetan-2-yl)methyl)-1H-benzo[d]imidazole-5-carboximidamide (80 mg. 76.2% yield). MS Calcd.:606.2 ; MS Found: 607.3[M+H]+.

*Step E:3-(2-((4-((S)-2-(5-chloropyridin-2-yl)-2-methylbenzo[d][1,3]dioxol-4-yl)piperidin-1-yl)methyl)-7-fluoro-1-(((S)-oxetan-2-yl)methyl)-1H-benzo[d]imidazol-5-yl)-1,2,4-oxadiazol-5(4H)-one*

A mixture of 2-((4-((S)-2-(5-chloropyridin-2-yl)-2-methylbenzo[d][1,3]dioxol-4-yl)piperidin-1-yl)methyl)-7-fluoro-N-hydroxy-1-(((S)-oxetan-2-yl)methyl)-1H-benzo[d]imidazole-5-carboximidamide (80 mg, 0.13 mmol) and CDI (43 mg, 0.26 mmol) in dioxane (1.0 mL) was stirred at 50°C for 16 hours. After the reaction was completed, the reaction was washed with water (10 mL) and extracted with ethyl acetate (20 mL x 3). The organic layer was combined and washed with brine (10 mL), dried over sodium sulfate, filtered and concentrated in vacuum. the mixture was purified directly by Prep-HPLC to give 3-(2-((4-((S)-2-(5-chloropyridin-2-yl)-2-methylbenzo[d][1,3]dioxol-4-yl)piperidin-1-yl)methyl)-7-fluoro-1-(((S)-oxetan-2-yl)methyl)-1H-benzo[d]imidazol-5-yl)-1,2,4-oxadiazol-5(4H)-one (25.4 mg, yield: 30.1%).

MS Calcd.:632.20 ; MS Found: 633.4 [M+H]+.

1H NMR: δ 8.72 (d, *J*=2.4 Hz, 1 H), 8.01 (dd, *J*=2.4 Hz, *J*=8.4 Hz, 1 H), 7.93 (d, *J*=1.2 Hz, 1 H), 7.60 (d, *J*=8.4 Hz, 1 H), 7.50 (d, *J*=12.0 Hz, 1 H), 6.71-6.85 (m, 3 H), 5.07-5.17 (m, 1 H), 4.79-4.90 (m, 1 H), 4.67-4.71 (m, 1 H), 4.45-4.50 (m, 1 H), 4.34-4.39 (m, 1 H), 3.97 (d, *J*=13.6 Hz, 1 H), 3.84 (d, *J*=13.6 Hz, 1 H), 2.99-3.08 (m, 1 H), 2.88-2.97 (m, 1 H), 2.62-2.77 (m, 2 H), 2.41-2.50 (m, 1 H), 2.17-2.31 (m, 2 H), 2.01 (s, 3 H), 1.68-1.83 (m, 4 H). $^{19}F$ NMR (377 MHz, DMSO-d6): δ -130.81.

**Example 38: (S)-3-(2-((4-(6-((4-chloro-2-fluorobenzyl)oxy)pyridin-2-yl)piperidin-1-yl)methyl)-7-fluoro-1-(oxetan-2-ylmethyl)-1H-benzo[d]imidazol-5-yl)-1,2,4-oxadiazol-5(4H)-one (Compound 38)**

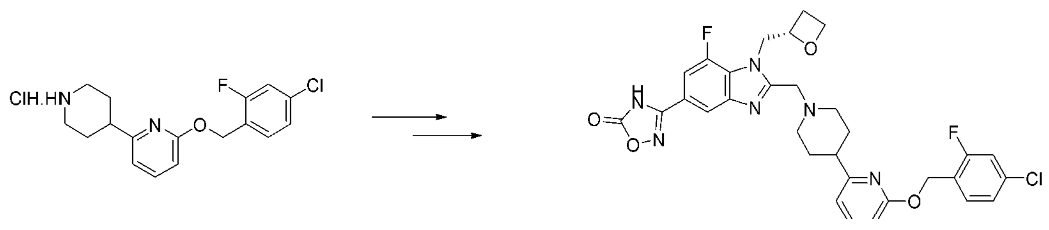

Starting from 2-((4-chloro-2-fluorobenzyl)oxy)-6-(piperidin-4-yl)pyridine hydrochloric acid salt,

(S)-3-(2-((4-(6-((4-chloro-2-fluorobenzyl)oxy)pyridin-2-yl)piperidin-1-yl)methyl)-7-fluoro-1-(oxetan-2-ylmethyl)-1H-benzo[d]imidazol-5-yl)-1,2,4-oxadiazol-5(4H)-one was obtained with the similar method of Example 37.

MS Calcd: 622.2; MS Found: 623.3 $[M+H]^+$.

$^1H$ NMR (400 MHz, $CD_3OD-d_4$): δ 7.97 (s, 1 H), 7.62 - 7.50 (m, 3 H), 7.24 - 7.19 (m, 2 H), 6.86 (d, *J* = 7.2 Hz, 1 H), 6.66 (d, *J* = 8.0 Hz, 1 H), 5.44 (s, 2 H), 5.33 - 5.26 (m, 1 H), 5.03 (dd, *J* = 7.2 Hz/*J* = 15.6 Hz, 1 H), 4.86 - 4.85 (m, 1 H), 4.69 - 4.63 (m, 1 H), 4.51 - 4.45 (m, 1 H), 4.08 (dd, *J* = 13.2 Hz/*J* = 39.6 Hz, 2 H), 3.16 - 3.14 (m, 1 H), 3.08 - 3.04 (m, 1 H), 2.90 - 2.81 (m, 1 H), 2.75 - 2.66 (m, 1 H), 2.59 - 2.53 (m, 1 H), 2.50 - 2.36 (m, 2 H), 1.99 - 1.87 (m, 4 H). $^{19}F$ NMR (377 MHz, $CD_3OD-d_4$): δ (-117.63), (-133.66).

**Example 39: 3-(2-((4-(2-(4-chloro-2-fluorophenyl)-2-methylbenzo[d][1,3]dioxol-4-yl)-3,6-dihydropyridin-1(2H)-yl)methyl)-7-fluoro-1-(((S)-oxetan-2-yl)methyl)-1H-benzo[d]imidazol-5-yl)-1,2,4-oxadiazol-5(4H)-one (Compound 39)**

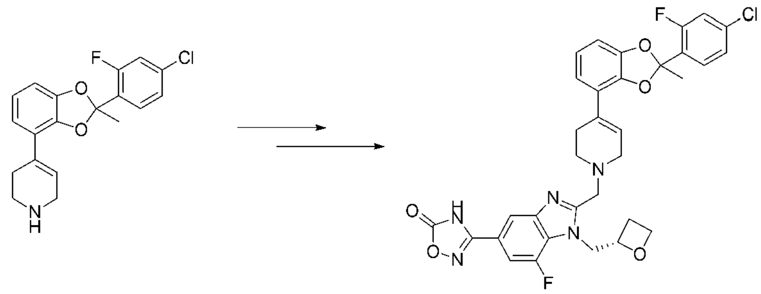

Starting from 4-(2-(4-chloro-2-fluorophenyl)-2-methylbenzo[d][1,3]dioxol-4-yl)-1,2,3,6-tetrahydropyridine HCl salt, 3-(2-((4-(2-(4-chloro-2-fluorophenyl)-2-methylbenzo[d][1,3]dioxol-4-yl)-3,6-dihydropyridin-1(2H)-yl)methyl)-7-fluoro-1-(((S)-oxetan-2-yl)methyl)-1H-benzo[d]imidazol-5-yl)-1,2,4-oxadiazol-5(4H)-one was obtained with the similar method of Example 37.

MS Calcd.:647.17 ; MS Found: 648.3$[M+H]^+$.

1H NMR: δ 7.93(s, 1 H), 7.45-7.62 (m, 3 H), 7.32-7.35 (m, 1 H), 6.82-6.88 (m, 3 H), 6.35-6.42 (m, 1 H), 5.04-5.14 (m, 1 H), 4.80-4.90 (m, 1 H), 4.65-4.75 (m, 1 H), 4.47-4.52 (m, 1 H), 4.32-4.43 (m, 1 H), 4.02-4.10 (m, 1 H), 3.88-3.99 (m, 1 H), 3.20-3.27 (m, 2 H), 2.69-2.82 (m, 3 H), 2.55-2.62 (m, 1 H), 2.36-2.46 (m, 2 H), 2.03 (s, 3 H). $^{19}F$ NMR (377 MHz, DMSO-d6): δ -110.90, -131.21.

**Example 40: 2-((4-((S)-2-(4-chloro-2-fluorophenyl)-2-methylbenzo[d][1,3]dioxol-4-yl)piperidin-1-yl)methyl)-4-fluoro-5-(5-methyl-4H-1,2,4-triazol-3-yl)-1-(((S)-oxetan-2-yl)methyl)-1H-benzo[d]imidazole (Compound 40)**

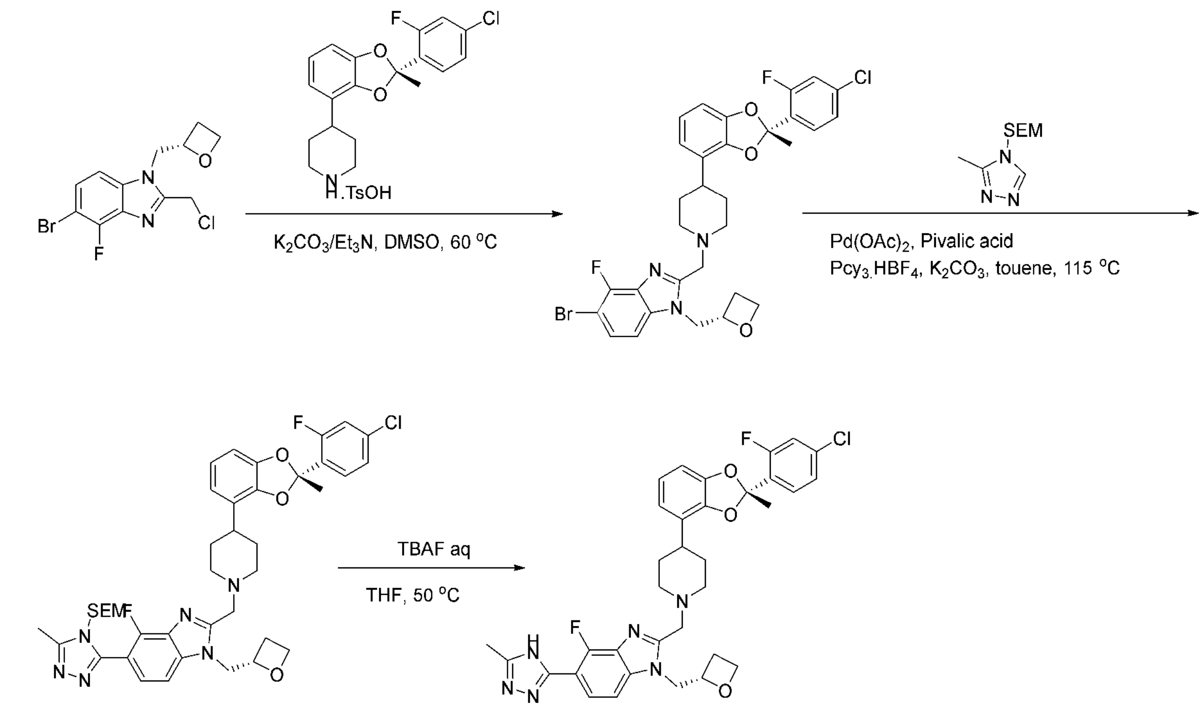


*Step A: 5-bromo-2-((4-((S)-2-(4-chloro-2-fluorophenyl)-2-methylbenzo[d][1,3]dioxol-4-yl)piperidin-1-yl)methyl)-4-fluoro-1-(((S)-oxetan-2-yl)methyl)-1H-benzo[d]imidazole*

A mixture of (S)-5-bromo-2-(chloromethyl)-4-fluoro-1-(oxetan-2-ylmethyl)-1H-benzo[d]imidazole (78 mg, 0.15 mmol), $K_2CO_3$ (62 mg, 0.45 mmol) and TEA (76 mg, 0.75 mmol) in DMSO (5 mL) were stirred at RT for 10 min. The mixture was added (S)-4-(2-(4-chloro-2-fluorophenyl)-2-methylbenzo[d][1,3]dioxol-4-yl)piperidine. TsOH (50 mg, 0.15 mmol) and the mixture was stirred for 3 h. The mixture was poured into water (50 mL) and extracted with EA (2 x 50 mL), the combined organic layer was washed with brine, dried over sodium sulfate, filtered and purified by column chromatography (DCM : MeOH = 30: 1) on silica gel to obtain 5-bromo-2-((4-((S)-2-(4-chloro-2-fluorophenyl)-2-methylbenzo[d][1,3]dioxol-4-yl) piperidin-1-yl)methyl)-4-fluoro-1-(((S)-oxetan-2-yl)methyl)-1H-benzo[d]imidazole (84 mg, yield: 87.3%). MS Calcd: 643.1; MS Found: 644.2 $[M+H]^+$

*Step B: 2-((4-((S)-2-(4-chloro-2-fluorophenyl)-2-methylbenzo[d][1,3]dioxol-4-yl)piperidin-1-yl)methyl)-4-fluoro-5-(5-methyl-4-((2-(trimethylsilyl)ethoxy)methyl)-4H-1,2,4-triazol-3-yl)-1-(((S)-oxetan-2-yl)methyl)-1H-benzo(d]imidazole*

A mixture of 5-bromo-2-((4-((S)-2-(4-chloro-2-fluorophenyl)-2-methylbenzo[d] [1,3]dioxol-4-yl)piperidin-1-yl) methyl)-4-fluoro-1-(((S)-oxetan-2-yl)methyl)-1H-benzo[d]imidazole (84 mg, 0.13 mmol), 3-methyl-4-((2-(trimethylsilyl) ethoxy)methyl)-4H-1,2,4-triazole (83 mg, 0.39 mmol), $Pd(OAc)_2$ (3.0 mg, 0.013 mmol), Pivalic acid (2.7 mg, 0.026 mmol), $Pcy_3.HBF_4$ (3.0 mg, 0.0079 mmol ) and $K_2CO_3$ (133 mg, 0.79 mmol) were stirred with toluene (5 mL) at 115 °C for 16 h under Ar. The mixture was poured into water (50 mL) and extracted with EA (2 x 50 mL), the combined organic layer was washed with brine, dried over sodium sulfate, filtered and purified by column chromatography (DCM : MeOH = 30: 1) on silica gel to obtain 2-((4-((S)-2-(4-chloro-2-fluorophenyl)-2-methylbenzo[d][1,3]dioxol-4-yl)piperidin-1-yl)methyl)-4-fluoro-5-(5-methyl-4-((2-(trimethylsilyl)ethoxy)methyl)-4H-1,2,4-triazol-3-yl)-1-(((S)-oxetan-2-yl)methyl)-1H-benzo[d] imidazole (10 mg, yield: 9.8%). MS Calcd: 776.3; MS Found: 777.5 $[M+H]^+$

*Step C: 2-((4-((S)-2-(4-chloro-2-fluorophenyl)-2-methylbenzo[d][1,3]dioxol-4-yl)piperidin-1-yl)methyl)-4-fluoro-5-(5-methyl-4H-1,2,4-triazol-3-yl)-1-(((S)-oxetan-2-yl)methyl)-1H-benzo[d]imidazole*

A mixture of 2-((4-((S)-2-(4-chloro-2-fluorophenyl)-2-methylbenzo[d][1,3]dioxol-4-yl)piperidin-1-yl)methyl)-4-fluoro-5-(5-methyl-4-((2-(trimethylsilyl)ethoxy)methyl)-4H-1,2,4-triazol-3-yl)-1-(((S)-oxetan-2-yl)methyl)-1H-benzo[d] imidazole (10 mg, 0.0129 mmol) and TBAF aq in THF (1M, 0.5 mL) in THF (3 mL) was stirred at 50 °C for 4 h. The mixture was concentrated and the residue was purified by prep-HPLC to obtain 2-((4-((S)-2-(4-chloro-2-fluorophenyl)-2-methylbenzo[d][1,3]dioxol-4-yl)piperidin-1-yl)methyl)-4-fluoro-5-(5-methyl-4-((2-(trimethylsilyl)ethoxy)methyl)-4H-1,2,4-triazol-3-yl)-1-(((S)-oxetan-2-yl)methyl)-1H-benzo[d]imidazole (4.7 mg, yield: 51.4%). MS Calcd: 646.2; MS Found: 647.4

[M+H]+

$^1$H NMR (400 MHz, $CD_3OD-d_4$): δ 7.84 (s, 1 H), 7.58 (t, *J* = 8.4 Hz, 2 H), 7.28 (dd, $J_1$ = 10.4 Hz/$J_2$ = 8.8 Hz, 1 H), 7.21 (d, *J* = 8.8 Hz, 1 H), 6.79 - 6.68 (m, 3 H), 5.31 - 5.26 (m, 1 H), 4.92 - 4.81 (m, 1 H), 4.72 (d, *J* = 15.2 Hz, 1 H), 4.66 - 4.61 (m, 1 H), 4.50 - 4.45 (m, 1 H), 4.04 (d, *J* = 14.4 Hz, 1 H), 3.91 (d, *J* = 13.6 Hz, 1 H), 3.13 - 3.07 (m, 1 H), 2.97 - 2.93 (m, 1 H), 2.84 - 2.79 (m, 1 H), 2.74 - 2.69 (m, 1 H), 2.57 - 2.44 (m, 4 H), 2.37 - 2.26 (m, 2 H), 2.03 (s, 3 H), 1.95 - 1.78 (m, 4 H). $^{19}$F NMR (377 MHz, $CD_3OD-d_4$): δ (-112.35), (-130.41).

**Example 41: 2-((4-((S)-2-(4-chloro-2-fluorophenyl)-2-methylbenzo[d][1,3]dioxol-4-yl)piperidin-1-yl)methyl)-7-fluoro-5-(5-methyl-4H-1,2,4-triazol-3-yl)-1-(((S)-oxetan-2-yl)methyl)-1H-benzo[d]imidazole (Compound 41)**

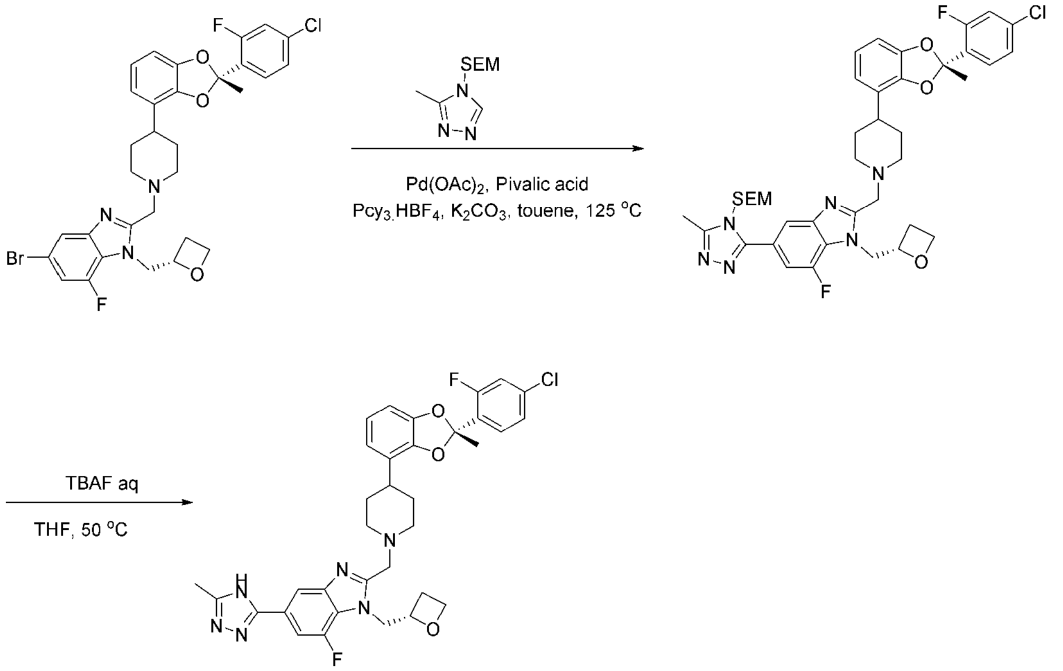


*Step A: 2-((4-((S)-2-(4-chloro-2-fluorophenyl)-2-methylbenzo[d][1,3]dioxol-4-yl)piperidin-1-yl)methyl)-7-fluoro-5-(5-methyl-4-((2-(trimethylsilyl)ethoxy)methyl)-4H-1,2,4-triazol-3-yl)-1-(((S)-oxetan-2-yl)methyl)-1H-benzo[d]imidazole*

A mixture of 5-bromo-2-((4-((S)-2-(4-chloro-2-fluorophenyl)-2-methylbenzo[d][1,3]dioxol-4-yl)piperidin-1-yl)methyl)-7-fluoro-1-(((S)-oxetan-2-yl)methyl)-1H-benzo[d]imidazole (50 mg, 0.078 mmol), $Pd(OAc)_2$(1.7 mg, 0.0078 mmol), Pivalic acid (0.47 mg, 0.0047 mmol), $Pcy_3.HBF_4$ (5.7 mg, 0.016 mmol) and $K_2CO_3$ (65 mg, 0.47 mmol) were stirred with toluene(5 ml) at 120 °C for 16 h under Ar. The mixture was poured into water (50 mL) and extracted with EA (2 x 50 mL), the combined organic layer was washed with brine, dried over sodium sulfate, filtered and purified by column chromatography (DCM : MeOH = 30: 1) on silica gel to obtain 2-((4-((S)-2-(4-chloro-2-fluorophenyl)-2-methylbenzo[d][1,3]dioxol-4-yl)piperidin-1-yl)methyl)-7-fluoro-5-(5-methyl-4-((2-(trimethylsilyl)ethoxy)methyl)-4H-1,2,4-triazol-3-yl)-1-(((S)-oxetan-2-yl)methyl)-1H-benzo[d]imidazole (20 mg, yield: 33.1%). MS Calcd: 776.3; MS Found: 777.4 [M+H]+

*Step B: 2-((4-((S)-2-(4-chloro-2-fluorophenyl)-2-methylbenzo[d][1,3]dioxol-4-yl)piperidin-1-yl)methyl)-7-fluoro-5-(5-methyl-4H-1,2,4-triazol-3-yl)-1-(((S)-oxetan-2-yl)methyl)-1H-benzo[d]imidazole*

A mixture of 2-((4-((S)-2-(4-chloro-2-fluorophenyl)-2-methylbenzo[d][1,3]dioxol-4-yl)piperidin-1-yl)methyl)-7-fluoro-5-(5-methyl-4-((2-(trimethylsilyl)ethoxy)methyl)-4H-1,2,4-triazol-3-yl)-1-(((S)-oxetan-2-yl)methyl)-1H-benzo[d]imidazole (24 mg, 0.032 mmol) and TBAF aq in THF (1 M, 1 ml) in THF (4 mL) was stirred at 50 °C for 4 h. The mixture was concentrated, and the residue was purified by prep-HPLC to obtain 2-((4-((S)-2-(4-chloro-2-fluorophenyl)-2-methylbenzo[d][1,3]dioxol-4-yl)piperidin-1-yl)methyl)-7-fluoro-5-(5-methyl-4H-1,2,4-triazol-3-yl)-1-(((S)-oxetan-2-yl)methyl)-1H-benzo[d]imidazole (4.3 mg, yield: 22.2%). MS Calcd: 646.2; MS Found: 647.2 [M+H]+

$^1$H NMR (400 MHz, $CD_3OD-d_4$): δ 8.12 (s, 1 H), 7.69 (d, *J* = 13.2 Hz, 1 H), 7.58 (t, *J* = 8.4 Hz, 1 H), 7.27 (dd, $J_1$ = 10.8 Hz/$J_2$ = 9.2 Hz, 2 H), 7.20 (d, *J* = 9.2 Hz, 1 H), 6.77 - 6.68 (m, 3 H), 5.28 (d, *J* = 5.2 Hz, 1 H), 5.05 - 5.00 (m, 1 H), 4.80 -

4.79 (m, 1 H), 4.66 - 4.58 (m, 2 H), 4.49 - 4.45 (m, 1 H), 4.05 (d, *J* = 12.8 Hz, 1 H), 3.94 (d, *J* = 14.4 Hz, 1 H), 3.13 - 3.08 (m, 1 H), 3.02 - 2.98 (m, 1 H), 2.86 - 2.80 (m, 1 H), 2.75 - 2.70 (m, 1 H), 2.57 - 2.50 (m, 4 H), 2.36 - 2.31 (m, 2 H), 2.03 (s, 3 H), 1.97 - 1.79 (m, 4 H). $^{19}$F NMR (377 MHz, $CD_3OD-d_4$): δ (-112.37), (-134.90).

**Example 42: 2-((4-((S)-2-(5-chloropyridin-2-yl)-2-methylbenzo[d][1,3]dioxol-4-yl)piperidin-1-yl)methyl)-7-fluoro-5-(5-methyl-4H-1,2,4-triazol-3-yl)-1-(((S)-oxetan-2-yl)methyl)-1H-benzo[d]imidazole (Compound 42)**

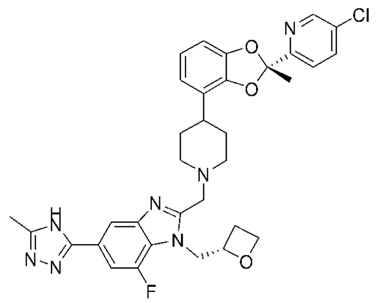

2-((4-((S)-2-(5-chloropyridin-2-yl)-2-methylbenzo[d][1,3]dioxol-4-yl)piperidin-1-yl)methyl)-7-fluoro-5-(5-methyl-4H-1 ,2,4-triazol-3-yl)-1-(((S)-oxetan-2-yl)methyl)-1H-benzo[d]imidazole was obtained with the similar method of Example 41. MS Calcd: 629.2; MS Found: 630.4 $[M+H]^+$

$^1$H NMR (400 MHz, $CD_3OD-d_4$): δ 8.60 - 8.59 (m, 1 H), 8.13 (s, 1 H), 7.87 (dd, $J_1$ = 8.4 Hz/$J_2$ = 6 Hz, 1 H), 7.72 - 7.64 (m, 2 H), 6.79 - 6.65 (m, 3 H), 5.28 (s, 1 H), 5.03 - 4.92 (m, 1 H), 4.81 - 4.79 (m, 1 H), 4.63 (d, *J* = 6.8 Hz, 1 H), 4.45 (d, *J* = 13.2 Hz, 1 H), 4.09 - 4.04 (m, 1 H), 3.98 - 3.95 (m, 1 H), 3.15 - 3.04 (m, 2 H), 2.84 - 2.76 (m, 2 H), 2.50 (s, 4 H), 2.39 - 2.31 (m, 2 H), 2.01 (s, 3 H), 1.94 - 1.77 (m, 4 H).$^{19}$F NMR (377 MHz, $CD_3OD-d_4$): δ (-74.00), (-75.88).

**Example 43: 2-((4-(2-(4-chloro-2-fluorophenyl)-2-methylbenzo[d][1,3]dioxol-4-yl)-3,6-dihydropyridin-1(2H)-yl) methyl)-7-fluoro-5-(5-methyl-4H-1,2,4-triazol-3-yl)-1-(((S)-oxetan-2-yl)methyl)-1H-benzo[d]imidazole (Compound 43)**

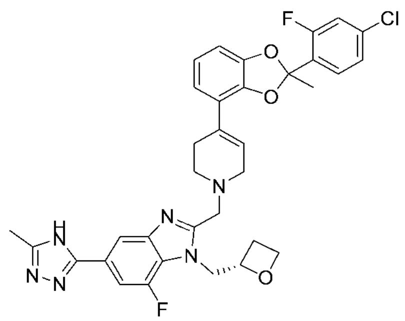

2-((4-(2-(4-chloro-2-fluorophenyl)-2-methylbenzo[d][1,3]dioxol-4-yl)-3,6-dihydropyridin-1(2H)-yl)methyl)-7-fluoro-5-(5-methyl-4H-1,2,4-triazol-3-yl)-1-(((S)-oxetan-2-yl)methyl)-1H-benzo[d]imidazole was obtained with the similar methods of Example 41. MS Calcd: 644.2; MS Found: 645.3 $[M+H]^+$.

$^1$H NMR (400 MHz, $CD_3OD-d_4$): δ 8.13 (s, 1 H), 7.69 (d, *J* = 13.2 Hz, 1 H), 7.56 (t, *J* = 9.2 Hz, 1H), 7.27 (d, *J* = 9.6 Hz, 1 H), 7.20 (d, *J* = 8.8 Hz, 1 H), 6.87 - 6.78 (m, 2 H), 6.74 (d, *J* = 7.2 Hz, 1 H), 6.40 (s, 1 H), 4.65 - 4.58 (m, 5 H), 4.14 (d, *J* = 14.0 Hz, 1 H), 4.03 (d, *J* = 13.2 Hz, 1 H), 2.91 - 2.77 (m, 3 H), 2.70 - 2.53 (m, 3 H), 2.50 (s, 5 H), 2.03 (s, 3 H). $^{19}$F NMR (377 MHz, $CD_3OD-d_4$): δ (-112.51), (-134.62).

**Example 44: 2-((4-((S)-2-(4-chloro-2-fluorophenyl)-2-methylbenzo[d][1,3]dioxol-4-yl)-3,6-dihydropyridin-1(2H)-yl)methyl)-3-(((S)-oxetan-2-yl)methyl)-6-(5-(trifluoromethyl)-4H-1,2,4-triazol-3-yl)-3H-imidazo[4,5-c] pyridine (Compound 44)**

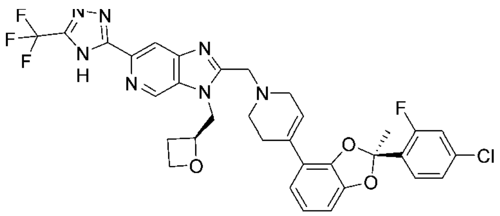

2-((4-((S)-2-(4-chloro-2-fluorophenyl)-2-methylbenzo[d][1,3]dioxol-4-yl)-3,6-dihydropyridin-1(2H)-yl)methyl)-3-(((S)-oxetan-2-yl)methyl)-6-(5-(trifluoromethyl)-4H-1,2,4-triazol-3-yl)-3H-imidazo[4,5-c]pyridine was obtained. MS Calcd.: 681.19; MS Found: 682.0 $[M+H]^+$.

1H NMR (400 MHz, DMSO-d6): δ 9.13 (s, 1 H), 8.28 (s, 1 H), 7.51-7.60 (m, 2 H), 7.33 (d, *J*=8.0 Hz , 1 H), 6.81-6.88 (m, 3 H), 6.35-6.42 (m, 1 H), 5.09-5.18 (m, 1 H), 4.85-4.95 (m, 1 H), 4.70-4.78 (m, 1 H), 4.36-4.53 (m, 1 H), 4.07-4.15 (m, 1 H), 3.94-4.02 (m, 1 H), 3.17-3.32 (m, 2 H), 2.65-2.84 (m, 3 H), 2.39-2.46 (m, 3 H), 2.03 (s, 3 H). $^{19}$F NMR(377 MHz, DMSO-d6): δ -63.15, -110.92.

**Example 45: 2-((4-((S)-2-(4-chloro-2-fluorophenyl)-2-methylbenzo[d][1,3]dioxol-4-yl)-3,6-dihydropyridin-1(2H)-yl)methyl)-3-(((S)-oxetan-2-yl)methyl)-6-(5-(trifluoromethyl)-4H-1,2,4-triazol-3-yl)-3H-imidazo[4,5-b]pyridine (Compound 45)**

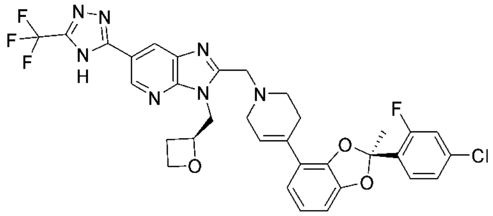

2-((4-((S)-2-(4-chloro-2-fluorophenyl)-2-methylbenzo[d][1,3]dioxol-4-yl)-3,6-dihydropyridin-1(2H)-yl)methyl)-3-(((S)-oxetan-2-yl)methyl)-6-(5-(trifluoromethyl)-4H-1,2,4-triazol-3-yl)-3H-imidazo[4,5-b]pyridine was obtained. MS Calcd.: 681.19; MS Found: 682.0 $[M+H]^+$.

$^1$H NMR (400 MHz, DMSO-d6): δ 9.00 (d, *J*=2.0 Hz, 1 H), 8.58 (d, *J*=1.6 Hz, 1 H), 7.52-7.61 (m, 2 H), 7.34 (dd, *J*=8.4 Hz, 2.0 Hz, 1 H), 6.81-6.90 (m, 3 H), 6.38-6.45 (m, 1 H), 5.13-5.22 (m, 1 H), 4.66-4.87 (m, 2 H), 4.33-4.53 (m, 2 H), 4.11 (d, *J*=13.6 Hz, 1 H), 4.02 (d, *J*=13.6 Hz, 1 H), 2.65-2.83 (m, 5 H), 2.39-2.45 (m, 3 H), 2.04 (s, 3 H). $^{19}$F NMR(377 MHz, DMSO-d6): δ -62.97, -110.90.

**Example 46: 6-((4-((S)-2-(4-chloro-2-fluorophenyl)-2-methylbenzo[d][1,3]dioxol-4-yl)-3,6-dihydropyridin-1(2H)-yl)methyl)-7-(((S)-oxetan-2-yl)methyl)-3-(5-(trifluoromethyl)-4H-1,2,4-triazol-3-yl)-7H-imidazo[4,5-c]pyridazine (Compound 46)**

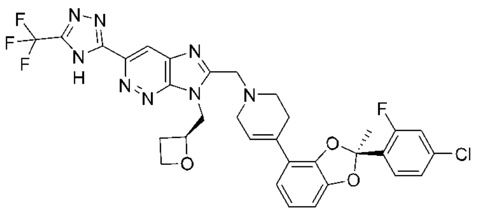

6-((4-((S)-2-(4-chloro-2-fluorophenyl)-2-methylbenzo[d][1,3]dioxol-4-yl)-3,6-dihydropyridin-1 (2H)-yl)methyl)-7-(((S)-oxetan-2-yl)methyl)-3-(5-(trifluoromethyl)-4H-1,2,4-triazol-3-yl)-7H-imidazo[4,5-c]pyridazine was obtained. MS Calcd.: 682.18; MS Found: 683.0 $[M+H]^+$.

$^1$H NMR (400 MHz, DMSO-d6): δ 8.53 (s, 1 H), 7.50-7.62 (m. 2 H), 7.33 (dd, J=8.4 Hz, 1.6 Hz, 1 H), 6.80-6.91 (m, 3 H), 6.36-6.44 (m, 1 H), 5.20-5.31 (m, 1 H), 4.84-5.06 (m, 2 H), 4.33-4.55 (m, 2 H), 4.09-4.24 (m, 2 H), 2.57-2.91 (m, 6 H), 2.30-2.46 (m, 2 H), 2.03 (s, 3 H). $^{19}$F NMR(377 MHz, DMSO-d6): δ -63.65, -110.89.

**Example 47: 6-[(4-chloro-2-fluorophenyl)methoxy]-3-fluoro-2-{1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl}pyridine (Compound 47)**

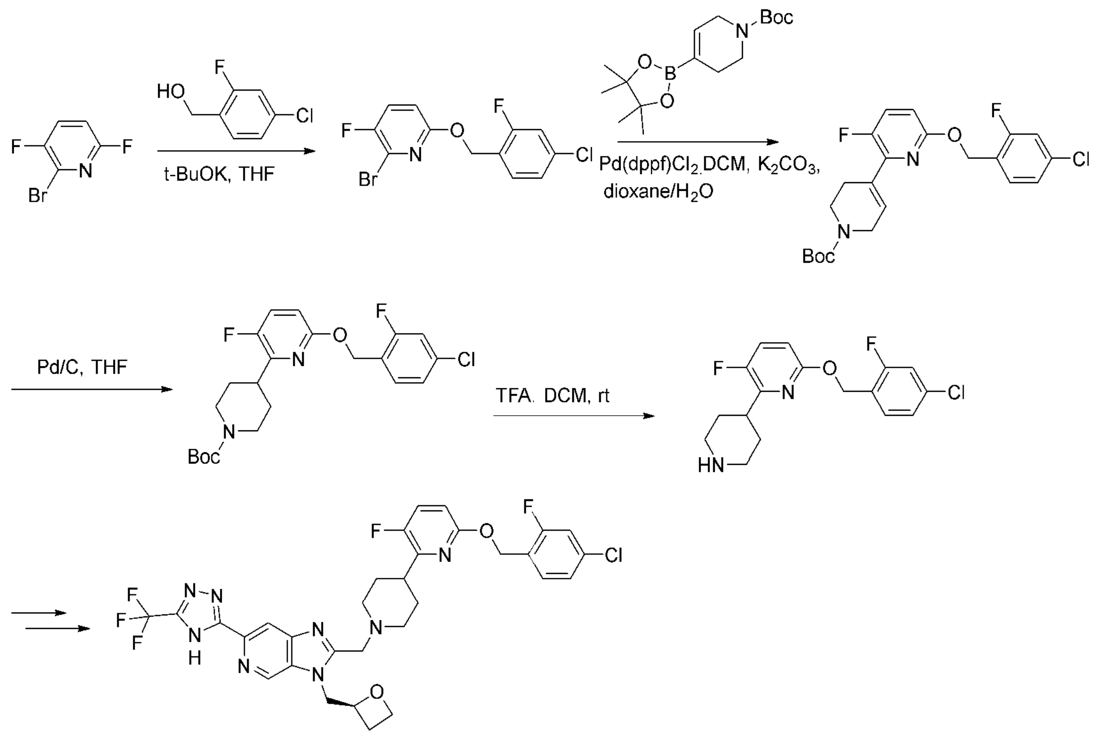


*Step A: 2-bromo-6-((4-chloro-2-fluorobenzyl)oxy)-3-fluoropyridine*

To a solution of 2-bromo-3,6-difluoropyridine (300 mg, 1.55 mmol) and (4-chloro-2-fluorophenyl)methanol (250 mg, 1.55 mmol) in THF (6 mL) was added t-BuOK (1.71 ml, 1 mol/L, 1.71 mmol) at 0 °C. The mixture was stirred at 0 °C for 1 hour under $N_2$. The reaction was quenched with $H_2O$ (10 mL), extracted with ethyl acetate (20 mL × 2). The organic layer was dried over anhydrous sodium sulfate, filtered, and evaporated to dryness. The residue was purified by column chromatography on silica gel (PE/EA=10/1) to give 2-bromo-6-((4-chloro-2-fluorobenzyl)oxy)-3-fluoropyridine (446 mg, 86.2% yield) as a white solid.

*Step B: tert-butyl 6-((4-chloro-2-fluorobenzyl)oxy)-3-fluoro-3',6'-dihydro-[2,4'-bipyridine]-1'(2'H)-carboxylate*

To a solution of 2-bromo-6-((4-chloro-2-fluorobenzyl)oxy)-3-fluoropyridine (446 mg, 1.34 mmol) in dioxane (15 mL) and $H_2O$ (1.5 mL) were added Pd(dppf)$Cl_2$.DCM (109 mg, 0.13 mmol), $K_2CO_3$ (554 mg, 4.01 mmol) and tert-butyl 4-(4,4,5,5-tetramethyl-1,3,2-dioxaborolan-2-yl)-3,6-dihydropyridine-1(2H)-carboxylate (455 mg, 1.47 mmol). The mixture was stirred at 95 °C for 16 hours under $N_2$. The reaction was quenched with $H_2O$(50 mL), extracted with ethyl acetate (70 mL × 2). The organic layer was dried over anhydrous sodium sulfate, filtered and evaporated to dryness. The residue was purified by column chromatography on silica gel (PE/EA=10/1) to give tert-butyl 6-((4-chloro-2-fluorobenzyl)oxy)-3-fluoro-3',6'-dihydro-[2,4'-bipyridine]-1'(2'H)-carboxylate (575 mg, 98.4% yield) as colorless oil. MS Calcd.: 436.1; MS Found: 437.0 $[M+H]^+$.

*Step C: tert-butyl 4-(6-((4-chloro-2-fluorobenzyl)oxy)-3-fluoropyridin-2-yl)piperidine-1-carboxylate*

A mixture of tert-butyl 6-((4-chloro-2-fluorobenzyl)oxy)-3-fluoro-3',6'-dihydro-[2,4'-bipyridine]-1'(2'H)-carboxylate (555 mg, 1.27 mmol) in THF (6 mL) was stirred at room temperature for 2 hours under $H_2$. The reaction mixture was filtered, and the filtrate evaporated to give tert-butyl 4-(6-((4-chloro-2-fluorobenzyl)oxy)-3-fluoropyridin-2-yl)piperidine-1-carboxylate (450 mg, crude) as a yellow oil. MS Calcd.: 438.2; MS Found: 383.0 $[M+H-56]^+$.

*Step D: 6-((4-chloro-2-fluorobenzyl)oxy)-3-fluoro-2-(piperidin-4-yl)pyridine*

To a solution of tert-butyl 4-(6-((4-chloro-2-fluorobenzyl)oxy)-5-fluoropyridin-2-yl)-3-oxopiperazine-1-carboxylate (250 mg) in DCM (2.4 mL) was added TFA (0.6 mL). The solution was stirred at room temperature for 1 hour. The reaction mixture was evaporated in vacuo to give 6-((4-chloro-2-fluorobenzyl)oxy)-3-fluoro-2-(piperidin-4-yl)pyridine TFA Salt (325 mg, crude). MS Calcd.: 338.1; MS Found: 339.3 $[M+H]^+$.

*Step E : 6-[(4-chloro-2-fluorophenyl)methoxy]-3-fluoro-2-{1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl}pyridine*

6-[(4-chloro-2-fluorophenyl)methoxy]-3-fluoro-2-{1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl}pyridine (Compound 47) (61 mg) was obtained with the similar method of Example 1. MS Calcd.: 674.2; MS Found: 675.2 $[M+H]^+$.

$^1$H NMR (400 MHz, DMSO-*d6*): δ 9.15 (s, 1 H), 8.27 (s, 1 H), 7.52-7.65 (m, 2 H), 7.47 (dd, *J=10.0* Hz, 2.0 Hz, 1 H), 7.27-7.33 (m, 1 H), 6.75 (dd, J = 8.8, 2.8 Hz, 1 H), 5.37 (s, 2 H), 5.16-5.25 (m, 1 H), 4.87-4.97 (m, 1 H), 4.73-4.81 (m, 1 H), 4.39-4.54 (m, 2 H), 4.01 (d, J = 13.6 Hz, 1 H), 3.86 (d, J = 14.0 Hz, 1 H), 2.98-3.08 (m, 1 H), 2.84-2.96 (m, 2 H), 2.65-2.79 (m, 1 H), 2.18-2.36 (m, 3 H), 1.73-1.90 (m, 2 H), 1.61-1.73 (m, 2 H). $^{19}$F NMR (377 MHz, DMSO-$d_6$): δ -63.37, -115.28, -138.88.

**Example 48: 1-{6-[(4-chloro-2-fluorophenyl)methoxy]-3,5-difluoropyridin-2-yl}-4-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperazine (Compound 48)**

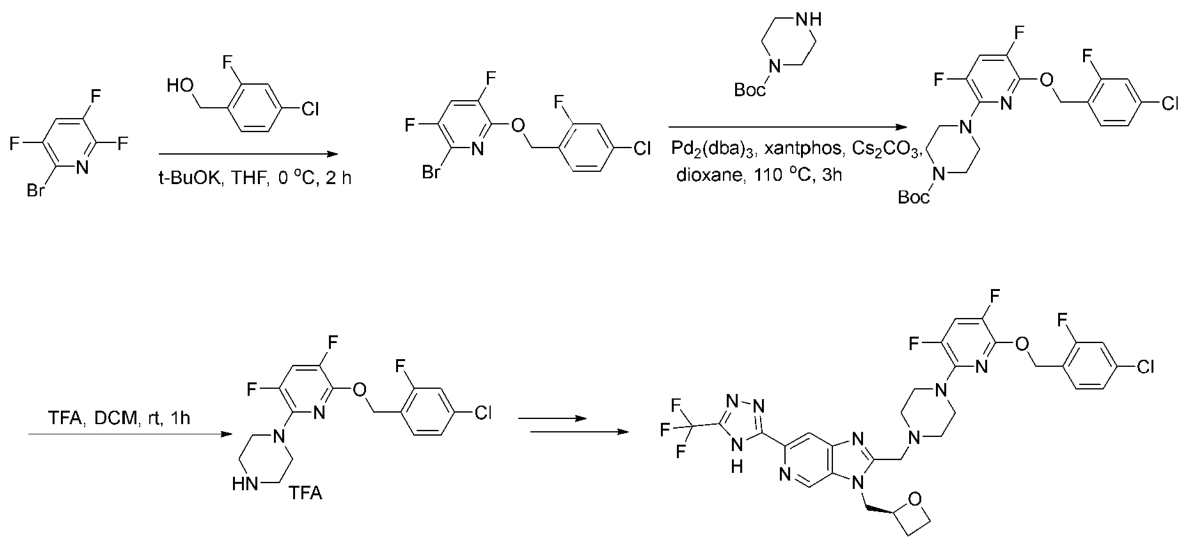


*Step A: 2-bromo-6-((4-chloro-2-fluorobenzyl)oxy)-3,5-difluoropyridine*

To a solution of 2-bromo-3,5,6-trifluoropyridine (2 g, 9.43 mmol) in THF (40 mL) was added (4-chloro-2-fluorophenyl)methanol (1.59 g, 9.91 mmol) and t-BuOK (1 M, 10.37 mL). The solution was stirred at 0°C for 2 hours. After the reaction was completed, the mixture was diluted with $H_2O$ (100 mL), extracted with EA (60 mL×2). The combined organic layer was dried over anhydrous sodium sulfate, filtered and evaporated to dryness. The residue was purified by column chromatography on silica gel (PE) to give 2-bromo-6-((4-chloro-2-fluorobenzyl)oxy)-3,5-difluoropyridine (2.8 g, yield: 85%) as a white solid. MS Calcd.: 350.9; MS Found: 351.9 $[M+H]^+$.

*Step B: tert-butyl 4-(6-((4-chloro-2-fluorobenzyl)oxy)-3,5-difluoropyridin-2-yl)piperazine-1-carboxylate*

A mixture of 2-bromo-6-((4-chloro-2-fluorobenzyl)oxy)-3,5-difluoropyridine (400 mg, 1.14 mmol), tert-butyl piperazine-1-carboxylate (318 mg, 1.71 mmol), $Pd_2(dba)_3$ (104 mg, 0.12 mmol), Xantphos (139 mg, 0.24 mmol), $Cs_2CO_3$ (741 mg, 42.3 mmol) in dioxane (10.0 mL) was stirred at 110 °C for 3 hours. After the reaction was completed, the mixture was diluted with $H_2O$ (40 mL), extracted with EA (30 mL×2). The combined organic layer was dried over anhydrous sodium sulfate, filtered and evaporated to dryness. The residue was purified by column chromatography on silica gel

(PE/EA=20/1) to give tert-butyl 4-(6-((4-chloro-2-fluorobenzyl)oxy)-3,5-difluoropyridin-2-yl)piperazine-1-carboxylate (660 mg, crude) as yellow oil. MS Calcd.: 457.1; MS Found: 402.1 $[M-56+H]^+$.

*Step C: 1-(6-((4-chloro-2-fluorobenzyl)oxy)-3,5-difluoropyridin-2-yl)piperazine TFA Salt*

To a solution of tert-butyl 4-(6-((4-chloro-2-fluorobenzyl)oxy)-3,5-difluoropyridin-2-yl)piperazine-1-carboxylate (350 mg, crude) in DCM (4 mL) was added TFA (1.0 mL). The solution was stirred at room temperature for 1 hour. The reaction was removed in vacuo to give 1-(6-((4-chloro-2-fluorobenzyl)oxy)-3,5-difluoropyridin-2-yl)piperazine TFA Salt (400 mg, crude) as yellow oil. MS Calcd.: 357.1; MS Found: 358.1 $[M+H]^+$.

*Step D: 1-16-[(4-chloro-2-fluorophenyl)methoxy]-3,5-difluoropyridin-2-yl}-4-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperazine (Compound 48)*

1-{6-[(4-chloro-2-fluorophenyl)methoxy]-3,5-difluoropyridin-2-yl}-4-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperazine (Compound 48) (12.8 mg) was obtained with the similar method of Example 1.

MS Calcd.: 693.2; MS Found: 693.9 $[M+H]^+$.

$^1$H NMR (400 MHz, DMSO-d6): δ 9.28 (s, 1 H), 8.37 (s, 1 H), 7.85-7.95 (m, 1 H), 7.56 (t, *J*=8.0 Hz, 1 H), 7.49 (d, *J*=10.0 Hz, 2.0 Hz, 1 H), 7.33 (d, *J*=8.4 Hz, 2.0 Hz, 1 H), 5.42 (s, 2 H), 5.06-5.18 (m, 1 H), 4.87-4.98 (m, 1 H), 4.72-4.81 (m, 1 H), 4.48-4.56 (m, 1 H), 4.37-4.46 (m, 1 H), 3.11-3.40 (m, 7 H), 2.64-2.84 (m, 4 H), 2.29-2.46 (m, 1 H). $^{19}$F NMR(377 MHz, DMSO-d6): δ -63.72, -115.08, - 135.46, -148.17.

**Example 49: 2-[(4-chloro-2-fluorophenyl)methoxy]-3,5-difluoro-6-{1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl}pyridine (Compound 49)**

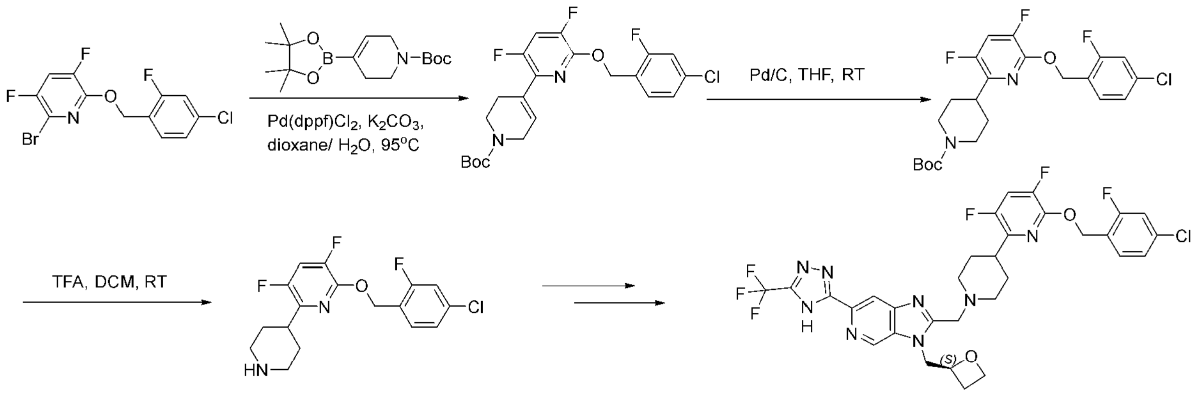


*Step A: tert-butyl 6-((4-chloro-2-fluorobenzyl)oxy)-3,5-difluoro-3',6'-dihydro-[2,4'-bipyridine]-1'(2'H)-carboxylate*

To a solution of 2-bromo-6-((4-chloro-2-fluorobenzyl)oxy)-3,5-difluoropyridine (1.0 g, 2.85 mmol) in dioxane(30 mL) and $H_2O$ (3 mL) was added tert-butyl 4-(4,4,5,5-tetramethyl-1,3,2-dioxaborolan-2-yl)-3,6-dihydropyridine-1(2H)-carboxylate (968 mg, 3.13 mmol) , $K_2CO_3$ (1.2 g, 8.55 mmol) and Pd(dppf)$Cl_2$DCM (204 mg, 0.28 mmol) at RT under $N_2$. The mixture was stirred at 95°C for 16 hours, cooled to room temperature, diluted with ethyl acetate (100 mL), extracted with $H_2O$ (50 mL*2). The organic layer was dried over anhydrous sodium sulfate, filtered and evaporated to dryness. The residue was purified by column chromatography on silica gel (PE/EA=20/1) to give tert-butyl 6-((4-chloro-2-fluorobenzyl) oxy)-3,5-difluoro-3',6'-dihydro-[2,4'-bipyridine]-1'(2'H)-carboxylate (1.15 g, yield: 88.0%) as yellow solid. MS Calcd.: 454.1; MS Found: 399.0 $[M-56+H]^+$.

Step B: tert-butyl 4-(6-((4-chloro-2-fluorobenzyl)oxy)-3,5-difluoropyridin-2-yl)piperidine-1-carboxylate

To a solution of tert-butyl 6-((4-chloro-2-fluorobenzyl)oxy)-3,5-difluoro-3',6'-dihydro-[2,4'-bipyridine]-1'(2'H)-carboxylate (600 mg, 1.32 mmol) in THF (6.0 mL) was added Pd/C (10% w/w, 360 mg). The solution was stirred at room temperature for 2 hours under $H_2$. The mixture was filtered, and the filtrate evaporated to give tert-butyl

4-(6-((4-chloro-2-fluorobenzyl)oxy)-3,5-difluoropyridin-2-yl)piperidine-1-carboxylate (690 mg, crude) as yellow oil. MS Calcd.: 456.1; MS Found: 401.0$[M-56+H]^+$.

*Step C: 2-((4-chloro-2-fluorobenzyl)oxy)-3,5-difluoro-6-(piperidin-4-yl)pyridine TFA salt*

To a solution of tert-butyl 4-(6-((4-chloro-2-fluorobenzyl)oxy)-3,5-difluoropyridin-2-yl)piperidine-1-carboxylate (690 mg, crude) in DCM (6.0 mL) was added TFA (2 mL). The solution was stirred at room temperature for 2 hours. The solvent was removed to give 2-((4-chloro-2-fluorobenzyl)oxy)-3,5-difluoro-6-(piperidin-4-yl)pyridine TFA salt (890 mg, crude) as yellow oil. MS Calcd.: 356.1; MS Found: 357.1 $[M+H]^+$.

*Step D : 2-[(4-chloro-2-fluorophenyl)methoxy]-3,5-difluoro-6-{1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl}pyridine*

2-[(4-chloro-2-fluorophenyl)methoxy]-3.5-difluoro-6-{1-[(3-{[(2S)-oxetan-2-yl]methyl}-6-[5-(trifiluoromethyl)-4H-1,2,4-triazol-3-yl]-3H-imidazo[4,5-c]pyridin-2-yl)methyl]piperidin-4-yl}pyridine (25.23mg) was obtained with the similar method of Example 1. MS Calcd.: 692.2; MS Found: 693.0 $[M+H]^+$.

$^1H$ NMR (400 MHz, DMSO-d6): δ 9.15 (s, 1 H), 8.27 (s, 1 H), 7.89 (t, *J*=9.6 Hz, 1 H), 7.60 (t, *J*=8.0 Hz, 1 H), 7.48 (dd, *J=10.0* Hz, 1.6 Hz, 1 H), 7.32 (dd, *J*=8.0 Hz, 1.6 Hz, 1 H), 5.47 (s, 2 H), 5.15-5.24 (m, 1 H), 4.88-4.97 (m, 1 H), 4.73-4.81 (m, 1 H), 4.40-4.53 (m, 2 H), 4.00 (d, *J*=14.0 Hz, 1 H), 3.86 (d, *J*=13.6 Hz, 1 H), 2.98-3.08 (m, 1 H), 2.85-2.97 (m, 2 H), 2.65-2.80 (m, 1 H), 2.42-2.49 (m, 1 H), 2.19-2.34 (m, 2 H), 1.60-1.90 (m, 4 H). $^{19}F$ NMR (377 MHz, DMSO-d6): δ -63.30, -115.08, -133.85, - 138.38.

**Example 50: 2-[(4-chloro-2-fluorophenyl)methoxy]-3,5-difluoro-6-{1-[(7-{[(2S)-oxetan-2-yl]methyl}-3-[5-(trifluoromethyl)-4H-1,2,4-triazol-3-yl]-7H-imidazo[4,5-c]pyridazin-6-yl)methyl]piperidin-4-yl}pyridine (Compound 50)**

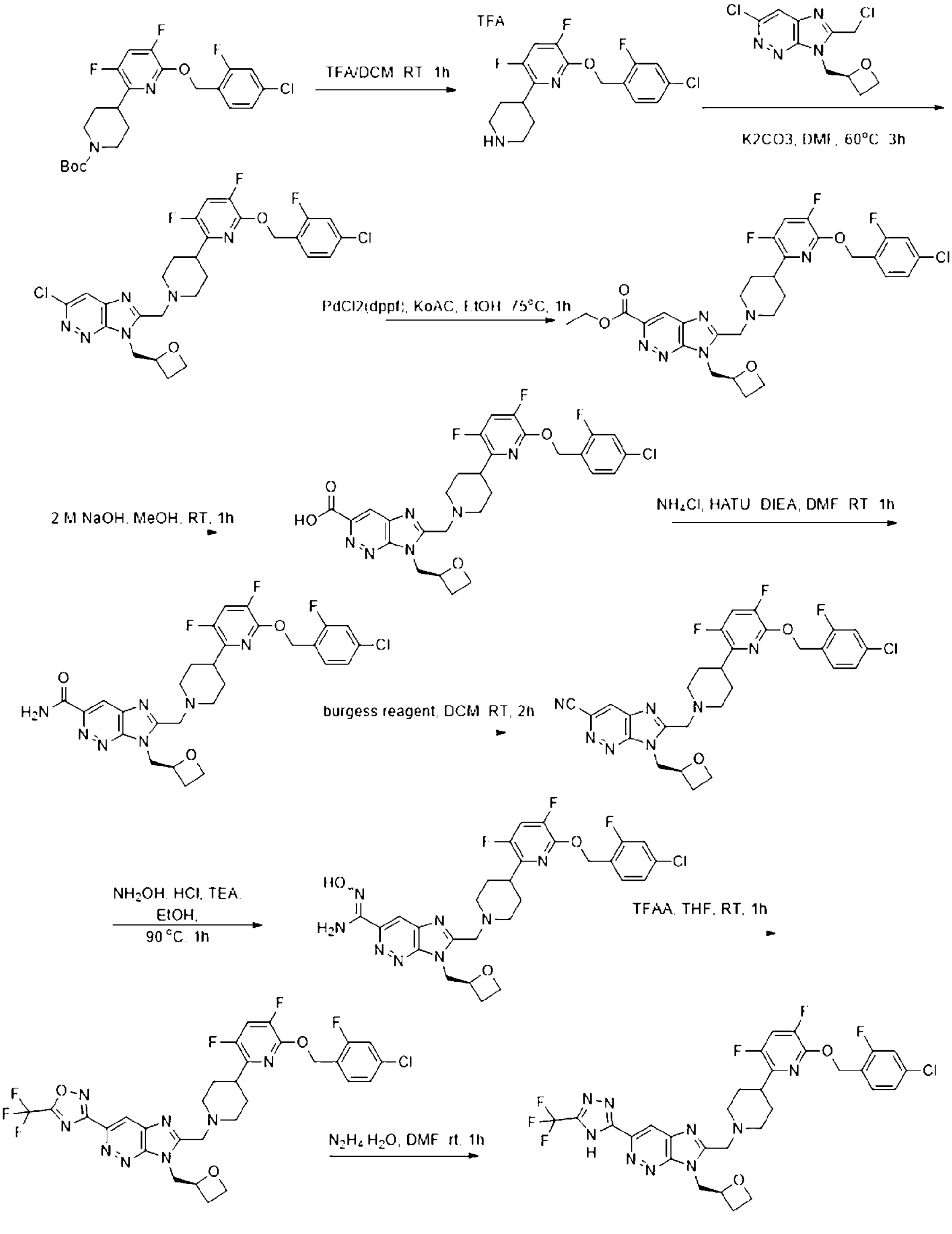


*Step A: 2-((4-chloro-2-fluorobenzyl)oxy)-3,5-difluoro-6-(piperidin-4-yl)pyridine TFA Salt*

To a solution of tert-butyl 4-(6-((4-chloro-2-fluorobenzyl)oxy)-3,5-difluoropyridin-2-yl)piperidine-1-carboxylate (690 mg, 1.51 mmol) in DCM (6 mL) was added TFA (2mL). The solution was stirred at room temperature for 1 hour. The reaction was removed in vacuo to give 2-((4-chloro-2-fluorobenzyl)oxy)-3,5-difluoro-6-(piperidin-4-yl)pyridine TFA Salt (890 mg, crude).

*Step B: (S)-3-chloro-6-((4-(6-((4-chloro-2-fluorobenzyl)oxy)-3,5-difluoropyridin-2-yl)piperidin-1-yl)methyl)-7-(oxetan-2-ylmethyl)-7H-imidazo[4,5-c]pyridazine*

A mixture of 2-((4-chloro-2-fluorobenzyl)oxy)-3,5-difluoro-6-(piperidin-4-yl)pyridine TFA salt (600 mg, crude),

(S)-3-chloro-6-(chloromethyl)-7-(oxetan-2-ylmethyl)-7H-imidazo[4,5-c]pyridazine (350 mg, 1.28mmol), $K_2CO_3$ (648 mg, 5.00 mmol) in DMF (6 mL) was stirred at 60 °C for 3 hours. After the reaction was completed, the mixture was diluted with $H_2O$ (30 mL), extracted with EA (30 mL×2). The combined organic layer was dried over anhydrous sodium sulfate, filtered and evaporated to dryness. The residue was purified by column chromatography on silica gel (PE/EA=5/1) to give (S)-3-chloro-6-((4-(6-((4-chloro-2-fluorobenzyl)oxy)-3,5-difluoropyridin-2-yl)piperidin-1-yl)methyl)-7-(oxetan-2-ylmethyl)-7H-imidazo[4,5-c]pyridazine (400 mg, yield: 53%). MS Calcd.: 592.1; MS Found: 593.0 [M+H]$^+$.

*Step C: ethyl (S)-6-((4-(6-((4-chloro-2-fluorobenzyl)oxy)-3,5-difluoropyridin-2-yl)piperidin-1-yl)methyl)-7-(oxetan-2-ylmethyl)-7H-imidazo[4,5-c]pyridazine-3-carboxylate*

To a mixture of (S)-3-chloro-6-((4-(6-((4-chloro-2-fluorobenzyl)oxy)-3,5-difluoropyridin-2-yl)piperidin-1-yl)methyl)-7-(oxetan-2-ylmethyl)-7H-imidazo[4,5-c]pyridazine (360 mg, 0.62 mmol) in EtOH (6.0 mL) were added $PdCl_2$(dppf) (90.5 mg, 0.12 mmol) and KOAc (182 mg, 1.86 mmol). The mixture was stirred at 75°C for 1 hour under CO. After the reaction was completed, the reaction mixture was filtered, and the filtrate evaporated to dryness. The residue was purified by column chromatography on silica gel (DCM/MeOH=80/1) to give ethyl (S)-6-((4-(6-((4-chloro-2-fluorobenzyl)oxy)-3,5-difluoropyridin-2-yl)piperidin-1-yl)methyl)-7-(oxetan-2-ylmethyl)-7H-imidazo[4,5-c]pyridazine-3-carboxylate (330 mg, yield: 76%). MS Calcd.: 630.2; MS Found: 631.2 [M+H]$^+$.

*Step D: (S)-6-((4-(6-((4-chloro-2-fluorobenzyl)oxy)-3,5-difluoropyridin-2-yl)piperidin-1-yl)methyl)-7-(oxetan-2-ylmethyl)-7H-imidazo[4,5-c]pyridazine-3-carboxylic acid*

To a mixture of ethyl (S)-6-((4-(6-((4-chloro-2-fluorobenzyl)oxy)-3,5-difluoropyridin-2-yl)piperidin-1-yl)methyl)-7-(oxetan-2-ylmethyl)-7H-imidazo[4,5-c]pyridazine-3-carboxylate (330 mg, 0.52 mmol) in MeOH (4.0 mL) was added NaOH (2 M, 1.0 mL, aq.). The mixture was stirred at RT for 1 h. After the reaction was completed, the mixture was adjusted to pH=7 with 1 N HCl (aq.) and extracted with EA (30 mL×2). The combined organic layer was dried over anhydrous sodium sulfate, filtered and evaporated to give (S)-6-((4-(6-((4-chloro-2-fluorobenzyl)oxy)-3,5-difluoropyridin-2-yl)piperidin-1-yl)methyl)-7-(oxetan-2-ylmethyl)-7H-imidazo[4,5-c]pyridazine-3-carboxylic acid (300 mg, yield: 95%). MS Calcd.: 602.2; MS Found: 603.0 [M+H]$^+$.

*Step E: (S)-6-((4-(6-((4-chloro-2-fluorobenzyl)oxy)-3,5-difluoropyridin-2-yl)piperidin-1-yl)methyl)-7-(oxetan-2-ylmethyl)-7H-imidazo[4,5-c]pyridazine-3-carboxamide*

A mixture of (S)-6-((4-(6-((4-chloro-2-fluorobenzyl)oxy)-3,5-difluoropyridin-2-yl)piperidin-1-yl)methyl)-7-(oxetan-2-ylmethyl)-7H-imidazo[4,5-c]pyridazine-3-carboxylic acid (300 mg, 0.50 mmol), $NH_4Cl$ (40 mg, 0.75 mmol), HATU (285 mg, 0.75 mmol), DIEA (129 mg, 1.00 mmol) in DMF (5.0 mL) was stirred at RT for 1 hour. After the reaction was completed, the mixture was diluted with $H_2O$ (30 mL), extracted with EA (20 mL×2). The combined organic layer was dried over anhydrous sodium sulfate, filtered and evaporated to dryness. The residue was purified by column chromatography on silica gel (DCM/MeOH=80/1) to give (S)-6-((4-(6-((4-chloro-2-fluorobenzyl)oxy)-3,5-difluoropyridin-2-yl)piperidin-1-yl)methyl)-7-(oxetan-2-ylmethyl)-7H-imidazo[4,5-c]pyridazine-3-carboxamide (220 mg, yield: 73%). MS Calcd.: 601.2; MS Found: 602.2 [M+H]$^+$.

*Step F: (S)-6-((4-(6-((4-chloro-2-fluorobenzyl)oxy)-3,5-difluoropyridin-2-yl)piperidin-1-yl)methyl)-7-(oxetan-2-ylmethyl)-7H-imidazo[4,5-c]pyridazine-3-carbonitrile*

A mixture of (S)-6-((4-(6-((4-chloro-2-fluorobenzyl)oxy)-3,5-difluoropyridin-2-yl)piperidin-1-yl)methyl)-7-(oxetan-2-ylmethyl)-7H-imidazo[4,5-c]pyridazine-3-carboxamide (220 mg, 0.37 mmol), Burgess reagent (349 mg, 1.46 mmol) in DCM (4.0 mL) was stirred at RT for 2 hours. After the reaction was completed, the mixture was diluted with $H_2O$ (20 mL), extracted with EA (10 mL×2). The combined organic layer was dried over anhydrous sodium sulfate, filtered and evaporated to dryness. The organic layer was dried over sodium sulfate, filtered and concentrated in vacuum. The residue was purified by column chromatography on silica gel (DCM/MeOH=60/1) to give (S)-6-((4-(6-((4-chloro-2-fluorobenzyl)oxy)-3,5-difluoropyridin-2-yl)piperidin-1-yl)methyl)-7-(oxetan-2-ylmethyl)-7H-imidazo[4,5-c]pyridazine-3-carbonitrile (140 mg, yield: 66%). MS Calcd.: 583.2; MS Found: 584.1 [M+H]$^+$.

*Step G: (S)-6-((4-(6-((4-chloro-2-fluorobenzyl)oxy)-3,5-difluoropyridin-2-yl)piperidin-1-yl)methyl)-N'-hydroxy-7-(oxetan-2-ylmethyl)-7H-imidazo[4,5-c]pyridazine-3-carboximidamide*

A solution of (S)-6-((4-(6-((4-chloro-2-fluorobenzyl)oxy)-3,5-difluoropyridin-2-yl)piperidin-1-yl)methyl)-7-(oxetan-2-ylmethyl)-7H-imidazo[4,5-c]pyridazine-3-carbonitrile (140 mg, 0.24 mmol), $HONH_2 \cdot HCl$ (100.13 mg, 1.44 mmol),

TEA (96.96 mg, 0.96 mmol) in EtOH (4 mL) was stirred at 90°C for 1 h. After the reaction was completed, the mixture was filtered and the solid dried to give (S)-6-((4-(6-((4-chloro-2-fluorobenzyl)oxy)-3,5-difluoropyridin-2-yl)piperidin-1-yl)methyl)-N'-hydroxy-7-(oxetan-2-ylmethyl)-7H-imidazo[4,5-c]pyridazine-3-carboximidamide (110 mg, yield: 74%) as a white solid. MS Calcd.: 616.2; MS Found: 617.2 $[M+H]^+$.

*Step H:(S)-3-(6-((4-(6-((4-chloro-2-fluorobenzyl)oxy)-3,5-difluoropyridin-2-yl)piperidin-1-yl)methyl)-7-(oxetan-2-ylmethyl)-7H-imidazo[4,5-c]pyridazin-3-yl)-5-(trifluoromethyl)-1,2,4-oxadiazole*

To a solution of (S)-6-((4-(6-((4-chloro-2-fluorobenzyl)oxy)-3,5-difluoropyridin-2-yl)piperidin-1-yl)methyl)-N'-hydroxy-7-(oxetan-2-ylmethyl)-7H-imidazo[4,5-c]pyridazine-3-carboximidamide (110 mg, 0.18 mmol) in THF (2 mL) was added TFAA (74.99 mg, 0.36 mmol) and the mixture stirred at RT for 1 hour. After the reaction was completed, the reaction was quenched with sat. sodium bicarbonate aqueous solution (5.0 mL), extracted with ethyl acetate (15 mL×3). The organic layers were combined, dried over $Na_2SO_4$, filtered and concentrated under vacuum to dryness. The residue was purified by Prep-TLC (DCM/MeOH=30/1) to give (S)-3-(6-((4-(6-((4-chloro-2-fluorobenzyl)oxy)-3,5-difluoropyridin-2-yl)piperidin-1-yl)methyl)-7-(oxetan-2-ylmethyl)-7H-imidazo[4,5-c]pyridazin-3-yl)-5-(trifluoromethyl)-1,2,4-oxadiazole (80 mg, yield: 65%) as a yellow solid. MS Calcd.: 694.2; MS Found: 695.2 $[M+H]^+$.

*Step I: 2-[(4-chloro-2-fluorophenyl)methoxy]-3,5-difluoro-6-{1-[(7-{[(2S)-oxetan-2-yl]methyl}-3-[5-(trifluoromethyl)-4H-1,2,4-triazol-3-yl]-7H-imidazo[4,5-c]pyridazin-6-yl)methyl]piperidin-4-yl}pyridine (Compound 50)*

A mixture of (S)-3-(6-((4-(6-((4-chloro-2-fluorobenzyl)oxy)-3,5-difluoropyridin-2-yl)piperidin-1-yl)methyl)-7-(oxetan-2-ylmethyl)-7H-imidazo[4,5-c]pyridazin-3-yl)-5-(trifluoromethyl)-1,2,4-oxadiazole (80 mg, 0.12 mmol) and $N_2H_4 \cdot H_2O$ (12.00 mg, 0.24 mmol) in DMF (2 mL) was stirred at RT for 1 hour. After the reaction was completed, the mixture was purified directly by Prep-HPLC (0.1% FA/$H_2O$/$CH_3CN$) to give 2-[(4-chloro-2-filuorophenyl)methoxy]-3,5-difiluoro-6-{1-[(7-{ [(2S)-oxetan-2-yl]methyl}-3-[5-(trifluoromethyl)-4H-1,2,4-triazol-3-yl]-7H-imidazo[4,5-c]pyridazin-6-yl)methyl]piperidin-4-yl}pyridine (30.8 mg, yield: 39%) as a white solid. MS Calcd.: 693.2; MS Found: 694.0 $[M+H]^+$.

$^1$H NMR (400 MHz, DMSO-d6): δ 8.49 (s, 1 H), 7.89 (t, J=9.2 Hz, 1 H), 7.58 (t, J=8.4 Hz, 1 H), 7.49 (dd, *J*=10.0 Hz, 2.0 Hz, 1 H), 7.32 (dd, *J*=8.0 Hz, 1.6 Hz, 1 H), 5.46 (s, 2 H), 5.25-5.36 (m. 1 H), 4.99-5.09 (m. 1 H), 4.87-4.96 (m, 1 H), 4.39-4.55 (m, 2 H), 4.01-4.09 (m, 2 H), 2.86-3.06 (m, 3 H), 2.70-2.82 (m, 1 H), 2.50-2.63 (m, 1 H), 2.26-2.38 (m, 2 H), 1.77-1.93 (m, 2 H), 1.64-1.75 (m, 2 H). $^{19}$F NMR(377 MHz, DMSO-d6): δ -63.28, -114.99, -133.85, -138.37.

**Example 51: 1-{3-[(4-chloro-2-fluorophenyl)methoxy]-4-fluorophenyl}-4-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperazin-2-one (Compound 51)**

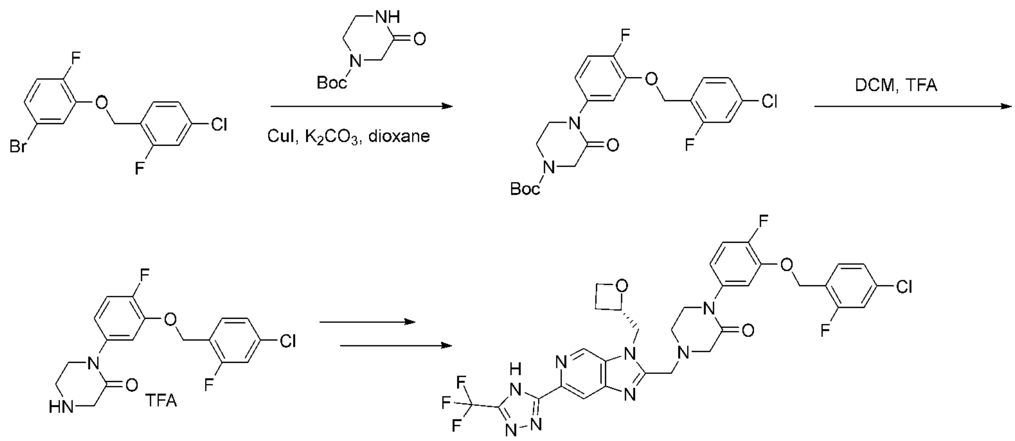


*Step A: tert-butyl 4-(3-((4-chloro-2-fluorobenzyl)oxy)-4-fluorophenyl)-3-oxopiperazine-1-carboxylate*

A mixture of 4-bromo-2-((4-chloro-2-fluorobenzyl)oxy)-1-fluorobenzene (1.0 g, 3.0 mmol), tert-butyl 3-oxopiperazine-1-carboxylate (602 mg, 3.0 mmol), CuI (57 mg, 0.3 mmol), $K_2CO_3$ (831 mg, 6.0 mmol) and (*1R,2R*)-$N^1$,$N^2$-dimethylcyclohexane-1,2-diamine (43 mg, 0.3 mmol) in dioxane (15 mL) was stirred at 120°C for 16 hours. After the reaction was completed, the reaction was filtered, and the filtrate concentrated in vacuum. The crude product was purified by column chromatography (PE:EA=2:1) to give tert-butyl 4-(3-((4-chloro-2-fluorobenzyl)oxy)-4-fluorophenyl)-3-oxopiperazine-1-carboxylate (498 mg, yield: 81%) as a white solid. MS Calcd.:452.1; MS Found: 397.2 [M-56+H]$^+$.

*Step B: 1-(3-((4-chloro-2-fluorobenzyl)oxy)-4-fluorophenyl)piperazin-2-one TFA salt*

A mixture of tert-butyl 4-(3-((4-chloro-2-fluorobenzyl)oxy)-4-fluorophenyl)-3-oxopiperazine-1-carboxylate (150 mg, 0.33 mmol), TFA (1 mL) in DCM (3 mL) was stirred at room temperature for 1 hour. After the reaction was completed, the reaction was concentrated in vacuum to give 1-(3-((4-chloro-2-fluorobenzyl)oxy)-4-fluorophenyl)piperazin-2-one TFA salt (176 mg, crude) as yellow oil. MS Calcd: 352.1; MS Found:353.1 [M+H]$^+$.

*Step C: 1-(3-[(4-chloro-2-fluorophenyl)methoxy]-4-fluorophenyl}-4-[(3-{[(2S)-oxetan-2-yl]methyl}-6-[5-(trifluoromethyl)-4H-1,2,4-triazol-3-yl]-3H-imidazo[4,5-c]pyridin-2-yl)methylpiperazin-2-one*

1-{3-[(4-chloro-2-fluorophenyl)methoxy]-4-fluorophenyl}-4-[(3-{[(2S)-oxetan-2-yl]methyl}-6-[5-(trifiluoromethyl)-4H-1,2,4-triazol-3-yl]-3H-imidazo[4,5-c]pyridin-2-yl)methyl]piperazin-2-one was obtained with the similar method of Example 1 (41.7 mg). MS Calcd.:688.2; MS Found: 689.3 [M+H]$^+$.

$^1$H NMR (400 MHz, DMSO-$d_6$): 1H NMR: δ 9.19 (s, 1 H), 8.32 (s, 1 H), 7.61 (t, *J*=8.4 Hz, 1 H), 7.51 (dd, *J*=10.0 Hz, *J*=2.0 Hz, 1 H), 7.32-7.38 (m, 2 H), 7.25 (dd, *J*=11.2 Hz, *J*=8.8 Hz, 1 H), 6.90-6.98 (m, 1 H), 5.10-5.19 (m, 3 H), 4.87-4.96 (m, 1 H), 4.73-4.81 (m, 1 H), 4.47-4.55 (m, 1 H), 4.38-4.44 (m, 1 H), 4.14 (d, *J*=14.0 Hz, 1 H), 4.05 (d, *J=1*4.0 Hz, 1 H), 3.62-3.69 (m, 2 H), 3.38-3.47 (m, 2 H), 2.93-3.00 (m, 2 H), 2.69-2.80 (m, 1 H), 2.40-2.51 (m, 1 H). $^{19}$F NMR (377 MHz, DMSO-*d*6): δ -63.70, - 115.03, -136.54.

**Example 52: 1-{6-[(4-chloro-2-fluorophenyl)methoxy]-5-fluoropyridin-2-yl}-4-[(3-{[(2S)-oxetan-2-yl] methyl}-6-[5-(trifluoromethyl)-4H-1,2,4-triazol-3-yl]-3H-imidazo[4,5-c]pyridin-2-yl)methyl]piperazin-2-one (Compound 52)**

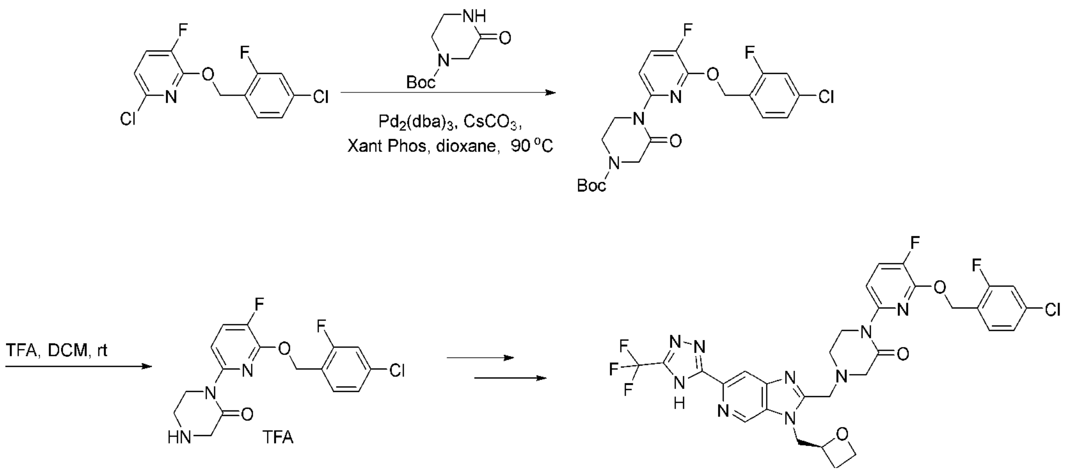


*Step A: tert-butyl 4-(6-((4-chloro-2-fluorobenzyl)oxy)-5-fluoropyridin-2-yl)-3-oxopiperazine-1-carboxylate*

To a solution of 6-chloro-2-((4-chloro-2-fluorobenzyl)oxy)-3-fluoropyridine (600 mg, 2.08 mmol) in dioxane (25 mL) was added Xantphos (361 mg, 0.62 mmol), $Pd_2(dba)_3$ (380 mg, 0.42 mmol), $Cs_2CO_3$ (1.35 g, 4.14 mmol) and tert-butyl 3-oxopiperazine-1-carboxylate (830 mg, 4.15 mmol). The mixture was stirred at 90 °C for 16 hours under $N_2$. The reaction was quenched with $H_2O$ (50 mL), extracted with ethyl acetate (70 mL × 2). The organic layer was dried over anhydrous sodium sulfate, filtered and evaporated to dryness. The residue was purified by column chromatography on silica gel (PE/EA=4/1) to give tert-butyl 4-(6-((4-chloro-2-fluorobenzyl)oxy)-5-fluoropyridin-2-yl)-3-oxopiperazine-1-carboxylate (230 mg, 24.5% yield) as yellow oil. MS Calcd.: 453.1; MS Found: 454.1 [M+H]$^+$.

*Step B: 1-(6-((4-chloro-2-fluorobenzyl)oxy)-5-fluoropyridin-2-yl)piperazin-2-one*

To a solution of tert-butyl 4-(6-((4-chloro-2-fluorobenzyl)oxy)-5-fluoropyridin-2-yl)-3-oxopiperazine-1-carboxylate (210 mg, 0.46 mmol) in DCM (5 mL) was added TFA (1.25 mL). The solution was stirred at room temperature for 1 hour. The reaction was removed in vacuo to give 1-(6-((4-chloro-2-fluorobenzyl)oxy)-5-fluoropyridin-2-yl)piperazin-2-one TFA

Salt (256 mg, crude) as yellow oil. MS Calcd.: 353.1; MS Found: 354.0 [M+H]+.

*Step C: 1-{6-[(4-chloro-2-fluorophenyl)methoxy]-5-fluoropyridin-2-yl}-4-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperazin-2-one*

1-{6-[(4-chloro-2-fluorophenyl)methoxy]-5-fluoropyridin-2-yl}-4-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperazin-2-one (66.4 mg) was obtained with the similar method of Example 1. MS Calcd.: 689.2; MS Found: 690.1 [M+H]+.

$^1$H NMR (400 MHz, DMSO-*d6*): δ 9.18 (s, 1 H), 8.31 (s, 1 H), 7.75 (t, *J*=9.2 Hz, 1 H), 7.51-7.60 (m, 2 H), 7.48 (dd, *J*=10.4 Hz, 2.0 Hz, 1 H), 7.33 (dd, *J*=8.0 Hz, 1.6 Hz,1 H), 5.44 (s, 2 H), 5.09-5.18 (m, 1 H), 4.86-4.96 (m, 1 H), 4.72-4.81 (m, 1 H), 4.46-4.54 (m, 1 H), 4.37-4.43 (m, 1 H), 4.13 (d, *J=13.6* Hz, 1 H), 4.03 (d, *J=13.6* Hz, 1 H), 3.82-3.90 (m, 2 H), 3.383.51 (m, 2 H), 2.93-3.00 (m, 2 H), 2.65-2.77 (m, 1 H), 2.37-2.47 (m, 1 H). $^{19}$F NMR (377 MHz, DMSO-$d_6$): δ -63.58, -115.00, -144.94.

**Example 53: 4-[2-(2,4-difluorophenyl)-2-methyl-2H-1,3-benzodioxol-4-yl]-1-[(3-{[(2S)-oxetan-2-yl]methyl}-6-[5-(trifluoromethyl)-4H-1,2,4-triazol-3-yl]-3H-imidazo[4,5-b]pyridin-2-yl)methyl]piperidine (Compound 53)**

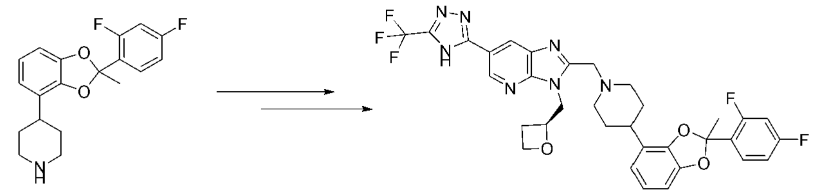

Starting from 4-(2-(2,4-difluorophenyl)-2-methylbenzo[d][1,3]dioxol-4-yl)piperidine,4-[2-(2,4-difluorophenyl)-2-methyl-2H-1,3-benzodioxol-4-yl]-1-[(3-{[(2S)-oxetan-2-yl]methyl}-6-[5-(trifluoromethyl)-4H-1,2,4-triazol-3-yl]-3H-imidazo[4,5-b]pyridin-2-yl)methyl]piperidine was obtained with the similar method of Example 1. MS Calcd.: 667.2; MS Found: 668.0 [M+H]+.

$^1$H NMR (400 MHz, DMSO-d6): δ 9.00 (d, *J*=2.0 Hz, 1 H), 8.59 (d, *J*=2.0 Hz, 1 H), 7.55-7.65 (m. 1 H), 7.30-7.40 (m. 1 H), 7.07-7.16 (m. 1 H), 6.70-6.83 (m. 3 H), 5.15-5.27 (m. 1 H), 4.82-4.91 (m. 1 H), 4.69-4.78 (m, 1 H), 4.44-4.53 (m, 1 H), 4.34-4.43 (m, 1 H), 3.90-4.04 (m, 2 H), 2.91-3.06 (m, 2 H), 2.61-2.74 (m, 2 H), 2.42-2.52 (m, 1 H), 2.20-2.36 (m, 2 H), 2.02 (s, 3 H), 1.69-1.87 (m, 4 H). $^{19}$F NMR(377 MHz, DMSO-d6): δ -63.64, -108.51, -108.53, -109.09, -109.10, -109.11.

**Example 54: 4-[2-(2,4-difluorophenyl)-2-methyl-2H-1,3-benzodioxol-4-yl]-1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidine (Compound 54)**

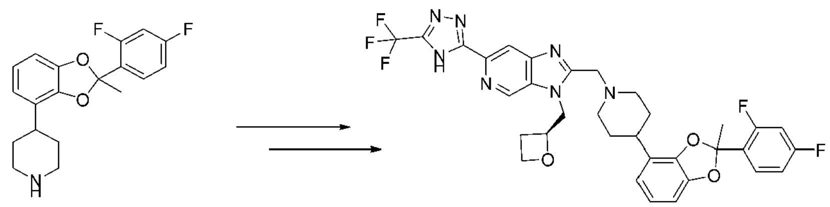

Starting from 4-(2-(2,4-difluorophenyl)-2-methylbenzo[d][1,3]dioxol-4-yl)piperidine, 4-[2-(2,4-difluorophenyl)-2-methyl-2H-1,3-benzodioxol-4-yl]-1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidine was obtained with the similar method of Example 1. MS Calcd.: 667.2; MS Found: 668.4 [M+H]+.

$^1$H NMR (400 MHz, DMSO-d6): δ 9.17 (s, 1 H), 8.29 (s, 1 H), 7.57-7.64 (m. 1 H), 7.32-7.40 (m. 1 H), 7.08-7.15 (m. 1 H), 6.70-6.81 (m. 3 H), 5.14-5.23 (m. 1 H), 4.88-4.97 (m. 1 H), 4.72-4.79 (m, 1 H), 4.39-4.53 (m, 2 H), 4.02 (d, *J*=12.8 Hz, 1 H), 3.86 (d, *J=13.6* Hz, 1 H), 3.00-3.08 (m, 1 H), 2.85-2.93 (m, 1 H), 2.60-2.80 (m, 2 H), 2.43-2.52 (m, 1 H), 2.17-2.35 (m, 2 H), 2.02 (s, 3 H), 1.68-1.85 (m, 4 H). $^{19}$F NMR(377 MHz, DMSO-d6): δ -63.72, -108.48, -108.50, -109.07, -109.10, -109.11,

-109.14.

**Example 55: 2-methyl-5-[2-methyl-4-{1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl}-2H-1,3-benzodioxol-2-yl]pyridine (Compound 55)**

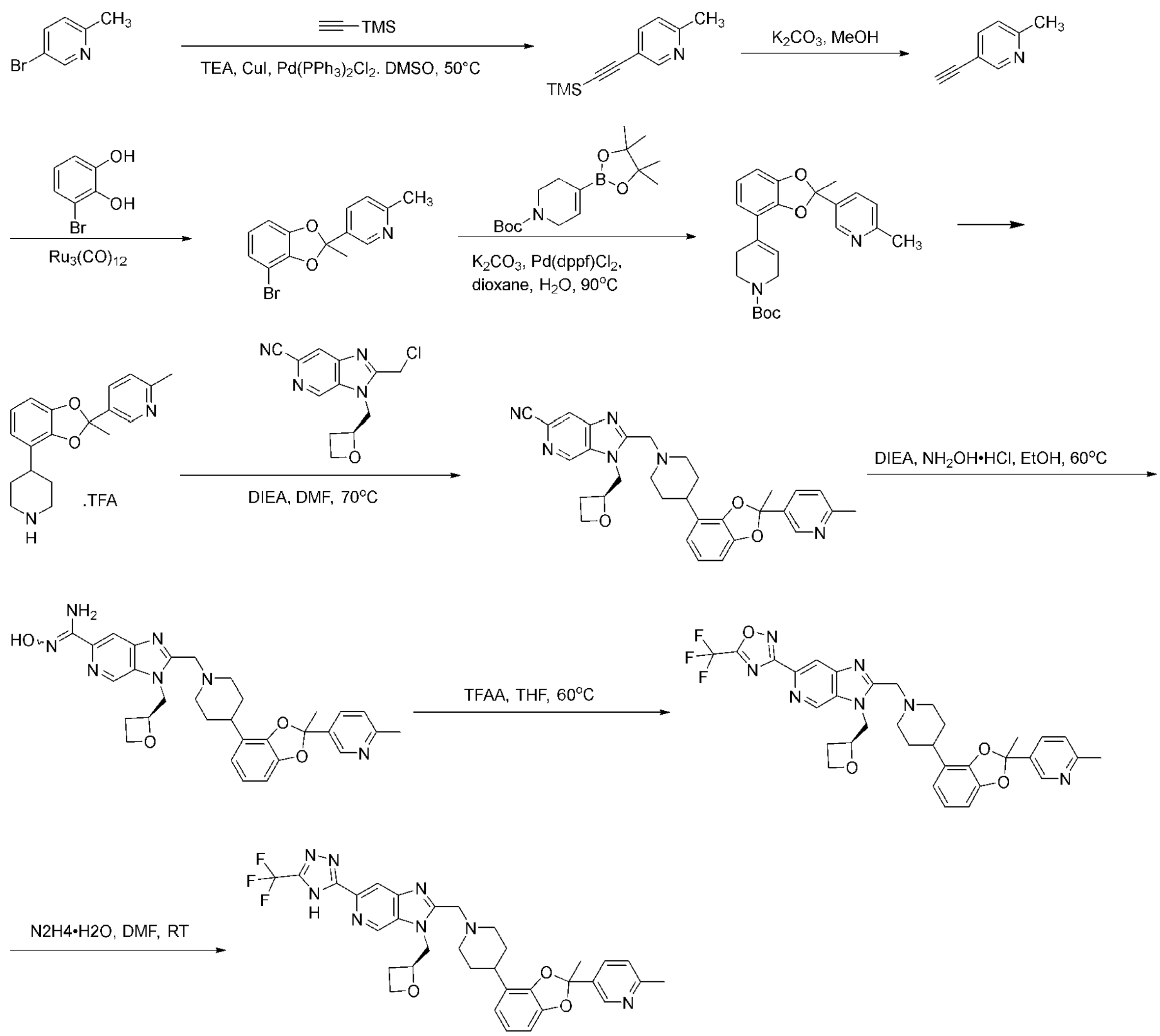


*Step A: 2-methyl-5-((trimethylsilyl)ethynyl)pyridine*

A mixture of 5-bromo-2-methylpyridine (30.0 g, 174 mmol), ethynyltrimethylsilane (18.8 g, 192 mmol), CuI (663 mg, 3.5 mmol), $Pd(PPh_3)_2Cl_2$ (4.9 g, 7.0 mmol) in DMSO (300 mL) and TEA (24 ml) was stirred at 50°C for 16h. After the reaction was completed, the mixture was washed with $H_2O$ (3 L X 2), extracted with EA (1.5 L). The organic layer was dried over anhydrous sodium sulfate, filtered and evaporated to dryness. The residue was purified by column chromatography on silica gel (PE/EA=10/1) to give 2-methyl-5-((trimethylsilyl)ethynyl)pyridine (29.5 g, 90% yield) as yellow oil. MS Calcd.: 189.1; MS Found: 190.1 $[M+H]^+$.

*Step B: 5-ethynyl-2-methylpyridine*

A mixture of 2-methyl-5-((trimethylsilyl)ethynyl)pyridine (29.5 g, 156 mmol), $K_2CO_3$(107.7 g, 780 mmol) in MeOH (300 mL) was stirred at RT overnight. After the reaction was completed, the mixture was filtered and the filtrate evaporated to dryness and the residue was purified by column chromatography on silica gel (PE/EA=15/1) to give 5-ethynyl-2-methylpyridine (4.1 g, 22.6% yield) as a light yellow solid. MS Calcd.: 117.1; MS Found: 118.2 $[M+H]^+$.

*Step C: 5-(4-bromo-2-methylbenzo[d][1,3]dioxol-2-yl)-2-methylpyridine*

A mixture of 3-bromobenzene-1,2-diol (5.4 g, 28.5 mmol), 5-ethynyl-2-methylpyridine (4.0 g, 34.2 mmol) and $Ru_3(CO)_{12}$ (910 mg, 1.42 mmol) in toluene (40 mL) was stirred at 110°C for 16 hours under $N_2$. After the reaction was completed, the reaction mixture was filtered, and the filtrate evaporated to dryness. The residue was purified by column chromatography on silica gel (PE/EA=20/1) to give 5-(4-bromo-2-methylbenzo[d][1,3]dioxol-2-yl)-2-methylpyridine (2.3 g, yield: 26.3%) as a yellow oil. MS Calcd.: 305.0; MS Found: 305.9 $[M+H]^+$.

*Step D: tert-butyl 4-(2-methyl-2-(6-methylpyridin-3-yl)benzo[d][1,3]dioxol-4-yl)-3,6-dihydropyridine-1(2H)-carboxylate*

A mixture of 5-(4-bromo-2-methylbenzo[d][1 ,3]dioxol-2-yl)-2-methylpyridine (2.3 g, 7.5 mmol), tert-butyl 4-(4,4,5,5-tetramethyl-1,3,2-dioxaborolan-2-yl)-3,6-dihydropyridine-1(2H)-carboxylate (2.7 g, 8.6 mmol), $K_2CO_3$ (3.0 g, 21.6 mmol), Pd(dppf)$Cl_2$ (294 mg, 0.36 mmol) in dioxane/$H_2O$ (20 ml/2 ml) was stirred at 90°C for 16 hours. After the reaction was completed, the mixture was filtered and the filtrate evaporated to dryness and the residue was purified by column chromatography on silica gel (PE/EA=3/1) to give tert-butyl 4-(2-methyl-2-(6-methylpyridin-3-yl)benzo[d][1,3]dioxol-4-yl)-3,6-dihydropyridine-1(2H)-carboxylate (2.5 g, yield: 84.9%) as yellow oil. MS Calcd.: 408.2; MS Found: 409.3 $[M+H]^+$.

*Step E: 2-((4-(2-methyl-2-(6-methylpyridin-3-yl)benzo[d][1,3]dioxol-4-yl)piperidin-1-yl)methyl)-3-(((S)-oxetan-2-yl)methyl)-3H-imidazo[4,5-c]pyridine-6-carbonitrile*

To a solution of 2-methyl-5-(2-methyl-4-(piperidin-4-yl)benzo[d][1,3]dioxol-2-yl)pyridine TFA salt (225 mg, crude) in DMF (2 mL) was added DIEA (315 mg, 2.4 mmol). After 2 minutes, (S)-2-(chloromethyl)-3-(oxetan-2-ylmethyl)-3H-imidazo[4,5-c]pyridine-6-carbonitrile (71 mg, 0.27 mmol) was added. The mixture was stirred at 70°C for 1 hours. After the reaction was completed, the mixture was evaporated to dryness. The residue was purified by Prep-TLC (EA=100%) to give 2-((4-(2-methyl-2-(6-methylpyridin-3-yl)benzo[d][1,3]dioxol-4-yl)piperidin-1-yl)methyl)-3-(((S)-oxetan-2-yl)methyl)-3H-imidazo[4,5-c]pyridine-6-carbonitrile (110 mg, 84.1% yield) as a light yellow solid. MS Calcd.: 536.2; MS Found: 537.4 $[M+H]^+$

*Step H: N-hydroxy-2-((4-(2-methyl-2-(6-methylpyridin-3-yl)benzo[d][1,3]dioxol-4-yl)piperidin-1-yl)methyl)-3-(((S)-oxetan-2-yl)methyl)-3H-imidazo[4,5-c]pyridine-6-carboximidamide*

A solution of 2-((4-(-2-methyl-2-(6-methylpyridin-3-yl)benzo[d][1,3]dioxol-4-yl)piperidin-1-yl)methyl)-3-(((S)-oxetan-2-yl)methyl)-3H-imidazo[4,5-c]pyridine-6-carbonitrile (110 mg, 0.20 mmol), $NH_2OH \cdot HCl$ (43 mg, 0.62 mmol), DIEA (80 mg, 0.62 mmol) in EtOH (1 mL) was stirred at 60°C for 1 h. After the reaction was completed, the mixture was filtered to give N-hydroxy-2-((4-(2-methyl-2-(6-methylpyridin-3-yl)benzo[d][1,3]dioxol-4-yl)piperidin-1-yl)methyl)-3-(((S)-oxetan-2-yl)methyl)-3H-imidazo[4,5-c]pyridine-6-carboximidamide (150 mg, crude) as yellow solid. MS Calcd.: 569.3; MS Found: 570.4 $[M+H]^+$.

*Step I: 2-methyl-5-(2-methyl-4-{1-[(3-{[(2S)-oxetan-2-yl]methyl}-6-[5-(trifluoromethyl)-1,2,4-oxadiazol-3-yl]-3H-imidazo[4,5-c]pyridin-2-yl)methyl]piperidin-4-yl}-2H-1,3-benzodioxol-2-yl)pyridine*

To a solution of N-hydroxy-2-((4-(2-methyl-2-(6-methylpyridin-3-yl)benzo[d][1,3]dioxol-4-yl)piperidin-1-yl)methyl)-3-(((S)-oxetan-2-yl)methyl)-3H-imidazo[4,5-c]pyridine-6-carboximidamide (150 mg) in THF (2 mL) was added TFAA (5 drops) and the mixture stirred at 60°C for 1 hour. After the reaction was completed, the reaction was quenched with sodium bicarbonate aqueous solution (10 mL), extracted with ethyl acetate (15 mL×3). The organic layers were combined, dried over $Na_2SO_4$, filtered and concentrated under vacuum to dryness. The residue was purified by prep-TLC (DCM/MeOH=60/1) to give 2-methyl-5-(2-methyl-4-{1-[(3-{[(2S)-oxetan-2-yl]methyl}-6-[5-(trifluoromethyl)-1,2,4-oxadiazol-3-yl]-3H-imidazo[4,5-c]pyridin-2-yl)methyl]piperidin-4-yl}-2H-1,3-benzodioxol-2-yl)pyridine (105 mg, yield: 79.1 %) as yellow oil. MS Calcd.: 647.2; MS Found: 648.4 $[M+H]^+$.

*Step J: 2-methyl-5-[2-methyl-4-{1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl}-2H-1,3-benzodioxol-2-yl]pyridine*

A mixture of 2-methyl-5-(2-methyl-4-{1-[(3-{[(2S)-oxetan-2-yl]methyl}-6-[5-(trifluoromethyl)-1,2,4-oxadiazol-3-yl]-3H-imidazo[4,5-c]pyridin-2-yl)methyl]piperidin-4-yl}-2H-1,3-benzodioxol-2-yl)pyridine (105 mg, 0.16 mmol) and $N_2H_4 \cdot H_2O$ (2 drops) in DMF (1 mL) was stirred at RT for 1 hour. After the reaction was completed, the reaction mixture was purified directly by Prep-HPLC to give 2-methyl-5-[2-methyl-4-{1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl}-2H-1,3-benzodioxol-2-yl]pyridine (32.8 mg, yield: 31.3%). MS Calcd.: 646.3; MS Found: 647.3 [M+H]$^+$.

$^1$H NMR (400 MHz, DMSO-d6): δ 9.15 (d, *J*=0.8 Hz, 1 H), 8.64 (d, *J*=2.0 Hz, 1 H), 8.28 (d, *J*=0.8 Hz, 1 H), 7.83 (dd, *J*=8.0 Hz, *J*=2.4 Hz 1 H), 7.30 (d, *J*=8.0 Hz, 1 H), 6.70-6.82 (m, 3 H), 5.13-5.22 (m, 1 H), 4.86-4.98 (m, 1 H), 4.71-4.79 (m. 1 H), 4.38-4.55 (m, 2 H), 4.02 (d, *J*=13.6 Hz, 1 H), 3.85 (d, *J*=13.6 Hz, 1 H), 3.00-3.10 (m, 1 H), 2.84-2.92 (m, 1 H), 2.60-2.80 (m, 2 H), 2.45-2.49 (m, 4 H), 2.17-2.37 (m, 2 H), 2.00 (s, 3 H), 1.65-1.86 (m, 4 H). $^{19}$F NMR (377 MHz, DMSO-d6): δ -63.56.

**Example 56: 2-methyl-5-[2-methyl-4-{1-[(3-{[(2S)-oxetan-2-yl]methyl}-6-[5-(trifluoromethyl)-4H-1,2,4-triazol-3-yl]-3H-imidazo[4,5-b]pyridin-2-yl)methyl]piperidin-4-yl }-2H-1,3-benzodioxol-2-yl]pyridine (Compound 56)**

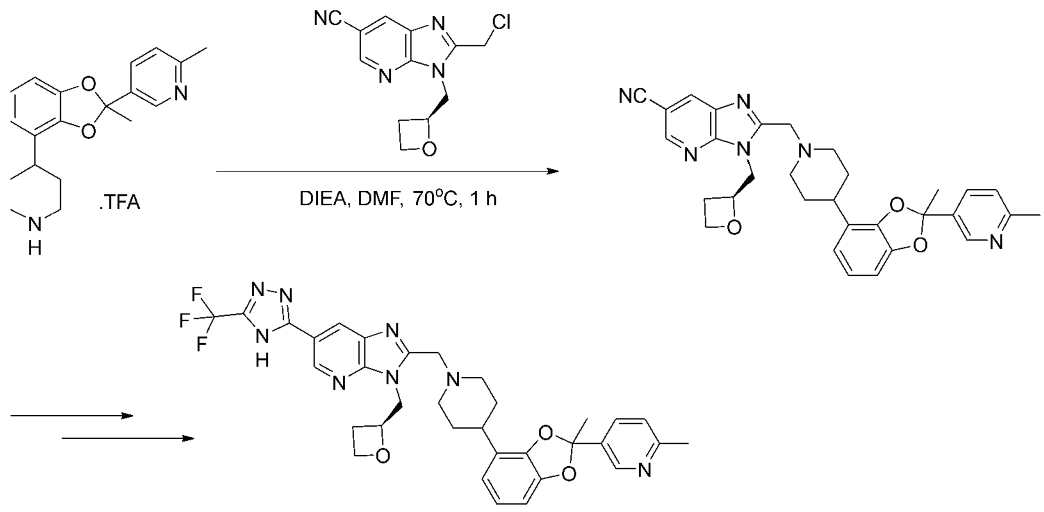


*Step A: 2-((4-((S)-2-methyl-2-(6-methylpyridin-3-yl)benzo[d][1,3]dioxol-4-yl)piperidin-1-yl)methyl)-3-(((S)-oxetan-2-yl) methyl)-3H-imidazo[4,5-b]pyridine-6-carbonitrile*

A mixture of (S)-2-methyl-5-(2-methyl-4-(piperidin-4-yl)benzo[d][1,3]dioxol-2-yl)pyridine TFA salt (144 mg, crude), (S)-2-(chloromethyl)-3-(oxetan-2-ylmethyl)-3H-imidazo[4,5-b]pyridine-6-carbonitrile (80 mg, 0.31 mmol), TEA (0.6 mL) in DMF (2.5 mL) was stirred at 50°C for 2 hours. After the reaction was completed, the reaction was quenched with $H_2O$ (20 mL) and extracted with ethyl acetate (25 mL x 3). The organic layer was combined and washed with brine (20 mL x 2), dried over sodium sulfate, filtered and concentrated in vacuum. The reaction was purified by Pre-TLC (DCM:MeOH=30:1) to give 2-((4-((S)-2-methyl-2-(6-methylpyridin-3-yl)benzo[d][1,3]dioxol-4-yl)piperidin-1-yl) methyl)-3-(((S)-oxetan-2-yl)methyl)-3H-imidazo[4,5-b]pyridine-6-carbonitrile (100 mg, 61 % yield) as yellow oil. MS Calcd.: 536.2; MS Found: 537.4 [M+H]$^+$.

*Step B: 2-methyl-5-[2-methyl-4-{1-[(3-{[(2S)-oxetan-2-yl]methyl}-6-[5-(trifluoromethyl)-4H-1,2,4-triazol-3-yl]-3H-imidazo[4,5-b]pyridin-2-yl)methyl]piperidin-4-yl}-2H-1,3-benzodioxol-2-yl]pyridine*

2-methyl-5-[2-methyl-4-{1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl}-2H-1,3-benzodioxol-2-yl]pyridine (33.1 mg) was obtained with the similar method of Example 55. MS Calcd.:646.3; MS Found: 647.5 [M+H]$^+$.

$^1$H NMR (400 MHz, DMSO-$d_6$): $^1$H NMR: δ 8.99 (d, *J*=2.0 Hz, 1 H), 8.64 (d, *J*=2.4 Hz, 1 H), 8.56 (d, *J*=2.0 Hz, 1 H), 7.84 (dd, *J*=8.0 Hz, *J*=2.4 Hz 1 H), 7.31 (d, *J*=8.0 Hz, 1 H), 6.70-6.82 (m, 3 H), 5.16-5.25 (m, 1 H), 4.79-4.88 (m, 1 H), 4.68-4.76 (m. 1 H), 4.44-4.51 (m, 1 H), 4.34-4.42 (m, 1 H), 3.89-4.02 (m, 2 H), 2.91-3.05 (m, 2 H), 2.60-2.75 (m, 2 H), 2.45-2.49 (m, 4 H), 2.20-2.35 (m, 2 H), 2.00 (s, 3 H), 1.68-1.84 (m, 4 H). $^{19}$F NMR(377 MHz, DMSO-d6): δ -63.05.

**Example 57: (3,4-trans)-4-{6-[(4-chloro-2-fluorophenyl)methoxy]pyridin-2-yl}-1-[(3-{[(2S)-oxetan-2-yl] methyl}-6-[5-(trifluoromethyl)-4H-1,2,4-triazol-3-yl]-3H-imidazo[4,5-c]pyridin-2-yl)methyl]piperidin-3-ol (Compound 57)**

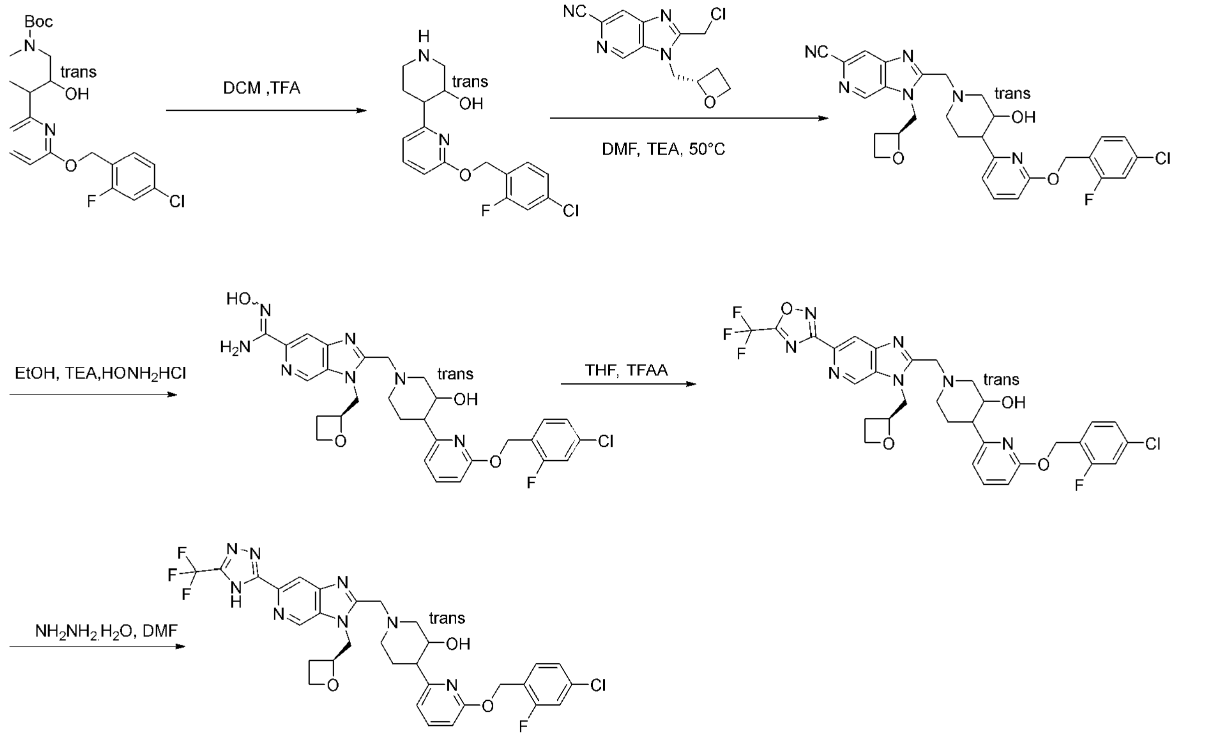


*Step A: trans-4-(6-((4-chloro-2-fluorobenzyl)oxy)pyridin-2-yl)piperidin-3-ol TFA salt*

A mixture of tert-butyl trans-4-(6-((4-chloro-2-fluorobenzyl)oxy)pyridin-2-yl)-3-hydroxypiperidine-1-carboxylate (175 mg, 0.40 mmol) in DCM (2 mL) was added TFA (1 mL). The reaction was stirred at room temperature for 1 hour. After the reaction was completed, the reaction was concentrated in vacuum to give trans-4-(6-((4-chloro-2-fluorobenzyl)oxy) pyridin-2-yl)piperidin-3-ol TFA salt (235 mg, crude) as yellow oil. MS Calcd : 336.1; MS Found:337.3 $[M+H]^+$.

*Step B: 2-((trans-4-(6-((4-chloro-2-fluorobenzyl)oxy)pyridin-2-yl)-3-hydroxypiperidin-1-yl)methyl)-3-(((S)-oxetan-2-yl) methyl)-3H-imidazo[4,5-c]pyridine-6-carbonitrile*

A mixture of trans-4-(6-((4-chloro-2-fluorobenzyl)oxy)pyridin-2-yl)piperidin-3-ol TFA salt (235 mg, crude) and (S)-2-(chloromethyl)-3-(oxetan-2-ylmethyl)-3H-imidazo[4,5-c]pyridine-6-carbonitrile (100 mg, 0.38 mmol) and TEA (0.5 mL) in DMF (3.5 mL) was stirred at 50°C for 2 hours. After the reaction was completed, the reaction was quenched with $H_2O$ (20 mL) and extracted with ethyl acetate (25 mL x 3). The organic layer was combined and washed with brine (20 mL x 2), dried over sodium sulfate, filtered and concentrated in vacuum. The reaction was purified by Prep-TLC (DCM: MeOH=20:1) to give 2-((trans-4-(6-((4-chloro-2-fluorobenzyl)oxy)pyridin-2-yl)-3-hydroxypiperidin-1-yl) methyl)-3-(((S)-oxetan-2-yl)methyl)-3H-imidazo[4,5-c]pyridine-6-carbonitrile (180 mg, 80% yield) as yellow oil.MS Calcd.: 562.2; MS Found: 563.3 $[M+H]^+$.

*Step C: 2-((trans-4-(6-((4-chloro-2-fluorobenzyl)oxy)pyridin-2-yl)-3-hydroxypiperidin-1-yl)methyl)-N'-hydroxy-3-(((S)-oxetan-2-yl)methyl)-3H-imidazo[4,5-c]pyridine-6-carboximidamide*

To a solution of 2-((trans-4-(6-((4-chloro-2-fluorobenzyl)oxy)pyridin-2-yl)-3-hydroxypiperidin-1-yl) methyl)-3-(((S)-oxetan-2-yl)methyl)-3H-imidazo[4,5-c]pyridine-6-carbonitrile (180 mg, 0.32 mmol) and TEA (0.3 ml) in EtOH (3 mL) was added hydroxylamine hydrochloride (44 mg, 0.64 mmol). The reaction was stirred at 60°C for 0.5 hour. After the reaction was completed, After the reaction was completed, the reaction was filtered and the solid was collected and dried to give 2-((trans-4-(6-((4-chloro-2-filuorobenzyl)oxy)pyridin-2-yl)-3-hydroxypiperidin-1-yl)methyl)-N'-hydroxy-3-(((S)-oxetan-2-yl)methyl)-3H-imidazo[4,5-c]pyridine-6-carboximidamide (132 mg, 69% yield) as a white solid. MS Calcd.: 595.2; MS Found: 596.2 $[M+H]^+$.

*Step D: (3,4-trans)-4-(6-((4-chloro-2-fluorobenzyl)oxy)pyridin-2-yl)-1-((3-(((S)-oxetan-2-yl)methyl)-6-(5-(trifluoromethyl)-1,2,4-oxadiazol-3-yl)-3H-imidazo[4,5-c]pyridin-2-yl)methyl)piperidin-3-ol*

To a solution of 2-((trans-4-(6-((4-chloro-2-fluorobenzyl)oxy)pyridin-2-yl)-3-hydroxypiperidin-1-yl)methyl)-N'-hydroxy-3-(((S)-oxetan-2-yl)methyl)-3H-imidazo[4,5-c]pyridine-6-carboximidamide (130 mg, 0.22 mmol) in THF (3 mL) was added TFAA (101 mg,0.48 mmol). The reaction was stirred at 60°C for 1 hour. After the reaction was completed, the reaction was quenched with saturated $NaHCO_3$ solution (20 mL) and extracted with ethyl acetate (20 mL x 3). The organic layer was combined and washed with brine (20 mL x 2), dried over sodium sulfate, filtered and concentrated in vacuum to get (3,4-trans)-4-(6-((4-chloro-2-fluorobenzyl)oxy)pyridin-2-yl)-1-((3-(((S)-oxetan-2-yl)methyl)-6-(5-(trifluoromethyl)-1,2,4-oxadiazol-3-yl)-3H-imidazo[4,5-c]pyridin-2-yl)methyl)piperidin-3-ol (100 mg, crude) as yellow oil. MS Calcd.:673.2; MS Found: 674.4$[M+H]^+$.

*Step E: (3,4-trans)-4-{6-[(4-chloro-2-fluorophenyl)methoxy]pyridin-2-yl}-1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-3-ol*

To a solution of (3,4-trans)-4-(6-((4-chloro-2-fluorobenzyl)oxy)pyridin-2-yl)-1-((3-(((S)-oxetan-2-yl)methyl)-6-(5-(trifluoromethyl)-1,2,4-oxadiazol-3-yl)-3H-imidazo[4,5-c]pyridin-2-yl)methyl)piperidin-3-ol (100 mg, crude) in DMF (2.5 mL) was added hydrazine hydrate (30 mg, 0.60mmol) at room temperature. The reaction was stirred at room temperature for 2 hours. After the reaction was completed, the reaction was purified by Prep-HPLC to give (3,4-trans)-4-{ 6-[(4-chloro-2-fluorophenyl)methoxy]pyridin-2-yl}-1-[(3-{ [(2S)-oxetan-2-yl]methyl}-6-[5-(trifluoromethyl)-4H-1,2,4-triazol-3-yl]-3H-imidazo[4,5-c]pyridin-2-yl)methyl]piperidin-3-ol (39.9 mg, 40% yield) as a white solid. MS Calcd.:672.2; MS Found: 673.2 $[M+H]^+$.

$^1H$ NMR (400 MHz, DMSO-$d_6$): 1H NMR: δ 9.16 (s,1 H), 8.28 (s,1 H), 7.56-7.62 (m, 2 H), 7.48 (d, *J*=10.0 Hz, 1 H), 7.31 (d, *J*=8.0 Hz, 1 H), 6.87 (d, *J*=7.2 Hz, 1 H), 6.68 (d, *J*=8.0 Hz, 1 H), 5.33-5.45(m. 2 H), 5.13-5.23 (m. 1 H), 4.88-4.98 (m, 1 H), 4.73-4.81 (m, 1 H), 4.52-4.70 (m, 1 H), 4.39-4.52 (m, 2 H), 3.75-4.12 (m, 3 H), 3.07-3.14 (m, 1 H), 2.90-2.99 (m, 1 H), 2.68-2.82 (m, 2 H), 2.41-2.50 (m, 1 H), 1.95-2.24 (m, 2 H), 1.63-1.86 (m, 2 H).$^{19}F$ NMR(377 MHz, DMSO-d6): δ -63.43, -115.38.

**Example 58 and 59: 2-[(4-chloro-2-fluorophenyl)methoxy]-6-[(3,4-trans)-3-fluoro-1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl]pyridine (Diastereomer 1) (Compound 58) and**

**2-[(4-chloro-2-fluorophenyl)methoxy]-6-[(3,4-trans)-3-fluoro-1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl]pyridine (Diastereomer 2) (Compound 59)**

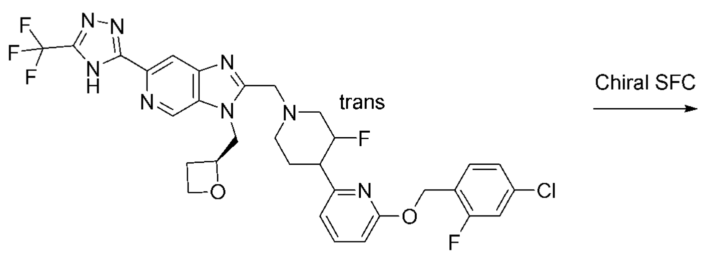


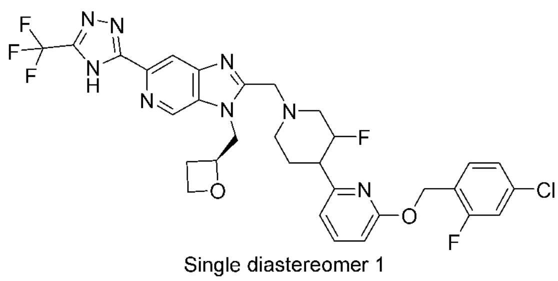

Single diastereomer 1

+

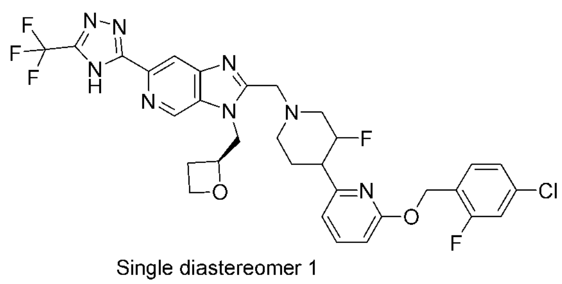

Single diastereomer 1

2-[(4-chloro-2-fluorophenyl)methoxy]-6-[(3,4-trans)-3-fluoro-1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl]pyridine (300 mg) was separated by the chiral SFC (DAICELCHIRALPAK®OZ, Supercritical $CO_2$ / MEOH (+0.1% 7.0mol/l Ammonia in MEOH) = 50/50) to give Diastereomer 1 which was further purified by Prep-HPLC (58 mg, yield: 34.7%) and Diastereomer 2 (102 mg, yield: 34.0%) as white solid.

*2-[(4-chloro-2-fluorophenyl)methoxy]-6-[(3,4-trans)-3-fluoro-1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl]pyridine Diastereomer 1:*

MS Calcd.: 674.2; MS Found: 675.3 $[M+H]^+$. SFC Retention Time: 4.46 min.

1H NMR: δ 9.16 (s, 1 H), 8.49 (s, 1 H), 7.68 (t, *J*=7.6 Hz, 1 H), 7.50 (t, *J*=8.0 Hz, 1 H), 7.17-7.27 (m, 2 H), 6.99 (d, *J*=7.2 Hz, 1 H), 6.78 (d, *J*=8.4 Hz, 1 H), 5.38-5.49 (m, 2 H), 5.15-5.35 (m, 2 H), 4.80-4.96 (m, 2 H), 4.64-4.80 (m, 4 H), 4.43-4.54 (m, 1 H), 3.80-4.00 (m, 1 H), 3.43-3.58 (m, 1 H), 3.10-3.29 (m, 2 H), 2.79-2.90 (m, 1 H), 2.50-2.62(m, 1 H), 2.13-2.26 (m, 2 H). $^{19}F$ NMR: (377 MHz, $CD_3OD$): δ -66.72, -117.76, -185.42.

*2-[(4-chloro-2-fluorophenyl)methoxy]-6-[(3,4-trans)-3-fluoro-1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl]pyridine Diastereomer 2:*

MS Calcd.: 674.2; MS Found: 675.2 $[M+H]^+$. SFC Retention Time: 4.73 min.

$^1H$ NMR: δ 9.19 (d, *J*=1.2 Hz, 1 H), 8.31 (d, *J*=0.8 Hz, 1 H), 7.66 (dd, *J*=8.0 Hz, 7.2 Hz, 1 H), 7.57 (t, *J*=8.4 Hz, 1 H), 7.46 (dd, *J*=10.0 Hz, 2.0 Hz, 1 H), 7.30 (dd, *J*=8.0 Hz, 1.6 Hz, 1 H), 6.95 (d, *J*=7.2 Hz, 1 H), 6.74 (d, *J*=8.4 Hz, 1 H), 5.40 (s, 2 H), 5.13-5.23 (m, 1 H), 4.72-5.00 (m, 3 H), 4.44-4.54 (m, 1 H), 4.36-4.44 (m, 1 H), 4.10 (d, *J*=13.6 Hz, 1 H), 4.00 (d, *J*=13.6 Hz, 1 H), 3.21-3.29 (m, 1 H), 2.88-2.97 (m, 1 H), 2.78-2.88 (m, 1 H), 2.65-2.78 (m, 1 H), 2.40-2.52 (m, 1 H), 2.24-2.37 (m, 2 H), 1.70-1.83 (m, 2 H). $^{19}F$ NMR: (377 MHz, DMSO-d6): δ -63.73, -115.26, -181.88.

**Example 60 and 61: 5-chloro-2-[(2S)-4-[(3,4-trans)-3-fluoro-1-[(3-{[(2S)-oxetan-2-yl]methyl}-6-[5-(trifluoromethyl)-4H-1,2,4-triazol-3-yl]-3H-imidazo[4,5-b]pyridin-2-yl)methyl]piperidin-4-yl]-2-methyl-2H-1,3-benzodioxol-2-yl]pyridine (Diastereomer 1) (Compound 60) and**

**5-chloro-2-[(2S)-4-[(3,4-trans)-3-fluoro-1-[(3-{[(2S)-oxetan-2-yl]methyl}-6-[5-(trifluoromethyl)-4H-1,2,4-triazol-3-yl]-3H-imidazo[4,5-b]pyridin-2-yl)methyl]piperidin-4-yl]-2-methyl-2H-1,3-benzodioxol-2-yl]pyridine (Diastereomer 2) (Compound 61)**

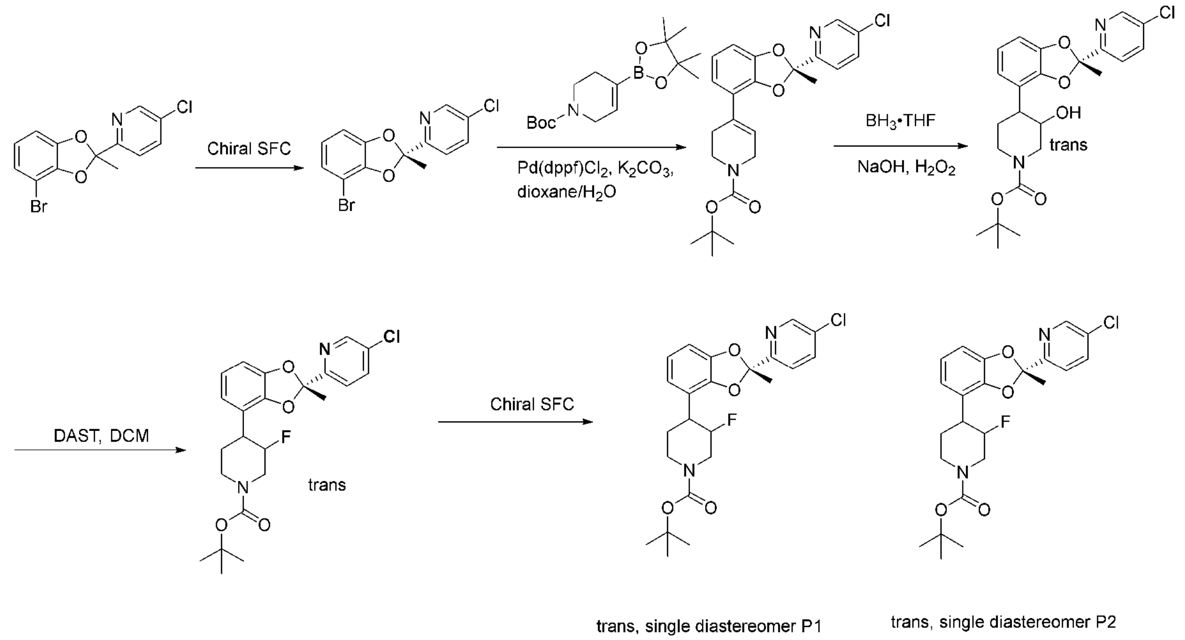

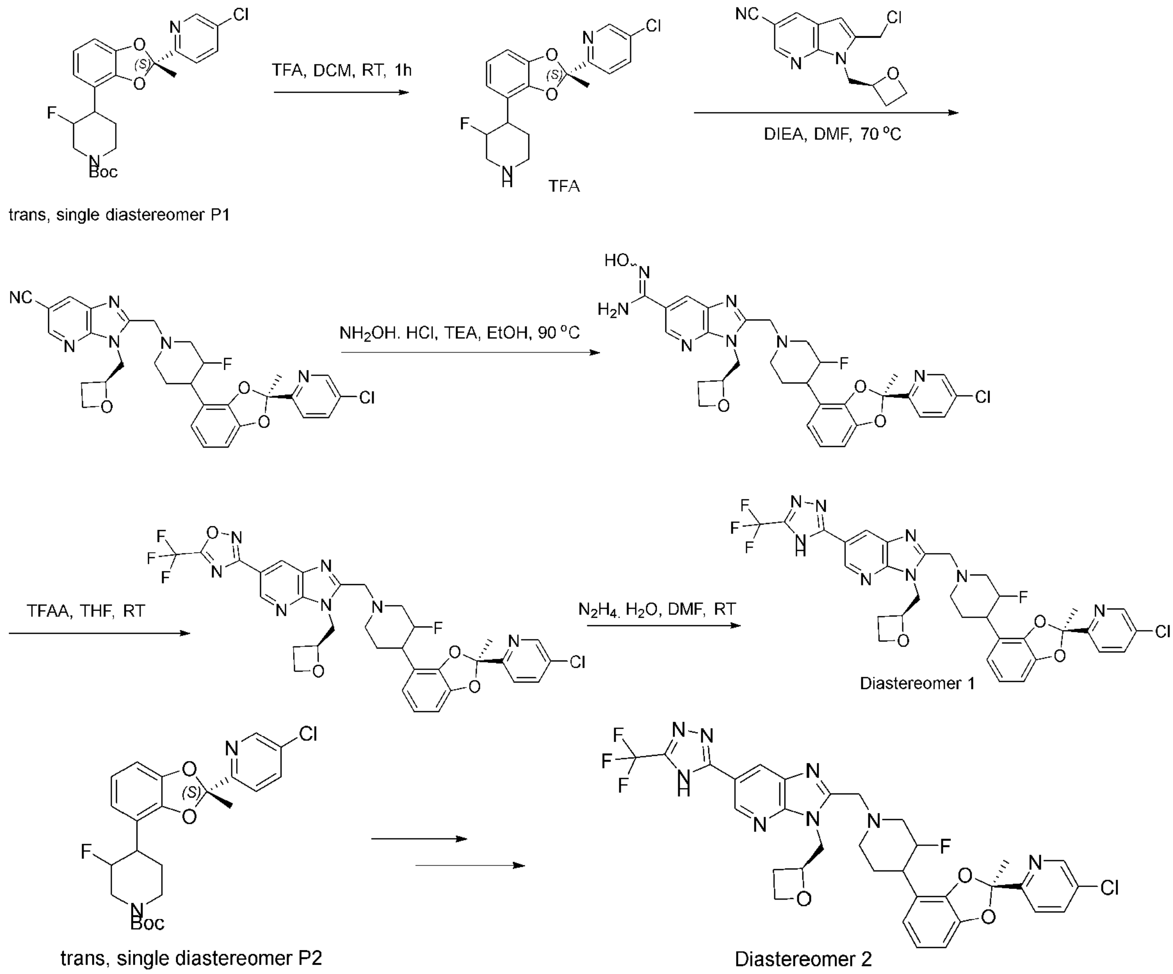


*Step A: (S)-2-(4-bromo-2-methylbenzo[d][1,3]dioxol-2-yl)-5-chloropyridine*

2-(4-bromo-2-methylbenzo[d][1,3]dioxol-2-yl)-5-chloropyridine (4.4 g, 13.5 mmol) was separated by chiral SFC (DAICELCHIRALCEL®AD, Supercritical $CO_2$/ MEOH (+0.1% 7.0mol/l Ammonia in MEOH) = 70/30) to give (S)-2-(4-bromo-2-methylbenzo[d][1,3]dioxol-2-yl)-5-chloropyridine (2.2 g, yield: 50%) as a colorless oil. MS Calcd.: 325.0; MS Found: 325.9 $[M+H]^+$. SFC Retention Time: 0.825 min.

And (R)-2-(4-bromo-2-methylbenzo[d][1,3]dioxol-2-yl)-5-chloropyridine (2.2 g, yield: 50%) as a colorless oil. MS Calcd.: 325.0; MS Found: 326.1 $[M+H]^+$. SFC Retention Time : 0.913 min.

*Step B: tert-butyl (S)-4-(2-(5-chloropyridin-2-yl)-2-methylbenzo[d][1,3]dioxol-4-yl)-3,6-dihydropyridine-1(2H)-carboxylate*

A mixture of (S)-2-(4-bromo-2-methylbenzo[d][1,3]dioxol-2-yl)-5-chloropyridine (2.2 g, 6.8 mmol), tert-butyl 4-(4,4,5,5-tetramethyl-1,3,2-dioxaborolan-2-yl)-3,6-dihydropyridine-1(2H)-carboxylate (2.1 g, 6.8 mmol), $Pd(dppf)Cl_2$ (495 mg, 0.68 mmol), $K_2CO_3$ (2.8 g, 20.4 mmol) in dioxane/$H_2O$ (20.0 mL/2.0 mL) was stirred at 90 °C for 3 hours. After the reaction was completed, the mixture was diluted with $H_2O$ (100 mL), extracted with EA (50 mL×2). The combined organic layer was dried over anhydrous sodium sulfate, filtered and evaporated to dryness. The residue was purified by column chromatography on silica gel (PE/EA=20/1) to give tert-butyl (S)-4-(2-(5-chloropyridin-2-yl)-2-methylbenzo[d][1,3]dioxol-4-yl)-3,6-dihydropyridine-1(2H)-carboxylate (2.1 g, yield: 73%) as yellow oil. MS Calcd.: 428.2; MS Found: 373.0 $[M-56+H]^+$.

*Step C: tert-butyl (3,4-trans)-4-[(2S)-2-(5-chloropyridin-2-yl)-2-methyl-2H-1,3-benzodioxol-4-yl]-3-hydroxypiperidine-1-carboxylate*

To a solution of *tert*-butyl (S)-4-(2-(5-chloropyridin-2-yl)-2-methylbenzo[d][1,3]dioxol-4-yl)-3,6-dihydropyridine-1(2H)-carboxylate (2 g, 4.7 mmol) in THF (40 mL) was added $BH_3 \cdot THF$ (9.35 mL, 9.4 mmol) slowly at 0°C. The

mixture was stirred at 0°C for 2 hours. Then NaOH solution (7 mL, 2 M in water) was added, followed by the addition of $H_2O_2$ (7 mL, 30% in water). The reaction was stirred at 50°C for 1 hour. After the reaction was completed, the mixture was diluted with EA (100 mL) and wished with water (30 mL x 2). The organic layer was dried over $Na_2SO_4$, filtered and evaporated to dryness. The residue was purified by column chromatography on silica gel (PE:EA=2:1) to give tert-butyl (3,4-trans)-4-[(2S)-2-(5-chloropyridin-2-yl)-2-methyl-2H-1,3-benzodioxol-4-yl]-3-hydroxypiperidine-1-carboxylate (1.55 g, yield: 75%) as a colorless oil. MS Calcd.: 446.2; MS Found: 469.1 $[M+Na]^+$.

*Step D: tert-butyl (3,4-trans)-4-[(2S)-2-(5-chloropyridin-2-yl)-2-methyl-2H-1,3-benzodioxol-4-yl]-3-fluoropiperidine-1-carboxylate*

To a mixture of tert-butyl (3,4-*trans*)-4-[(2S)-2-(5-chloropyridin-2-yl)-2-methyl-2H-1,3-benzodioxol-4-yl]-3-hydroxypiperidine-1-carboxylate (1.35 g, 3.03 mmol) in DCM (20 mL) was added DAST(975 mg, 6.05 mmol) slowly at 0°C. The mixture was stirred at 0°C for 2 minutes. After the reaction was completed, the mixture was diluted with EA (100 mL) and wished with water (30 mL x 2). The organic layer was dried over $Na_2SO_4$, filtered and evaporated to dryness. The residue was purified by column chromatography on silica gel (PE:EA=10:1) to give to give tert-butyl (3,4-*trans*)-4-[(2S)-2-(5-chloropyridin-2-yl)-2-methyl-2H-1,3-benzodioxol-4-yl]-3-fluoropiperidine-1-carboxylate (1.2 g, yield: 88.9%) as a colorless oil. MS Calcd.: 448.2; MS Found: 449.2 $[M+H]^+$.

*Step E: tert-butyl (3,4-trans)-4-[(2S)-2-(5-chloropyridin-2-yl)-2-methyl-2H-1,3-benzodioxol-4-yl]-3-fluoropiperidine-1-carboxylate trans, single diastereomer P1 and trans, single diastereomer P2*

tert-Butyl (3,4-trans)-4-[(2S)-2-(5-chloropyridin-2-yl)-2-methyl-2H-1,3-benzodioxol-4-yl]-3-fluoropiperidine-1-carboxylate (1.2 g, 2.68 mmol) was separated by chiral SFC (DAICELCHIRALCELRAD, Supercritical $CO_2$ / MEOH (+0.1% 7.0 mol/L Ammonia in MEOH) = 60/40) to give trans, single diastereomer P1 (670 mg, yield: 55.8%) as a colorless oil and trans, single diastereomer P2 (430 mg, yield: 35.8%) as a white solid.

Trans, single diastereomer P1 (670 mg, yield: 55.8%): MS Calcd.: 448.2; MS Found: 449.1 $[M+H]^+$. SFC Retention Time: 1.022 min. $^{19}F$ NMR: (377 MHz, DMSO-d6): δ-182.46.

Trans, single diastereomer P2 (430 mg, yield: 35.8%). MS Calcd.: 448.16; MS Found: 449.1 [M+H]+. SFC Retention Time: 1.373 min.

1H NMR: δ 8.72 (d, *J*=0.2 Hz, 1 H), 8.02 (dd, *J*=2.4 Hz, 8.4 Hz, 1 H), 7.59 (d, *J*=8.4 Hz, 1 H), 6.80-6.90 (m, 3 H), 4.56-4.81 (m, 1 H), 4.21-4.34 (m, 1 H), 3.87-4.03 (m, 1 H), 2.96-3.07 (m, 1 H), 2.80-2.95 (m, 2 H), 2.02 (s, 3 H), 1.65-1.85 (m, 2 H), 1.42 (s, 9 H). $^{19}F$ NMR: (377 MHz, DMSO-d6): δ: - 182.27.

*Step F: 5-chloro-2-((S)-4-((3,4-trans)-3-fluoropiperidin-4-yl)-2-methylbenzo[d][1,3]dioxol-2-yl)pyridine TFA Salt (from single diastereomer P1)*

To a solution of tert-butyl (3,4-trans)-4-((S)-2-(5-chloropyridin-2-yl)-2-methylbenzo[d][1,3]dioxol-4-yl)-3-fluoropiperidine-1-carboxylate (isomer P1) (100 mg, 0.22 mmol) in DCM (4 mL) was added TFA (1.0 mL). The solution was stirred at room temperature for 1 hour. The reaction was removed in vacuo to give 5-chloro-2-((S)-4-((3,4-trans)-3-fluoropiperidin-4-yl)-2-methylbenzo[d][1,3]dioxol-2-yl)pyridine TFA Salt (from single diastereomer P1) (120 mg, crude) as red oil. MS Calcd.: 348.1; MS Found: 349.1 [M+H]+.

*Step G: 2-(((3,4-trans)-4-((S)-2-(5-chloropyridin-2-yl)-2-methylbenzo[d][1,3]dioxol-4-yl)-3-fluoropiperidin-1-yl) methyl)-3-(((S)-oxetan-2-yl)methyl)-3H-imidazo[4,5-b]pyridine-6-carbonitrile (from single diastereomer P1)*

A mixture of 5-chloro-2-((S)-4-((3,4-trans)-3-fluoropiperidin-4-yl)-2-methylbenzo[d][1,3]dioxol-2-yl)pyridine TFA salt (from single diastereomer P1) (120 mg, 0.34 mmol), (S)-2-(chloromethyl)-3-(oxetan-2-ylmethyl)-3H-imidazo[4,5-b] pyridine-6-carbonitrile (50 mg, 0.19 mmol), DIEA (24.62 mg, 1.91 mmol) in DMF (3.0 mL) was stirred at 70 °C for 1 hour. After the reaction was completed, the mixture was diluted with $H_2O$ (40 mL), extracted with EA (30 mL×2). The combined organic layer was dried over anhydrous sodium sulfate, filtered and evaporated to dryness. The residue was purified by column chromatography on silica gel (DCM/MeOH=100/1) to give 2-(((3,4-trans)-4-((S)-2-(5-chloropyridin-2-yl)-2-methylbenzo[d][1,3]dioxol-4-yl)-3-fluoropiperidin-1-yl)methyl)-3-(((S)-oxetan-2-yl)methyl)-3H-imidazo[4,5-b]pyridine-6-carbonitrile (from single diastereomer P1) (130 mg, impure) as a yellow solid. MS Calcd.: 574.2; MS Found: 575.2 $[M+H]^+$.

*Step H: 2-(((3,4-trans)-4-((S)-2-(5-chloropyridin-2-yl)-2-methylbenzo[d][1,3]dioxol-4-yl)-3-fluoropiperidin-1-yl)methyl)-N'-hydroxy-3-(((S)-oxetan-2-yl)methyl)-3H-imidazo(4,5-b)pyridine-6-carboximidamide (from single diastereomer P1)*

A solution of 2-(((3,4-trans)-4-((S)-2-(5-chloropyridin-2-yl)-2-methylbenzo[d][1,3]dioxol-4-yl)-3-fluoropiperidin-1-yl)methyl)-3-(((S)-oxetan-2-yl)methyl)-3H-imidazo[4,5-b]pyridine-6-carbonitrile(from isomer P1) (130 mg, 0.23 mmol), $HONH_2 \cdot HCl$ (95 mg, 1.36 mmol), TEA (93 mg, 0.96 mmol) in EtOH (4 mL) was stirred at 90°C for 1 h. After the reaction was completed, the mixture was filtered and the solid was dried to give 2-(((3,4-*trans*)-4-((S)-2-(5-chloropyridin-2-yl)-2-methylbenzo[d][1,3]dioxol-4-yl)-3-fluoropiperidin-1-yl)methyl)-N'-hydroxy-3-(((S)-oxetan-2-yl)methyl)-3H-imidazo[4,5-b]pyridine-6-carboximidamide (from single diastereomer P1) (130 mg, crude) as yellow oil. MS Calcd.: 607.2; MS Found: 608.2 $[M+H]^+$.

*Step I: 3-(2-(((3,4-trans)-4-((S)-2-(5-chloropyridin-2-yl)-2-methylbenzo[d][1,3]dioxol-4-yl)-3-fluoropiperidin-1-yl)methyl)-3-(((S)-oxetan-2-yl)methyl)-3H-imidazo[4,5-b]pyridin-6-yl)-5-(trifluoromethyl)-1,2,4-oxadiazole (from single diastereomer P1)*

To a solution of 2-(((3,4-*trans*)-4-((S)-2-(5-chloropyridin-2-yl)-2-methylbenzo[d][1,3]dioxol-4-yl)-3-fluoropiperidin-1-yl)methyl)-N'-hydroxy-3-(((S)-oxetan-2-yl)methyl)-3H-imidazo[4,5-b]pyridine-6-carboximidamide (from isomer P1) (130 mg) in THF (4 mL) was added TFAA (89.95 mg, 0.43 mmol) and the mixture stirred at RT for 1 hour. After the reaction was completed, the reaction was quenched with sat. sodium bicarbonate aqueous solution (5.0 mL), extracted with ethyl acetate (15 mL×3). The organic layers were combined, dried over $Na_2SO_4$, filtered and concentrated under vacuum to dryness. The residue was purified by Prep-TLC (PE/EA=1/1) to give 3-(2-(((3,4-*trans*)-4-((S)-2-(5-chloropyridin-2-yl)-2-methylbenzo[d][1,3]dioxol-4-yl)-3-fluoropiperidin-1-yl)methyl)-3-(((S)-oxetan-2-yl)methyl)-3H-imidazo[4,5-b]pyridin-6-yl)-5-(trifluoromethyl)-1,2,4-oxadiazole (from single diastereomer P1) (90 mg, yield: 61%) as yellow oil. MS Calcd.: 685.2; MS Found: 708.0 $[M+Na]^+$.

*Step J: 5-chloro-2-[(2S)-4-[(3,4-trans)-3-fluoro-1-[(3-{[(2S)-oxetan-2-yl]methyl}-6-[5-(trifluoromethyl)-4H-1,2,4-triazol-3-yl]-3H-imidazo[4,5-b]pyridin-2-yl)methyl]piperidin-4-yl]-2-methyl-2H-1,3-benzodioxol-2-yl]pyridine (Diastereomer 1)*

A mixture of 3-(2-(((3,4-*trans*)-4-((S)-2-(5-chloropyridin-2-yl)-2-methylbenzo[d][1,3]dioxol-4-yl)-3-fluoropiperidin-1-yl)methyl)-3-(((S)-oxetan-2-yl)methyl)-3H-imidazo[4,5-b]pyridin-6-yl)-5-(trifluoromethyl)-1,2,4-oxadiazole (from isomer P1) (45 mg, 0.07 mmol) and $N_2H_4 \cdot H_2O$ (6.57 mg, 0.13 mmol) in DMF (1.5 mL) was stirred at RT for 1 hour. After the reaction was completed, the mixture was purified directly by Prep-HPLC (0.1% FA/$H_2O$/$CH_3CN$) to give 5-chloro-2-[(2S)-4-[(3,4-*trans*)-3-fluoro-1-[(3-{ [(2S)-oxetan-2-yl]methyl}-6-[5-(trifluoromethyl)-4H-1,2,4-triazol-3-yl]-3H-imidazo[4,5-b]pyridin-2-yl)methyl]piperidin-4-yl]-2-methyl-2H-1,3-benzodioxol-2-yl]pyridine (Diastereomer 1) (5.8 mg, yield: 13%) as a white solid. MS Calcd.: 684.2; MS Found: 685.0 $[M+H]^+$.

$^1H$ NMR (400 MHz, DMSO-d6): δ 9.00 (d, *J*=1.6 Hz, 1 H), 8.72 (d, *J*=2.0 Hz, 1 H), 8.60 (d, *J*=2.0 Hz, 1 H), 8.01 (dd, *J*=8.8 Hz, 2.4 Hz, 1 H), 7.62 (d, *J*=8.8 Hz, 1 H), 6.80-6.89 (m, 3 H), 5.16-5.22 (m, 1 H), 4.78-4.99 (m, 2 H), 4.60-4.71 (m, 1 H), 4.35-4.50 (m, 2 H), 4.00-4.11 (m, 2 H), 3.32-3.40 (m, 1 H), 2.76-2.95 (m, 2 H), 2.64-2.74 (m, 1 H), 2.44-2.52 (m, 1 H), 2.25-2.40 (m, 2 H), 2.01 (s, 3 H), 1.75-1.87 (m, 2 H). $^{19}F$ NMR(377 MHz, DMSO-d6): δ -63.44, -181.60.

*Step K: 5-chloro-2-[(2S)-4-[(3,4-trans)-3-fluoro-1-[(3-{[(2S)-oxetan-2-yl]methyl}-6-[5-(trifluoromethyl)-4H-1,2,4-triazol-3-yl]-3H-imidazo[4,5-b]pyridin-2-yl)methyl]piperidin-4-yl]-2-methyl-2H-1,3-benzodioxol-2-yl]pyridine (Diastereomer 2)*

Diastereomer 2 was obtained using the similar procedure described for Example 60 by using tert-butyl (3,4-*trans*)-4-((S)-2-(5-chloropyridin-2-yl)-2-methylbenzo[d][1,3]dioxol-4-yl)-3-fluoropiperidine-1-carboxylate (from single diastereomer P2) as starting material.

MS Calcd.: 684.2; MS Found: 685.2$[M+H]^+$.

$^1H$ NMR (400 MHz, DMSO-d6): δ 9.00 (d, *J*=1.6 Hz, 1 H), 8.72 (d, *J*=2.0 Hz, 1 H), 8.57 (d, *J*=2.0 Hz, 1 H), 8.01 (dd, *J*=8.4 Hz, *J*=2.4 Hz, 1H), 7.60 (d, *J*=8.4 Hz, 1H), 6.77-6.90 (m, 3 H), 5.12-5.26 (m, 1 H), 4.63-4.93 (m, 3 H), 4.40-4.50 (m, 1 H), 4.29-4.39 (m, 1 H), 3.99-4.10 (m, 2 H), 3.21-3.25 (m, 1 H), 2.80-2.96 (m, 2 H), 2.60-2.74 (m, 1 H), 2.40-2.52 (m, 1 H), 2.26-2.40 (m, 2 H), 2.02 (s, 3 H), 1.77-1.91 (m, 2 H). $^{19}F$ NMR(377 MHz, DMSO-d6): (377 MHz, DMSO-d6): δ -63.05, -181.50.

**Example 62 and 63: 5-chloro-2-[(2S)-4-[(3,4-*trans*)-3-fluoro-1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl]-2-methyl-2H-1,3-benzodioxol-2-yl]pyridine (Diastereomer 1) (Compound 62) and**

**5-chloro-2-[(2S)-4-[(3,4-*trans*)-3-fluoro-1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl]-2-methyl-2H-1,3-benzodioxol-2-yl]pyridine (Diastereomer 2) (Compound 63)**

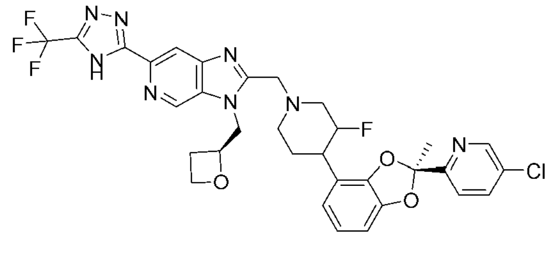

Diastereomer1

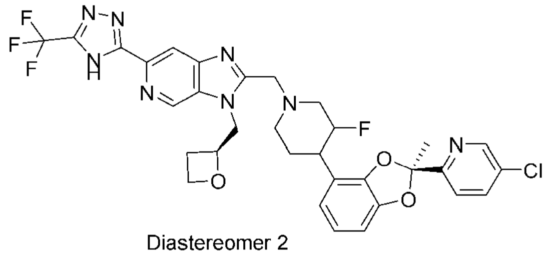

Diastereomer 2

5-chloro-2-[(2S)-4-[(3,4-*trans*)-3-fluoro-1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl]-2-methyl-2H-1,3-benzodioxol-2-yl]pyridine (Diastereomer 1) (62) was obtained using the similar procedure described for Example 60 by using (S)-2-(chloromethyl)-1-(oxetan-2-ylmethyl)-1H-pyrrolo[2,3-c]pyridine-5-carbonitrile as the starting material.

MS Calcd.: 684.2; MS Found: 685.1 $[M+H]^+$.

$^1$H NMR (400 MHz, DMSO-d6): δ 9.14 (s, 1 H), 8.72 (d, *J*=2.4 Hz, 1 H), 8.27 (s, 1 H), 8.01 (dd, *J*=8.8 Hz, 2.4 Hz, 1 H), 7.61 (d, *J*=8.8 Hz, 1 H), 6.80-6.86 (m, 3 H), 5.09-5.18 (m, 1 H), 4.74-4.94 (m, 2 H), 4.66-4.74 (m, 1 H), 4.39-4.51 (m, 2 H), 4.09 (d, *J*=14.0 Hz, 1 H), 3.95 (d, *J*=13.6 Hz, 1 H), 3.38-3.45 (m, 1 H), 2.77-2.88 (m, 2 H), 2.65-2.77 (m, 1 H), 2.41-2.52 (m, 1 H), 2.30-2.40 (m, 1 H), 2.20-2.30 (m, 1 H), 2.02 (s, 3 H), 1.72-1.85 (m, 2 H). $^{19}$F NMR (377 MHz, DMSO-d6): δ -63.22, -181.57.

*5-chloro-2-[(2S)-4-[(3,4-trans)-3-fluoro-1-[(3-{[(2S)-oxetan-2-yl]methyl}-6-[5-(trifluoromethyl)-4H-1,2,4-triazol-3-yl]-3H-imidazo[4,5-c]pyridin-2-yl)methyl]piperidin-4-yl]-2-methyl-2H-1,3-benzodioxol-2-yl]pyridine (Diastereomer 2)*

MS Calcd.: 684.2; MS Found: 685.0 $[M+H]^+$.

$^1$H NMR (400 MHz, DMSO-$d_6$): 1H NMR: δ 9.16 (d, *J*=0.4 Hz, 1 H), 8.69-8.75 (m, 1 H), 8.29 (d, *J*=0.8 Hz, 1 H), 8.01 (dd, *J*=8.4 Hz, *J*=2.4 Hz, 1H), 7.59 (d, *J*=8.4 Hz, 1H), 6.79-6.89 (m, 3 H), 5.11-5.20 (m, 1 H), 4.68-4.93 (m, 3 H), 4.42-4.51 (m, 1 H), 4.33-4.41 (m, 1 H), 4.08 (d, *J*=14.0 Hz, 1 H), 3.99 (d, *J*=13.6 Hz, 1 H), 3.24-3.29 (m, 1 H), 2.90-2.98 (m, 1 H), 2.78-2.91 (m, 1 H), 2.65-2.75 (m, 1 H), 2.36-2.52 (m, 1 H), 2.27-2.36 (m, 2 H), 2.03 (s, 3 H), 1.77-1.85 (m, 2 H). $^{19}$F NMR (377 MHz, DMSO-$d_6$): δ -63.49, -181.50.

## BIOLOGICAL EXAMPLES

### Biological Example 1: cAMP Assays

Activation of GLP-1 receptor is known to stimulate cyclic AMP (cAMP) production in cells which indicates primary coupling to the Gαs subunit of the G protein heterotrimeric complex. Evidence suggests signaling through Gas induced cAMP stimulation elicits the desired pharmacological response regarding insulin release from pancreatic β-cells.

To optimize functional activity directed toward Gas coupling, a HEK293/CRE-Luc cell line developed by HDB stably expressing the GLP-1 Receptor was used. 200× concentration of compound working solutions were prepared (Agilent Technologies Bravo) with 1/2log serial dilution in 384-well Echo LDV plate (Labcyte, Cat# LP-0200). 50 nL/well 200x concentration of compound working solutions were moved to 384-well white low volume plate (Greiner, Cat #784075) using Labcyte ECHO550. 1×105 cells/mL HEK293/GLP1R/CRE-LUC (HD Biosciences) cell suspensions prepared with assay buffer [DPBS containing 0.5 mM IBMX (Sigma, Cat # I5879) and 0.1% BSA (GENVIEW, Cat# FA016-100g)], 10 μL cell suspensions were added to each well of previous generated assay plate which already contains 50 nL compound at 200xconcentration using ThermoFisher Multidrop Combi (1000 cells/well). Seal the plate and incubate at 37 °C with 5% $CO_2$ for 30 min.

After incubation the cAMP assay signal was generated using cAMP dynamic 2 Kit (Cisbio). 5μL cAMP-d2

working solution was added to each well, followed with 5 μL Anti-cAMP antibody-cryptate working solution added to each well using ThermoFisher Multidrop Combi. Incubate at room temperature for 1 hour protected from light. Read the fluorescence at 665 and 615 nm with Reader PerkinElmer EnVision.

%Activity = 100% x (mean RLU of test sample - mean RLU of vehicle control) / (mean RLU of MAX control - mean RLU of vehicle control))

Table 2 shows the biological activity of compounds in GLP-1R agonist cAMP stimulation assay ($EC_{50}$). Activity of the tested compounds is provided in Table 2 below as follows: +++ = $EC_{50}$ < 1 nM; ++ = $EC_{50}$ 1-100 nM; + = $EC_{50}$ > 100 nM.

**Table 2**

| No. | Activity | Activity (μM) |
|---|---|---|
| 1 | +++ | 0.031 |
| 2 | +++ | 0.009 |
| 3 | +++ | 0.14 |
| 4 | +++ | 0.088 |
| 5 | +++ | 0.1 |
| 6 | +++ | 0.04 |
| 7 | +++ | 0.18 |
| 8 | +++ | 0.069 |
| 9 | +++ | 0.051 |
| 10 | +++ | 0.13 |
| 11 | +++ | 0.62 |
| 12 | +++ | 0.028 |
| 13 | +++ | 0.047 |
| 14 | +++ | 0.031 |
| 15 | +++ | 0.013 |
| 16 | +++ | 0.012 |
| 17 | +++ | 0.03 |
| 18 | +++ | 0.086 |
| 19 | +++ | 0.056 |
| 20 | +++ | 0.068 |
| 21 | +++ | 0.55 |
| 22 | ++ | 4.3 |
| 23 | +++ | 0.079 |
| 24 | +++ | 0.24 |
| 25 | +++ | 0.93 |
| 26 | +++ | 0.52 |
| 27 | +++ | 0.17 |
| 28 | +++ | 0.17 |
| 29 | +++ | 0.25 |
| 30 | ++ | 5 |
| 31 | +++ | 0.043 |
| 32 | +++ | 0.18 |

(continued)

| No. | Activity | Activity (μM) |
|---|---|---|
| 33 | +++ | 0.02 |
| 34 | +++ | 0.014 |
| 35 | +++ | 0.03 |
| 36 | +++ | 0.08 |
| 37 | ++ | 2.2 |
| 38 | ++ | 1.4 |
| 39 | ++ | 2.3 |
| 40 | +++ | 0.016 |
| 41 | +++ | 0.09 |
| 42 | +++ | 0.35 |
| 43 | ++ | 2.7 |
| 44 | +++ | 0.052 |
| 45 | +++ | 0.066 |
| 46 | +++ | 0.074 |
| 47 | +++ | 0.21 |
| 48 | +++ | 0.1 |
| 49 | +++ | 0.26 |
| 50 | +++ | 0.95 |
| 51 | +++ | 0.27 |
| 52 | +++ | 0.24 |
| 53 | +++ | 0.066 |
| 54 | +++ | 0.042 |
| 55 | +++ | 0.017 |
| 56 | +++ | 0.04 |
| 57 | +++ | 0.19 |
| 58 | +++ | 0.024 |
| 59 | +++ | 0.15 |
| 60 | +++ | 0.03 |
| 61 | +++ | 0.63 |
| 62 | +++ | 0.013 |
| 63 | +++ | 0.18 |

## Claims

**1.** A compound selected from:

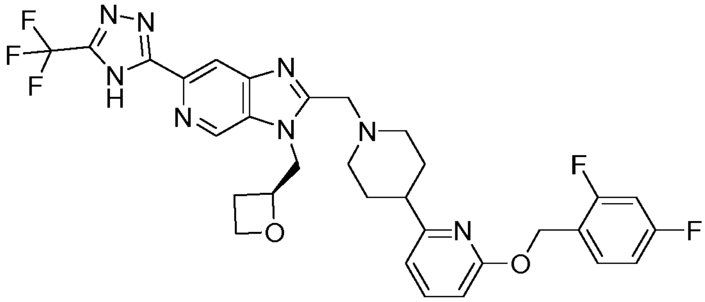

,

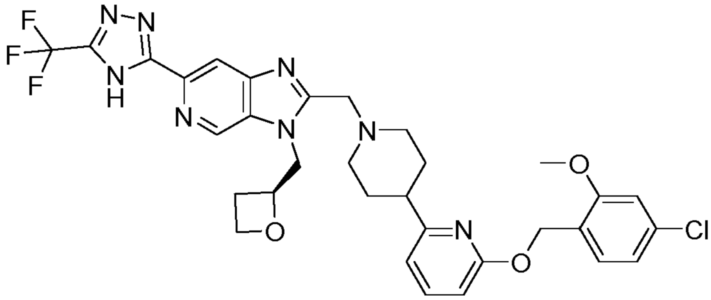

,

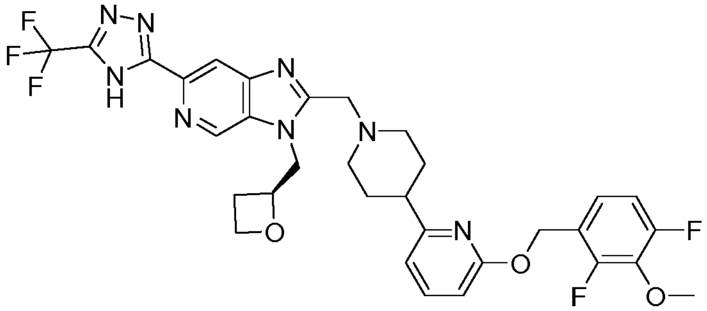

,

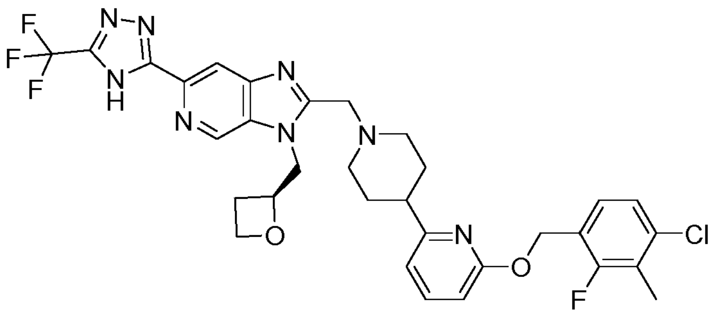

,

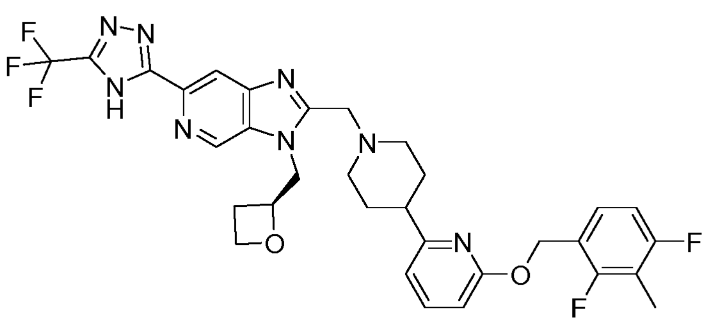

,

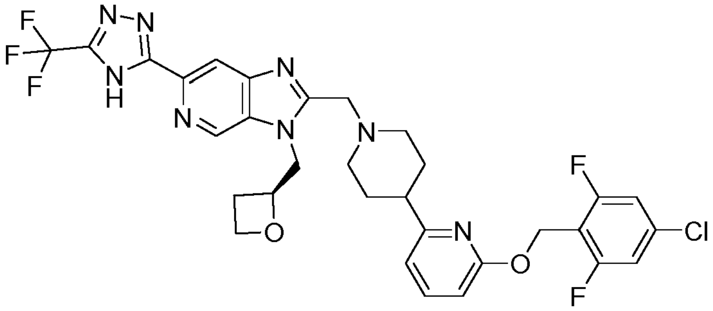

,

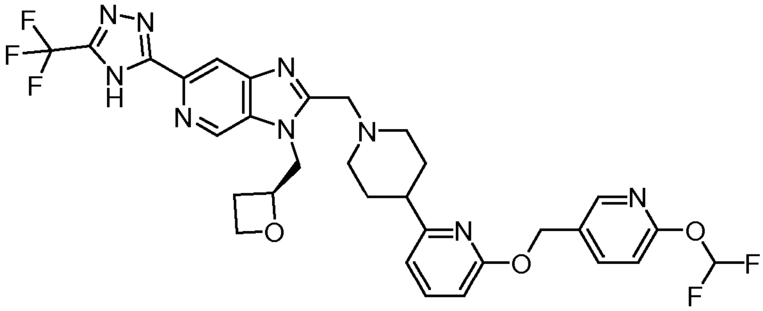

,

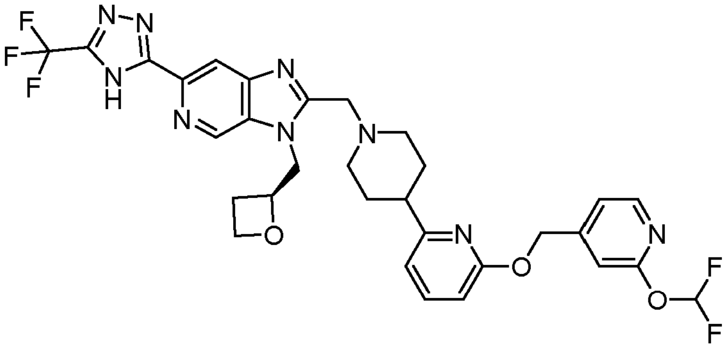

,

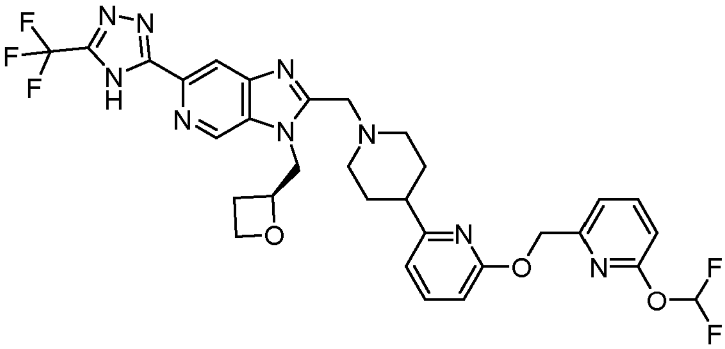

,

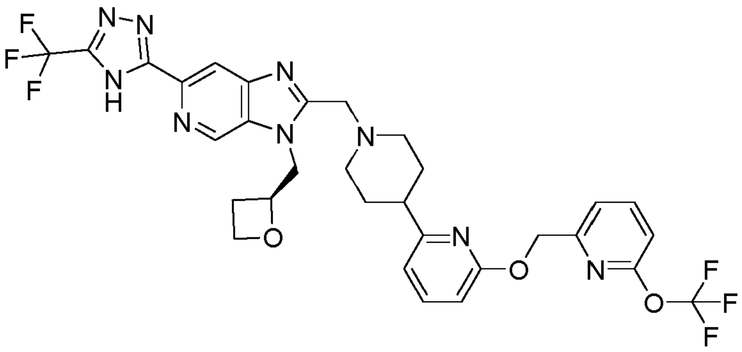

,

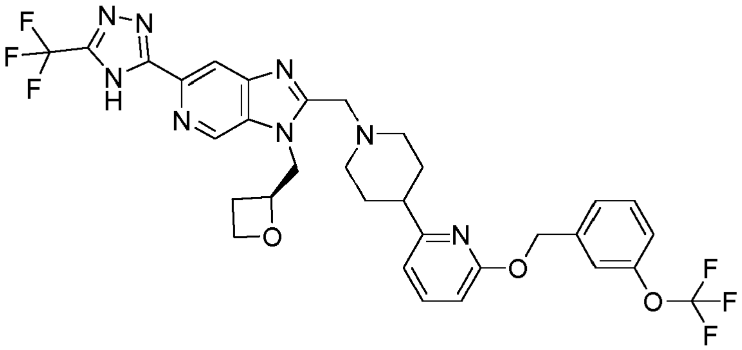

,

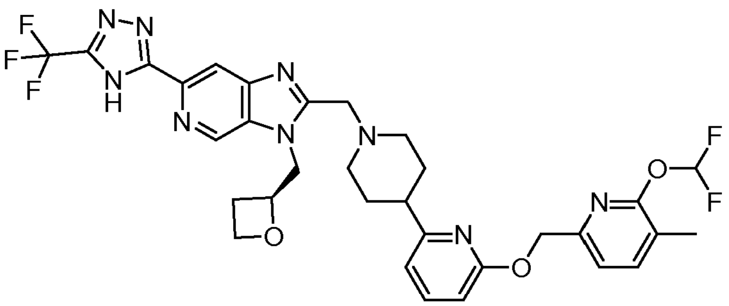

,

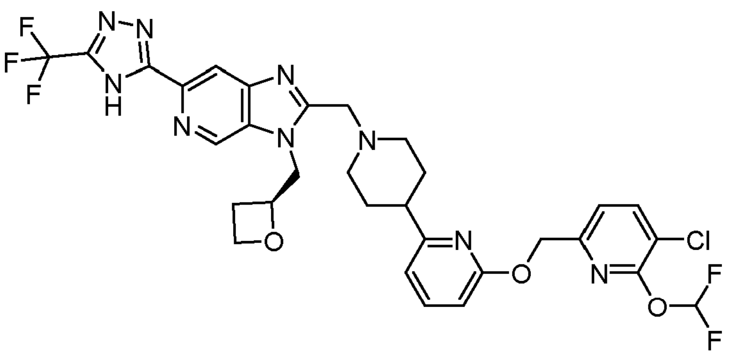

,

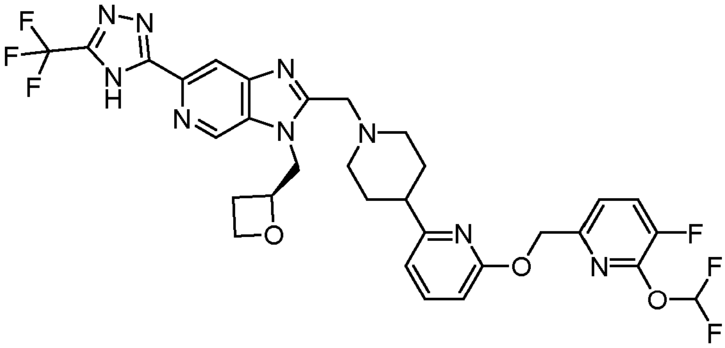

,

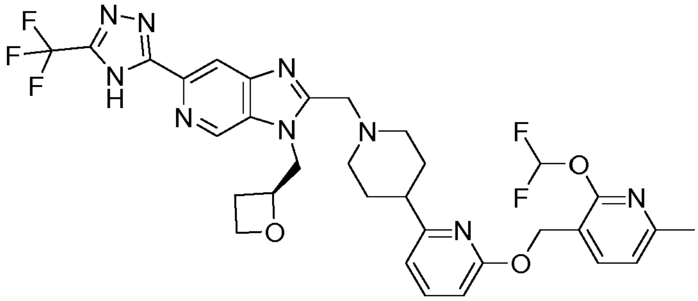

,

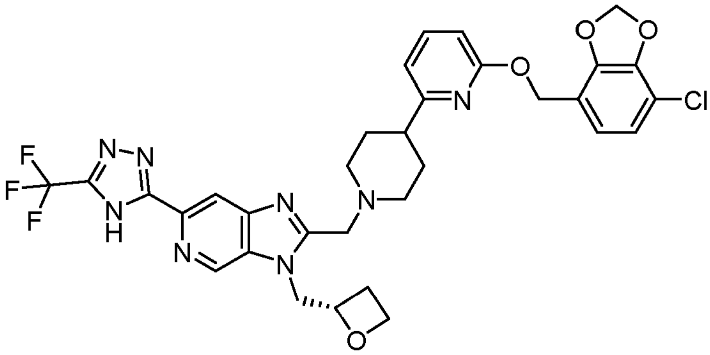

,

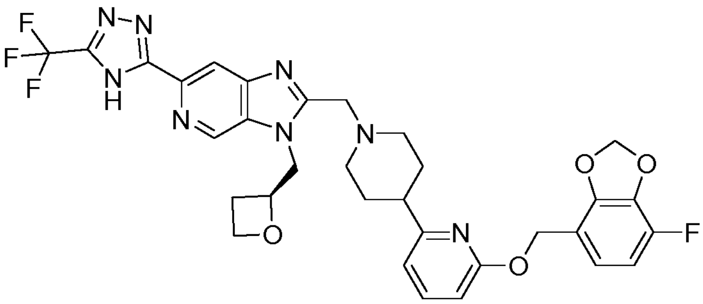

,

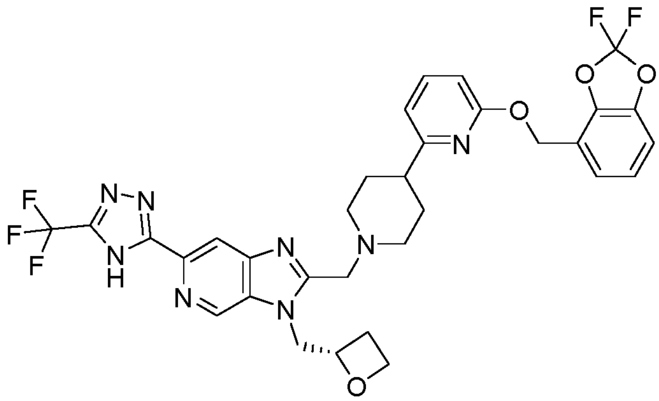

,

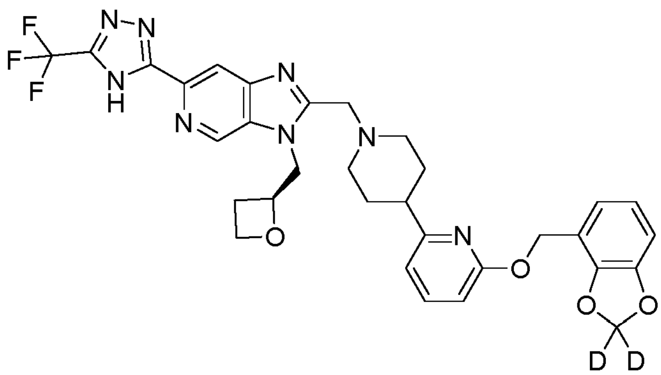

,

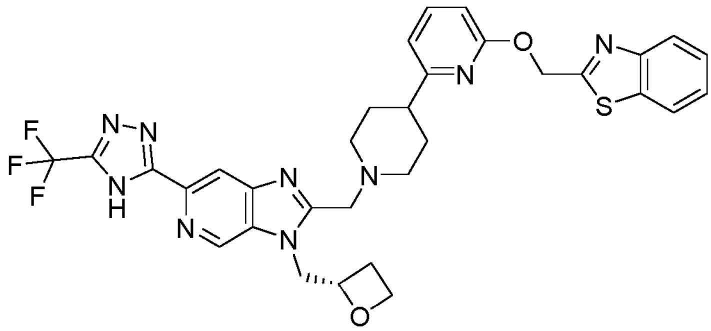

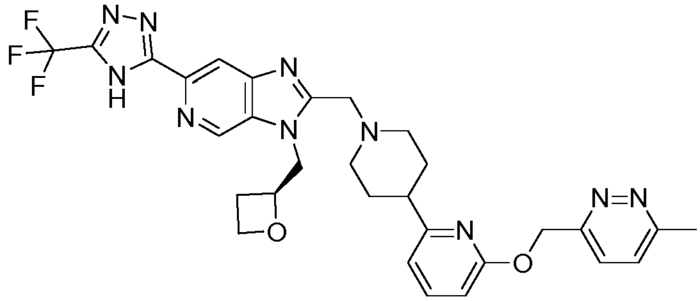

,

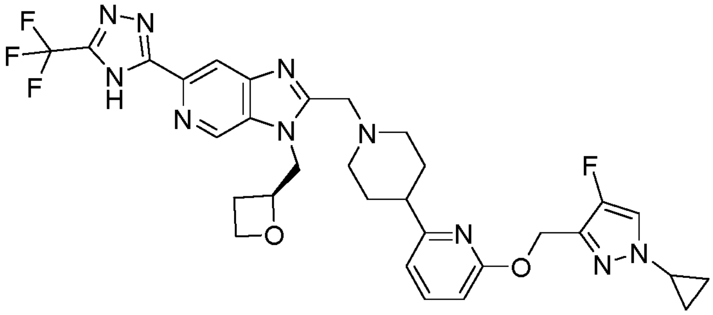

,

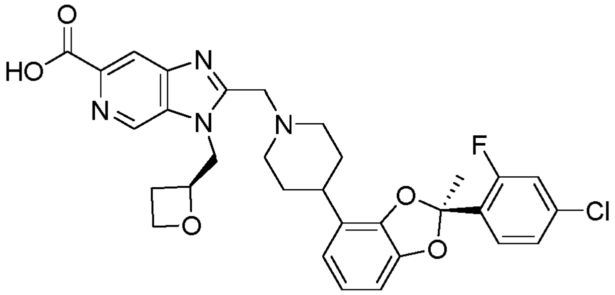

,

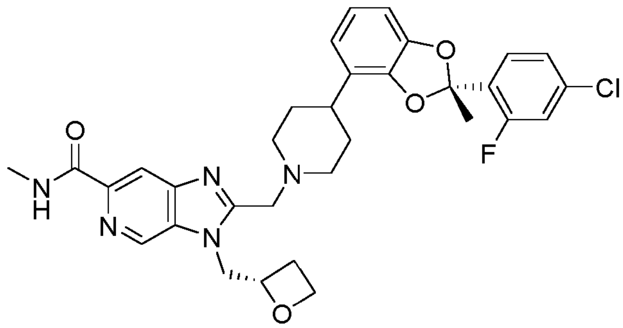

,

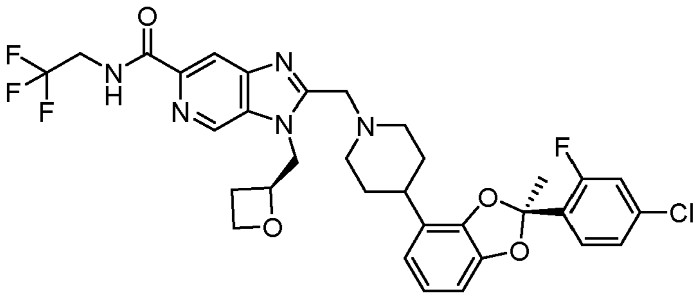

,

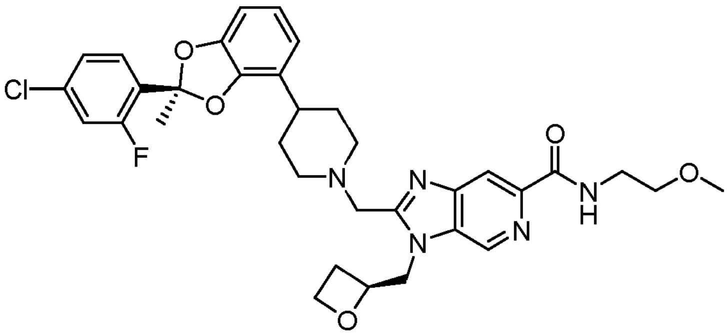

,

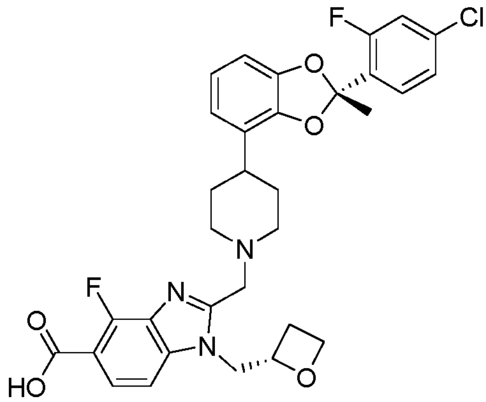

,

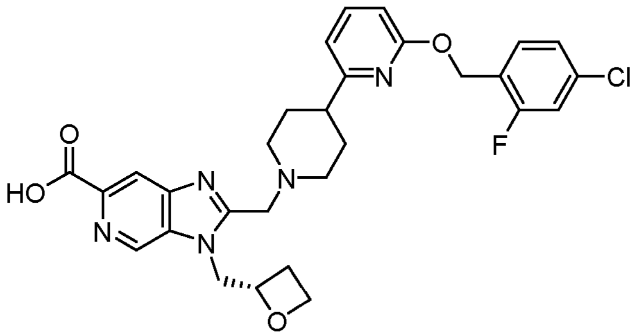

,

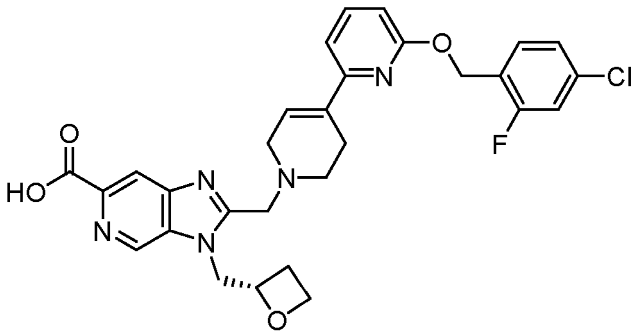

,

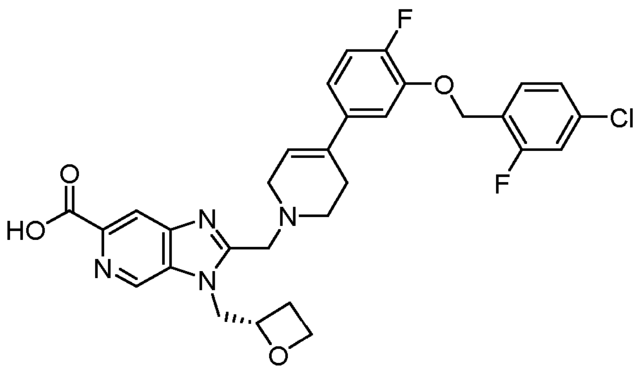

,

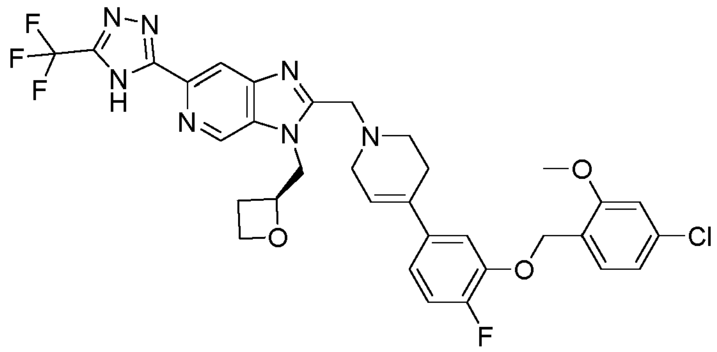

,

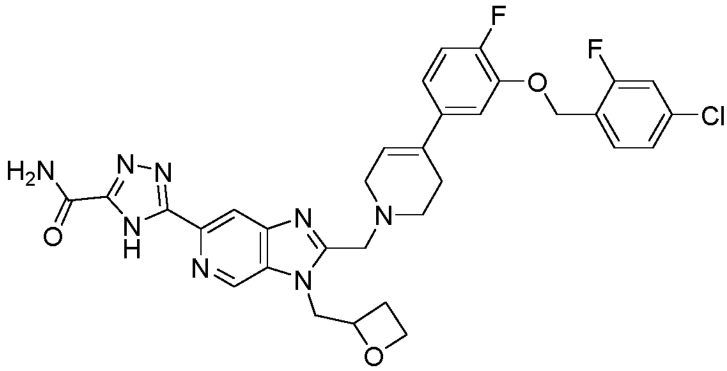

,

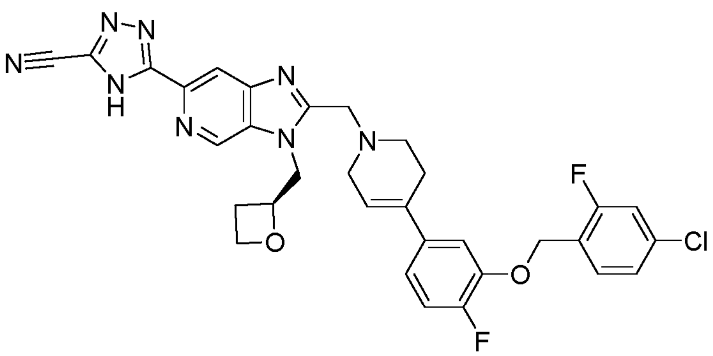

,

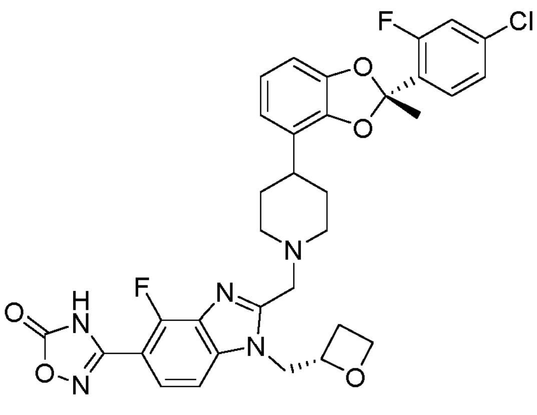

,

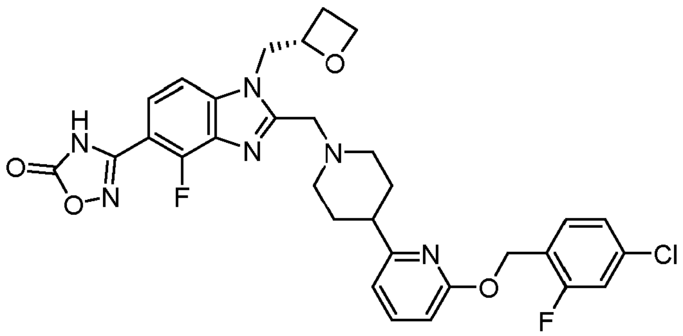

,

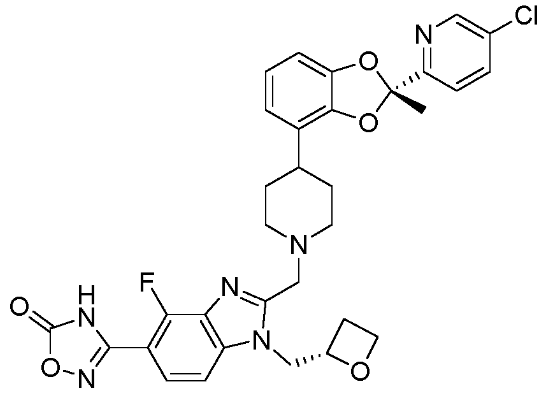

,

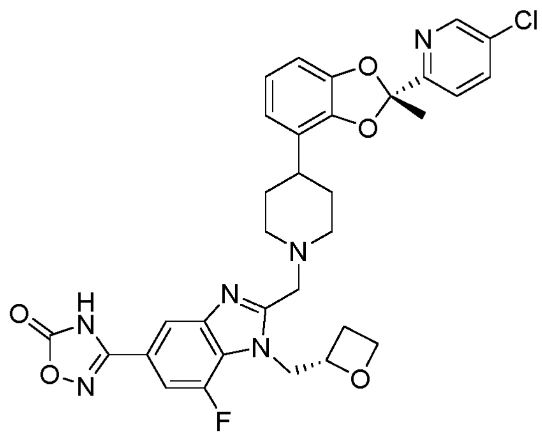

,

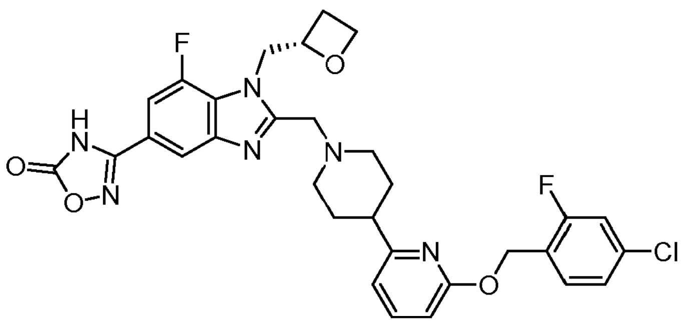

,

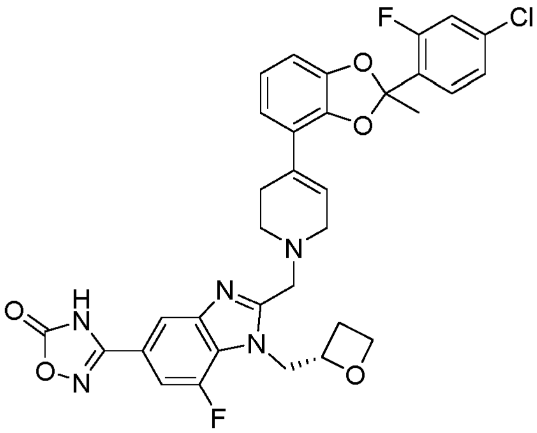

,

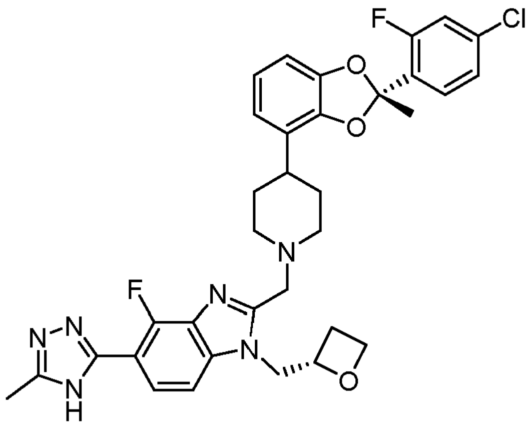

,

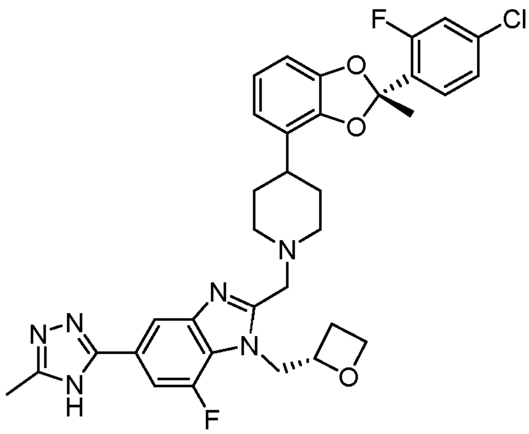

,

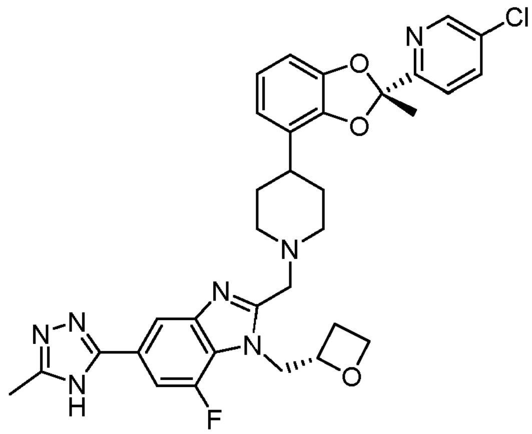

,

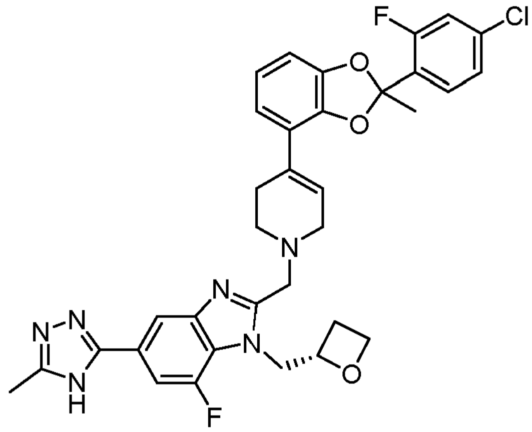

,

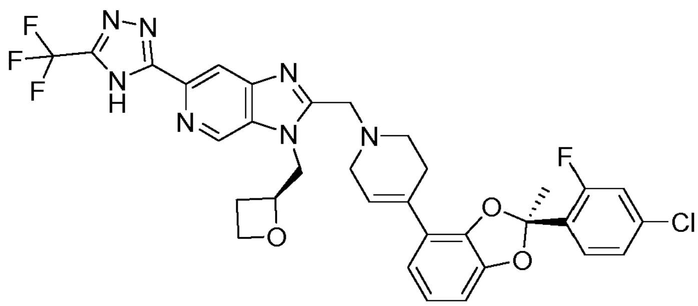

,

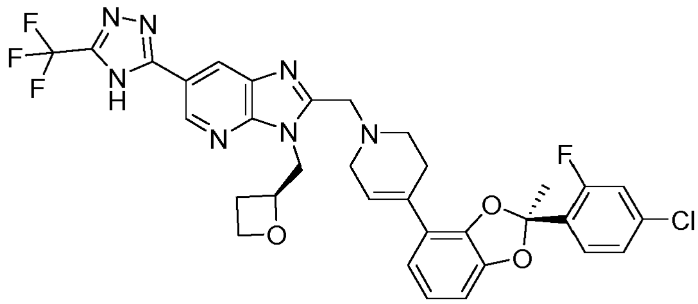

,

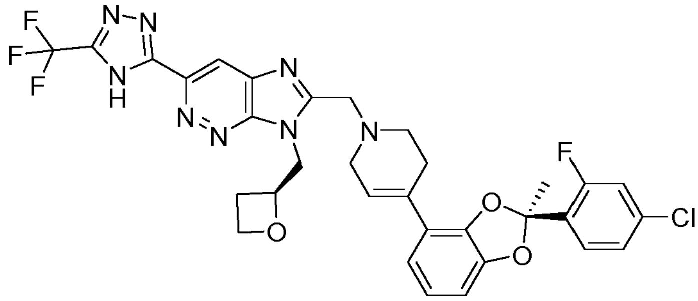

,

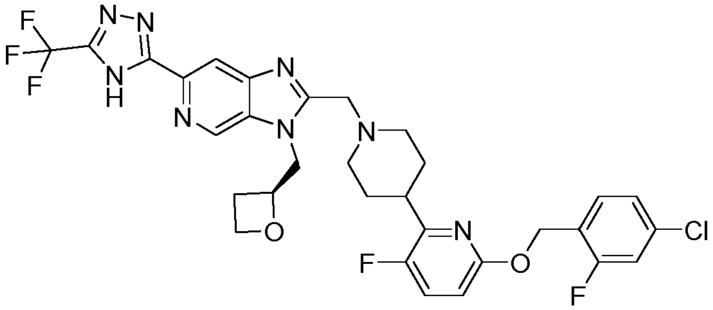

,

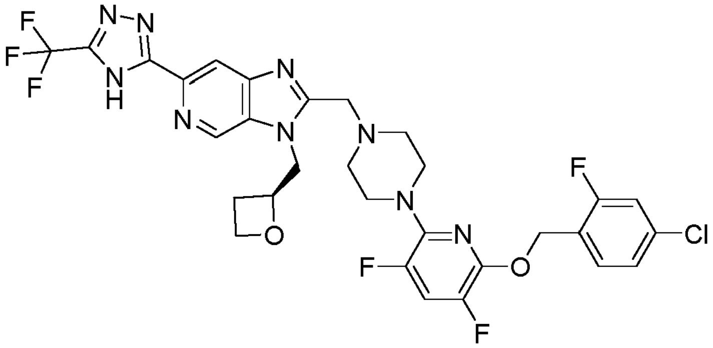

,

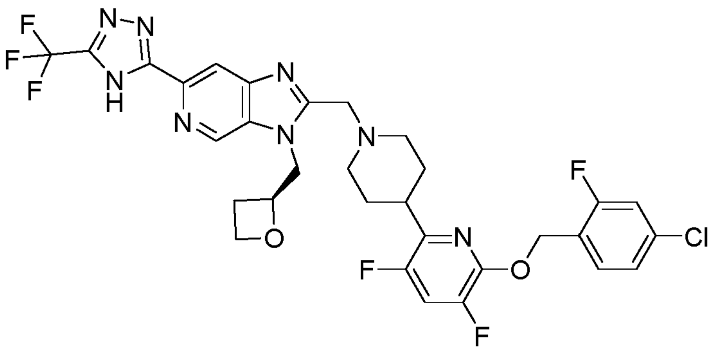

,

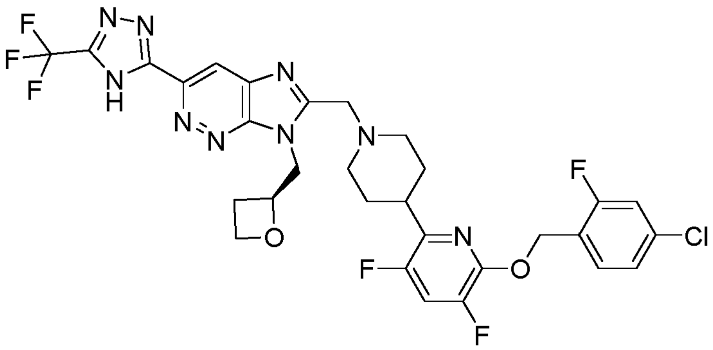

,

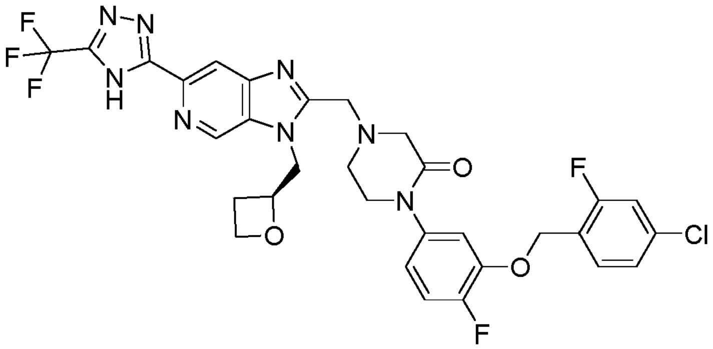

,

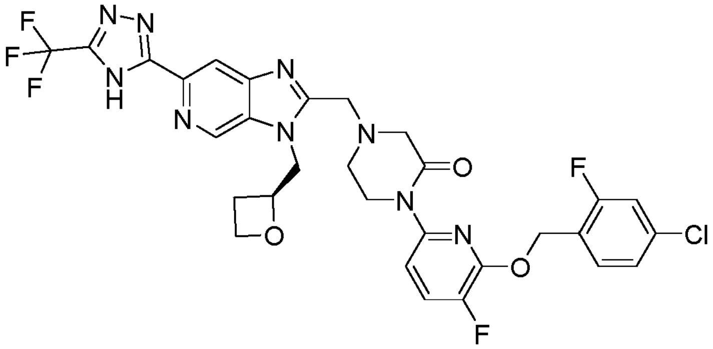

,

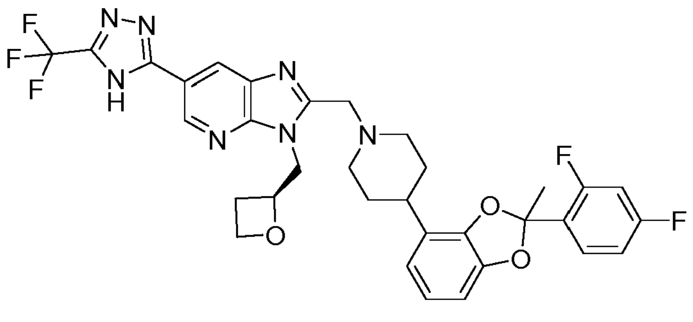

,

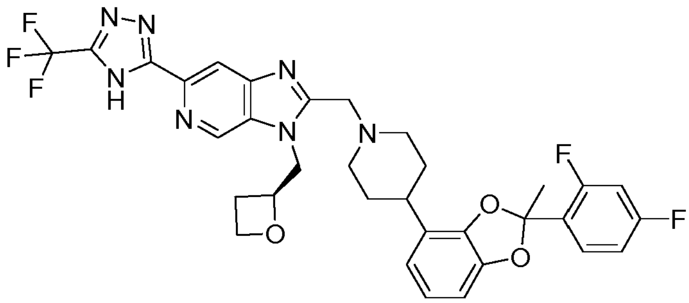

,

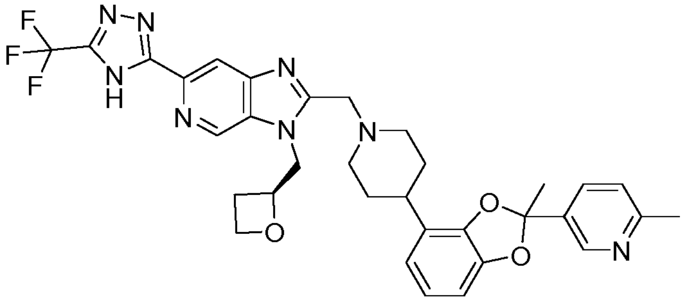

,

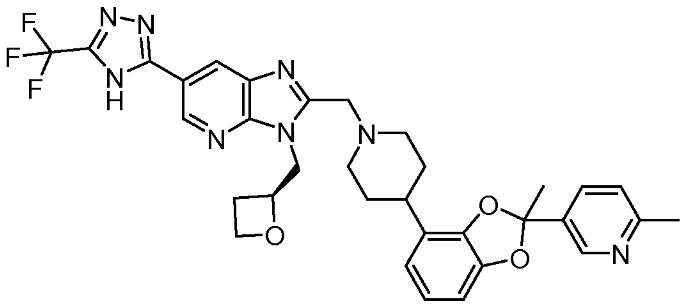

,

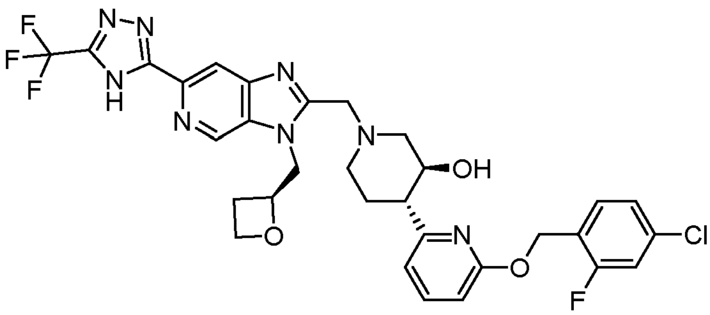

,

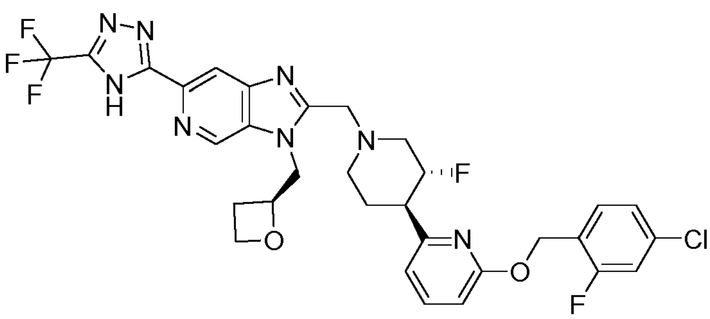

,

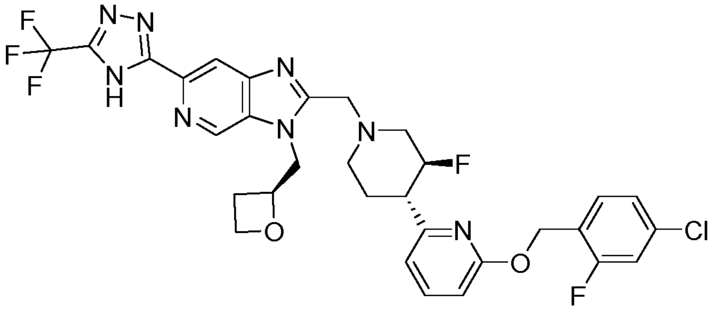

,

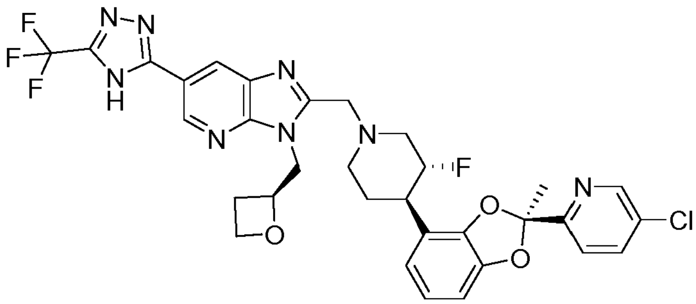

,

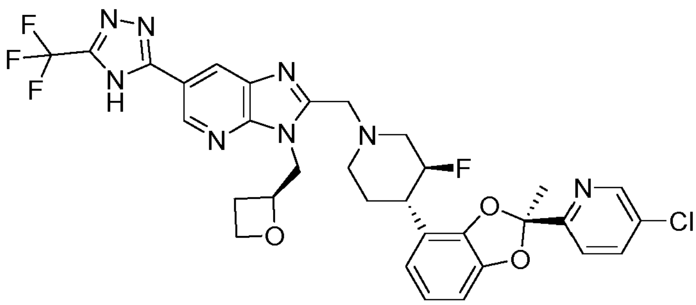

,

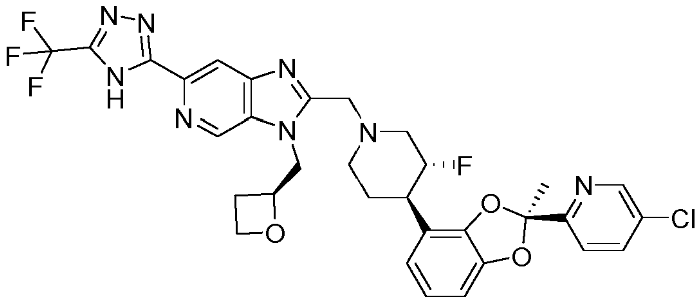

,

and

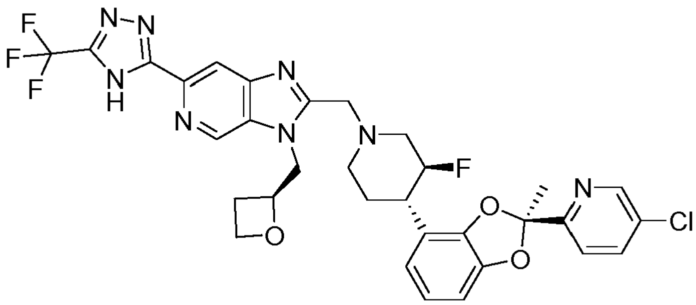

,

or a pharmaceutically acceptable salt or solvate thereof.

2. A pharmaceutical composition comprising a compound of claim 1, or a pharmaceutically acceptable salt or solvate thereof, and a pharmaceutically acceptable excipient.

3. A compound of claim 1, or a pharmaceutically acceptable salt or solvate thereof, or a pharmaceutical composition according to claim 2, for use in a method for treating a GLP-1 associated disease, disorder, or condition, the method comprising administering to a patient in need thereof an effective amount of the compound of claim 1, or a pharmaceutically acceptable salt or solvate thereof, or the pharmaceutical composition according to claim 2, wherein the disease, disorder, or condition is selected from the group consisting of type 1 diabetes mellitus, type 2 diabetes mellitus, early onset type 2 diabetes mellitus, idiopathic type 1 diabetes mellitus (Type 1b), youth-onset atypical diabetes (YOAD), maturity onset diabetes of the young (MODY), latent autoimmune diabetes in adults (LADA), obesity, weight gain from use of other agents, gout, excessive sugar craving, hypertriglyceridemia, dyslipidemia, malnutrition-related diabetes, gestational diabetes, kidney disease, adipocyte dysfunction, sleep apnea, visceral adipose deposition, eating disorders, cardiovascular disease, congestive heart failure, myocardial infarction, left ventricular hypertrophy, peripheral arterial disease, stroke, hemorrhagic stroke, ischemic stroke, transient ischemic attacks, atherosclerotic cardiovascular disease, traumatic brain injury, peripheral vascular disease, endothelial dysfunction, impaired vascular compliance, vascular restenosis, thrombosis, hypertension, pulmonary hypertension, restenosis after angioplasty, intermittent claudication, hyperglycemia, postprandial lipemia, metabolic acidosis, ketosis, hyperinsulinemia, impaired glucose metabolism, insulin resistance, hepatic insulin resistance, alcohol use disorder, chronic renal failure, metabolic syndrome, syndrome X, smoking cessation, premenstrual syndrome, angina pectoris, diabetic nephropathy, impaired glucose tolerance, diabetic neuropathy, diabetic retinopathy, macular degeneration, cataract, glomerulosclerosis, arthritis, osteoporosis, treatment of addiction, cocaine dependence, bipolar disorder/major depressive disorder, skin and connective tissue disorders, foot ulcerations, psoriasis, primary polydipsia, non-alcoholic steatohepatitis (NASH), non-alcoholic fatty liver disease (NAFLD), ulcerative colitis, inflammatory bowel disease, colitis, irritable bowel syndrome, Crohn's disease, short bowel syndrome, Parkinson's, Alzheimer's disease, impaired cognition, schizophrenia, Polycystic Ovary Syndrome (PCOS), or any combination thereof.

4. A compound of claim 1, or a pharmaceutically acceptable salt or solvate thereof, or a pharmaceutical composition according to claim 2, for use in a method of treating type 2 diabetes mellitus in a patient in need thereof, the method comprising administering to a patient in need thereof an effective amount of the compound of claim 1, or a pharmaceutically acceptable salt or solvate thereof, or the pharmaceutical composition according to claim 2.

5. A compound of claim 1, or a pharmaceutically acceptable salt or solvate thereof, or a pharmaceutical composition according to claim 2, for use in a method for modulating insulin levels in a patient in need of such modulating, the method comprising administering to a patient in need thereof an effective amount of the compound of claim 1, or a pharmaceutically acceptable salt or solvate thereof, or the pharmaceutical composition according to claim 2.

6. A compound of claim 1, or a pharmaceutically acceptable salt or solvate thereof, or a pharmaceutical composition according to claim 2, for use in a method for modulating glucose levels in a patient in need of such modulating, the method comprising administering to a patient in need thereof an effective amount of the compound of claim 1, or a pharmaceutically acceptable salt or solvate thereof, or the pharmaceutical composition according to claim 2.

7. The compound, pharmaceutically acceptable salt, solvate or pharmaceutical composition for use of any one of claims 3-6, further comprising administering an additional therapy or therapeutic agent to the patient.

8. The compound, pharmaceutically acceptable salt, solvate or pharmaceutical composition for use of claim 7, wherein the additional therapy or therapeutic agent is selected from the group consisting of an antidiabetic agent, an anti-obesity agent, a GLP-1 receptor agonist, an anti-emetic agent, an agent to treat non-alcoholic steatohepatitis (NASH), gastric electrical stimulation, dietary monitoring, physical activity, or a combination thereof.

## Patentansprüche

1. Verbindung, ausgewählt aus:

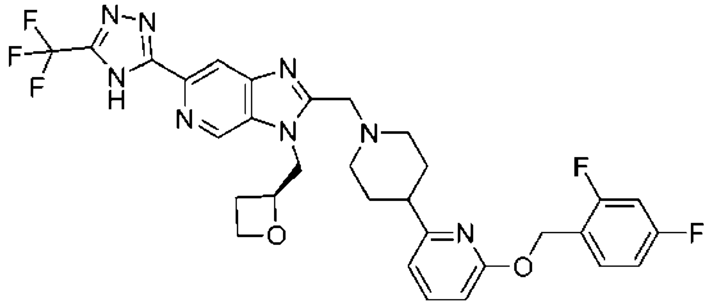

.

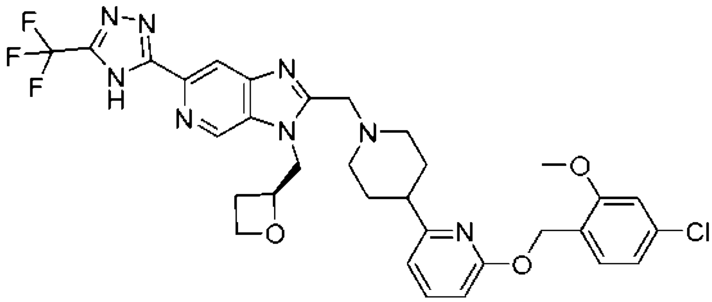

,

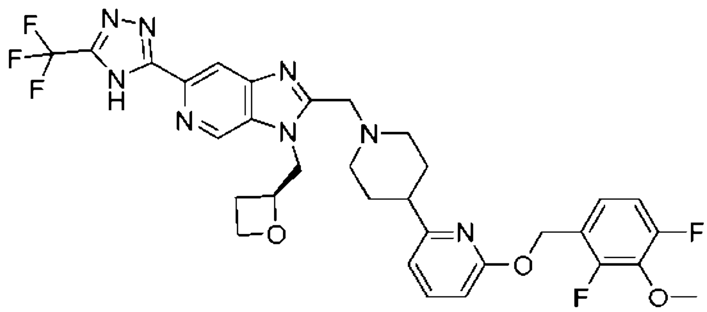

.

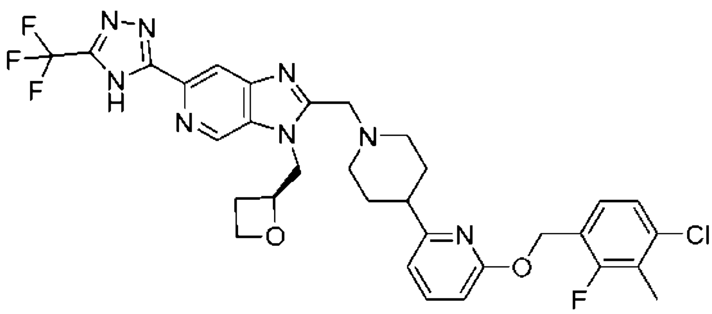

.

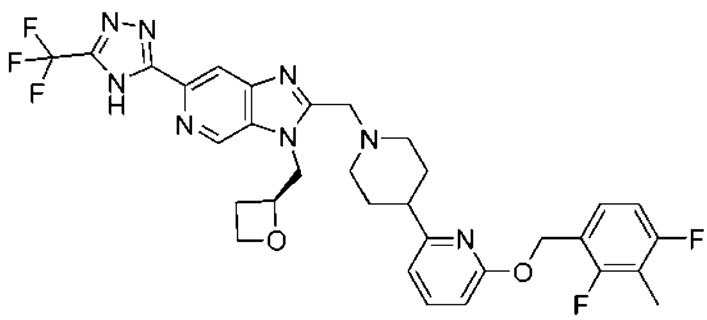

,

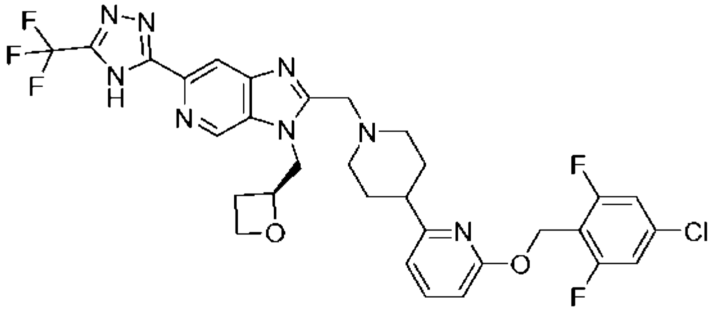

,

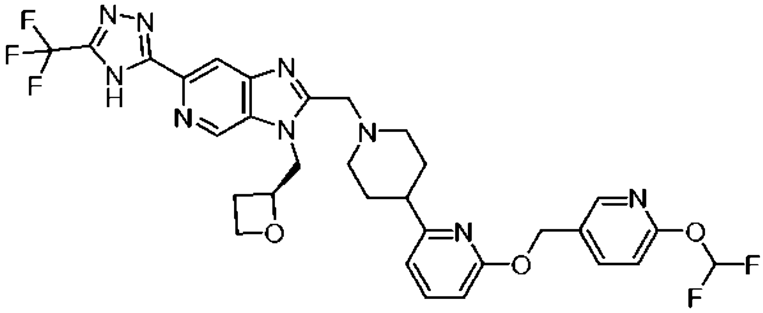

,

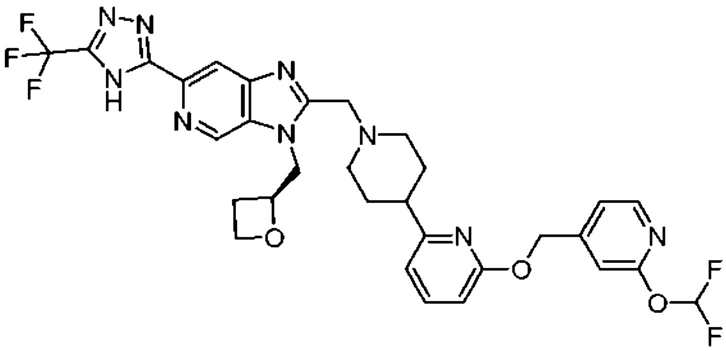

,

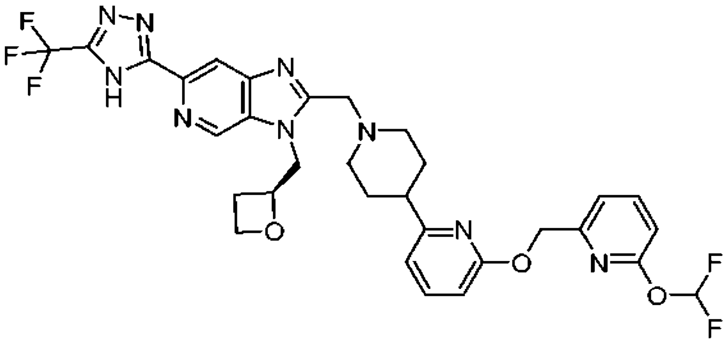

,

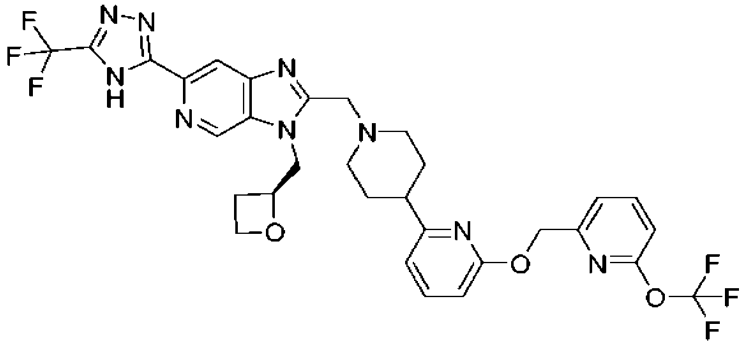

,

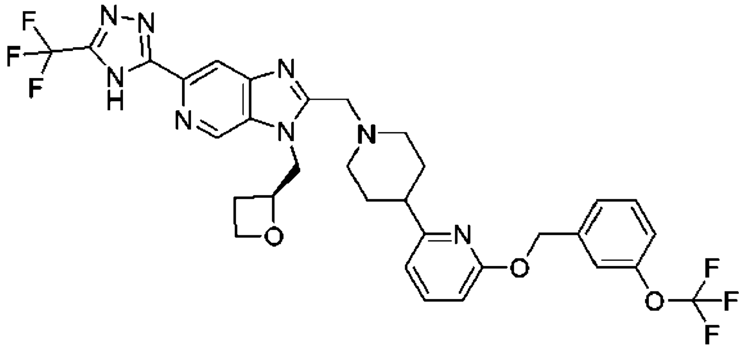

,

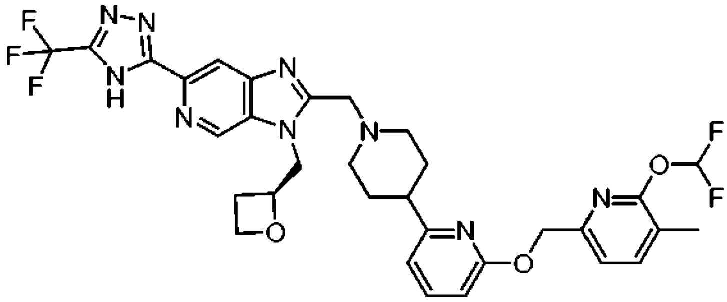

,

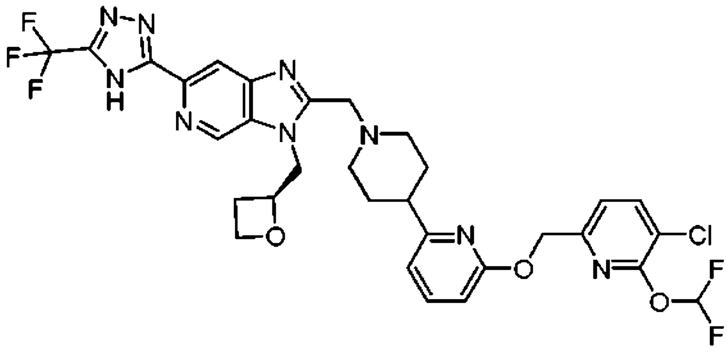

,

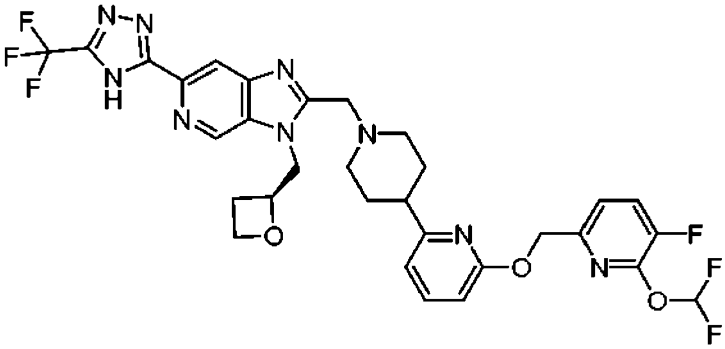

,

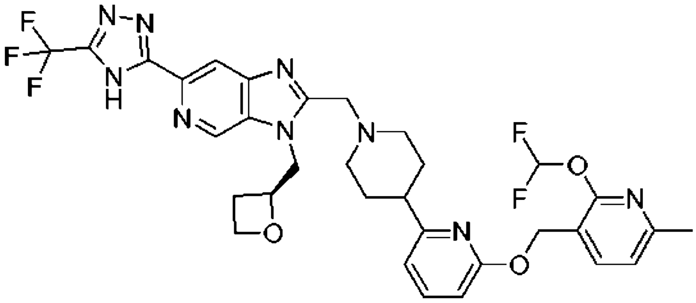

,

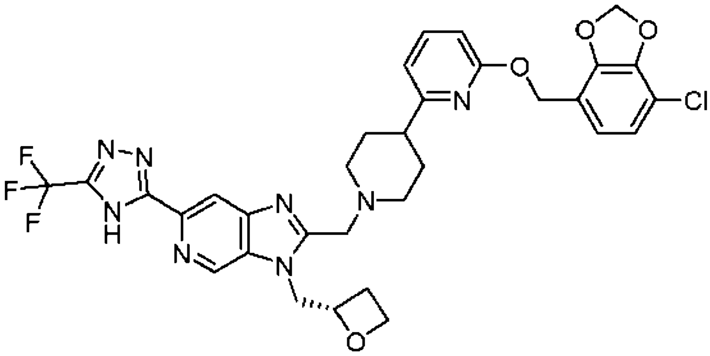

,

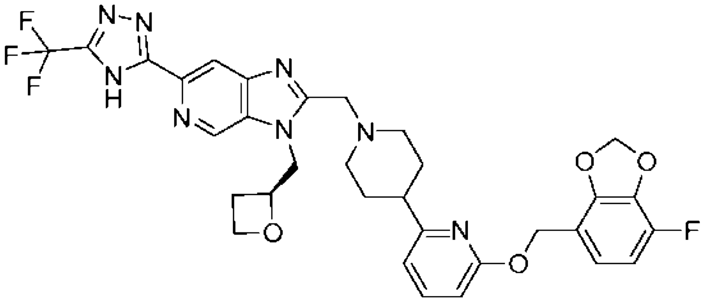

,

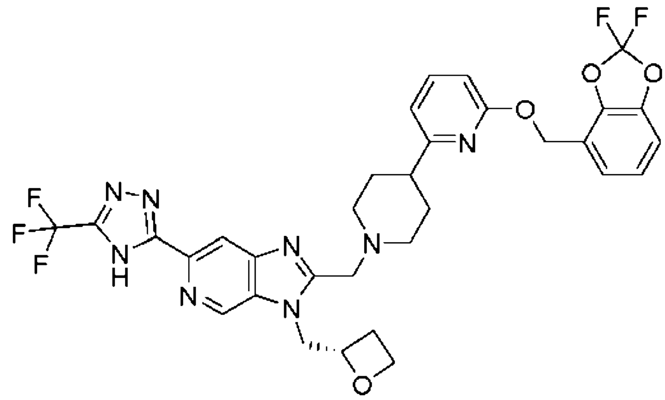

,

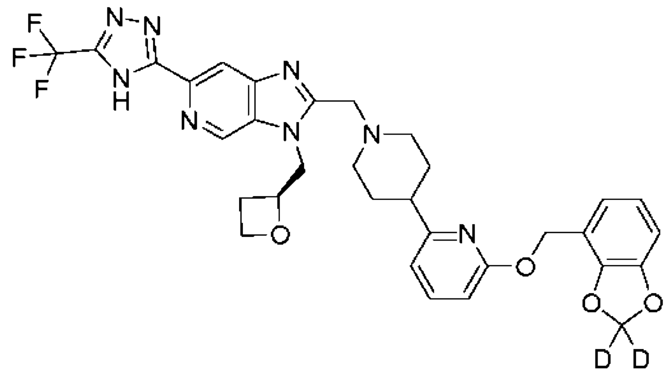

,

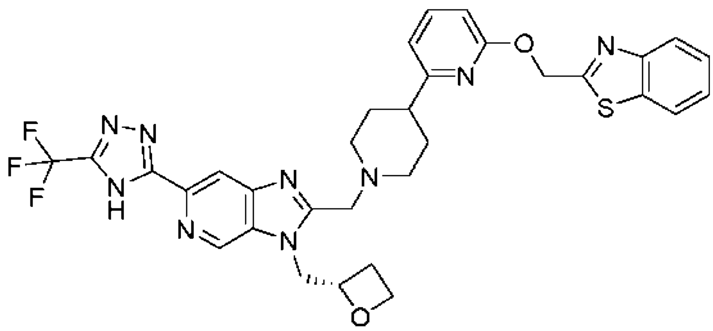

,

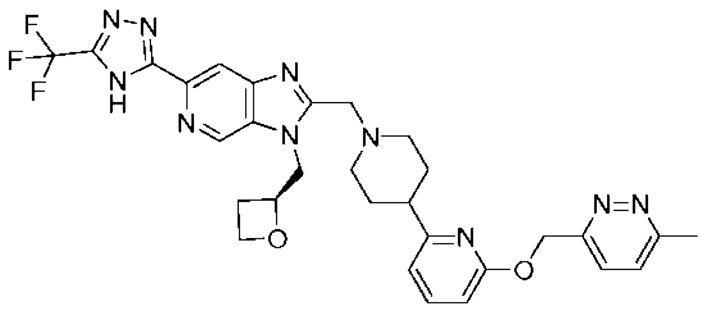

,

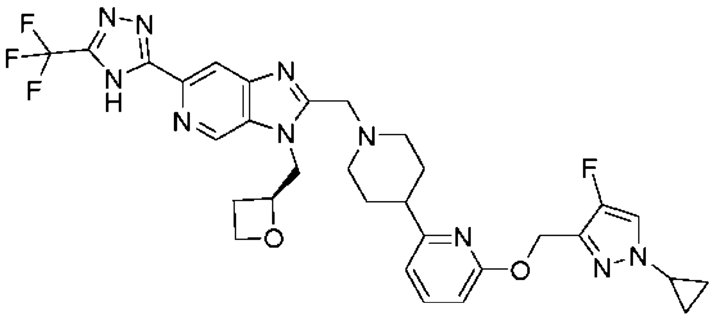

,

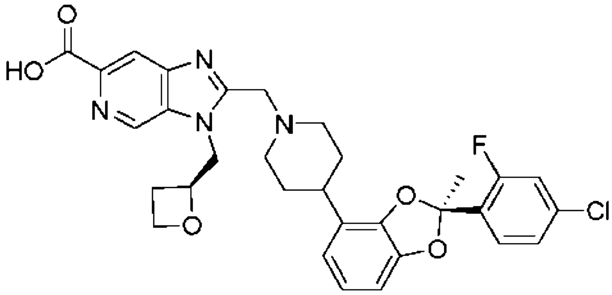

,

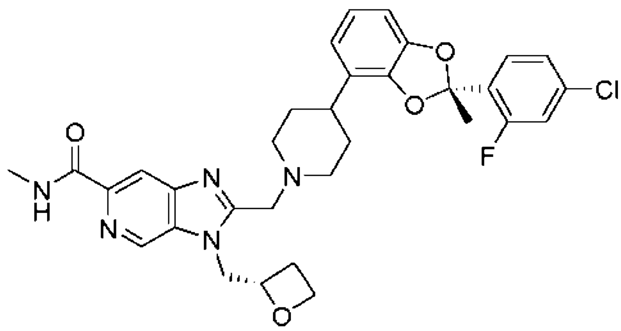

,

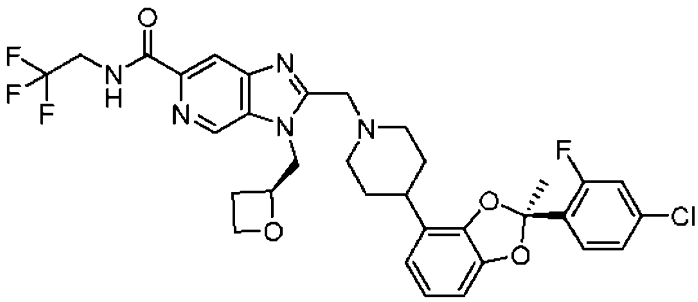

,

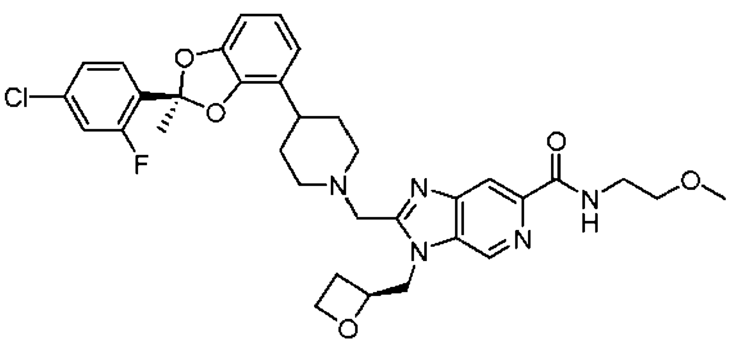

,

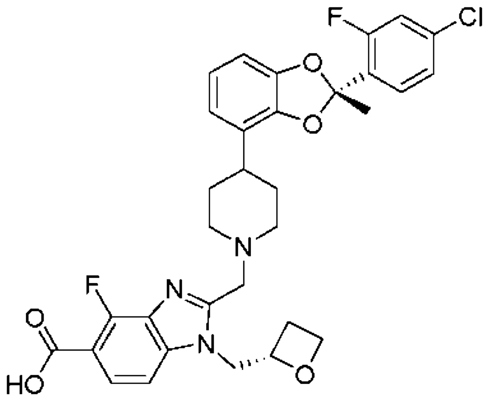

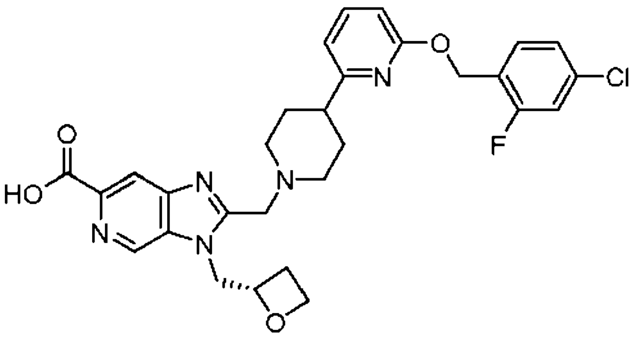

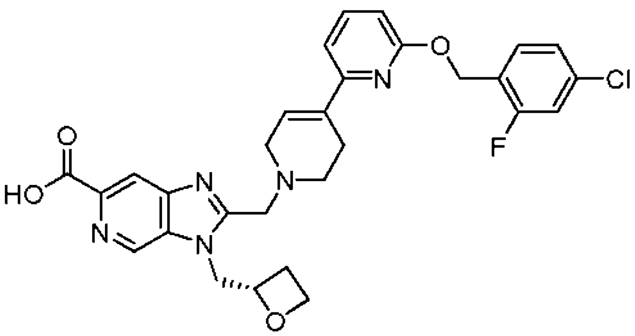

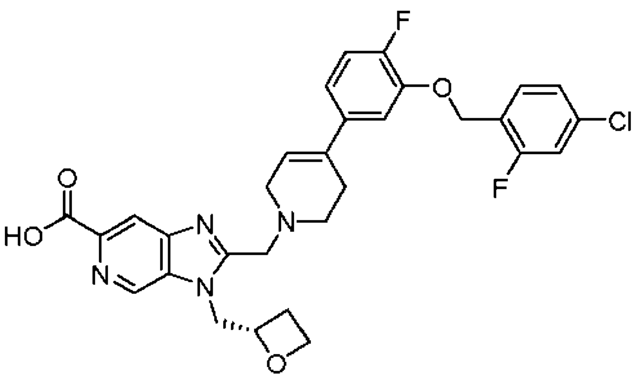

,

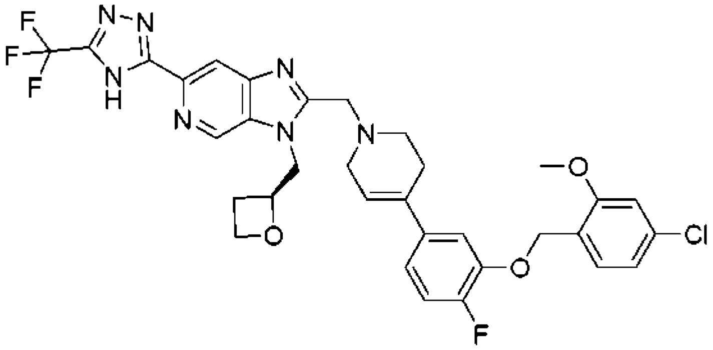

.

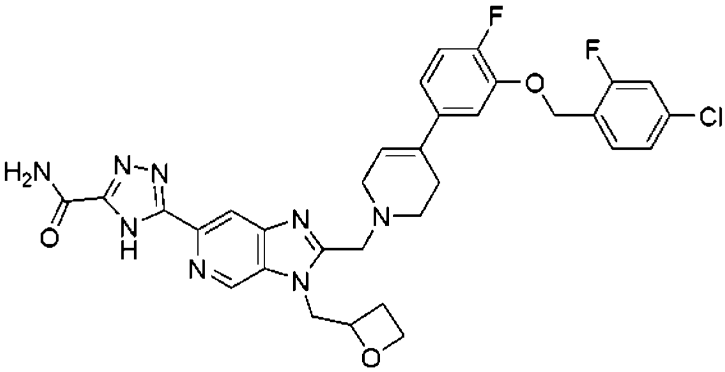

.

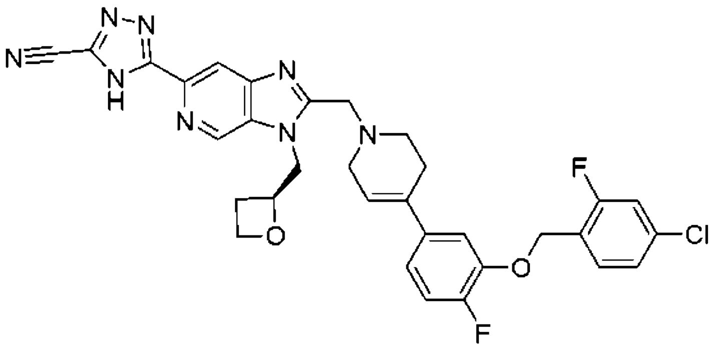

.

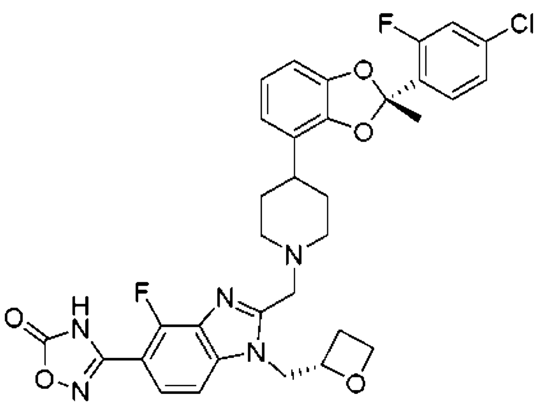

.

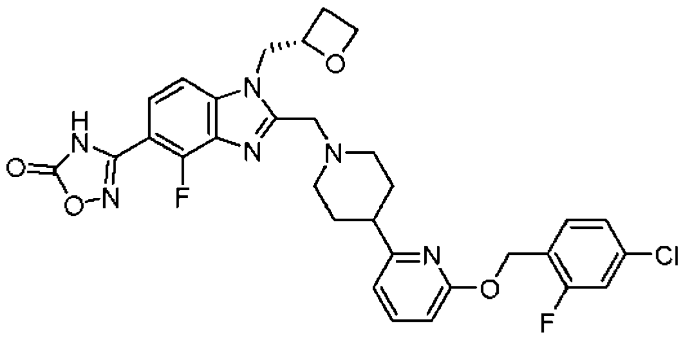

,

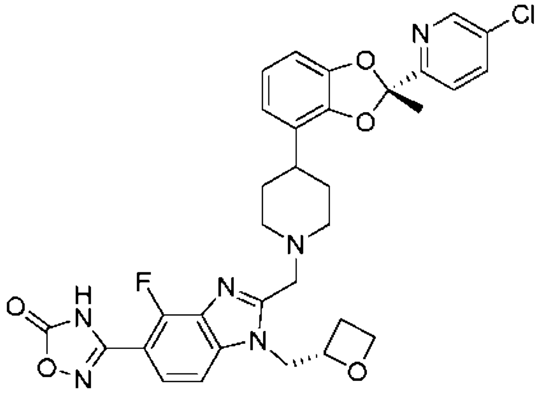

,

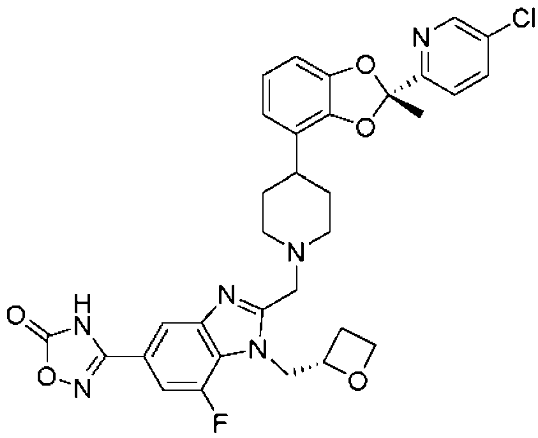

.

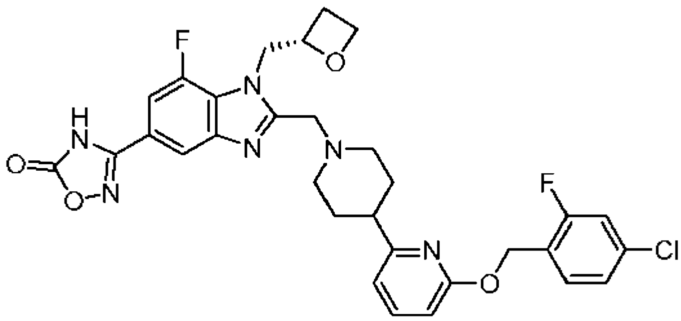

,

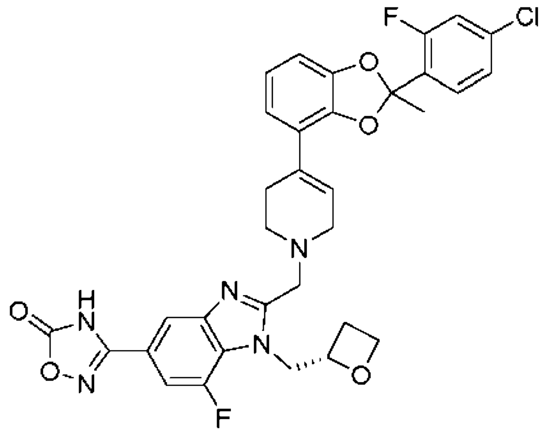

,

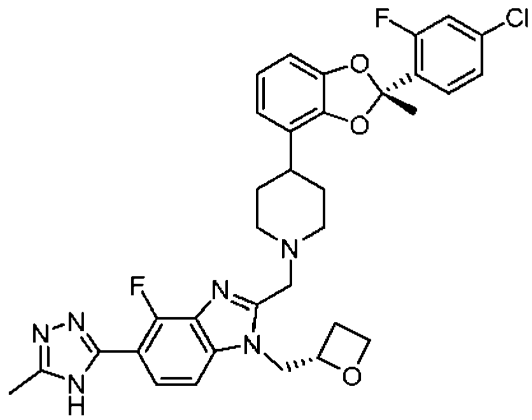

,

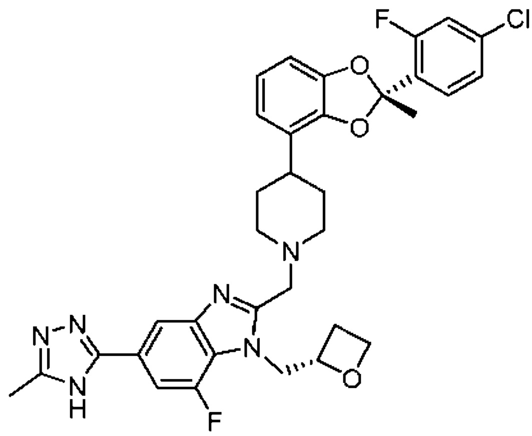

,

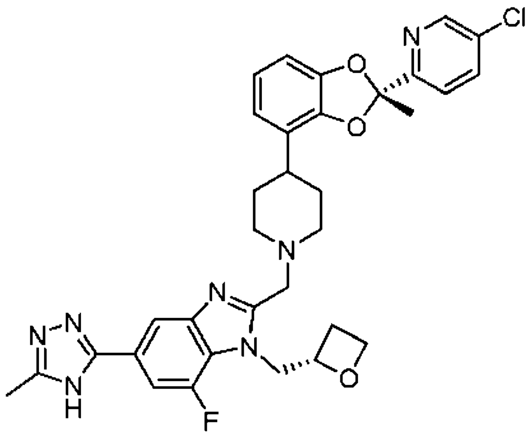

,

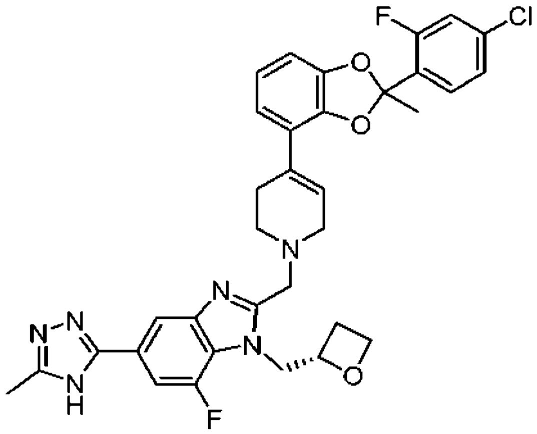

,

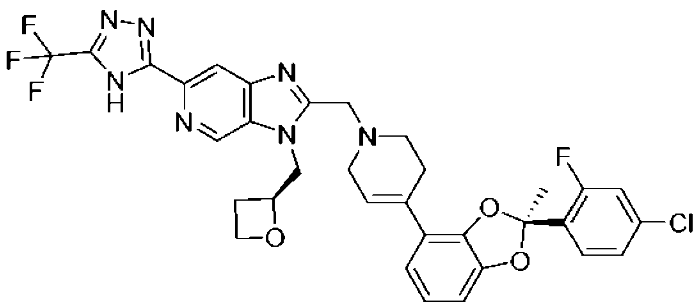

,

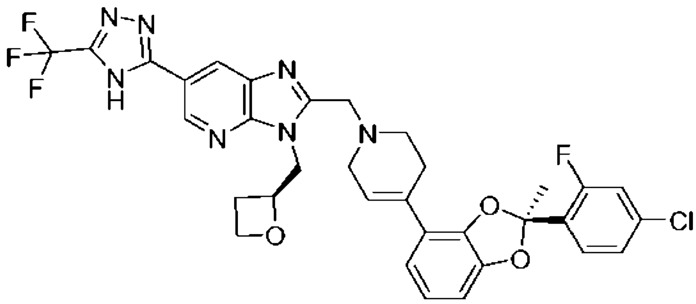

,

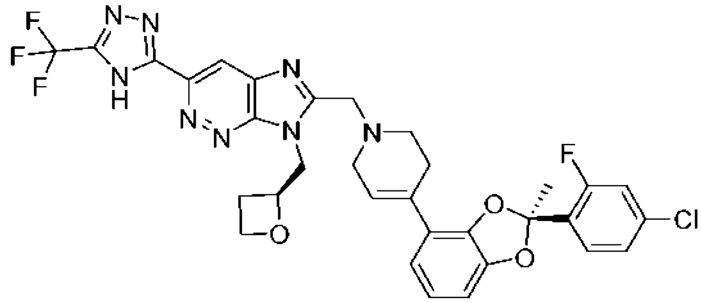

,

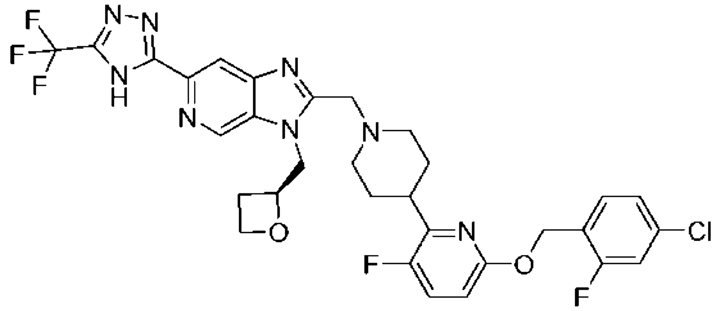

,

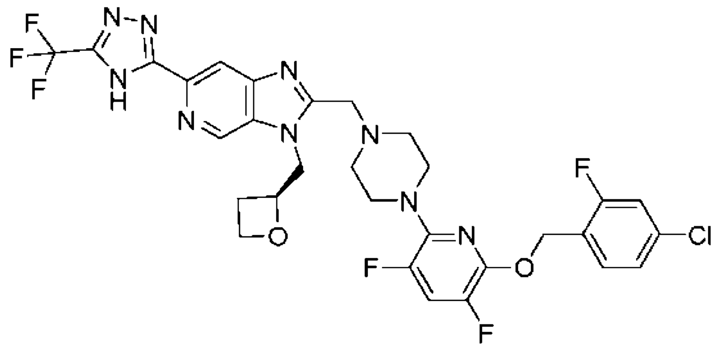

.

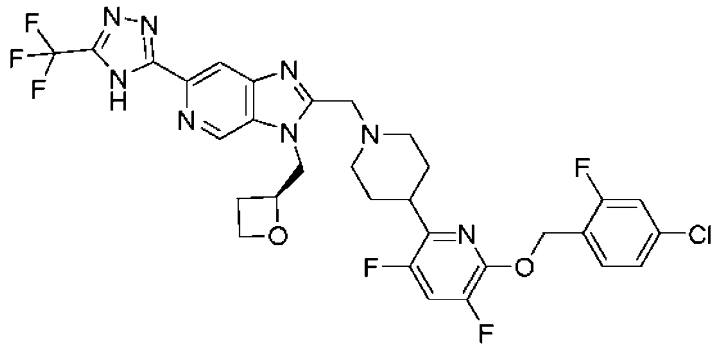

.

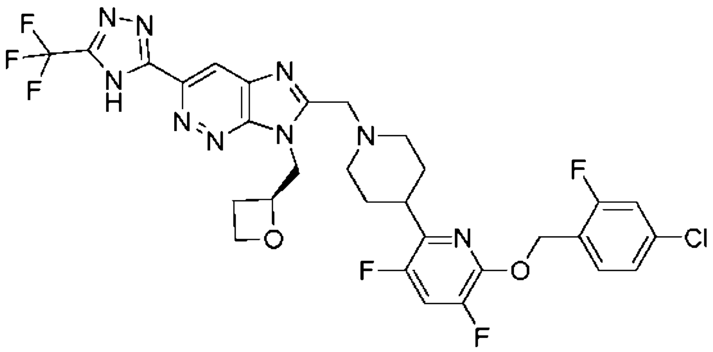

,

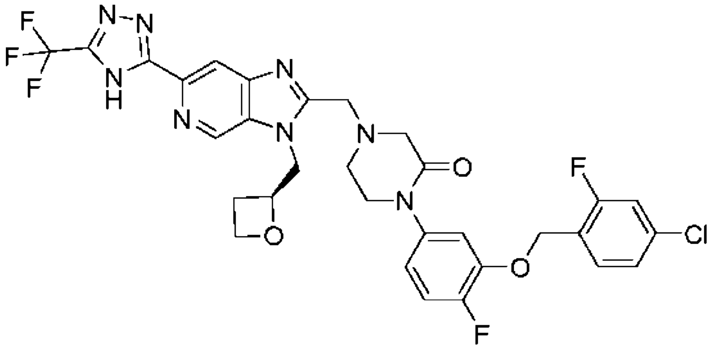

,

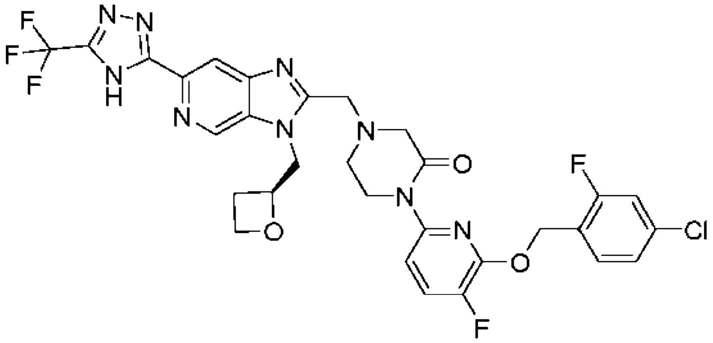

,

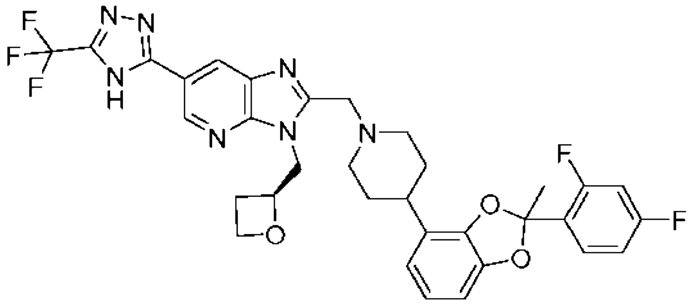

,

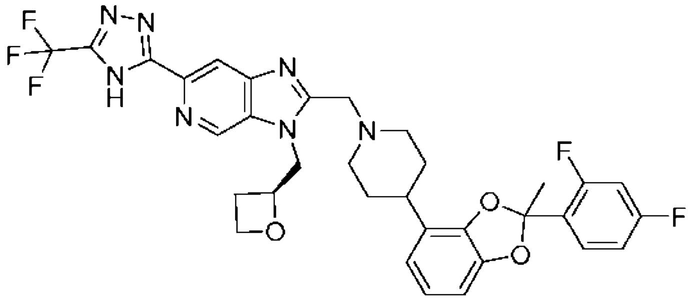

.

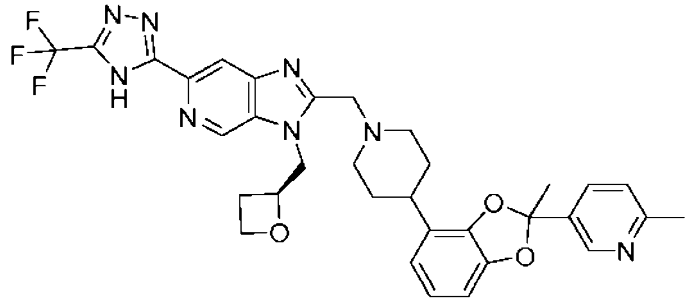

.

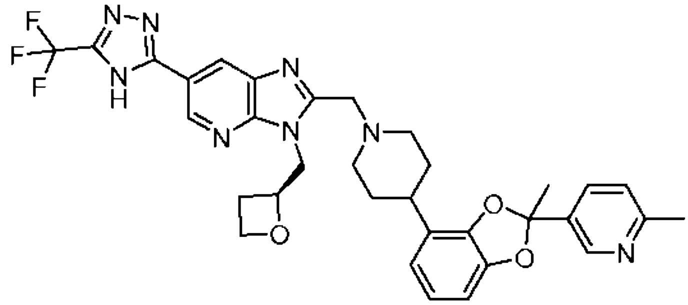

;

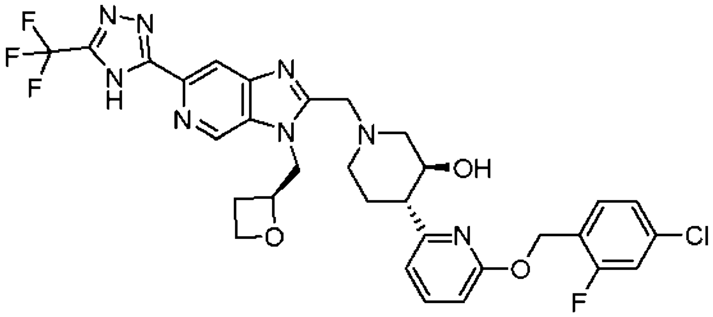

.

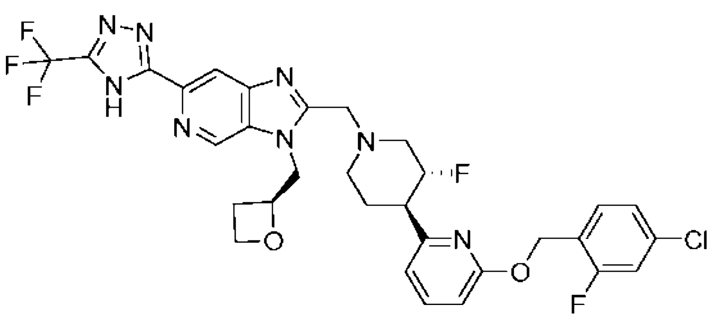

.

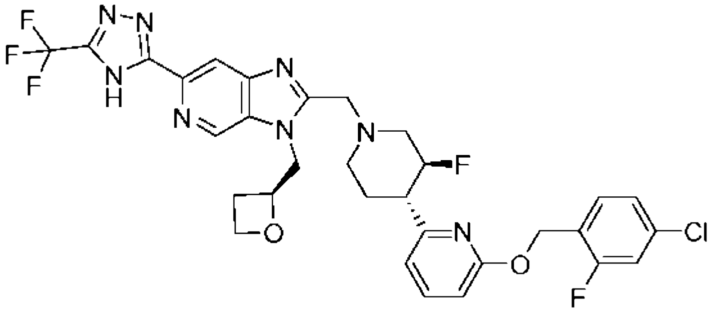

,

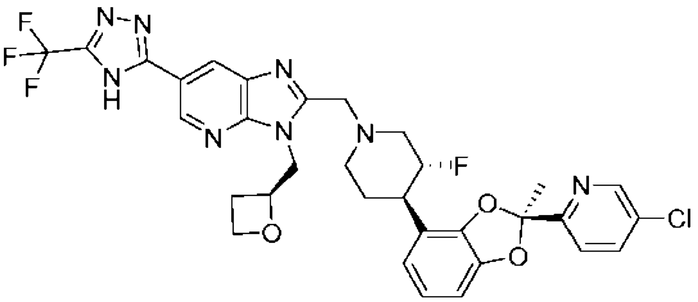

,

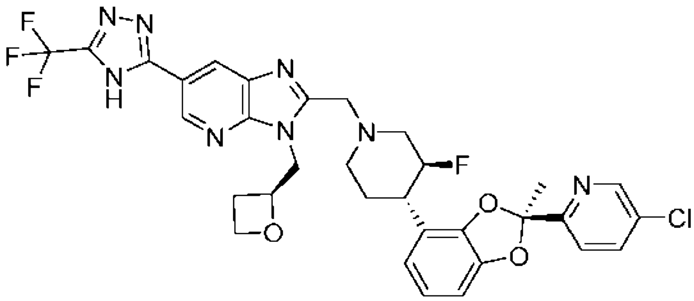

,

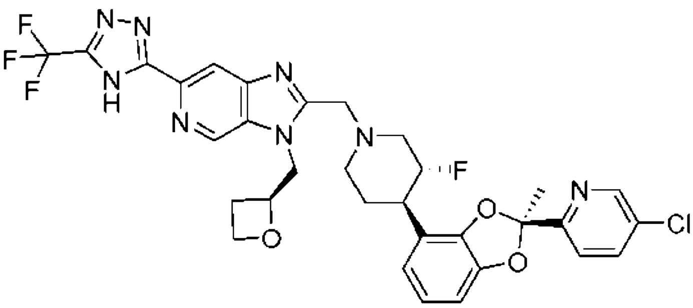

und

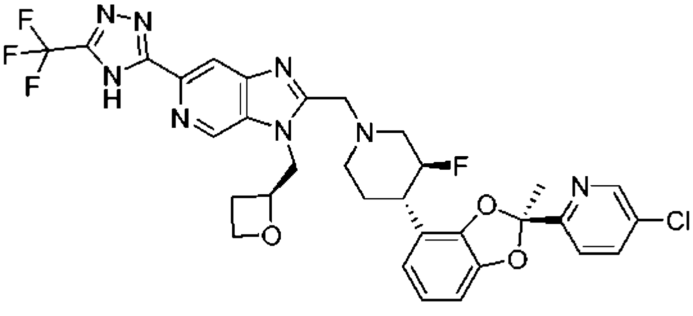

,

oder ein pharmazeutisch unbedenkliches Salz oder Solvat davon.

**2.** Pharmazeutische Zusammensetzung, umfassend eine Verbindung nach Anspruch 1 oder ein pharmazeutisch unbedenkliches Salz oder Solvat davon und einen pharmazeutisch unbedenklichen Hilfsstoff.

**3.** Verbindung nach Anspruch 1 oder ein pharmazeutisch unbedenkliches Salz oder Solvat davon oder pharmazeutische Zusammensetzung nach Anspruch 2 zur Verwendung bei einem Verfahren zur Behandlung einer mit GLP-1 assoziierten Erkrankung, einer mit GLP-1 assoziierten Störung oder einem mit GLP-1 assoziierten Leiden, wobei das Verfahren das Verabreichen einer wirksamen Menge der Verbindung nach Anspruch 1 oder eines pharmazeutisch unbedenklichen Salzes oder Solvats davon oder der pharmazeutischen Zusammensetzung nach Anspruch 2 an einen Patienten, bei dem diesbezüglicher Bedarf besteht, umfasst,
wobei die Erkrankung, die Störung oder das Leiden ausgewählt ist aus der Gruppe bestehend aus Diabetes mellitus Typ 1, Diabetes mellitus Typ 2, früh auftretendem Diabetes mellitus Typ 2, idiopathischem Diabetes mellitus Typ 1 (Typ 1b), in der Jugend auftretendem atypischem Diabetes (YOAD), Maturity Onset Diabetes of the Young (MODY), Latent Autoimmune Diabetes in Adults (LADA), Adipositas, Gewichtszunahme durch Anwendung anderer Mittel, Gicht, übermäßigem Verlangen nach Zucker, Hypertriglyceridämie, Dyslipidämie, durch Mangelernährung bedingtem Diabetes, Schwangerschaftsdiabetes, Nierenerkrankung, Adipozytendysfunktion, Schlafapnoe, adipöser Gefäßablagerung, Essstörungen, Herz-Kreislauf-Erkrankung, kongestiver Herzinsuffizienz, Myokardinfarkt, linksventrikulärer Hypertrophie, peripherer arterieller Erkrankung, Schlaganfall, hämorrhagischem Schlaganfall, ischämischem Schlaganfall, transitorischen ischämischen Attacken, atherosklerotischer Herz-Kreislauf-Erkrankung, traumatischer Hirnverletzung, peripherer Gefäßerkrankung, endothelialer Dysfunktion, gestörter Gefäß-Compliance, Gefäß-Restenose, Thrombose, Hypertonie, pulmonaler Hypertonie, Restenose nach Angioplastie, Claudicatio intermittens, Hyperglykämie, postprandialer Lipämie, metabolischer Azidose, Ketose, Hyperinsulinämie, gestörtem Glucose-Stoffwechsel, Insulinresistenz, hepatischer Insulinresistenz, Alkoholmissbrauch, chronischer Niereninsuffizienz, metabolischem Syndrom, Syndrom X, Raucherentwöhnung, prämenstruellem Syndrom, Angina pectoris, diabetischer Nephropathie, gestörter Glucosetoleranz, diabetischer Neuropathie, diabetischer Retinopathie, Makuladegeneration, Katarakt, Glomerulosklerose, Arthritis, Osteoporose, Suchtbehandlung, Kokainabhängigkeit, bipolarer Störung/starker depressiver Störung, Haut- und Bindegewebsstörungen, Fußgeschwülsten, Psoriasis, primärer Polydipsie, nichtalkoholischer Steatohepatitis (NASH), nichtalkoholischer Fettlebererkrankung (NAFLD), Colitis ulcerosa, entzündlicher Darmkrankheit, Kolitis, Reizdarmsyndrom, Morbus Crohn, Kurzdarmsyndrom, Parkinson-Krankheit, Alzheimer-Krankheit, gestörter Kognition, Schizophrenie, polyzystischem Ovarialsyndrom (PCOS) oder beliebigen Kombinationen davon.

**4.** Verbindung nach Anspruch 1 oder pharmazeutisch unbedenkliches Salz oder Solvat davon oder pharmazeutische Zusammensetzung nach Anspruch 2 zur Verwendung bei einem Verfahren zur Behandlung von Diabetes mellitus Typ 2 bei einem Patienten, bei dem diesbezüglicher Bedarf besteht, wobei das Verfahren das Verabreichen einer wirksamen Menge der Verbindung nach Anspruch 1 oder eines pharmazeutisch unbedenklichen Salzes oder Solvats davon oder der pharmazeutischen Zusammensetzung nach Anspruch 2 an einen Patienten, bei dem diesbezüglicher Bedarf besteht, umfasst.

**5.** Verbindung nach Anspruch 1 oder pharmazeutisch unbedenkliches Salz oder Solvat davon oder pharmazeutische Zusammensetzung nach Anspruch 2 zur Verwendung bei einem Verfahren zur Modulierung von Insulinspiegeln bei einem Patienten, der einer derartigen Modulierung bedarf, wobei das Verfahren das Verabreichen einer wirksamen Menge der Verbindung nach Anspruch 1 oder eines pharmazeutisch unbedenklichen Salzes oder Solvats davon oder der pharmazeutischen Zusammensetzung nach Anspruch 2 an einen Patienten, bei dem diesbezüglicher Bedarf besteht, umfasst.

**6.** Verbindung nach Anspruch 1 oder pharmazeutisch unbedenkliches Salz oder Solvat davon oder pharmazeutische Zusammensetzung nach Anspruch 2 zur Verwendung bei einem Verfahren zur Modulierung von Glucosespiegeln bei einem Patienten, der einer derartigen Modulierung bedarf, wobei das Verfahren das Verabreichen einer wirksamen Menge der Verbindung nach Anspruch 1 oder eines pharmazeutisch unbedenklichen Salzes oder Solvats davon oder der pharmazeutischen Zusammensetzung nach Anspruch 2 an einen Patienten, bei dem diesbezüglicher Bedarf besteht, umfasst.

**7.** Verbindung, pharmazeutisch unbedenkliches Salz, Solvat oder pharmazeutische Zusammensetzung zur Verwendung nach einem der Ansprüche 3-6, ferner umfassend das Verabreichen einer zusätzlichen Therapie oder eines zusätzlichen therapeutischen Mittels an den Patienten.

8. Verbindung, pharmazeutisch unbedenkliches Salz, Solvat oder pharmazeutische Zusammensetzung zur Verwendung nach Anspruch 7, wobei die zusätzliche Therapie bzw. das therapeutische Mittel aus der Gruppe bestehend aus einem Antidiabetikum, einem Mittel gegen Fettleibigkeit, einem GLP-1-Rezeptor-Agonisten, einem Antiemetikum, einem Mittel zur Behandlung von nichtalkoholischer Steatohepatitis (NASH), elektrischer Magenstimulation, Ernährungsüberwachung, körperlicher Aktivität oder einer Kombination davon ausgewählt ist.

## Revendications

1. Composé choisi parmi :

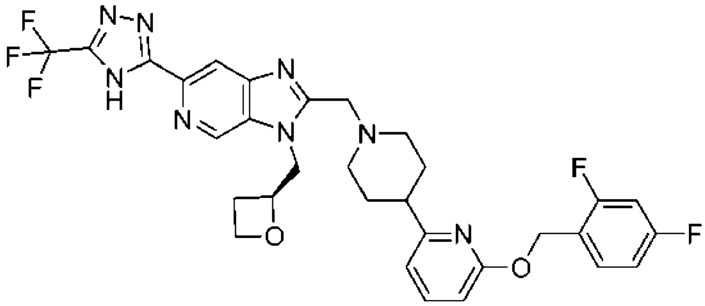

.

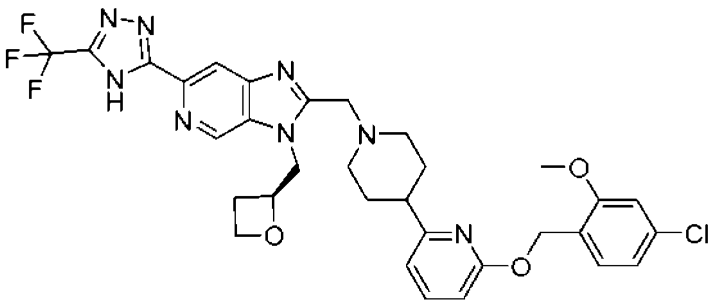

;

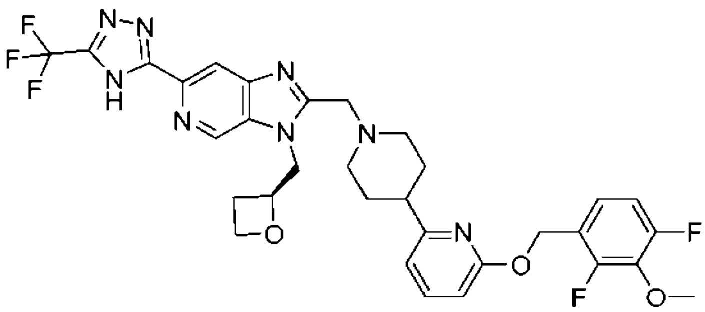

.

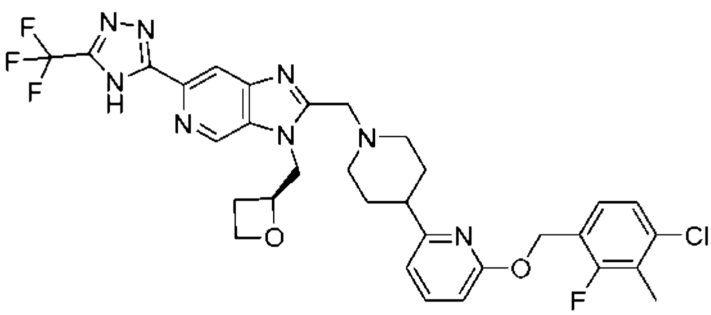

.

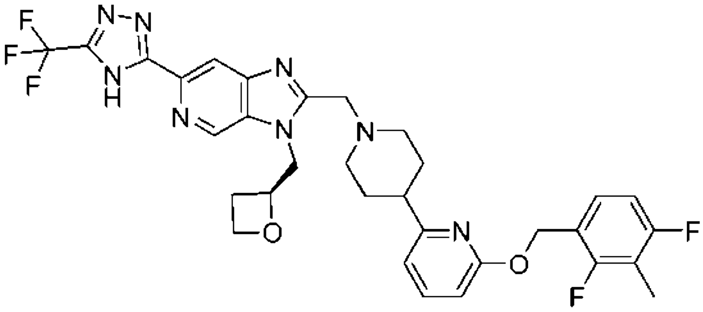

,

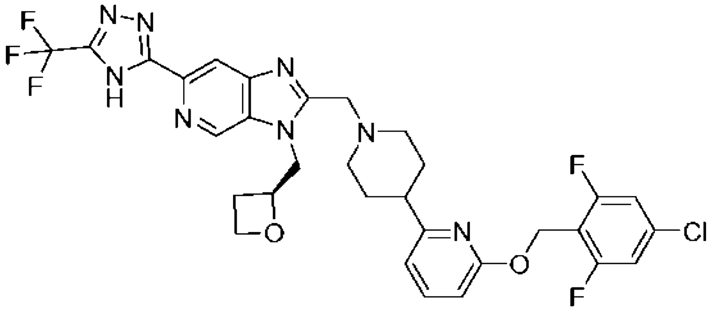

,

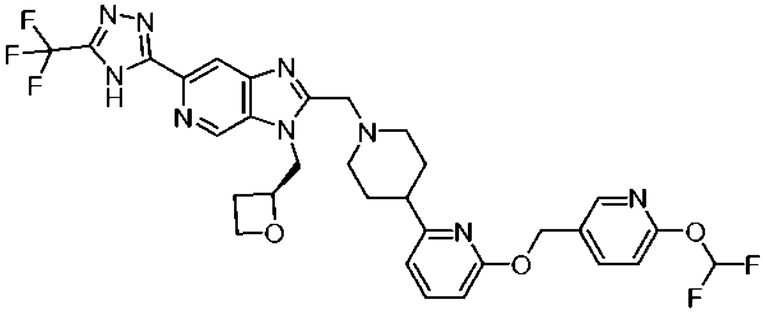

,

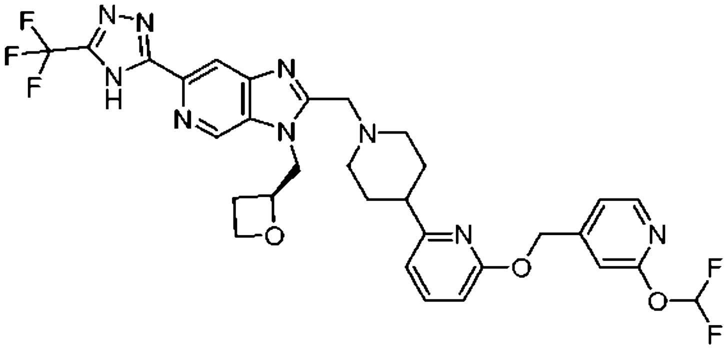

,

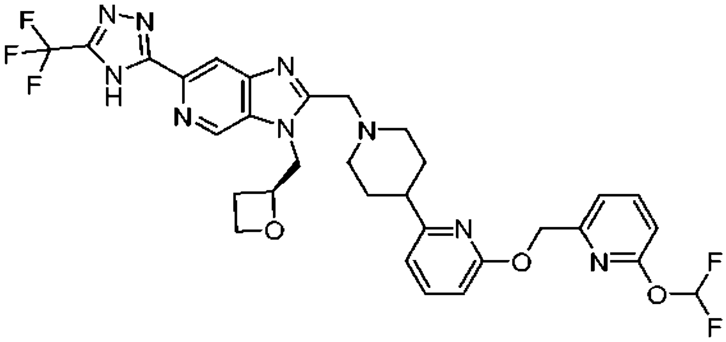

,

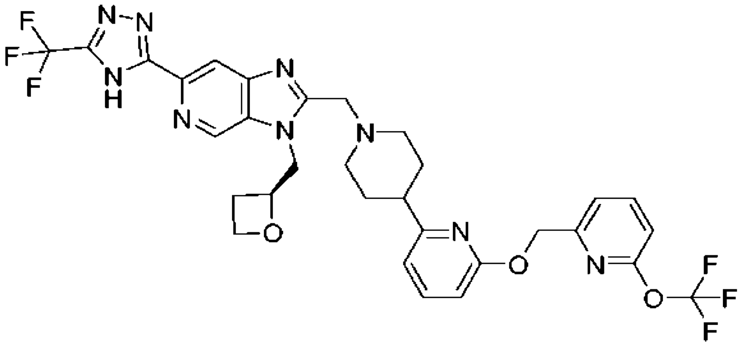

,

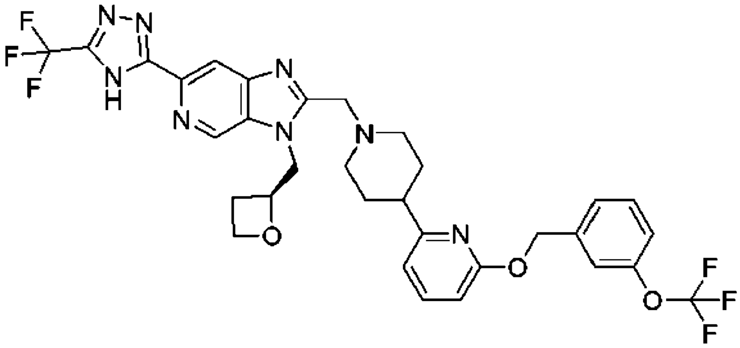

,

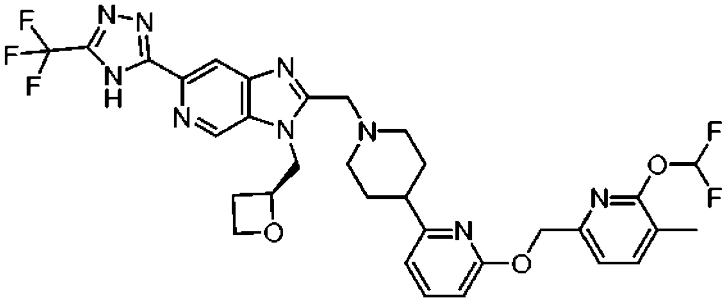

,

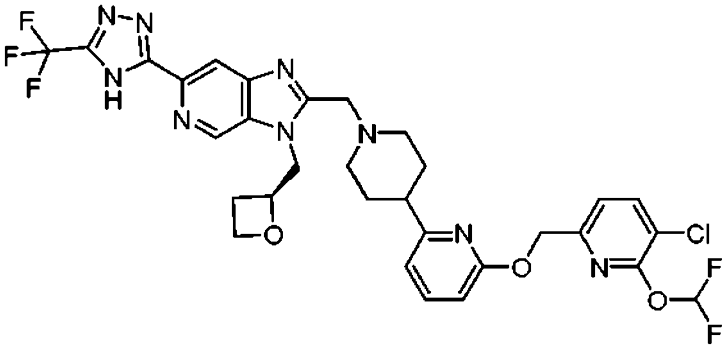

,

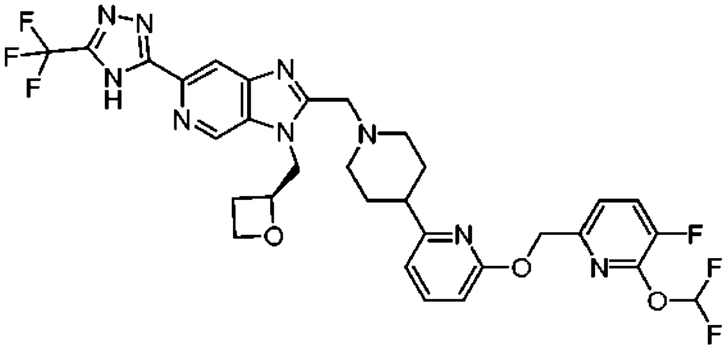

,

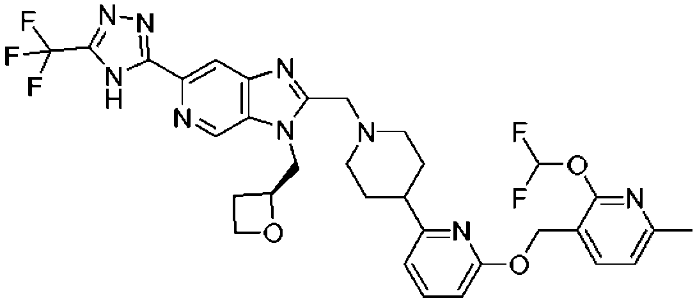

,

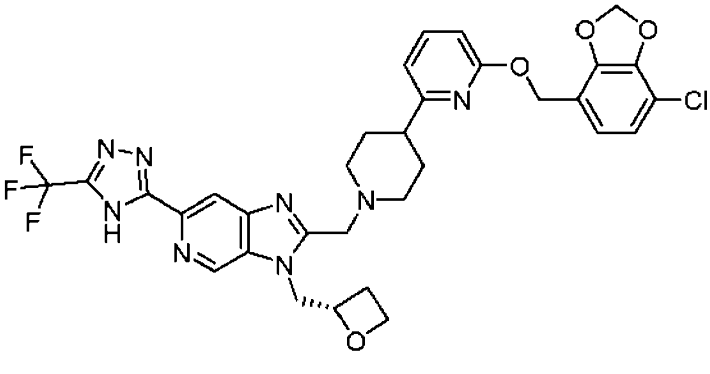

,

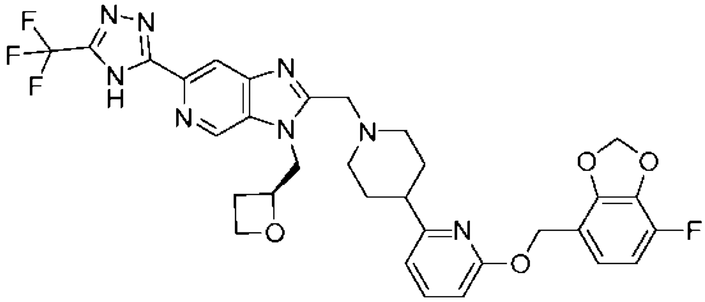

,

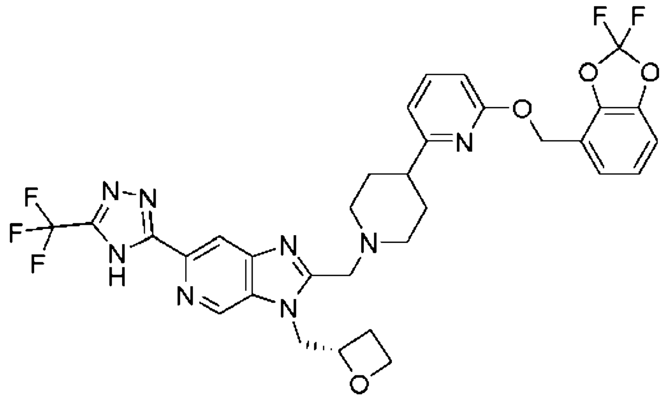

.

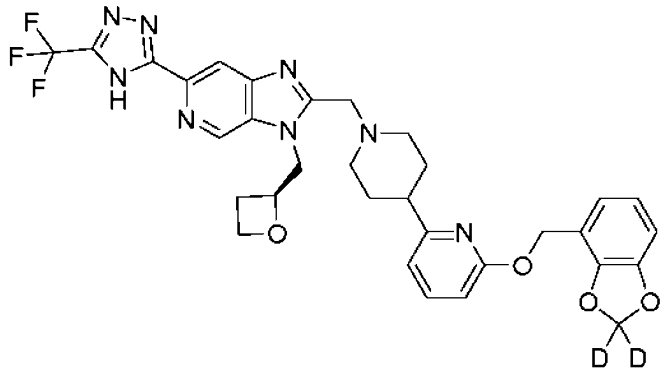

,

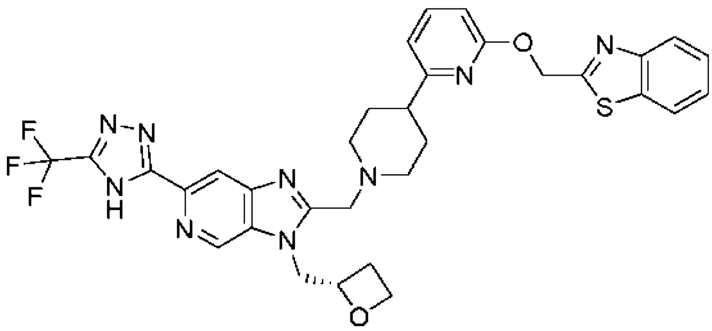

,

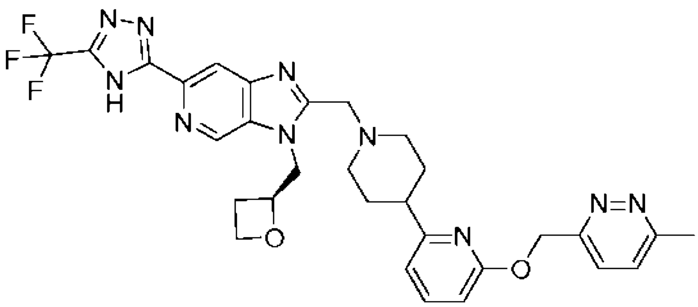

,

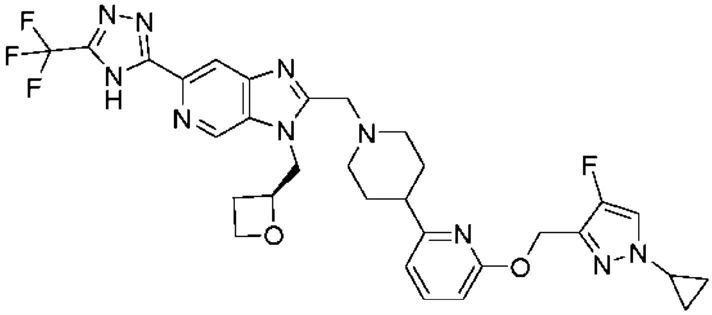

,

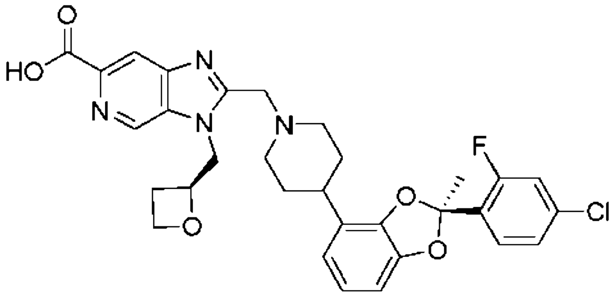

,

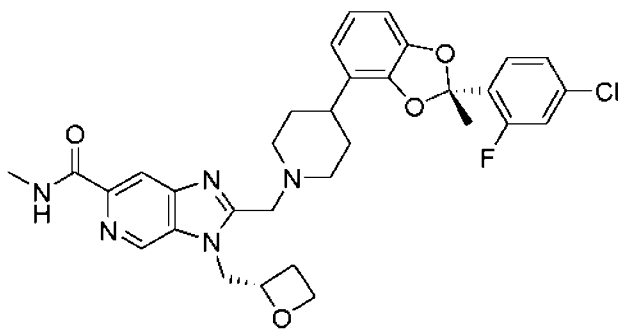

,

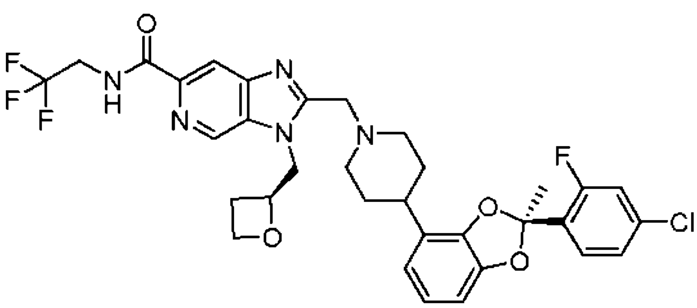

,

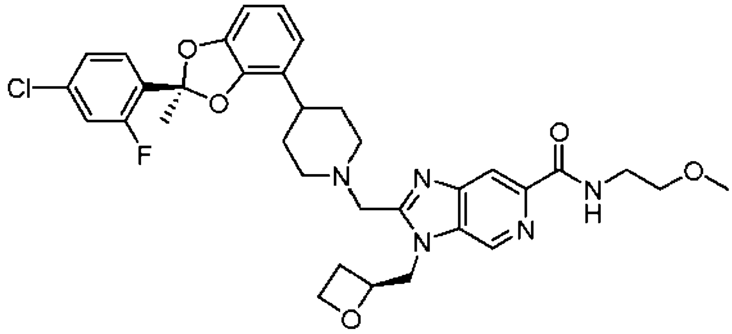

,

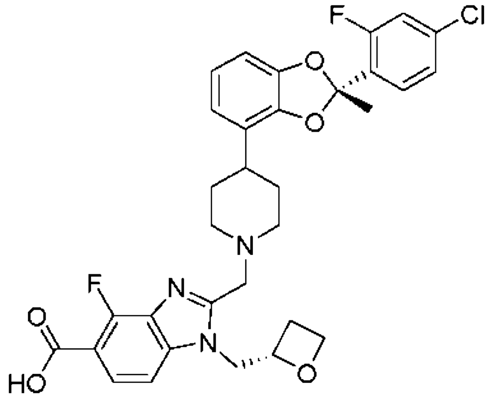

,

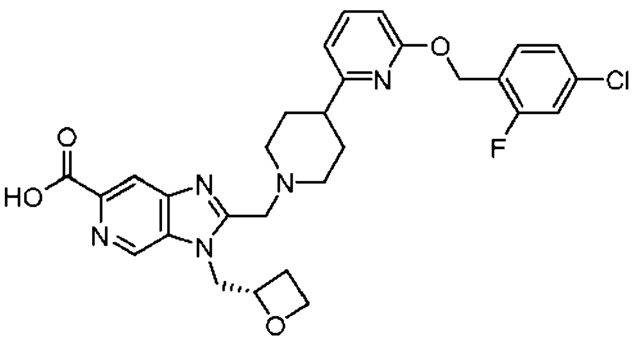

,

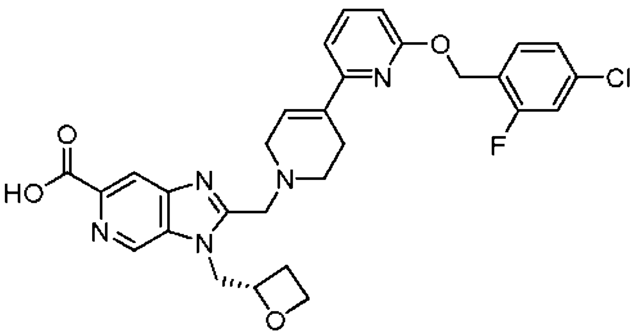

,

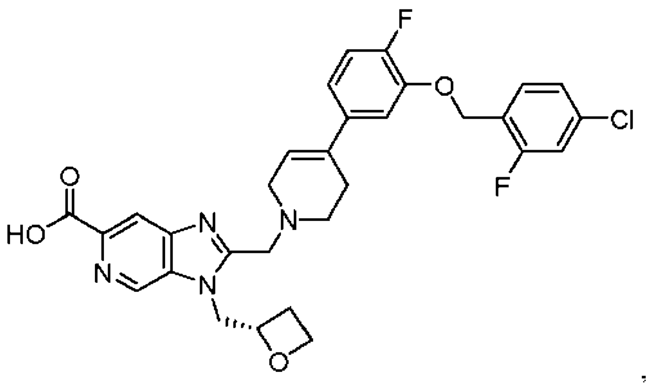

,

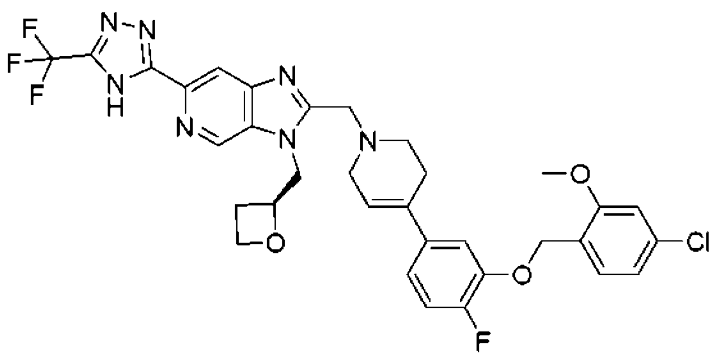

,

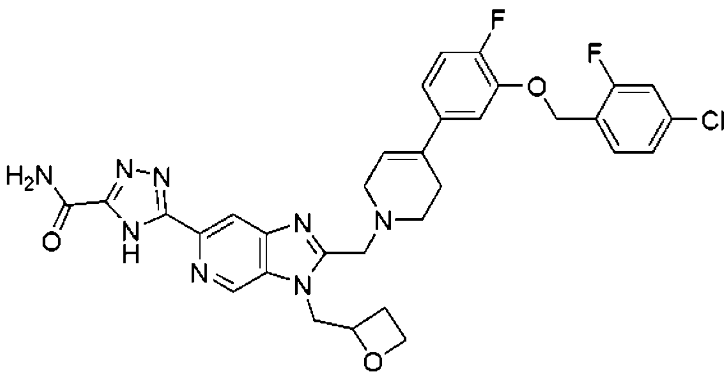

,

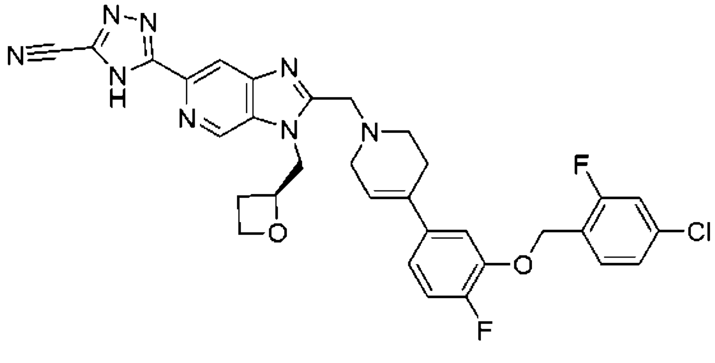

.

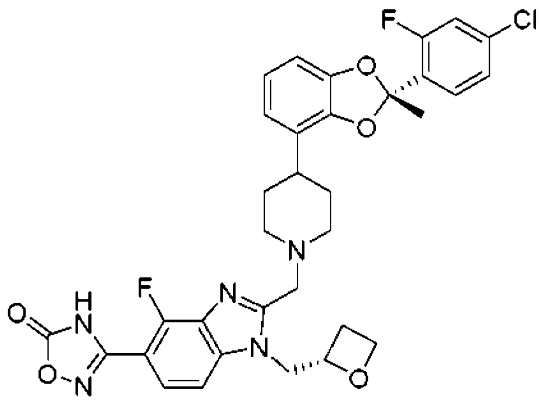

.

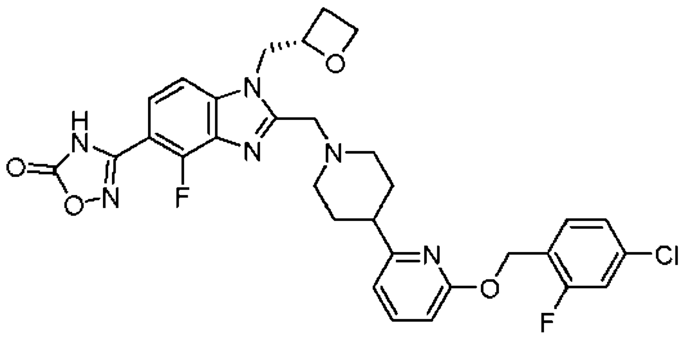

.

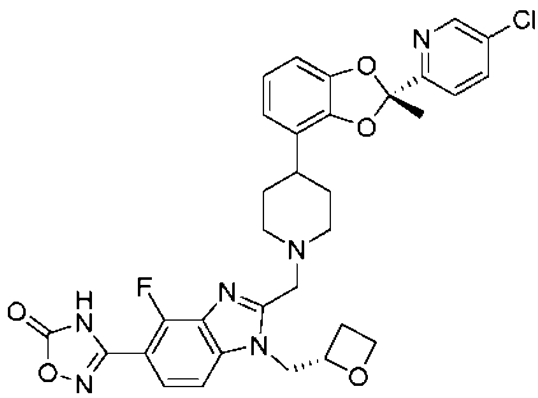

.

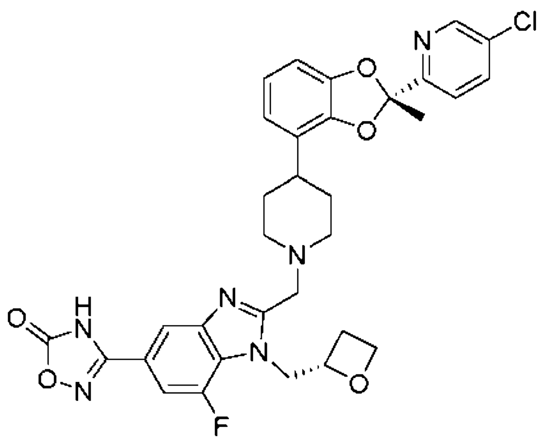

.

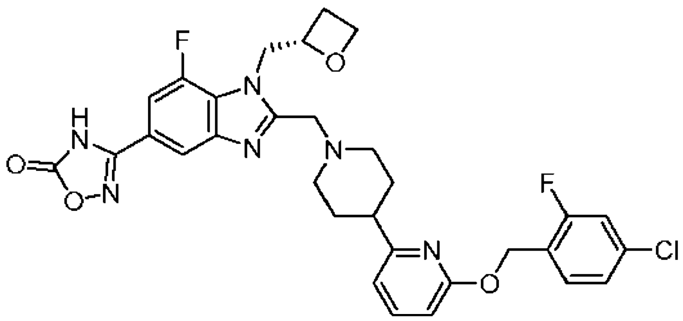

,

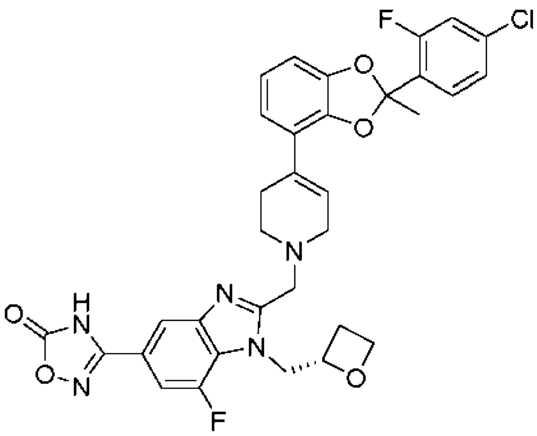

,

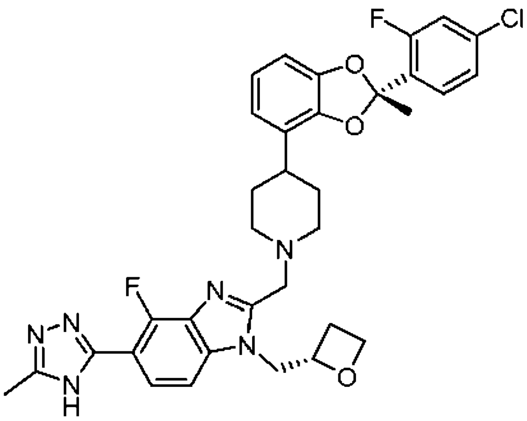

,

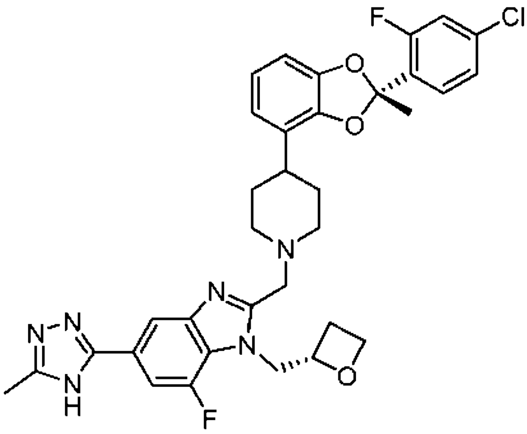

,

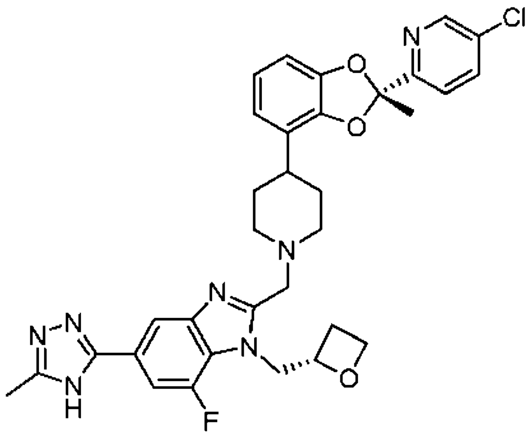

,

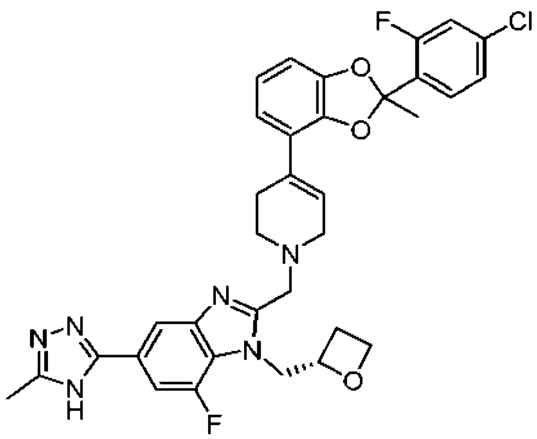

,

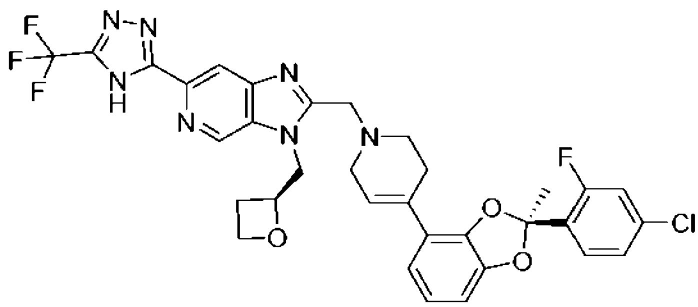

,

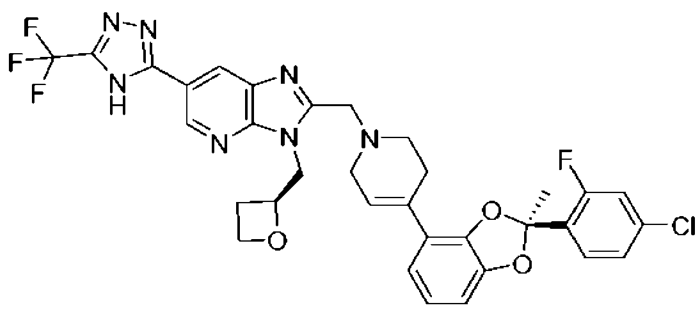

,

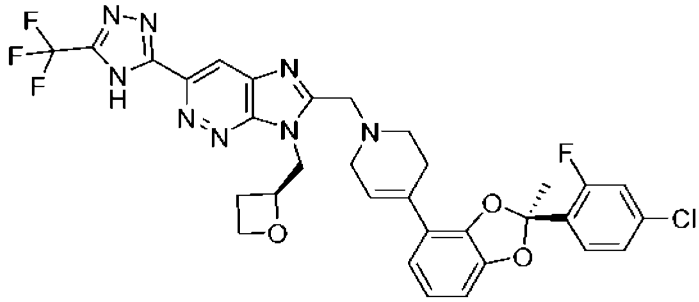

,

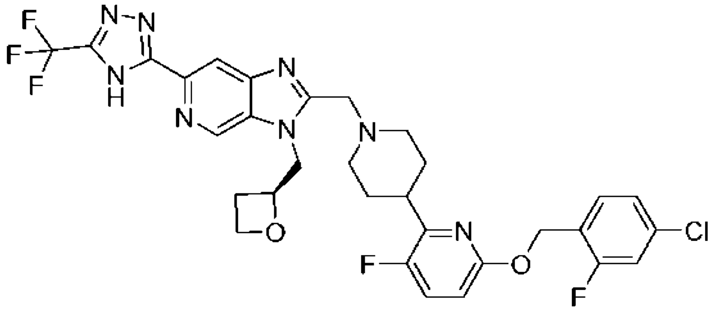

,

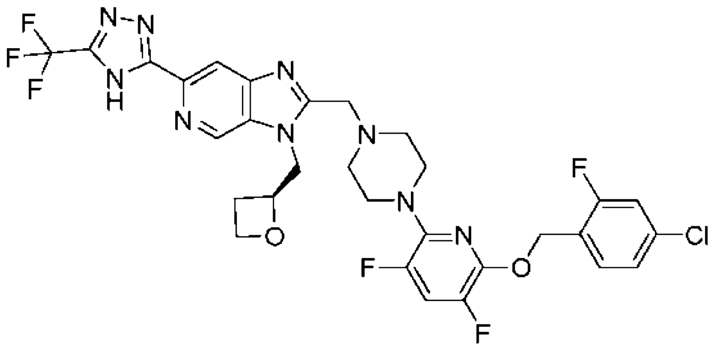

.

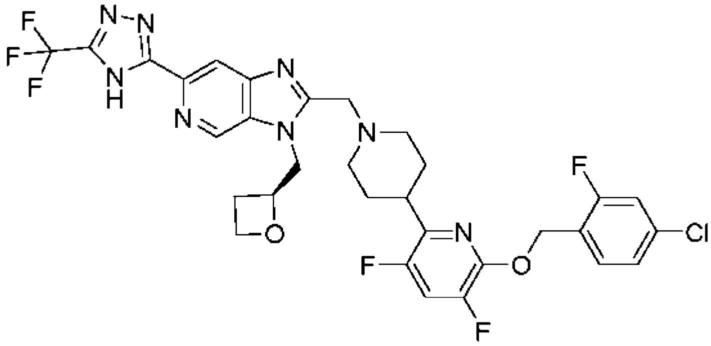

.

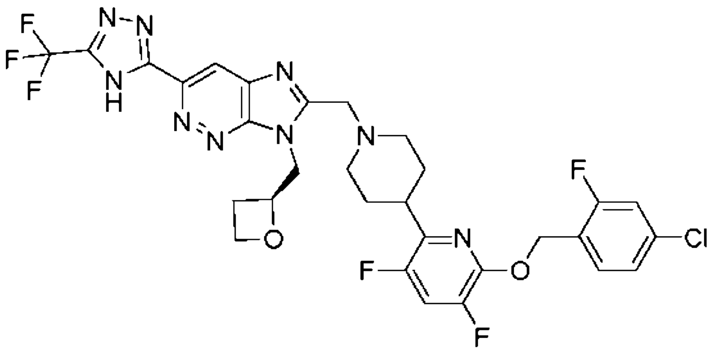

,

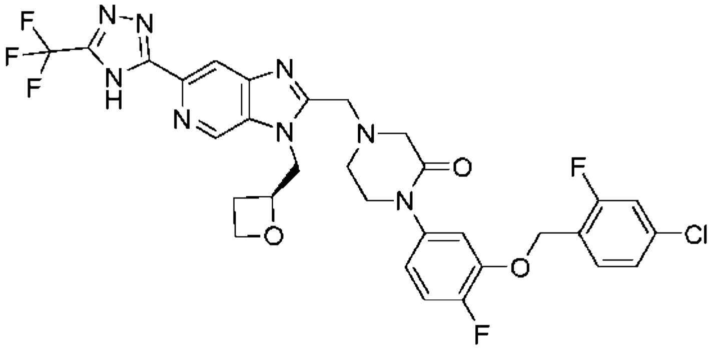

,

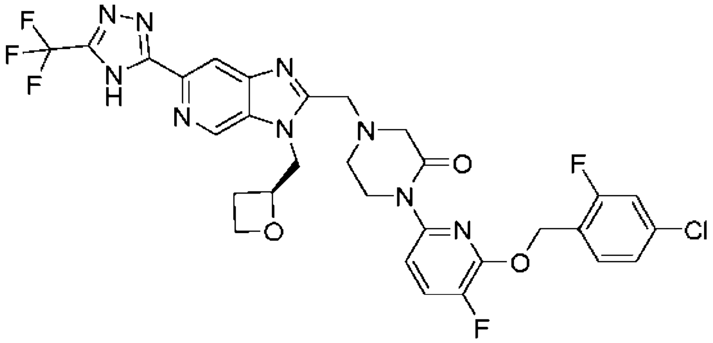

,

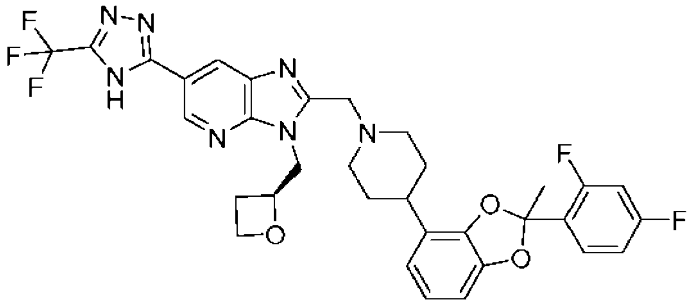

,

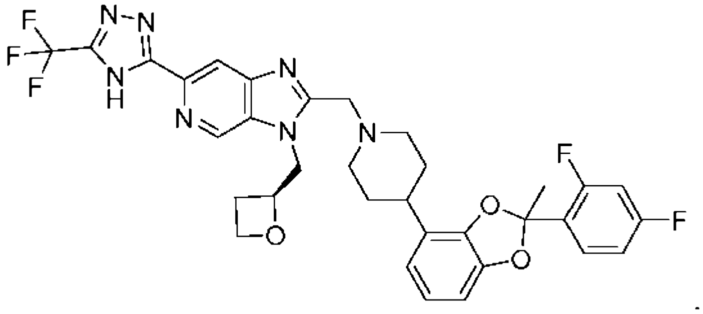

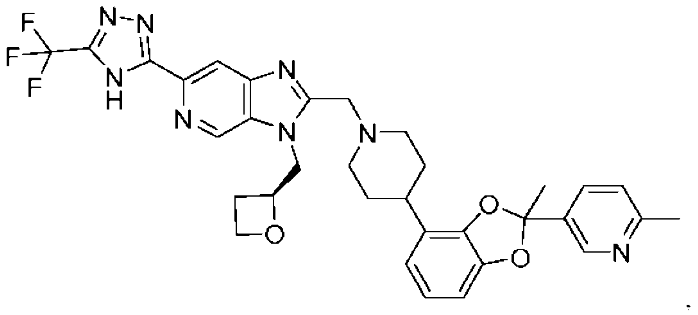

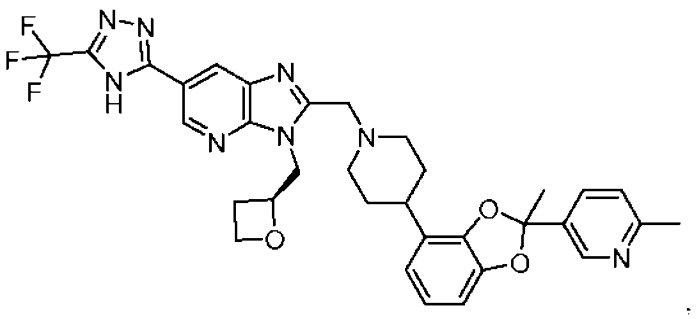

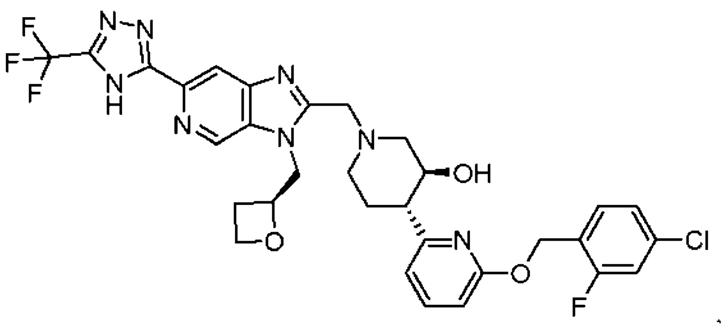

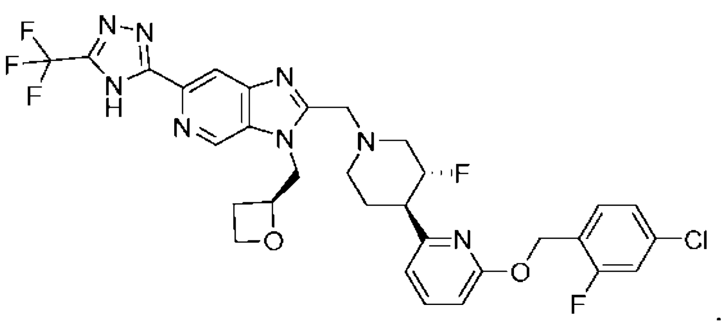

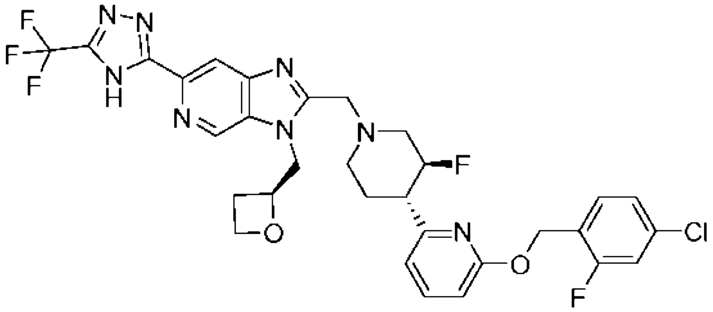

,

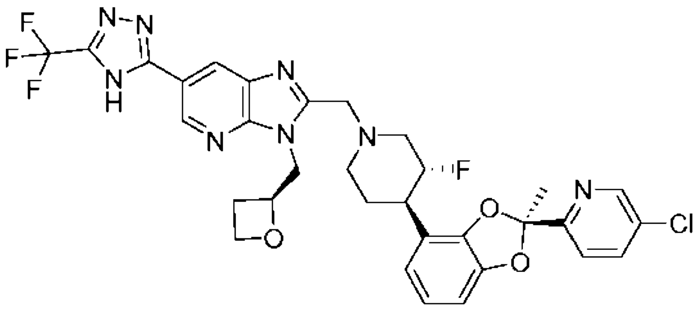

.

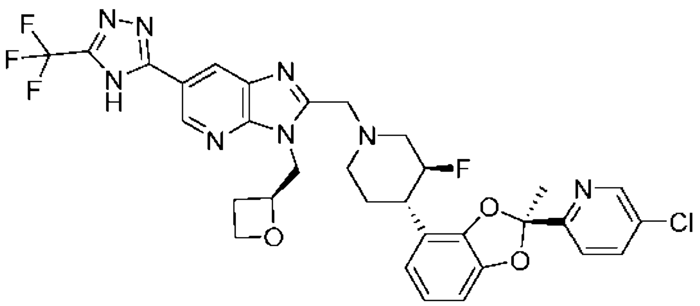

,

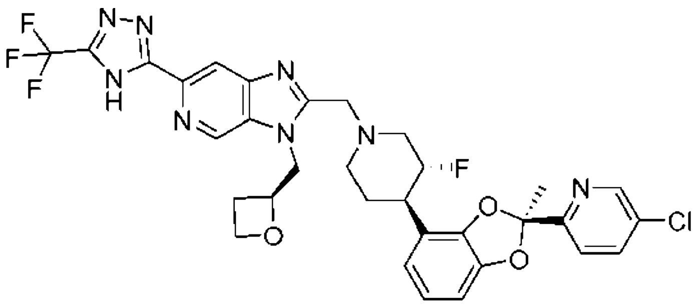

et

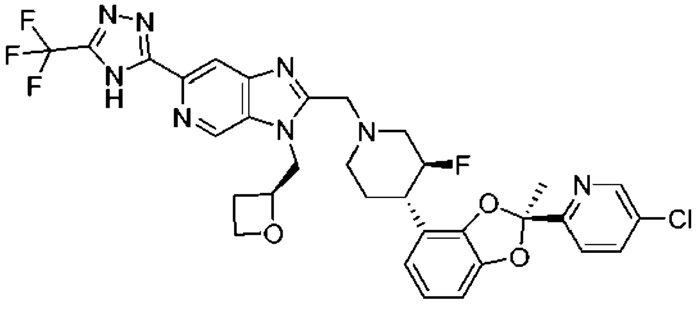

,

ou sel pharmaceutiquement acceptable ou solvate de celui-ci.

2. Composition pharmaceutique comprenant un composé selon la revendication 1, ou un sel pharmaceutiquement acceptable ou solvate de celui-ci, et un excipient pharmaceutiquement acceptable.

3. Composé selon la revendication 1, ou sel pharmaceutiquement acceptable ou solvate de celui-ci, ou composition pharmaceutique selon la revendication 2, destinés à être utilisés dans une méthode de traitement d'une maladie, d'un trouble ou d'une affection associés au GLP-1, la méthode comprenant l'administration à un patient qui en a besoin d'une quantité efficace du composé selon la revendication 1, ou d'un sel pharmaceutiquement acceptable ou solvate de celui-ci, ou de la composition pharmaceutique selon la revendication 2,
la maladie, le trouble ou l'affection étant choisis dans le groupe constitué par le diabète sucré de type 1, le diabète sucré de type 2, le diabète sucré de type 2 à début précoce, le diabète sucré de type 1 idiopathique (type 1b), le diabète atypique à début précoce chez l'adulte jeune (YOAD), le diabète sucré de type adulte apparaissant chez le sujet jeune (MODY), le diabète auto-immun latent chez l'adulte (LADA), l'obésité, la prise de poids due à l'utilisation d'autres agents, la goutte, l'envie excessive de sucre, l'hypertriglycéridémie, la dyslipidémie, le diabète lié à la malnutrition, le diabète gestationnel, une maladie rénale, un dysfonctionnement des adipocytes, l'apnée du sommeil, un dépôt adipeux viscéral, les troubles de l'alimentation, une maladie cardiovasculaire, une insuffisance cardiaque congestive, un infarctus du myocarde, une hypertrophie ventriculaire gauche, une artériopathie périphérique, un accident vasculaire cérébral, un accident vasculaire cérébral hémorragique, un accident vasculaire cérébral ischémique, les accidents ischémiques transitoires, une maladie cardiovasculaire athéroscléreuse, une lésion cérébrale traumatique, une maladie vasculaire périphérique, un dysfonctionnement endothélial, une altération de la compliance vasculaire, une resténose vasculaire, une thrombose, l'hypertension, l'hypertension pulmonaire, une resténose après angioplastie, une claudication intermittente, l'hyperglycémie, la lipémie post-prandiale, l'acidose métabolique, la cétose, l'hyperinsulinémie, une altération du métabolisme du glucose, l'insulinorésistance, l'insulinorésistance hépatique, un trouble lié à la consommation d'alcool, une insuffisance rénale chronique, le syndrome métabolique, le syndrome de l'X fragile, la désaccoutumance du tabac, un syndrome prémenstruel, une angine de poitrine, une néphropathie diabétique, une altération de la tolérance au glucose, une neuropathie diabétique, une rétinopathie diabétique, une dégénérescence maculaire, la cataracte, une glomérulosclérose, l'arthrite, l'ostéoporose, le traitement d'une addiction, la dépendance à la cocaïne, un trouble bipolaire/trouble dépressif majeur, les troubles de la peau et du tissu conjonctif, les ulcères du pied, le psoriasis, une polydipsie primaire, une stéatohépatite non alcoolique (NASH), la maladie du foie gras non alcoolique (NAFLD), une rectocolite hémorragique, une maladie intestinale inflammatoire, une colite, le syndrome du côlon irritable, la maladie de Crohn, le syndrome de l'intestin court, la maladie de Parkinson, la maladie d'Alzheimer, une altération des fonctions cognitives, la schizophrénie, le syndrome des ovaires polykystiques (PCOS) ou une quelconque combinaison de ceux-ci.

4. Composé selon la revendication 1, ou sel pharmaceutiquement acceptable ou solvate de celui-ci, ou composition pharmaceutique selon la revendication 2, destinés à être utilisés dans une méthode de traitement du diabète sucré de type 2 chez un patient qui en a besoin, la méthode comprenant l'administration à un patient qui en a besoin d'une quantité efficace du composé selon la revendication 1, ou d'un sel pharmaceutiquement acceptable ou solvate de celui-ci, ou de la composition pharmaceutique selon la revendication 2.

5. Composé selon la revendication 1, ou sel pharmaceutiquement acceptable ou solvate de celui-ci, ou composition pharmaceutique selon la revendication 2, destinés à être utilisés dans une méthode pour la modulation des taux d'insuline chez un patient qui a besoin d'une telle modulation, la méthode comprenant l'administration à un patient qui en a besoin d'une quantité efficace du composé selon la revendication 1, ou d'un sel pharmaceutiquement acceptable ou solvate de celui-ci, ou de la composition pharmaceutique selon la revendication 2.

6. Composé selon la revendication 1, ou sel pharmaceutiquement acceptable ou solvate de celui-ci, ou composition pharmaceutique selon la revendication 2, destinés à être utilisés dans une méthode pour la modulation des taux de glucose chez un patient qui a besoin d'une telle modulation, la méthode comprenant l'administration à un patient qui en a besoin d'une quantité efficace du composé selon la revendication 1, ou d'un sel pharmaceutiquement acceptable ou solvate de celui-ci, ou de la composition pharmaceutique selon la revendication 2.

7. Composé, sel pharmaceutiquement acceptable, solvate ou composition pharmaceutique destinés à être utilisés selon l'une quelconque des revendications 3 à 6, comprenant en outre l'administration d'une thérapie ou d'un agent thérapeutique supplémentaires au patient.

8. Composé, sel pharmaceutiquement acceptable, solvate ou composition pharmaceutique destinés à être utilisés

selon la revendication 7, la thérapie ou l'agent thérapeutique supplémentaires étant choisis dans le groupe constitué par un agent antidiabétique, un agent anti-obésité, un agoniste du récepteur du GLP-1, un agent antiémétique, un agent pour traiter la stéatohépatite non alcoolique (NASH), une stimulation électrique gastrique, un suivi diététique, l'activité physique ou une combinaison de ceux-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2022086863 W
- US 10370426 B
- US 10308700 B
- US 10259823 B
- US 10208019 B
- US 9920106 B
- US 9839664 B
- US 8129343 B
- US 8536122 B
- US 7919598 B
- US 6414126 B
- US 6628343 B
- RE 45313
- US 20060275288 A1
- US 20120021979 A1
- US 20120148586 A1
- WO 2020234726 A
- WO 2020044266 A
- US 8859577 B
- WO 2010140092 A
- WO 2010128425 A
- WO 2010128414 A
- WO 2010106457 A
- WO 2010103437 A
- WO 2010103438 A
- WO 2010013161 A
- WO 2007122482 A
- WO 2006112549 A
- WO 2007028135 A
- WO 2008047821 A
- WO 2008050821 A
- WO 2008136428 A
- WO 2008156757 A
- WO 2005116034 A
- US 20050287100 A
- WO 2010011439 A
- WO 2010023594 A
- WO 2009144554 A
- WO 2003072197 A
- WO 2009144555 A
- WO 2008065508 A
- US 20180051012 A
- WO 0114372 A
- WO 2004039365 A
- WO 9710224 A
- US 5612359 A
- US 6043265 A
- WO 200001389 A
- WO 0206234 A
- WO 2004048363 A
- WO 2005030740 A
- WO 2005058823 A
- WO 2005113504 A
- WO 2009016462 A
- WO 2010086820 A
- WO 2020117987 A
- WO 2007013694 A
- WO 2007018314 A
- WO 2008093639 A
- WO 2008099794 A
- WO 2004041266 A
- WO 2004106276 A
- WO 2005063729 A
- WO 2005063725 A
- WO 2005087710 A
- WO 2005095338 A
- WO 2007013689 A
- WO 2008001931 A
- US 20120101089 A1
- US 10071088 B2
- US 6673792 B1
- US 6197329 B1
- US 10828297 B2
- US 10322106 B2
- US 10525033 B2
- WO 2009080351 A1
- WO 2019203753 A2
- WO 2002020001 A2
- US 8119697 B2
- US 5039528 A
- US 20090305964 A1
- WO 2006111169 A


### Non-patent literature cited in the description


- **KARLA et al.** Glucagon-like peptide-1 receptor agonists in the treatment of type 2 diabetes: Past, present, and future. *Indian J Endocrinol Metab.*, March 2016, vol. 20 (2), 254-267
- Remington: The Science and Practice of Pharmacy. Lippincott Williams & Wilkins, 2005

- Handbook of Pharmaceutical Excipients. The Pharmaceutical Press and the American Pharmaceutical Association, 2009
- Handbook of Pharmaceutical Additives. Gower Publishing Company, 2007
- Pharmaceutical Preformulation and Formulation. CRC Press LLC, 2009
- The Handbook of Pharmaceutical Excipients. American Pharmaceutical Association and the Pharmaceutical Society of Great Britain
- Pharmaceutical Dosage Forms: Tablets, vol. 1-3
- Pharmaceutical Dosage Forms: Parenteral Medications, vol. 1-2
- Pharmaceutical Dosage Forms: Disperse Systems. Marcel Dekker, Inc, vol. 1-2
- Remington: The Science and Practice of Pharmacy. Pharmaceutical Press, 2012
- **SKELLY et al.** *J Hepatol*, 2001, vol. 35, 195-9
- **CHITTURI et al.** *Hepatology*, 2002, vol. 35 (2), 373-9
- **ANGULO et al.** *J Gastroenterol Hepatol*, 2002, vol. 17, 186-90
- **VIRDEE et al.** *Ophthalmol Ther.*, 2020, vol. 9 (4), 767-781
- **SEPPA et al.** *Sci Rep*, 2019, vol. 9, 15742
- **ZHANG et al.** *Drug Discovery Today*, 2007, vol. 12 (9-10), 373-381
- **BERHMAN RE** ; **KLIEGMAN R** ; **ARVIN AM** ; **NELSON WE**. Nelson Textbook of Pediatrics. W.B. Saunders Company, 1996
- **RUDOLPH AM et al.** Rudolph's Pediatrics. McGraw-Hill, 2002
- **AVERY MD** ; **FIRST LR**. Pediatric Medicine. Williams & Wilkins, 1994